# EUROPEAN PATENT SPECIFICATION

(11) **EP 1 315 490 B1**
(45) Date of publication and mention of the grant of the patent: **12.11.2008**
(21) Application number: 01968461.2
(22) Date of filing: 05.09.2001
(51) Int. Cl.: A61K 31/437, A61K 31/4162, A61K 31/44, A61K 31/536, A61P 37/08

(54) **A METHOD FOR TREATING ALLERGIES USING SUBSTITUTED PYRAZOLES**
METHODE ZUM BEKÄMPFEN DER ALLERGIEN DURCH VERWENDUNG VON SUBSTITUIERTEN PYRAZOLEN
METHODE DE TRAITEMENT DES ALLERGIES A L'AIDE DE PYRAZOLES SUBSTITUES

(30) Priority: 06.09.2000 US 230407 P; 10.08.2001 US 927324
(43) Date of publication of application: 04.06.2003
(73) Proprietor: Ortho-McNeil Pharmaceutical, Inc., Raritan, NJ 08869-0602 (US)
(72) Inventor: BUTLER, Christopher, R., San Diego, CA 92128 (US); CAI, Hui, San Diego, CA 92130 (US); EDWARDS, James, P., San Diego, CA 92129 (US); GRICE, Cheryl, A., Carlsbad, CA 92009 (US); GU, Yin, San Diego, CA 92130 (US); GUSTIN, Darin, J., Bothell, WA 98021 (US); KARLSSON, Lars, La Jolla, CA 92037 (US); KHATUYA, Haripada, San Diego, CA 92129 (US); MEDUNA, Steven, P., San Diego, CA 92129 (US); PIO, Barbara, A., San Diego, CA 92111 (US); SEHON, Clark, A., San Diego, CA 92126 (US); SUN, Siquan, San Diego, CA 92129 (US); TAYS, Kevin, L., Cardiff, CA 92007 (US); THURMOND, Robin, L., San Diego, CA 92115 (US); WEI, Jianmei, San Diego, CA 92129 (US)
(74) Representative: Fisher, Adrian John
(86) International application number: PCT/US2001/027429
(87) International publication number: WO 2002/020011

(56) References cited:
- EP-A- 0 254 241
- DATABASE CA [Online] CHEMICAL ABSTRACTS SERVICE, COLUMBUS, OHIO, US; NAKATSUKA, MASASHI ET AL: "Preparation of pyrazole derivatives as immunosuppressants" retrieved from STN Database accession no. 130:52417 XP002193692 -& WO 98 56785 A (SUMITOMO PHARMACEUTICALS CO., LTD., JAPAN) 17 December 1998 (1998-12-17)

## Description

### Field of the Invention

This invention relates to the use of a compound in the manufacture of a medicament for treating an allergic condition using substituted pyrazoles.

### Background of the Invention

Atopic allergies afflict at least 20% of populations in developed countries and comprise a wide range of IgE-mediated diseases such as hay fever, asthma, atopic dermatitis, and food allergies. Exposure of an allergic subject to relevant allergens cross-links allergen specific IgE bound to mast cells, triggering degranulation and release of proinflammatory mediators, such as histamine and eicosanoids, which cause the weal-and-flare response on a skin test. Characteristically, this early response is followed by a prolonged late reaction in which inflammatory cells, particularly eosinophils and activated TH-2 CD4 T cells, are recruited to the site of allergen exposure. Inflammatory cytokines such as IL-4 and IL-5, both produced by TH-2 cells, are important for IgE production by B cells and for eosinophilia, respectively. Immunotherapies targeting CD4 T cells have been shown to be effective in reducing the production of IgE, the activation of proinflammatory cells, and the release of inflammatory mediators.

Current allergy therapies targeting CD4 T cells have met with mixed success. Desensitization with allergen extracts or vaccines is effective for many allergens, such as the *Hymenoptera* insect sting which can induce life-threatening allergic reactions. The mechanism may be either induction of T cell tolerance or the conversion of TH-2 to TH-1. However, such treatment requires a long-term treatment regime, frequent doctor visits and prior stabilization by other medications, and is associated with a certain morbidity rate and rare deaths. Alternatively, immunosuppressive drugs such as steroids which effectively stabilize ongoing allergy responses, are often associated with severe side effects.

The activation of CD4 T cells is a major factor in the initiation and maintenance of the allergic response. Allergens are taken up by specialized antigen presenting cells (APCs) such as dendritic cells and B cells. Protein allergens pass through the endosomal or lysosomal system where they are degraded by different proteases. These peptide fragments are bound by the MHC class II molecules which, at the cell surface, are heterotrimeric complexes consisting of two transmembrane glycoprotein chains (α and β) that form a binding scaffold for the third component, a peptide of 11-20 amino acids. The antigen-MHC class II molecule complex is recognized by CD4 T cells and leads to the activation of the T cell. Activated T cells in turn activate several other components of the immune system, such as B cells and macrophages, that are crucial for the body's response to pathogens, but also lead to the symptoms of allergies.

Class II molecules, like other transmembrane proteins, are translocated into the endoplasmic reticulum (ER) after synthesis, where they associate with a third protein, the invariant chain (li). The invariant chain molecule is a type II transmembrane protein that serves as a class II-specific chaperone, promoting the exit of class II-li complexes from the ER and preventing class II molecules from binding to peptides and unfolded proteins in the ER and in the secretory pathway. A targeting motif in the cytoplasmic tail of li directs the class II-li complexes from the secretory pathway into the endosomal system.

Before the MHC class II molecules can present antigen the li must be removed by a series of proteases that break down li. The resultant li peptide fragments, called class II-associated invariant chain peptides (CLIP), occupy the peptide binding groove of the class II molecule, and in most cases are not spontaneously released. The CLIP protects the class II binding pocket from collapsing both during intracellular transport and after li degradation in the endosomal system. Binding of antigenic peptides generated from endocytosed proteins requires an empty, and yet open binding site. The CLIP therefore must be released while the open binding site is stabilized to allow the binding of other peptides. Human Leukocyte Antigen - DM ('HLA-DM') mediates both of these functions, thus promoting the binding of antigenic peptides. After acquiring peptides, the class II molecules are transported to the cell surface via routes that are largely unknown.

In view of the above, inhibition of invariant chain proteolysis will prevent removal of li from the class II binding pocket, which in turn will specifically block antigen binding to the MHC class II molecule.

Cathepsin S ('CatS') is a cysteine protease expressed in lymphatic tissues. CatS mediates invariant chain proteolysis, which is a prerequisite for peptide loading of MHC class II molecules (Riese et al. (1996) Immunity 4:357). CatS has 50-60% homology with cathepsins L and K, but differs from them in that it has a broad pH optimum that extends to alkaline pH. CatS modulates antigen presentation in animal models, and inhibitors are effective in an asthma model (Riese et al. (1998) J. Clin. Invest. 101:2351). Mice deficient in cathepsin S have an impaired ability to present exogenous proteins by professional antigen presenting cells (Nakagawa et al. (1999) Immunity 10:207; Shi et al. (1999) Immunity 10:197).

Compounds that inhibit the proteolytic activity of human cathepsin S are expected to find utility in the treatment of chronic autoimmune diseases including, but not limited to, lupus and rheumatoid arthritis; and have potential utility in modulating the immune response to tissue transplantation. Methods of modulating autoimmunity with an agent that modulates cathepsin S activity, e.g., proteolysis of the li chain, as well as methods of treating a subject having an autoimmune disorder, methods of evaluating a treatment for its ability to modulate an immune response are described in WO 99/58153.

Compounds somewhat similar to those of the present invention are described in the following references.

Winters, et. al. (Winters, G.; Sala, A.; Barone, D.; Baldoli, E. J. Med. Chem. 1985, 28, 934-940; Singh, P.; Sharma, R. C. Quant. Struct.-Act. Relat. 1990, 9, 29-32; Winters, G.; Sala, A.; Barone, D. in Ups-4500525 (1985)) have described bicyclic pyrazoles of the type shown below. R never contains a heterocyclic ring and no protease inhibitor activity is ascribed to these molecules; they are described as α1-adrenergic receptor modulators.

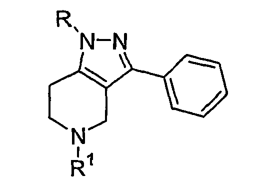

Shutske, et. al. claim the bicylic pyrazoles below. The pyridine ring is aromatic in their system (Shutske, G. M.; Kapples, K. J.; Tomer, J. D. US-5264576 (1993)). Although reference is made to R being a linker to a heterocycle, the claims specify only R = hydrogen. The compounds are referred to as serotonin reuptake inhibitors.

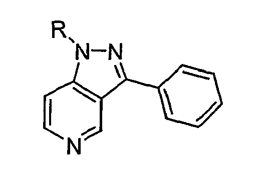

The compound 2-[4-[4-(3-methyl-5-phenyl-1H-pyrazol-1-yl)butyl]-1-piperazinyl]-pyrimidine is known from EP-382637, which describes pyrimidines having anxiolytic properties. This compound and analogs are further described in EP-502786 as cardiovascular and central nervous system agents. Pharmaceutical formulations with such compounds are disclosed in EP-655248 for use in the treatment of gastric secreation and as anti-ulcer agents. WO-9721439 describes medicaments with such compounds for treating obsessive-compulsive disorders, sleep apnea, sexual dysfunctions, emesis and motion sickness.

EP 0 254 241 relates to the use and preparation of pyrazolopyridine compounds and their salts thereof. The compounds disclosed in this document are said to possess antiinflammatory, analgesic, antipyretic, antiallergic, antiarthritic, and antirheumatic activities. In addition, the compounds disclosed therein are said to posses inhibitory activities on platelet aggregation.

The compounds 5-methyl-3-phenyl-1-[4-(4-phenyl-1-piperazinyl) butyl]-1H-indazole and 5-bromo-3-(2-chlorophenyl)-1-[4-(4-phenyl-1-piperazinyl) butyl]-1H-indazole, in particular the hydrochloride salts thereof, are known from WO-9853940 and CA 122:314528, where these and similar compounds are described as kinase inhibitors in the former reference and possessing affinity for benzodiazepine receptors in the latter reference.

### Summary of the Invention

The present invention features the use of cathepsin S inhibitors in the manufacture of a pharmaceutical composition to treat allergic conditions, including but not limited to atopic allergies. Examples of an allergic condition include hay fever, asthma, atopic dermatitis and food allergies. Allergens include dust, pollen, mold, and pet dander or pet hair.

In one aspect, the invention provides the use of a compound in the manufacture of a pharmaceutical composition for treating a subject suffering from an allergic condition, in particular an atopic allergic condition, said compound being of formula I as shown in claim 1.

In another aspect, the invention provides the use of a compound in the manufacture of a pharmaceutical composition for treating a subject suffering from an IgE-mediated allergic condition, in particular an atopic allergic condition, said compound being of formula I as shown in claim 1.

A third aspect of the invention provides the use, or the use for the manufacture of a medicament, of a cathepsin S inhibitor for treating an allergic condition, more in particular for treating IgE-mediated allergic conditions, still more in particular treating hay fever, asthma, atopic dermatitis or food allergies. The invention also features anti-allergic pharmaceutical compositions comprising as active ingredient an effective amount of a cathepsin S inhibitor, and a pharmaceutically acceptable carrier. The active ingredient can be formulated in any manner suitable for the particular allergic condition, including aerosol, oral and topical formulations and time-release formulations.

The present invention concerns the use of a pharmaceutical composition for the treatment of an allergic condition using one or more compounds which can be represented by formula (I): 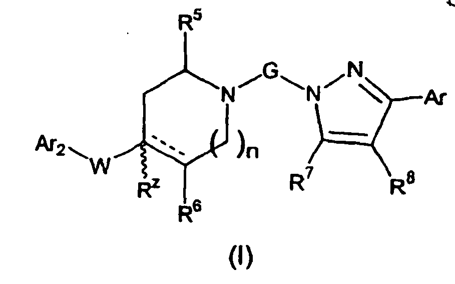 wherein:
Ar₂ is a monocyclic or bicyclic ring system, unsaturated, saturated or aromatic, optionally fused, optionally including between 1 and 5 heteroatom ring moieties independently selected from O, S, N, SO₂, and C=O; said Ar₂ ring system being optionally substituted with between 1 and 4 substituents;
R⁵ and R⁶ are independently selected from hydrogen and C₁₋₆alkyl;
R⁷ and R⁸ are independently hydrogen, C₁₋₅ alkyl, C₂₋₅ alkenyl, C₁₋₆ alkoxy, C₁₋₅ alkylthio, halogen, or a 4-7 membered carbocyclyl or heterocyclyl; alternatively, R⁷ and R⁸ can be taken together to form an optionally substituted 5- to 7- membered carbocyclic or heterocyclic ring, which ring may be unsaturated or aromatic, and may be optionally substituted with between one and three substituents independently selected from halo, cyano, amino, hydroxy, nitro, R⁴, R⁴O-, R⁴S-, R⁴O(C₁₋₅ alkylene)-, R⁴O(C=O)-, R⁴(C=O)-, R⁴(C=S)-, R⁴(C=O)O-, R⁴O(C=O)(C=O)-, R⁴SO₂, NHR⁴⁴(C=NH)-, NHR⁴⁴SO₂-, and NHR⁴⁴(C=O)-;
   - R⁴: is H, C₁₋₅ alkyl, C₂₋₅ alkenyl, C₁₋₅ heterocyclyl, (C₁₋₅ heterocyclyl)C₁₋₆ alkylene, phenyl, benzyl, phenethyl, NH₂, mono- or di(C₁₋₆ alkyl)N-, (C₁₋₆ alkoxy)carbonyl- or R⁴²OR⁴³-, wherein R⁴² is H, C₁₋₆ alkyl, C₂₋₅ alkenyl, phenyl, benzyl, phenethyl, C₁₋₅ heterocyclyl, or (C₁₋₅ heterocyclyl)C₁₋₆ alkylene and R⁴³ is C₁₋₅ alkylene, phenylene, or divalent C₁₋₅ heterocyclyl;
   - R⁴⁴: can be any of the values for R⁴;
   - n: is 0, 1, or 2;
   - G: is C₃₋₆ alkenediyl or C₃₋₆ alkanediyl, optionally substituted with hydroxy, halogen, C₁₋₅ alkoxy, C₁₋₅ alkyl, oxo, hydroximino, CO₂R^{k}, R^{k}R^{l}N, R^{k}R^{l}NCO₂, (L)-C₁₋₄ alkylene-, (L)-C₁₋₅ alkoxy, N₃ or [(L)-C₁₋₅ alkylene]amino;
   each of R^{k} and R^{l} is independently hydrogen, C₁₋₅ alkyl, C₃₋₅ alkenyl, phenyl, benzyl, phenethyl, or C₁₋₅ heterocyclyl; alternatively R^{k} and R^{l}, can be taken together to form an optionally substituted 4- to 7- membered heterocyclic ring, which ring may be saturated, unsaturated or aromatic;
   - L: is amino, mono- or di-C₁₋₅ alkylamino, pyrrolidinyl, morpholinyl, piperidinyl homopiperidinyl, or piperazinyl, wherein available ring nitrogens may be optionally substituted with C₁₋₅ alkyl, benzyl, C₂₋₅ acyl, C₁₋₅ alkylsulfonyl, or C₁₋₅ alkoxycarbonyl;
   - Ar: represents a monocyclic or bicyclic aryl or heteroaryl ring, optionally substituted with between 1 and 3 substituents independently selected from halogen, C₁₋₅alkoxy. C₁₋₅alkyl, C₂₋₅ alkenyl, cyano, azido, nitro, R²²R²³N, R²⁴SO_{2'} R²⁴S, R²⁴SO, R²⁴OC=O, R²²R²³NC=O, C₁₋₅ haloalkyl, C₁₋₅ haloalkoxy,
   C₁₋₅ haloalkylthio, and C₁₋₅ alkylthio;
   - R²²: is hydrogen, C₁₋₅ alkyl, C₃₋₅ alkenyl, phenyl, phenethyl, benzyl, or C₁₋₅ heterocyclyl, C₂₋₈ acyl, aroyl, R³⁸OC=O, R²⁵R²⁶NC=O, R³⁸SO, R³⁸SO₂, R³⁸S, or R²⁵R²⁶NSO₂;
   - R²³: is hydrogen, C₁₋₅ alkyl, C₃₋₅ alkenyl, phenyl, benzyl or C₁₋₅ heterocyclyl; alternatively, R²² and R²³ can be taken together to form an optionally substituted 4- to 7- membered heterocyclic ring, which ring may be saturated, unsaturated or aromatic;
   R²⁴ is C₁₋₅ alkyl, C₃₋₅ alkenyl, phenyl, benzyl, or C₁₋₅ heterocyclyl;
   - R²⁵ and R²⁶: independently are hydrogen, C₁₋₅ alkyl, C₃₋₅ alkenyl, phenyl, benzyl, or C₁₋₅ heterocyclyl;
   or, alternatively, R²⁵ and R²⁶ can be taken together to form an optionally substituted 4- to 7-membered heterocyclic ring, which ring may be saturated, unsaturated or aromatic;
   - W: represents O, S, NR²⁷, C=O, (C=O)NH, NH(C=O), CHR²⁸, or a covalent bond;
   - R^{z}: is H or OH and the dashed line is absent; or R^{z} is absent where the dashed line is an sp² bond;
   - R²⁷: is hydrogen, C₁₋₅ alkyl, C₃₋₅ alkenyl, phenyl, naphthyl, benzyl, phenethyl, C₁₋₅ heterocyclyl, C₂₋₈ acyl, aroyl, R²⁹OC=O, R³⁰R³¹NC=O, R²⁹SO, R²⁹S, R²⁹SO₂, or R³⁰R³¹NSO₂ ;
   or, alternatively, R²⁷ and part of Ar₂ can be taken together to form an optionally substituted 5- or 6-membered heterocyclic ring with optionally 1 to 3 additional heteroatom moieties in the ring selected from O, NR⁹, NR¹⁰, N, SO₂, C=O, and S; which ring may be saturated, unsaturated or aromatic; R⁹ and R¹⁰ are independently selected from H, C₁₋₃ alkyl and -CH₂CO₂(C₁₋₄ alkyl);
   - R²⁸: is hydrogen, C₁₋₅ alkyl, C₃₋₅ alkenyl, hydroxy, phenyl, benzyl, C₁₋₅ heterocyclyl, R²⁹O, R³⁰R³¹NC=O R²⁹S, R²⁹SO, R²⁹SO₂, or R³⁰R³¹NSO₂;
   - R²⁹: is C₁₋₅ alkyl, C₃₋₅ alkenyl, phenyl, benzyl, or C₁₋₅ heterocyclyl;
   - R³⁰ and R³¹: are independently selected from hydrogen, C₁₋₅ alkyl, C₃₋₅ alkenyl, phenyl, benzyl, phenethyl, naphthyl, and C ₁₋₅ heteroaryl; alternatively, R³⁰ and
   - R³¹: can be taken together to form an optionally substituted 4- to 7-membered ring carbocyclic or heterocyclic ring, which ring may be saturated, unsaturated or aromatic;
   wherein each of the above hydrocarbyl or heterocarbyl groups, unless otherwise indicated, and in addition to any specified substituents, is optionally and independently substituted with between 1 and 3 substituents selected from methyl, halomethyl, hydroxymethyl, halo, hydroxy, amino, nitro, cyano, C₁₋₅ alkyl, C₁₋₅ alkoxy, -COOH, C₂₋₆ acyl, [di(C₁₋₄ alkyl)amino]C₂₋₅ alkylene, [di(C₁₋₄ alkyl)amino]C₂₋₅ alkyl-NH-CO-, and C₁₋₅ haloalkoxy;
   or a pharmaceutically acceptable salt, amide, or ester thereof; or a stereoisomeric form thereof.

One embodiment of the invention is the use of a compound in the manufacture of a medicament for the treatment of an allergic condition using a compound of formula(I), wherein Ar₂ is selected from 5-7 membered monocyclic rings, and [5,6], [6,6], [6,5], and [5,5] fused bicyclic ring systems, said ring or ring system being carbocyclic or heterocyclic, saturated, unsaturated, or aromatic, optionally substituted with halo, C₁₋₄ alkyl, C₁₋₄ haloalkyl, C₁₋₄ hydroxyalkyl, nitro, hydroxy, amino, mono- or di-(C₁₋₆ alkyl)amino, C₁₋₄ alkoxy, C₂₋₄ alkoxycarbonyl, C₂₋₆ acyl, C₂₋₆ acyloxy, C₁₋₅ alkylsulfonyl, C₁₋₅ alkoxycarbonylC₁₋₄ alkoxy, cyano, and mono- or di-(C₁₋₆ alkyl)carbamoyl.

Another embodiment of the invention is the use of a compound of formula (I), wherein Ar₂ is selected from 2,5-di(C₁₋₆ alkyl)aminopyrrolyl and the following 6 formulae: 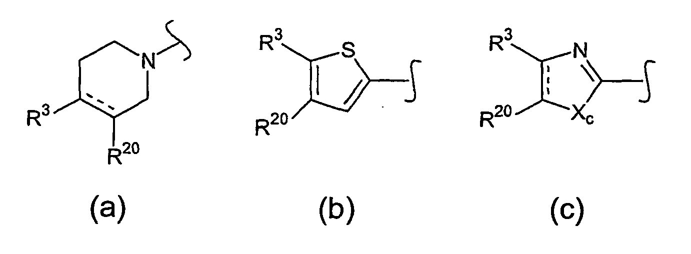 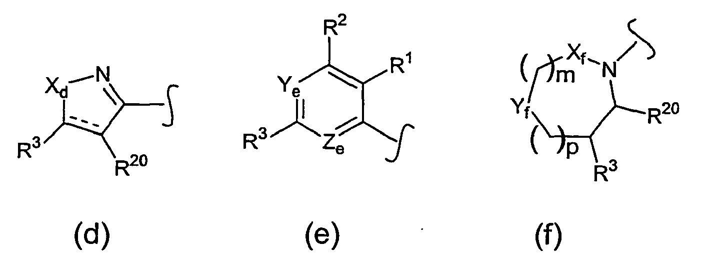 wherein
each dashed line may be an sp² bond or absent;
X_{c} is O, S, or N; and X_{d} is O or S;
   - R¹: is hydrogen, halogen, C₁₋₅ alkoxy, hydroxy, C₁₋₅ alkyl, C₂₋₅ alkenyl, cyano, nitro, R^{a}R^{b}N, C₂₋₈ acyl, C₁₋₅ heterocyclyl, (C₁₋₅ heterocyclyl)C₁₋₅ alkylene, R¹¹S, R¹¹SO, R¹¹SO₂, R^{c}OC=O, R^{c}R^{d}NC=O, or R^{c}R^{d}NSO₂; or R¹ can be taken together with R²⁷ as provided below;

   - R²: is hydrogen, halogen, C₁₋₅ alkoxy, hydroxy, C₁₋₅ alkyl, C₂₋₅ alkenyl, cyano, nitro, R^{e}R^{f}N, C₁₋₅ heterocyclyl, or C₂₋₈ acyl;
   - R³: is hydrogen, halogen, C₁₋₅ alkoxy, hydroxy, C₁₋₅ alkyl, C₂₋₅ alkenyl, cyano, nitro, R^{g}R^{h}N, C₂₋₈ acyl, C₁₋₅ heterocyclyl, R^{h}OC=O, R^{g}R^{h}NC=O, or R^{g}R^{h}NSO₂;
   - R^{a}: is selected from hydrogen, C₁₋₅ alkyl, C₃₋₅ alkenyl, phenyl, benzyl, phenethyl, C₁₋₅ heterocyclyl, C₂₋₈ acyl, aroyl, R^{j}OC=O, RⁱR^{j}NC=O, R¹²SO, R¹²SO₂, R¹²S , and RⁱR^{j}NSO₂;
   - R^{e}: is selected from hydrogen, C₁₋₅ alkyl, C₃₋₅ alkenyl, phenyl, benzyl, phenethyl, C₁₋₅ heterocyclyl, C₂₋₈ acyl, aroyl, R³²OC=O, R³²R³³NC=O, R¹³SO, R¹³SO₂, R¹³S, and R³²R³³NSO₂;
   - R^{m}: is selected from hydrogen, C₁₋₅ alkyl, C₃₋₅ alkenyl, phenyl, benzyl, phenethyl, C₁₋₅ heterocyclyl, C₂₋₈ acyl, aroyl, R³⁴OC=O, R³⁴R³⁵NC=O, R¹⁵SO, R¹⁵SO₂, R¹⁵S , and R³⁴R³⁵NSO₂;
   - R^{o}: is selected from hydrogen, C₁₋₅ alkyl, C ₃₋₅ alkenyl, phenyl, benzyl, phenethyl, C₁₋₅ heterocyclyl, C₂₋₈ acyl, aroyl, R³⁶OC=O, R³⁶R³⁷NC=O, R¹⁹SO, R¹⁹SO₂, R¹⁹S, and R³⁶R³⁷NSO2;
   each of R^{b}, R^{f}, Rⁿ, R^{p}, R³², R³³, R³⁴, R³⁵, R³⁶ , R³⁷, R³⁹, and R⁴⁰ is independently selected from hydrogen, C₁₋₅ alkyl, C₃₋₅ alkenyl, phenyl, benzyl, phenethyl, and C₁₋₅ heteroaryl;
   alternatively, R^{a} and R^{b}, R^{e} and R^{f}, R^{m} and Rⁿ, and R^{o} and R^{p}, independently, can be taken together to form an optionally substituted 4- to 7- membered heterocyclic ring, which ring may be saturated, unsaturated or aromatic;
   each of R¹¹, R¹², R¹³, R¹⁴, R¹⁵, R¹⁶, R¹⁹, R³⁸, and R⁴¹ is independently C₁₋₅alkyl, C₃₋₅ alkenyl, phenyl, benzyl, phenethyl, or C₁₋₅ heterocyclyl;
   each of R^{c} and R^{d} , and Rⁱ and R^{j} are independently are hydrogen, C₁₋₅ alkyl, C₃₋₅ alkenyl, phenyl, benzyl, phenethyl, or C₁₋₅ heteroaryl; alternatively, R^{c} and R^{d}, and Rⁱ and R^{j}, independently, can be taken together to form an optionally substituted 4- to 7- membered heterocyclic ring, which ring may be saturated, unsaturated or aromatic;
   - R^{g}: is hydrogen, C₁₋₅ alkyl, C₃₋₅ alkenyl, phenyl, benzyl, phenethyl, C₁₋₅ heterocyclyl, C₂₋₈ acyl, aroyl, R¹⁷OC=O, R¹⁷R¹⁸NC=O, R¹⁶S, R¹⁶SO, R¹⁶SO₂, or R¹⁷R¹⁸NSO₂;
   - R^{h}: is hydrogen, C₁₋₅ alkyl, C₃₋₅ alkenyl, phenyl, benzyl, phenethyl or C₁₋₅ heterocyclyl; alternatively, R^{g} and R^{h} can be taken together to form an optionally substituted 4- to 7- membered heterocyclic ring, which ring may be saturated, unsaturated or aromatic;
   - R¹⁷ and R¹⁸: independently are hydrogen, C₁₋₅ alkyl, C₃₋₅ alkenyl, phenyl, benzyl, or C₁₋₅ heterocyclyl;
alternatively, R¹⁷ and R¹⁸ can be taken together to form an optionally substituted 4- to 7- membered heterocyclic ring, which ring may be saturated, unsaturated or aromatic;
- Yₑ: is nitrogen or R²⁰C;
- Zₑ: is nitrogen or R²¹C ;
R²⁰ is hydrogen, halogen, C₁₋₅ alkoxy, C₁₋₅ alkyl, C₂₋₅ alkenyl, cyano, nitro, R^{m}RⁿN, C₂₋₈ acyl, R^{m}OC=O, R¹⁴S, R¹⁴SO, or R¹⁴SO₂;
- R²¹: is hydrogen, halogen, C₁₋₅ alkoxy, C₁₋₅ alkyl, C₂₋₅ alkenyl, cyano, nitro, R^{o}R^{p}N, C₂₋₈ acyl, R¹⁶OC=O, R¹¹S, R¹¹SO, or R¹¹SO₂;
alternatively, R³ and R²⁰ or R³ and R²¹ can be taken together to form an optionally substituted 5- or 6-membered carbocyclic or heterocyclic ring, which ring may be saturated, unsaturated or aromatic; wherein said ring may be optionally substituted with halo, di(C₁₋₅ alkyl)amino, C₂₋₅ acyl, and C₁₋₅ alkoxy;
- R²⁷: is hydrogen, C₁₋₅ alkyl, C₃₋₅ alkenyl, phenyl, naphthyl, benzyl, phenethyl, C₁₋₅ heterocyclyl, C₂₋₈ acyl, aroyl, R²⁹OC=O, R³⁰R³¹NC=O, R²⁹SO, R²⁹S, R²⁹SO₂, or R³⁰R³¹NSO₂ ;
or, alternatively, R²⁷ and R¹ can be taken together to form an optionally substituted 5- or 6-membered heterocyclic ring with optionally 1 to 3 additional heteroatom moieties in the ring selected from O, NR⁹, NR¹⁰, N, SO₂, C=O, and S; which ring may be saturated, unsaturated or aromatic; R⁹ and R¹⁰ are independently selected from H, C₁₋₃ alkyl, and -CH₂CO₂(C₁₋₄ alkyl);
- X_{f}: is CHR^{1f}, =N-, NH, C=O, SO₂, CHSR^{1f} wherein, in formula (f), R^{1f} is hydrogen, halogen, C₁₋₅ alkoxy, hydroxy, C₁₋₅ alkyl, C₃₋₅ alkenyl, cyano, nitro, R³⁹R⁴⁰N, C₂₋₈ acyl, C₁₋₅ heterocyclyl, (C₁₋₅ heterocyclyl)C₁₋₅ alkylene, R⁴¹S, R⁴¹SO, R⁴¹SO_{2'} R³⁹OC=O, R³⁹R⁴⁰NC=O, R³⁹R⁴⁰NSO₂, R⁴¹SO₃- or R³⁹(C=O)O-;
- Y_{f}: is CH₂, CHR^{2f}, =CR^{2f}, O, or NR^{2f}, wherein R^{2f} is H, C₁₋₇ alkyl, C ₃₋₅ alkenyl, 6₂₋₈ acyl, C₁₋₅ heterocyclyl, (C₁₋₅ heterocyclyl)-C₁₋₅ alkylene, phenyl, (phenyl)-C₁₋₅ alkylene, (C₃₋₇ cycloalkyl)-C₁₋₅ alkylene, (H₂NCO)- C₁₋₅ alkylene, C₁₋₅ haloalkyl, C₁₋₅ cyanoalkyl, (C₁₋₅ alkoxycarbonyl)C₁₋₅ alkylene, and (phenylcarbonyl)NH-;
- m: is 0 or 1;
- p: is 0 or 1;
wherein each of the above hydrocarbyl or heterocarbyl groups, unless otherwise indicated, and in addition to any specified substituents, is optionally and independently substituted with between 1 and 3 substituents selected from methyl, halomethyl, hydroxymethyl, halo, hydroxy, amino, nitro, cyano, C₁₋₅ alkyl, C₁₋₅ alkoxy, -COOH, C₂₋₆ acyl, [di(C₁₋₄ alkyl)amino]C₂₋₅ alkylene, [di(C₁₋₄ alkyl)amino] C ₂₋₅ alkyl-NH-CO-, and C ₁₋₅ haloalkoxy.

The disclosed compounds are high-affinity inhibitors of the proteolytic activity of human cathepsin S. For use in medicine, the preparation of pharmaceutically acceptable salts of compounds of formula (I) may be desirable.

Certain compounds of the present invention may have one stereogenic atom and may exist as two enantiomers. Certain compounds of the present invention may have two or more stereogenic atoms and may further exist as diastereomers. It is to be understood by those skilled in the art that all such stereoisomers and mixtures thereof in any proportion are encompassed within the scope of the present invention.

Another aspect of the invention provides pharmaceutical anti-allergic compositions comprising a compound of formula (I) and a pharmaceutically acceptable carrier. A further embodiment of the invention is a process for making a pharmaceutical anti-allergic composition comprising mixing a disclosed compound as described above, with a suitable pharmaceutically acceptable carrier.

The invention also contemplates pharmaceutical compositions comprising more than one compound of formula (I) and compositions comprising a compound of formula (I) and another pharmaceutically active agent.

The invention features the use of a compound in the manufacture of a pharmaceutical composition of treating allergic disorders or conditions mediated by the cathepsin S enzyme, in a subject in need thereof, comprising any of the compounds or pharmaceutical compositions described above. The compounds described herein inhibit the protease activity of human cathepsin S, an enzyme involved in the immune response. In preferred embodiments, cathepsin S inhibition is selective.

Additional features and advantages of the invention will become apparent from the detailed description below, including examples, and the appended claims.

### Brief Description of the Figures

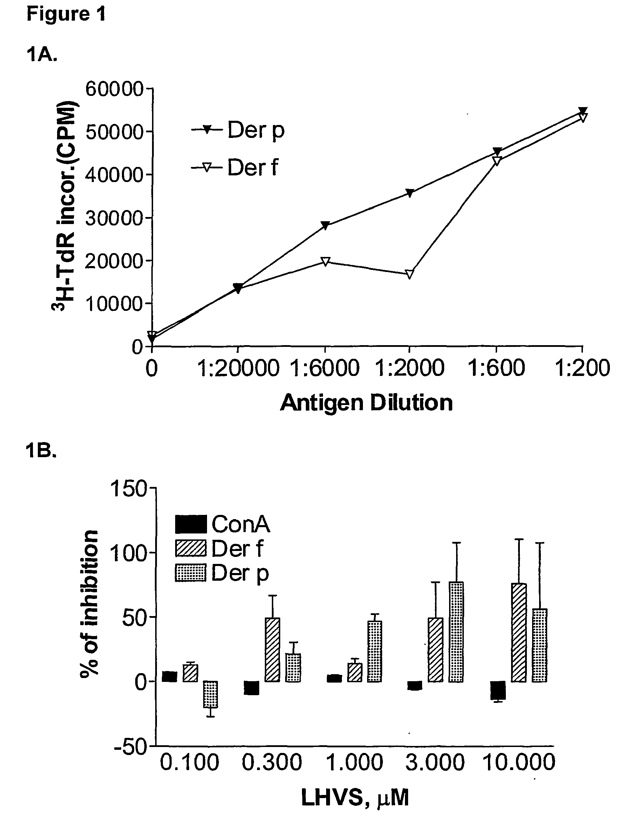

FIG. 1 shows the inhibition of human T cell proliferative responses to two species of dust mites, *Der p* and *Der f.* Top panel, FIG. 1A: Dilution curve for purified PBMC from an allergy donor were cultured with titrated doses of allergen extracts prepared from *Der p* and *Der f* for seven days. Proliferation of T cells was scored by measuring ³H-thymidine incorporation for 18 h at the end of culture. Bottom panel, FIG. 1 B: Effect of titrated doses of LHVS on proliferative responses of T cells to dust mite extracts.

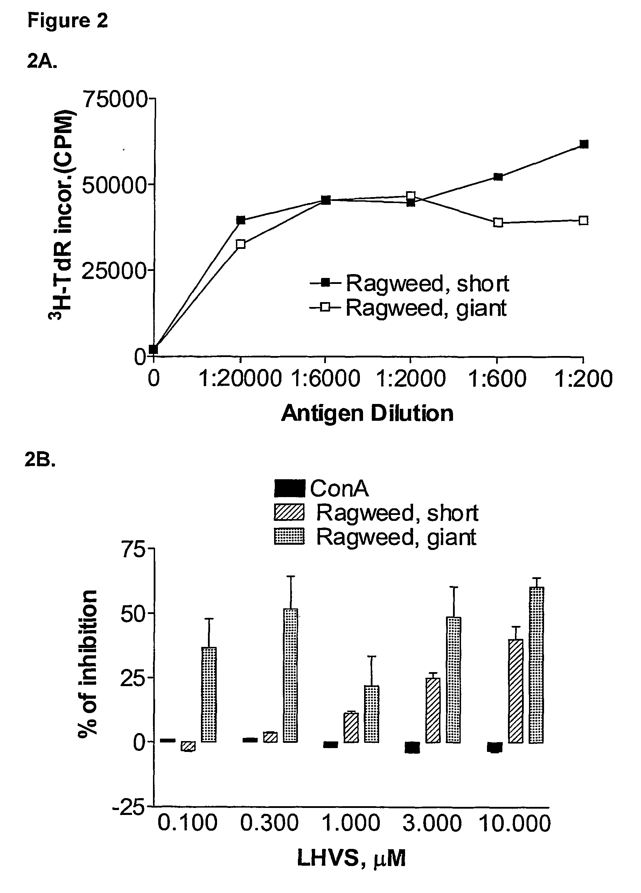

FIG. 2 shows the inhibition of human T cell proliferative responses to ragweeds but not ConA by LHVS. Top panel, FIG. 2A: Dilution curve for purified PBMC from an allergy donor were cultured with titrated doses of allergen extracts prepared from Ragweed short and Ragweed giant for seven days. Proliferation of T cells was scored by measuring ³H-thymidine incorporation for 18 h at the end of culture. Bottom panel, FIG. 2B: Effect of titrated doses of LHVS on proliferative responses of T cells to ragweed extracts.

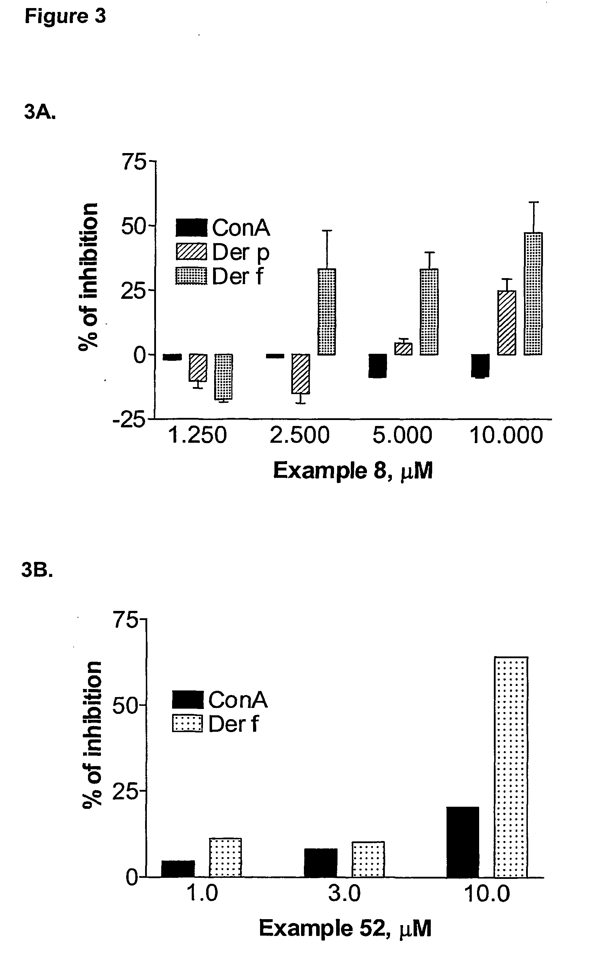

FIG. 3 shows the inhibition of human T cell proliferative responses to *Der f* but not ConA by two cathepsin S inhibitors. Purified PBMC from an allergy donor were cultured with allergen extracts prepared from *Der f* in the presence of titrated doses of indicated example compounds for seven days. Proliferation of T cells was scored by measuring ³H-thymidine incorporation for 18 h at the end of culture. Top panel, FIG. 3A shows the effect of titrated doses of Example 8. Bottom panel, FIG. 3B shows the effect of titrated doses of Example 52.

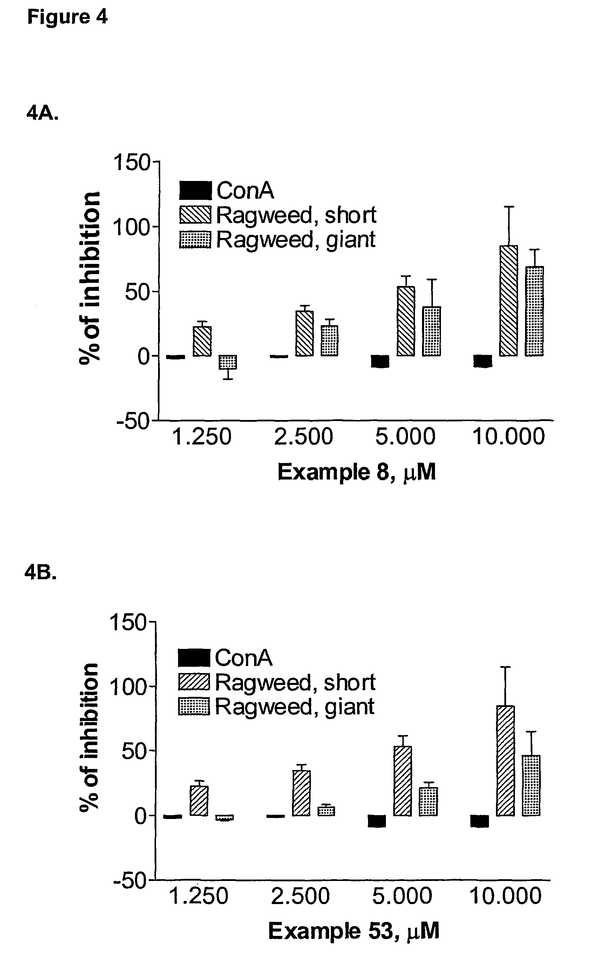

FIG. 4 shows the inhibition of human T cell proliferative responses to ragweeds but not ConA by two cathepsin S inhibitors. Top panel, FIG. 4A: Effect of titrated doses of Example 8 on proliferative responses of T cells to ragweed extracts. Bottom panel, FIG. 4B: Effect of titrated doses of Example 53 on proliferative responses of T cells to ragweed extracts.

### Detailed Description of the Invention

A target of the present invention was to determine whether the presentation of particular antigens in a human system is affected by the inhibition of cathepsin S. According to the invention, it now has been found that inhibitors of cathepsin S block the presentation of several crude allergen extracts in a human *ex vivo* assay, thereby supporting the use of cathepsin S inhibitors in the manufacture of a pharmaceutical composition for the treatment of allergic conditions.

Blocking li degradation should decrease antigen presentation to CD4 T cells and disrupt the normal immune response. A cathepsin S inhibitor should specfically affect the activation of CD4 T cells, thus limiting the extent of concomitant immunosuppression, an undesirable side effect of corticosteroid therapy.

By using cathepsin S inhibitors according to the methods of the present invention, the immunological component of the allergic reaction can be blocked to varying degrees, with the advantage over current therapies of being more selective, having fewer or reduced side effects, or both. The present invention is based, in part, on the finding that cathepsin S inhibitors can block the presentation of crude allergen extracts in a human *ex vivo* assay. This *ex vivo* system closely mimics the process that occurs in the whole body wherein antigens enter the blood stream,and are presented by antigen presenting cells, which in turn activate CD4 T cells. In the case of treating a subject, the inhibitor or a metabolite thereof would also be present in the blood as in the ex *vivo* assay.

The invention features the manufacture of a pharmaceutical composition for the treatment of an allergic condition using one or more pyrazole compounds of formula (I).

### A. Terms

The following terms are defined below and by their usage throughout this disclosure.

"Alkyl" includes optionally substituted straight chain and branched hydrocarbons with at least one hydrogen removed to form a radical group. Alkyl groups include methyl, ethyl, propyl, isopropyl, butyl, isobutyl, t-butyl, 1-methylpropyl, pentyl, isopentyl, sec-pentyl, hexyl, heptyl, octyl, and so on. Alkyl includes cycloalkyl, such as cyclopropyl, cyclobutyl, cyclopentyl, and cyclohexyl.

"Alkenyl" includes optionally substituted straight chain and branched hydrocarbon radicals as above with at least one carbon-carbon double bond (sp²). Alkenyls include ethenyl (or vinyl), prop-1-enyl, prop-2-enyl (or allyl), isopropenyl (or 1-methylvinyl), but-1-enyl, but-2-enyl, butadienyls, pentenyls, hexa-2,4-dienyl, and so on. Hydrocarbon radicals having a mixture of double bonds and triple bonds, such as 2-penten-4-ynyl, are grouped as alkynyls herein. Alkenyl includes cycloalkenyl. *Cis* and *trans* or (E) and (Z) forms are included within the invention.

"Alkynyl" includes optionally substituted straight chain and branched hydrocarbon radicals as above with at least one carbon-carbon triple bond (sp). Alkynyls include ethynyl, propynyls, butynyls, and pentynyls. Hydrocarbon radicals having a mixture of double bonds and triple bonds, such as 2-penten-4-ynyl, are grouped as alkynyls herein. Alkynyl does not include cycloalkynyl.

"Alkoxy" includes an optionally substituted straight chain or branched alkyl group with a terminal oxygen linking the alkyl group to the rest of the molecule. Alkoxy includes methoxy, ethoxy, propoxy, isopropoxy, butoxy, t-butoxy, pentoxy and so on. "Aminoalkyl", "thioalkyl", and "sulfonylalkyl" are analogous to alkoxy, replacing the terminal oxygen atom of alkoxy with, respectively, NH (or NR), S, and SO₂. Heteroalkyl includes alkoxy, aminoalkyl, thioalkyl, and so on.

"Aryl" includes phenyl, naphthyl, biphenylyl, tetrahydronaphthyl, and so on, any of which may be optionally substituted. Aryl also includes arylalkyl groups such as benzyl, phenethyl, and phenylpropyl. Aryl includes a ring system containing an optionally substituted 6-membered carbocyclic aromatic ring, said system may be bicyclic, bridge, and/or fused. The system may include rings that are aromatic, or partially or completely saturated. Examples of ring systems include indenyl, pentalenyl, 1-4-dihydronaphthyl, indanyl, benzimidazolyl, benzothiophenyl, indolyl, benzofuranyl, isoquinolinyl, and so on.

"Heterocyclyl" includes optionally substituted aromatic and nonaromatic rings having carbon atoms and at least one heteroatom (O, S, N) or heteroatom moiety (SO₂, CO, CONH, COO) in the ring. Unless otherwise indicated, a heterocyclic radical may have a valence connecting it to the rest of the molecule through a carbon atom, such as 3-furyl or 2-imidazolyl, or through a heteroatom, such as N-piperidyl or 1-pyrazolyl. Preferably a monocyclic heterocyclyl has between 4 and 7 ring atoms, or between 5 and 6 ring atoms; there may be between 1 and 5 heteroatoms or heteroatom moieties in the ring, and preferably between 1 and 3. A heterocyclyl may be saturated, unsaturated, aromatic (e.g., heteroaryl), nonaromatic, or fused.

Heterocyclyl also includes fused, e.g., bicyclic, rings, such as those optionally condensed with an optionally substituted carbocyclic or heterocyclic five- or six-membered aromatic ring. For example, "heteroaryl" includes an optionally substituted six-membered heteroaromatic ring containing 1, 2 or 3 nitrogen atoms condensed with an optionally substituted five- or six-membered carbocyclic or heterocyclic aromatic ring. Said heterocyclic five- or six-membered aromatic ring condensed with the said five- or six-membered aromatic ring may contain 1, 2 or 3 nitrogen atoms where it is a six-membered ring, or 1, 2 or 3 heteroatoms selected from oxygen, nitrogen and sulfur where it is a five-membered ring.

Examples of heterocyclyls include thiazoylyl, furyl, pyranyl, isobenzofuranyl, pyrrolyl, imidazolyl, pyrazolyl, isothiazolyl, isoxazolyl, pyridyl, pyrazinyl, pyrimidinyl, pyridazinyl, indolizinyl, isoindolyl, indolyl, indazolyl, purinyl, quinolyl, furazanyl, pyrrolidinyl, pyrrolinyl, imdazolidinyl, imidazolinyl, pyrazolidinyl, pyrazolinyl, piperidyl, piperazinyl, indolinyl, and morpholinyl. For example, preferred heterocyclyls or heterocyclic radicals include morpholinyl, piperazinyl, pyrrolidinyl, pyridyl, cyclohexylimino, cycloheptylimino, and more preferably, piperidyl.

Examples illustrating heteroaryl are thienyl, furanyl, pyrrolyl, imidazolyl, oxazolyl, thiazolyl, benzothienyl, benzofuranyl, benzimidazolyl, benzoxazolyl, benzothiazolyl.

"Acyl" refers to a carbonyl moiety attached to either a hydrogen atom (i.e., a formyl group) or to an optionally substituted alkyl or alkenyl chain, or heterocyclyl.

"Halo" or "halogen" includes fluoro, chloro, bromo, and iodo, and preferably chloro or bromo as a substituent.

"Alkanediyl" or "alkylene" represents straight or branched chain optionally substituted bivalent alkane radicals such as, for example, methylene, ethylene, propylene, butylene, pentylene or hexylene.

"Alkenediyl" represents, analogous to the above, straight or branched chain optionally substituted bivalent alkene radicals such as, for example, propenylene, butenylene, pentenylene or hexenylene. In such radicals, the carbon atom linking a nitrogen preferably should not be unsaturated.

"Aroyl" refers to a carbonyl moiety attached to an optionally substituted aryl or heteroaryl group, wherein aryl and heteroaryl have the definitions provided above. In particular, benzoyl is phenylcarbonyl.

As defined herein, two radicals, together with the atom(s) to which they are attached may form an optionally substituted 4- to 7-, 5 - to 7-, or a 5- to 6-membered ring carbocyclic or heterocyclic ring, which ring may be saturated, unsaturated or aromatic. Said rings may be as defined above in the Summary of the Invention section. Particular examples of such rings are as follows in the next section.

"Pharmaceutically acceptable salts, esters, and amides" include carboxylate salts (e.g., C₁₋₈ alkyl, cycloalkyl, aryl, heteroaryl, or non-aromatic heterocyclic) amino acid addition salts, esters, and amides which are within a reasonable benefit/risk ratio, pharmacologically effective and suitable for contact with the tissues of patients without undue toxicity, irritation, or allergic response. Representative salts include hydrobromide, hydrochloride, sulfate, bisulfate, nitrate, acetate, oxalate, valerate, oleate, palmitate, stearate, laurate, borate, benzoate, lactate, phosphate, tosylate, citrate, maleate, fumarate, succinate, tartrate, naphthylate, mesylate, glucoheptonate, lactiobionate, and laurylsulfonate. These may include alkali metal and alkali earth cations such as sodium, potassium, calcium, and magnesium, as well as non-toxic ammonium, quaternary ammonium, and amine cations such as tetramethyl ammonium, methylamine, trimethylamine, and ethylamine. See example, S.M. Berge, et al., "Pharmaceutical Salts," J. Pharm. Sci., 1977, 66:1-19 which is incorporated herein by reference. Representative pharmaceutically acceptable amides of the invention include those derived from ammonia, primary C₁₋₆ alkyl amines and secondary di (C₁₋₆ alkyl) amines. Secondary amines include 5- or 6-membered heterocyclic or heteroaromatic ring moieties containing at least one nitrogen atom and optionally between 1 and 2 additional heteroatoms. Preferred amides are derived from ammonia, C₁₋₃ alkyl primary amines, and di (C₁₋₂ alkyl)amines. Representative pharmaceutically acceptable esters of the invention include C₁₋₇ alkyl, C₅₋₇ cycloalkyl, phenyl, and phenyl(C₁₋₆)alkyl esters. Preferred esters include methyl esters.

"Patient" or "subject" includes mammals such as humans and animals (dogs, cats, horses, rats, rabbits, mice, non-human primates) in need of observation, experiment, treatment or prevention in connection with the relevant disease or condition. Preferably, the patient or subject is a human.

"Composition" includes a product comprising the specified ingredients in the specified amounts as well as any product which results directly or indirectly from combinations of the specified ingredients in the specified amounts.

"Therapeutically effective amount" or "effective amount" means that amount of active compound or pharmaceutical agent that elicits the biological or medicinal response in a tissue system, animal or human that is being sought by a researcher, veterinarian, medical doctor or other clinician, which includes alleviation of the symptoms of the disease or disorder being treated.

Concerning the various radicals in this disclosure and in the claims, three general remarks are made. The first remark concerns valency. As with all hydrocarbon radicals, whether saturated, unsaturated or aromatic, and whether or not cyclic, straight chain, or branched, and also similarly with all heterocyclic radicals, each radical includes substituted radicals of that type and monovalent, bivalent, and multivalent radicals as indicated by the context of the claims. The context will indicate that the substituent is an alkylene or hydrocarbon radical with at least two hydrogen atoms removed (bivalent) or more hydrogen atoms removed (multivalent). An example of a bivalent radical linking two parts of the molecule is G in formula (I) which links two rings.

Second, radicals or structure fragments as defined herein are understood to include substituted radicals or structure fragments. Hydrocarbyls include monovalent radicals containing carbon and hydrogen such as alkyl, alkenyl, alkynyl, cycloalkyl, and cycloalkenyl (whether aromatic or unsaturated), as well as corresponding divalent radicals such as alkylene, alkenylene, phenylene, and so on. Heterocarbyls include monovalent and divalent radicals containing carbon, hydrogen, and at least one heteroatom. Examples of monovalent heterocarbyls include acyl, acyloxy, alkoxyacyl, heterocyclyl, heteroaryl, aroyl, benzoyl, dialkylamino, hydroxyalkyl, and so on.

Using "alkyl" as an example, "alkyl" should be understood to include substituted alkyl having one or more substitutions, such as between 1 and 5, 1 and 3, or 2 and 4 substituents. The substituents may be the same (dihydroxy, dimethyl), similar (chlorofluoro), or different (chlorobenzyl- or aminomethyl-substituted). Examples of substituted alkyl include haloalkyl (such as fluoromethyl, chloromethyl, difluoromethyl, perchloromethyl, 2-bromoethyl, perfluoromethyl, and 3-iodocyclopentyl), hydroxyalkyl (such as hydroxymethyl, hydroxyethyl, 2-hydroxypropyl, aminoalkyl (such as aminomethyl, 2-aminoethyl, 3-aminopropyl, and 2-aminopropyl), nitroalkyl, alkylalkyl, and so on. A di(C₁₋₆ alkyl)amino group includes independently selected alkyl groups, to form, for example, methylpropylamino and isopropylmethylamino, in addition dialkylamino groups having two of the same alkyl group such as dimethyl amino or diethylamino.

Third, only stable compounds are intended. For example, where there is an NR'R" group, and R can be an alkenyl group, the double bond is at least one carbon removed from the nitrogen to avoid enamine formation. Similarly, where a dashed line is an optional sp² bond, if it is absent, the appropriate hydrogen atom(s) is(are) included.

Preferred substitutions for Ar or Ar₁ include methyl, methoxy, fluoromethyl, difluoromethyl, perfluoromethyl (trifluoromethyl), 1-fluoroethyl, 2-fluoroethyl, ethoxy, fluoro, chloro, and bromo, and particularly methyl, bromo, chloro, perfluoromethyl, perfluoromethoxy, methoxy, and fluoro. Preferred substitution patterns for Ar or Ar₁ are 4-substituted or 3,4-disubstituted phenyl.

Compounds of the invention are further described in the next section.

### B. Compounds

The invention features the treatment of an allergic condition with one or more compounds of formula (I) as described in the Summary section.

Preferred compounds include those wherein:
(a) Ar₂ is selected from formulae (e);
(b) Ar₂ is selected from formulae (f);
(c) Ar₂ is selected from formula (a)-(d);
(d) R¹ is halogen, C₁₋₅ alkoxy, hydroxy, C₁₋₅ alkyl, cyano, nitro, R^{a}R^{b}N or is taken together with R²⁷;
(e) R¹ is taken together with R²⁷;
(f) R¹ and R²⁷ taken together are selected from:
   (1) -CH₂NR⁹-(C=O)-
   (2) OCH₂(C=O)-
   (3) -CH₂CH₂(C=O)-
   (4) -CH₂-O(C=O)-
   (5) -CH₂S(C=O)-
   (6) -O(C=O)-
   (7) -CH₂(C=O)-
   (8) -NR⁹(C=O)-
   (9) -NR⁹(SO₂)-
   (10) -CH₂NR⁹SO₂₋
   (11) -NR⁹CH₂(C=O)- and -SCH2(C=O)-
(g) R¹ and R²⁷ taken together are selected from:
   a) -CH₂-(C=O)-
   b) -O(C=O)-
   c) -CH₂CH₂-
   d) -S(C=O)-
   e) -N=N-
   f) -NR⁹SO₂-
   g) -N=CR⁹-
   h) -NR⁹(C=O)- and
   i) -CH=CH-;
(h) R² is hydrogen, halogen, C₁₋₅ alkoxy, C₁₋₅ alkyl, cyano, or R^{e}R^{f}N, where R^{e} and R^{f} are H or C₁₋₅ alkyl, or are taken together to form a 5-7 membered heterocyclic ring;
(i) R³ is hydrogen, halogen, C₁₋₅ alkoxy, C₁₋₅ alkyl, cyano, nitro, or R^{g}R^{h}N, where R^{e} and R^{f} are H or C₁₋₅ alkyl, or are taken together to form a 5-7 membered heterocyclic ring;
(j) R⁵ and R⁶ are independently selected from hydrogen and C₁₋₃ alkyl;
(k) one of R⁵ and R⁶ is H;
(I) R⁵ and R⁶ are each H;
(m) one of R⁷ and R⁸ is H and the other is 5-7 membered carbocyclyl or heterocyclyl;
(n) R⁷ and R⁸ are taken together to form an optionally substituted 5- to 7-membered carbocyclic or heterocyclic ring;
(o) R⁷ and R⁸ taken together form a six-membered heterocyclyl;
(p) R⁷ and R⁸ taken together form pyridinyl, pyrimidinyl, or piperazinyl, optionally N-substituted with -(C=O)R⁴, -SO₂R⁴, or -(C=O)NHR⁴⁴;
(q) each of R^{a} , R^{e}, R^{m}, and R^{o} is independently selected from hydrogen, C₁₋₅ alkyl, C₂₋₈ acyl, and the respective ROC=O, RRNC=O, RSO, RSO₂, and RRNSO₂ groups;
(r) each of R^{a}, R^{e}, R^{m}, R^{o}, R^{b}, R^{f}, Rⁿ, and R^{p} is independently selected from hydrogen and C₁₋₅ alkyl; or , independently, R^{a} and R^{b}, R^{e} and R^{f}, R^{m} and Rⁿ, and R^{o} and R^{p}, taken together, form an optionally substituted 4- to 7-membered carbocyclic or heterocyclic ring;
(s) (1)R^{a} and R^{b} taken together are independently morpholinyl, piperidinyl, or pyrrolidinyl; (2) R^{e} and R^{f} taken together are morpholinyl, piperidinyl, or pyrrolidinyl; or (3) both (1) and (2) apply;
(t) each of R^{c} and R^{d} , Rⁱ and R^{j}, R^{k} and R^{l} is independently hydrogen or C₁₋₅ alkyl, alternatively, R^{c} and R^{d}, Rⁱ and Rj, and R^{k} and R^{l}, independently, can be taken together to form an optionally substituted 4- to 7- membered heterocyclic ring, which ring may be saturated, unsaturated or aromatic;
(u) R^{c} and R^{d}, Rⁱ and R^{j}, and R^{k} and R^{l} , independently, are taken together to form an optionally substituted 4- to 7- membered heterocyclic ring, which ring may be saturated, unsaturated or aromatic;
(v) each of R^{b}, R^{f}, Rⁿ, R^{p}, R³², R³³, R³⁴, R³⁵, R³⁶, R³⁷, R³⁹, and R⁴⁰ is independently H or C₁₋₅ alkyl;
(w) each of R¹¹, R¹² , R¹³ , R¹⁴ , R¹⁵, R¹⁶, R¹⁹ , R³⁸, and R⁴¹ is independently H or C₁₋₅ alkyl;
(x) R⁹ is C₁₋₅ alkyl, C₂₋₈ acyl, R¹⁷OC=O, R¹⁷R¹⁸NC=O, R¹⁶S, R¹⁶SO, R¹⁶SO₂, or R¹⁷R¹⁸NSO₂; and R^{h} hydrogen or C₁₋₅ alkyl; alternatively, R^{g} and R^{h} can be taken together to form an optionally substituted 4- to 7- membered heterocyclic ring;
(y) R¹⁷ and R¹⁸ independently are hydrogen or C₁₋₅ alkyl;
(z) n is 1;
(aa) n is 0;
(bb) G is C₃₋₄ alkanediyl, optionally substituted with hydroxy, halogen, (L)-C₁₋₅ alkyloxy, or [(L)-C₁₋₅ alkylene]amino;
(cc) G is C₃ alkanediyl, optionally substituted with hydroxy, (L)-C₁₋₅ alkyloxy, or [(L)-C₁₋₅ alkylene]amino;
(dd) each of R²⁰ and R²¹ is independently selected from hydrogen, halogen, C₁₋₅ alkoxy, C₁₋₅ alkyl, cyano, nitro, and R^{m}RⁿN or R^{o}R^{p}N, respectively;
(ee) each of R²⁰ and R²¹ is independently selected from hydrogen, halogen, C₁₋₃ alkyl, and R^{m}RⁿN or R^{o}R^{p}N, respectively;
(ff) Ar represents a monocyclic ring, optionally substituted with 1 to 2 substituents selected from halogen, C₁₋₅ alkyl, cyano, azido, nitro, R²²R²³N, halomethyl, and halomethoxy;
(gg) Ar is a six membered ring substituted with between 1 and 2 substituents independently selected from methyl, halogen, CF₃, and OCF₃, said substituent or substituents being at the 4- position, or at the 3- and 4-positions, respectively;
(hh) each of R²², R²³, and R²⁴ is hydrogen or C₁₋₅ alkyl;
(ii) R²⁵ and R²⁶ independently are hydrogen or C₁₋₅ alkyl; or, alternatively, R²⁵ and R²⁶ can be taken together to form an optionally substituted 4- to 7-membered heterocyclic ring;
(jj) each of R²⁵ and R²⁶ is independently hydrogen or C₁₋₅ alkyl;
(kk) W is NR²⁷;
(ll) W is CHR²⁸, and R²⁸ is hydrogen or C₁₋₅ alkyl;
(mm) R²⁹ is C₁₋₅ alkyl; or R³⁰ and R³¹ are independently selected from hydrogen and C₁₋₅ alkyl, or R³⁰ and R³¹ are taken together to form a 5-6 membered heterocyclyl;
(nn) Ar₂ is formula (e) and R¹ is halogen, C₁₋₅ alkoxy, hydroxy, C₁₋₅ alkyl, cyano, nitro, and R^{a}R^{b}N, or R¹ can be taken together with R²⁷ as provided below; R² is hydrogen, halogen, C₁₋₅ alkoxy, C₁₋₅ alkyl, or R^{e}R^{f}N; R³ is hydrogen, halogen, C₁₋₅ alkoxy, hydroxy, C₁₋₅ alkyl, cyano, R^{g}R^{h}N; R⁵ and R⁶ are independently selected from hydrogen and C₁₋₃ alkyl;
(oo) R⁷ and R⁸ independently are taken together to form an optionally substituted 5- to 7- membered carbocyclic or heterocyclic ring, which ring may be saturated, unsaturated or aromatic;
(pp) each of R^{a}, R^{e} , R^{m} , and R^{o} is independently selected from hydrogen, C₁₋₅ alkyl, C₂₋₈ acyl, and the respective ROC=O, RRNC=O, RS, RSO, RSO₂, and RRNSO₂ groups;
(qq) each of R^{b}, R^{f}, Rⁿ, and R^{p}, is independently selected from hydrogen and C₁₋₅ alkyl; each of R¹¹, R¹², R¹³, R¹⁴, R¹⁵, R¹⁶, R¹⁹, and R³⁸ is independently C₁₋₅ alkyl; each of R^{c} and R^{d}, Rⁱ and R^{j}, R^{k} and R^{l}, R³² and R³³, R³⁴ and R³⁵, R³⁶ and R³⁷ are independently are hydrogen or C₁₋₅ alkyl, or are taken together to form an optionally substituted 4- to 7- membered heterocyclic ring;
(rr) R^{g} is hydrogen, C₁₋₅ alkyl, C₂₋₈ acyl, R¹⁷OC=O, R¹⁷ R¹⁸NC=O, R¹⁶S, R¹⁶SO, R¹⁶SO₂, or R¹⁷R¹⁸NSO₂; R^{h} is hydrogen or C₁₋₅ alkyl;
   alternatively, R^{g} and R^{h} can be taken together to form an optionally substituted 4- to 7- membered heterocyclic ring; R¹⁷ and R¹⁸ independently are hydrogen or C₁₋₅ alkyl; n is 0 or 1;
(ss) G is C₃₋₄ alkenediyl or C₃₋₄ alkanediyl, optionally substituted with hydroxy, halogen, C₁₋₅ alkyloxy, (L)-C₁₋₅ alkoxy, or [(L)-C₁₋₅ alkylene]amino; L is amino, mono- or di-C₁₋₅ alkylamino, pyrrolidinyl, morpholinyl, piperidinyl homopiperidinyl, or piperazinyl, wherein available ring nitrogens may be optionally substituted with C₁₋₅ alkyl, benzyl, C₁₋₅ alkylcarbonyl, or C₁₋₅ alkyloxycarbonyl;
(tt) Yₑ is nitrogen or R²⁰C; Zₑ is nitrogen or R²¹C ;
(uu) R²⁰ and R²¹ are independently selected from hydrogen, halogen, C₁₋₅ alkoxy, C₁₋₅ alkyl, cyano, nitro, and R^{m}RⁿN or R^{o}R^{p}N, respectively; alternatively, R³ and R²⁰ or R³ and R²¹ can be taken together to form an optionally substituted 5- or 6-membered carbocyclic or heterocyclic ring;
(vv) Ar represents a monocyclic or bicyclic aryl or heteroaryl ring, optionally substituted with between 1 and 3 substituents independently selected from halogen, C₁₋₅ alkoxy, C₁₋₅ alkyl, cyano, azido, nitro, R²²R²³N, R²⁴SO₂, R²⁴OC=O, R²⁵R²⁶NC=O, CF₃, OCF₃, CF₃S, and C₁₋₅ alkylthio; R²² is hydrogen, C₁₋₅alkyl, phenyl, benzyl, phenethyl, C₁₋₅heterocyclyl, C₂₋₈acyl, aroyl, R²⁴OC=O, R²⁵R²⁶NC=O, R²⁴SO, R²⁴SO₂, or R²⁵R²⁶NSO₂; R²³ is hydrogen or C₁₋₅alkyl;
(ww) alternatively, R²² and R²³ can be taken together to form an optionally substituted 4- to 7- membered heterocyclic ring; R²⁴ is hydrogen or C₁₋₅ alkyl; R²⁵ and R²⁶ are independently hydrogen or C₁₋₅ alkyl; or, alternatively, R²⁵ and R²⁶ can be taken together to form an optionally substituted 4- to 7-membered heterocyclic; W is NR²⁷or CHR²⁸; R²⁷ is hydrogen, C₁₋₅ alkyl, R²⁹OC=O, R³⁰R³¹NC=O, R²⁹SO, R²⁹SO₂, or R³⁰R³¹NSO₂ ; or, alternatively, R²⁷ and R¹ can be taken together to form an optionally substituted 5- or 6-membered heterocyclic ring, which ring may be saturated, unsaturated or aromatic; R²⁸ is hydrogen, hydroxy, C₁₋₅ heterocyclyl, phenyl, or C₁₋₅ alkyl; R²⁹ is C₁₋₅ alkyl; R³⁰ and R³¹ are independently selected from hydrogen, C₁₋₅ alkyl; alternatively, R³⁰ and R³¹ can be taken together to form an optionally substituted 4- to 7-membered heterocyclic;
(xx) one of R⁵ and R⁶ is H; R⁷ and R⁸ are taken together to form an optionally substituted 6- membered carbocyclic or heterocyclic ring; and Ar represents a monocyclic ring, optionally substituted with 1 to 2 substituents selected from halogen, C₁₋₅ alkyl, cyano, azido, nitro, R²²R²³N, CF₃ and OCF₃;
(yy) both R⁵ and R⁶ are each H, and Ar is a six membered ring substituted with between 1 and 2 substituents independently selected from halogen, methyl, CF₃, and OCF₃, said substituent or substituents being at the 4-position, or at the 3- and 4-positions;
(zz) a R⁷ and R⁸ taken together form tetrahydropyridinyl, optionally N-substituted with -(C=O)R⁴, -SO₂R⁴, or -(C=O)NHR⁴⁴;
(aaa) X_{f} is C=O, SO₂, or CHR^{1f}, and Y_{f} is O or NR^{2f}, where R^{2f} is H, C₁₋₅ alkyl, C₂₋₅ heterocyclyl, C₁₋₅ cyanoalkyl, or (C₁₋₅ alkoxycarbonyl)C₁₋₅ alkylene;
(bbb) R^{2f} is H, C₁₋₃ alkyl, or a C₂₋₅ heterocyclyl;
(ccc)X_{f} is C=O, and Y_{f} is O, CHR^{2f} or NR^{2f}, where R^{2f} is H, C₁₋₅ alkyl, C₂₋₅ heterocyclyl, C₁₋₅ cyanoalkyl, or (C₁₋₅ alkoxycarbonyl)C₁₋₅ alkylene;
(ddd) X_{f} is C=O and Y_{f} is O;
(eee) m is 0 and p is 0; m is 0 and p is 1; or m is 1 and p is 0;
(fff) p is 0;
(ggg) R^{z} is H;
(hhh) R^{z} is OH;
(iii) R^{z} is absent;
(jjj) R²⁰ and R³ taken together are a six-membered carbocyclic or heterocyclic ring optionally substituted with between 1 and 3 substituents independently selected from halo, C₁₋₃ alkoxy, di(C₁₋₃ alkyl)amino, and C₂₋₅ acyl;
(kkk) each of R²⁰ and R³ is H; and
(III) combinations of the above.

Specific preferred compounds include those in the Examples provided, such as:
1-(1-{2-Hydroxy-3-[5-methanesulfonyl-3-(4-trifluoromethyl-phenyl)-4,5,6,7-tetrahydro-pyrazolo[4,3-c]pyridin-1-yl]-propyl}-piperidin-4-yl)-1,3-dihydro-benzoimidazol-2-one ; 1-(1-{3-[3-(3,4-Dichloro-phenyl)-5-methanesulfonyl-4,5,6,7-tetrahydro-pyrazolo[4,3-c]pyridin-1-yl]-propyl}-piperidin-4-yl)-1,3-dihydro-benzoimidazol-2-one ; 3-(3,4-Dichloro-phenyl)-1-{3-[4-(2-oxo-2,3-dihydro-benzoimidazol-1-yl)-piperidin-1-yl]-propyl}-1,4,6,7-tetrahydropyrazolo[4,3-c]pyridine-5-carboxylic acid amide ; 6-Chloro-1-(1-{3-[5-methanesulfonyl-3-(4-trifluoromethyl-phenyl)-4,5,6,7-tetrahydro-pyrazolo[4,3-c]pyridin-1-yl]-propyl}-piperidin-4-yl)-1,3-dihydro-benzoimidazol-2-one ; 3-(3,4-Dichloro-phenyl)-1-{3-[4-(3-methyl-2-oxo-2,3-dihydro-benzoimidazol-1-yl)-piperidin-1-yl]-propyl}-1,4,6,7-tetrahydro-pyrazolo[4,3-c]pyridine-5-carboxylic acid amide ; [3-(1-{2-Hydroxy-3-[5-methanesulfonyl-3-(4-trifluoromethylphenyl)-4,5,6,7-tetrahydro-pyrazolo[4,3-c]pyridin-1-yl]-propyl}-piperidin-4-yl)-2-oxo-2,3-dihydro-benzoimidazol-1-yl]-acetonitrile ; [3-(1-{2-Hydroxy-3-[5-methanesulfonyl-3-(4-trifluoromethyl-phenyl)-4,5,6,7-tetrahydro-pyrazolo[4,3-c]pyridin-1-yl]-propyl}-piperidin-4-yl)-2-oxo-2,3-dihydro-benzoimidazol-1-yl]-acetic acid ethyl ester ; 5-Chloro-3-(1-{2-hydroxy-3-[5-methanesulfonyl-3-(4-trifluoromethyl-phenyl)-4,5,6,7-tetrahydro-pyrazolo[4,3-c]pyridin-1-yl]-propyl}-piperidin-4-yl)-1-methyl-1,3-dihydro-benzoimidazol-2-one ; 1-{3-[4-(6-Chloro-3-methyl-2-oxo-2,3-dihydro-benzoimidazol-1-yl)-piperidin-1-yl]-propyl}-3-(3,4-dichloro-phenyl)-1,4,6,7-tetrahydro-pyrazolo[4,3-c]pyridine-5-carboxylic acid amide ; 3-(1-{2-Hydroxy-3-[5-methanesulfonyl-3-(4-trifluoromethyl-phenyl)-4,5,6,7-tetrahydro-pyrazolo[4,3-c]pyridin-1-yl]-propyl}-piperidin-4-yl)-1,5-dimethyl-1,3-dihydro-benzoimidazol-2-one ; 3-(1-{2-Hydroxy-3-[5-methanesulfonyl-3-(4-trifluoromethyl-phenyl)-4,5,6,7-tetrahydro-pyrazolo[4,3-c]pyridin-1-yl]-propyl}-piperidin-4-yl)-1,3-dihydro-imidazo[4,5-b]pyridin-2-one ; 3-(1-{3-[3-(4-Bromo-phenyl)-5-methanesulfonyl-4,5,6,7-tetrahydro-pyrazolo[4,3-c]pyridin-1-yl]-2-hydroxy-propyl}-piperidin-4-yl)-5-methoxy-1,3-dihydroimidazo[4,5-b]pyridin-2-one ; 3-(4-Bromo-phenyl)-1-{2-hydroxy-3-[4-(5-methoxy-2-oxo-1,2-dihydro-imidazo[4,5-b]pyridin-3-yl)-piperidin-1-yl]-propyl}-1,4,6,7-tetrahydro-pyrazolo[4,3-c]pyridine-5-carboxylic acid amide ; 3-(1-{2-Hydroxy-3-[5-methanesulfonyl-3-(4-trifluoromethyl-phenyl)-4,5,6,7-tetrahydropyrazolo[4,3-c]pyridin-1-yl]-propyl}-piperidin-4-yl)-5-methoxy-1-methyl-1,3-dihydro-imidazo[4,5-b]pyridin-2-one ; 5-Dimethylamino-3-(1-{2-hydroxy-3-[5-methanesulfonyl-3-(4-trifluoromethyl-phenyl)-4,5,6,7-tetrahydro-pyrazolo[4,3-c]pyridin-1-yl]-propyl}-piperidin-4-yl)-1,3-dihydro-imidazo[4,5-b]pyridin-2-one; 6-Chloro-1-(1-{3-[5-methanesulfonyl-3-(4-trifluoromethyl-phenyl)-4,5,6,7-tetrahydro-pyrazolo[4,3-c]pyridin-1-yl]-propyl}-piperidin-4-yl)-1,3-dihydro-indol-2-one ; 1-(1-{2-Hydroxy-3-[5-methanesulfonyl-3-(4-trifluoromethyl-phenyl)-4,5,6,7-tetrahydro-pyrazolo[4,3-c]pyridin-1-y1]-propyl}-piperidin-4-yl)-3,4-dihydro-1H-quinolin-2-one ; 4-(1-{3-[5-Methanesulfonyl-3-(4-trifluoromethylphenyl)-4,5,6,7-tetrahydro-pyrazolo[4,3-c]pyridin-1-yl]-propyl}-piperidin-4-yl)-4H-benzo[1,4]oxazin-3-one ; 4-(1-{2-Hydroxy-3-[5-methanesulfonyl-3-(4-trifluoromethyl-phenyl)-4,5,6,7-tetrahydro-pyrazolo[4,3-c]pyridin-1-yl]-propyl}-piperidin-4-yl)-4H-benzo[1,4]oxazin-3-one ; and 1-(1-{3-[5-Methanesulfonyl-3-(4-trifluoromethyl-phenyl)-4,5,6,7-tetrahydro-pyrazolo[4,3-c]pyridin-1-yl]-propyl}-piperidin-4-yl)-3,4-dihydro-1H-quinazolin-2-one.

Furthermore, preferred compounds include those wherein Ar or Ar₁ is selected from 4-trifluoromethylphenyl, 4-bromophenyl, 4-chlorophenyl, 4-chloro-3-methylphenyl and 3,4-dichlorophenyl.
More preferred compounds include Examples 37 and 50.

### Related Compounds

The invention provides the disclosed compounds and closely related, pharmaceutically acceptable forms of the disclosed compounds, such as salts, esters, amides, acids, hydrates or solvated forms thereof; masked or protected forms; and racemic mixtures, or enantiomerically or optically pure forms. Related compounds also include compounds of the invention that have been modified to be detectable, e.g., isotopically labelled with ¹⁸F for use as a probe in positron emission tomography (PET) or single-photon emission computed tomography (SPECT).

The invention also includes disclosed compounds having one or more functional groups (e.g., hydroxyl, amino, or carboxyl) masked by a protecting group. See, e.g., Greene and Wuts, Protective Groups in Organic Synthesis, 3rd ed., (1999) John Wiley & Sons, NY. Some of these masked or protected compounds are pharmaceutically acceptable; others will be useful as intermediates. Synthetic intermediates and processes disclosed herein, and minor modifications thereof, are also within the scope of the invention.

### HYDROXYL PROTECTING GROUPS

Protection for the hydroxyl group includes methyl ethers, substituted methyl ethers, substituted ethyl ethers, substitute benzyl ethers, and silyl ethers.

### Substituted Methyl Ethers

Examples of substituted methyl ethers include methyoxymethyl, methylthiomethyl, *t*-butylthiomethyl, (phenyldimethylsilyl)methoxymethyl, benzyloxymethyl, *p*-methoxybenzyloxymethyl, (4-methoxyphenoxy)methyl, guaiacolmethyl, *t*-butoxymethyl, 4-pentenyloxymethyl, siloxymethyl, 2-methoxyethoxymethyl, 2,2,2-trichloroethoxymethyl, bis(2-chloroethoxy)methyl, 2-(trimethylsilyl)ethoxymethyl, tetrahydropyranyl, 3-bromotetrahydropyranyl, tetrahydrothiopyranyl, 1-methoxycyclohexyl, 4-methoxytetrahydropyranyl, 4-methoxytetrahydrothiopyranyl, 4-methoxytetrahydrothiopyranyl S,S-dioxido, 1-[(2-chloro-4-methyl)phenyl]-4-methoxypiperidin-4-yl, 1,4-dioxan-2-yl, tetrahydrofuranyl, tetrahydrothiofuranyl and 2,3,3a,4,5,6,7,7a-octahydro-7,8,8-trimethyl-4,7-methanobenzofuran-2-yl.

### Substituted Ethyl Ethers

Examples of substituted ethyl ethers include 1-ethoxyethyl, 1-(2-chloroethoxy)ethyl, 1-methyl-1-methoxyethyl, 1-methyl-1-benzyloxyethyl, 1-methyl-1-benzyloxy-2-fluoroethyl, 2,2,2-trichloroethyl, 2-trimethylsilylethyl, 2-(phenylselenyl)ethyl, *t-*butyl, allyl, *p-*chlorophenyl, *p-*methoxyphenyl, 2,4-dinitrophenyl, and benzyl.

### Substituted Benzyl Ethers

Examples of substituted benzyl ethers include *p*-methoxybenzyl, 3,4-dimethoxybenzyl, *o*-nitrobenzyl, *p*-nitrobenzyl, *p*-halobenzyl, 2,6-dichlorobenzyl, *p*-cyanobenzyl, *p*-phenylbenzyl, 2- and 4-picolyl, 3-methyl-2-picolyl N-oxido, diphenylmethyl, *p*, *p*'-dinitrobenzhydryl, 5-dibenzosuberyl, triphenylmethyl, α-naphthyldiphenylmethyl, *p-*methoxyphenyldiphenylmethyl, di(*p-*methoxyphenyl)phenylmethyl, tri(*p-*methoxyphenyl)methyl, 4-(4'-bromophenacyloxy)phenyldiphenylmethyl, 4,4',4"-tris(4,5-dichlorophthalimidophenyl)methyl, 4,4',4"-tris(levulinoyloxyphenyl)methyl, 4,4',4"-tris(benzoyloxyphenyl)methyl, 3-(*I*midazol-1-ylmethyl)bis(4',4"-dimethoxyphenyl)methyl, 1,1-bis(4-methoxyphenyl)-1'-pyrenylmethyl, 9-anthryl, 9-(9-phenyl)xanthenyl, 9-(9-phenyl-10-oxo)anthryl, 1,3-benzodithiolan-2-yl, and benzisothiazolyl S,S-dioxido.

### Silyl Ethers

Examples of silyl ethers include trimethylsilyl, triethylsilyl, triisopropylsilyl, dimethylisopropylsilyl, diethylisopropylsilyl, dimethylthexylsilyl, *t-*butyldimethylsilyl, *t-*butyldiphenylsilyl, tribenzylsilyl, tri-*p-*xylylsilyl, triphenylsilyl, diphenylmethylsilyl, and *t-*butylmethoxyphenylsilyl.

### Esters

In addition to ethers, a hydroxyl group may be protected as an ester. Examples of esters include formate, benzoylformate, acetate, chloroacetate, dichloroacetate, trichloroacetate, trifluoroacetate, methoxyacetate, triphenylmethoxyacetate, phenoxyacetate, *p-*chlorophenoxyacetate, *p-*P-phenylacetate, 3-phenylpropionate, 4-oxopentanoate(levulinate), 4,4-(ethylenedithio)pentanoate, pivaloate, adamantoate, crotonate, 4-methoxycrotonate, benzoate, *p-*phenylbenzoate, 2,4,6-trimethylbenzoate(mesitoate)

### Carbonates

Examples of carbonate protecting groups include methyl, 9-fluorenylmethyl, ethyl, 2,2,2-trichloroethyl, 2-(trimethylsilyl)ethyl, 2-(phenylsulfonyl)ethyl, 2-(triphenylphosphonio)ethyl, isobutyl, vinyl, allyl, *p-*nitrophenyl, benzyl, *p*-methoxybenzyl, 3,4-dimethoxybenzyl, *o-*nitrobenzyl, *p-*nitrobenzyl, S-benzyl thiocarbonate, 4-ethoxy-1-naphthyl, and methyl dithiocarbonate.

### Assisted Cleavage

Examples of assisted cleavage include 2-iodobenzoate, 4-azidobutyrate, 4-nitro-4-methylpentanoate, *o-*(dibromomethyl)benzoate, 2-formylbenzenesulfonate, 2-(methylthiomethoxy)ethyl carbonate, 4-(methylthiomethoxy)butyrate, and 2-(methylthiomethoxymethyl)benzoate.

### Miscellaneous Esters

Examples of miscellaneous esters include 2,6-dichloro-4-methylphenoxyacetate, 2,6-dichloro-4-(1,1,3,3-tetramethylbutyl)phenoxyacetate, 2,4-bis(1,1-dimethylpropyl)phenoxyacetate, chlorodiphenylacetate, isobutyrate, monosuccinoate, (E)-2-methyl-2-butenoate(tigloate), *o*-(methoxycarbonyl)benzoate, *p-*P-benzoate, α-naphthoate, nitrate, alkyl N,N,N',N'-tetramethylphosphorodiamidate, N-phenylcarbamate, borate, dimethylphosphinothioyl, and 2,4-dinitrophenylsulfenate.

### Sulfonates

Examples of sulfonates include sulfate, methanesulfonate(mesylate), benzylsulfonate, and tosylate.

### AMINO PROTECTING GROUPS

Protection for the amino group includes carbamates, amides, and special -NH protective groups.

Examples of carbamates include methyl and ethyl carbamates, substituted ethyl carbamates, assisted cleavage carbamates, photolytic cleavage carbamates, urea-type derivatives, and miscellaneous carbamates.

### Carbamates

Examples of methyl and ethyl carbamates include methyl and ethyl, 9-fluorenylmethyl, 9-(2-sulfo)fluorenylmethyl, 9-(2,7-dibromo)fluorenylmethyl, 2,7-di-*t-*butyl-[9-(10,10-dioxo-10,10,10,10-tetrahydrothioxanthyl)]methyl, and 4-methoxyphenacyl.

### Substituted Ethyl

Examples of substituted ethyl carbamates include 2,2,2-trichloroethyl, 2-trimethylsilylethyl, 2-phenylethyl, 1-(1-adamantyl)-1-methylethyl, 1,1-dimethyl-2-haloethyl, 1,1-dimethyl-2,2-dibromoethyl, 1,1-dimethyl-2,2,2-trichloroethyl, 1-methyl-1-(4-biphenylyl)ethyl, 1-(3,5-di-*t*-butylphenyl)-1-methylethyl, 2-(2'- and 4'-pyridyl)ethyl, 2-(N,N-dicyclohexylcarboxamido)ethyl, *t-*butyl, 1-adamantyl, vinyl, allyl, 1-isopropylallyl, cinnamyl, 4-nitrocinnamyl, 8-quinolyl, N-hydroxypiperidinyl, alkyldithio, benzyl, *p-*methoxybenzyl, *p-*nitrobenzyl, *p-*bromobenzyl, *p-*chlorobenzyl, 2,4-dichlorobenzyl, 4-methylsulfinylbenzyl, 9-anthrylmethyl and diphenylmethyl.

### Assisted Cleavage

Examples of assisted cleavage include 2-methylthioethyl, 2-methylsulfonylethyl, 2-(*p-*toluenesulfonyl)ethyl, [2-(1,3-dithianyl)]methyl, 4-methylthiophenyl, 2,4-dimethylthiophenyl, 2-phosphonioethyl, 2-triphenylphosphonioisopropyl, 1,1-dimethyl-2-cyanoethyl, *m*-chloro-*p-*acyloxybenzyl, *p-*(dihydroxyboryl)benzyl, 5-benzisoxazolylmethyl, and 2-(trifluoromethyl)-6-chromonylmethyl.

### Photolytic Cleavage

Examples of photolytic cleavage include *m-*nitrophenyl, 3,5-dimethoxybenzyl, *o-*nitrobenzyl, 3,4-dimethoxy-6-nitrobenzyl, and phenyl(*o-*nitrophenyl)methyl.

### Urea-Type Derivatives

Examples of urea-type derivatives include phenothiazinyl-(10)-carbonyl derivative, N' -*p-*toluenesulfonylaminocarbonyl, and N'-phenylaminothiocarbonyl.

### Miscellaneous Carbamates

Examples of miscellaneous carbamates include *t-*amyl, S-benzyl thiocarbamate, *p-*cyanobenzyl, cyclobutyl, cyclohexyl, cyclopentyl, cyclopropylmethyl, *p-*decyloxybenzyl, diisopropylmethyl, 2,2-dimethoxycarbonylvinyl, *o-*(N,N-dimethylcarboxamido)benzyl, 1,1-dimethyl-3-(N,N-dimethylcarboxamido)propyl, 1,1-dimethylpropynyl, di(2-pyridyl)methyl, 2-furanylmethyl, 2-iodoethyl, isobomyl, isobutyl, isonicotinyl, *p-*(*p'-*methoxyphenylazo)benzyl, 1-methylcyclobutyl, 1-methylcyclohexyl, 1-methyl-1-cyclopropylmethyl, 1-methyl-1-(3,5-dimethoxyphenyl)ethyl, 1-methyl-1-(*p-*phenylazophenyl)ethyl, 1-methyl-1-phenylethyl, 1-methyl-1-(4-pyridyl)ethyl, phenyl, *p-*(phenylazo)benzyl, 2,4,6-tri-*t-*butylphenyl, 4-(trimethylammonium)benzyl, and 2,4,6-trimethylbenzyl.

### Examples of amides include:

### Amides

N-formyl, N-acetyl, N-chloroacetyl, N-trichloroacetyl, N-trifluoroacetyl, N-phenylacetyl, N-3-phenylpropionyl, N-picolinoyl, N-3-pyridylcarboxamide, N-benzoylphenylalanyl derivative, N-benzoyl, N-*p-*phenylbenzoyl.

### Assisted Cleavage

N-*o-*nitrophenylacetyl, N-*o-*nitrophenoxyacetyl, N-acetoacetyl, (N'-dithiobenzyloxycarbonylamino)acetyl, N-3-(*p-*hydroxyphenyl)propionyl, N-3-(*o-*nitrophenyl)propionyl, N-2-methyl-2-(*o-*nitrophenoxy)propionyl, N-2-methyl-2-(*o-*phenylazophenoxy)propionyl, N-4-chlorobutyryl, N-3-methyl-3-nitrobutyryl, N-*o-*nitrocinnamoyl, N-acetylmethionine derivative, N-*o-*nitrobenzoyl, N-*o-*(benzoyloxymethyl)benzoyl, and 4,5-diphenyl-3-oxazolin-2-one.

### Cyclic Imide Derivatives

N-phthalimide, N-dithiasuccinoyl, N-2,3-diphenylmaleoyl, N-2,5-dimethylpyrrolyl, N-1,1,4,4-tetramethyldisilylazacyclopentane adduct, 5-substituted 1,3-dimethyl-1,3,5-triazacyclohexan-2-one, 5-substituted 1,3-dibenzyl-1,3,5-triazacyclohexan-2-one, and 1-substituted 3,5-dinitro-4-pyridonyl.

### SPECIAL - NH PROTECTIVE GROUPS

Examples of special NH protective groups include

### N-Alkyl and N-Aryl Amines

N-methyl, N-allyl, N-[2-(trimethylsilyl)ethoxy]methyl, N-3-acetoxypropyl, N-(1-isopropyl-4-nitro-2-oxo-3-pyrrolin-3-yl), quaternary ammonium salts, N-benzyl, N-di(4-methoxyphenyl)methyl, N-5-dibenzosuberyl, N-triphenylmethyl, N-(4-methoxyphenyl)diphenylmethyl, N-9-phenylfluorenyl, N-2,7-dichloro-9-fluorenylmethylene, N-ferrocenylmethyl, and N-2-picolylamine N'-oxide.

### Imine Derivatives

N-1,1-dimethylthiomethylene, N-benzylidene, N-*p-*methoxybenzylidene, N-diphenylmethylene, N-[(2-pyridyl)mesityl]methylene, and N-(N',N'-dimethylaminomethylene).

### PROTECTION FOR THE CARBONYL GROUP

### Acyclic Acetals and Ketals

Examples of acyclic acetals and ketals include dimethyl, bis(2,2,2-trichloroethyl), dibenzyl, bis(2-nitrobenzyl) and diacetyl.

### Cyclic Acetals and Ketals

Examples of cyclic acetals and ketals include 1,3-dioxanes, 5-methylene-1,3-dioxane, 5,5-dibromo-1,3-dioxane, 5-(2-pyridyl)-1,3-dioxane, 1,3-dioxolanes, 4-bromomethyl-1,3-dioxolane, 4-(3-butenyl)-1,3-dioxolane, 4-phenyl-1,3-dioxolane, 4-(2-nitrophenyl)-1,3-dioxolane, 4,5-dimethoxymethyl-1,3-dioxolane, *O,O'*-phenylenedioxy and 1,5-dihydro-3H-2,4-benzodioxepin.

### Acyclic Dithio Acetals and Ketals

Examples of cyclic dithio acetals and ketals include S,S'-dimethyl, S,S'-diethyl, S,S'-dipropyl, S,S'-dibutyl, S,S'-dipentyl, S,S'-diphenyl, S,S'-dibenzyl and S,S'-diacetyl.

### Cyclic Dithio Acetals and Ketals

Examples of cyclic dithio acetals and ketals include 1,3-dithiane, 1,3-dithiolane and 1,5-dihydro-3H-2,4-benzodithiepin.

### Acyclic Monothio Acetals and Ketals

Examples of cyclic monothio acetals and ketals include *O-*trimethylsilyl-S-alkyl, *O-*methyl-S-alkyl or -S-phenyl and *O-*methyl-S-2-(methylthio)ethyl.

### Cyclic Monothio Acetals and Ketals

Examples of cyclic monothio acetals and ketals include 1,3-oxathiolanes.

### MISCELLANEOUS DERIVATIVES

### O-Substituted Cyanohydrins

Examples of *O-*substituted cyanohydrins include *O-*acetyl, *O-*trimethylsilyl, *O-*1-ethoxyethyl and *O-*tetrahydropyranyl.

### Substituted Hydrazones

Examples of substituted hydrazones include N,N-dimethyl and 2,4-dinitrophenyl.

### Oxime Derivatives

Examples of oxime derivatives include *O-*methyl, *O-*benzyl and *O-*phenylthiomethyl.

### Imines

### Substituted Methylene Derivatives, Cyclic Derivatives

Examples of substituted methylene and cyclic derivatives include oxazolidines, 1-methyl-2-(1'-hydroxyalkyl)imidazoles, N,N'-dimethylimidazolidines, 2,3-dihydro-1,3-benzothiazoles, diethylamine adducts, and methylaluminum bis(2,6-di-*t-*butyl-4-methylphenoxide)(MAD)complex.

### PROTECTION FOR THE CARBOXYL GROUP

### Esters

### Substituted Methyl Esters

Examples of substituted methyl esters include 9-fluorenylmethyl, methoxymethyl, methylthiomethyl, tetrahydropyranyl, tetrahydrofuranyl, methoxyethoxymethyl, 2-(trimethylsilyl)ethoxymethyl, benzyloxymethyl, phenacyl, *p-*bromophenacyl, α-methylphenacyl, *p-*methoxyphenacyl, carboxamidomethyl, and N-phthalimidomethyl.

### 2-Substituted Ethyl Esters

Examples of 2-substituted ethyl esters include 2,2,2-trichloroethyl, 2-haloethyl, ω-chloroalkyl, 2-(trimethylsilyl)ethyl, 2-methylthioethyl, 1,3-dithianyl-2-methyl, 2-(*p-*nitrophenylsulfenyl)ethyl, 2-(*p-*toluenesulfonyl)ethyl, 2-(2'-pyridyl)ethyl, 2-(diphenylphosphino)ethyl, 1-methyl-1-phenylethyl, *t-*butyl, cyclopentyl, cyclohexyl, allyl, 3-buten-1-yl, 4-(trimethylsilyl)-2-buten-1-yl, cinnamyl, α-methylcinnamyl, phenyl, *p-*(methylmercapto)phenyl and benzyl.

### Substituted Benzyl Esters

Examples of substituted benzyl esters include triphenylmethyl, diphenylmethyl, bis(*o-*nitrophenyl)methyl, 9-anthrylmethyl, 2-(9,10-dioxo)anthrylmethyl, 5-dibenzosuberyl, 1-pyrenylmethyl, 2-(trifluoromethyl)-6-chromylmethyl, 2,4,6-trimethylbenzyl, *p-*bromobenzyl, *o-*nitrobenzyl, *p-*nitrobenzyl, *p-*methoxybenzyl, 2,6-dimethoxybenzyl, 4-(methylsulfinyl)benzyl, 4-sulfobenzyl, piperonyl, 4-picolyl and *p-*P-benzyl.

### Silyl Esters

Examples of silyl esters include trimethylsilyl, triethylsilyl, *t-*butyldimethylsilyl, *i*-propyldimethylsilyl, phenyldimethylsilyl and di-*t* butylmethylsilyl.

### Activated Esters

Examples of activated esters include thiols.

### Miscellaneous Derivatives

Examples of miscellaneous derivatives include oxazoles, 2-alkyl-1,3-oxazolines, 4-alkyl-5-oxo-1,3-oxazolidines, 5-alkyl-4-oxo-1,3-dioxolanes, ortho esters, phenyl group and pentaaminocobalt(III) complex.

### Stannyl Esters

Examples of stannyl esters include triethylstannyl and tri-n-butylstannyl.

### AMIDES AND HYDRAZIDES

### Amides

Examples of amides include N,N-dimethyl, pyrrolidinyl, piperidinyl, 5,6-dihydrophenanthridinyl, *o-*nitroanilides, N-7-nitroindolyl, N-8-Nitro-1,2,3,4-tetrahydroquinolyl, and *p-*P-benzenesulfonamides.

### Hydrazides

Examples of hydrazides include N-phenyl and N,N'-diisopropyl hydrazides.

### C. Synthesis

The compounds of the present invention may be prepared by conventional synthetic organic chemistry and by matrix or combinatorial methods according to Schemes 1 to 10 below, and Examples 1 to 31. Those of ordinary skill in the art will be able to modify and adapt the guidance provided herein to make the disclosed compounds.

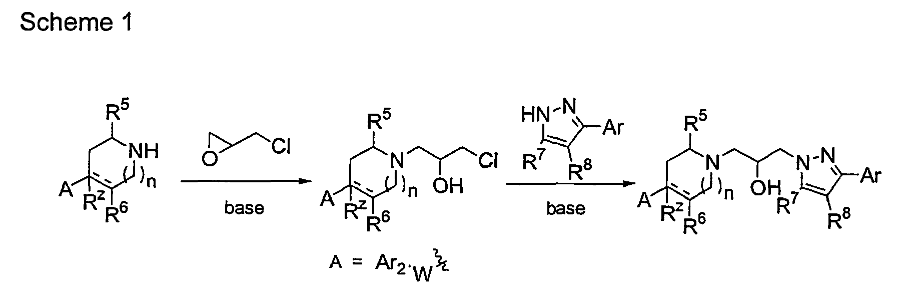

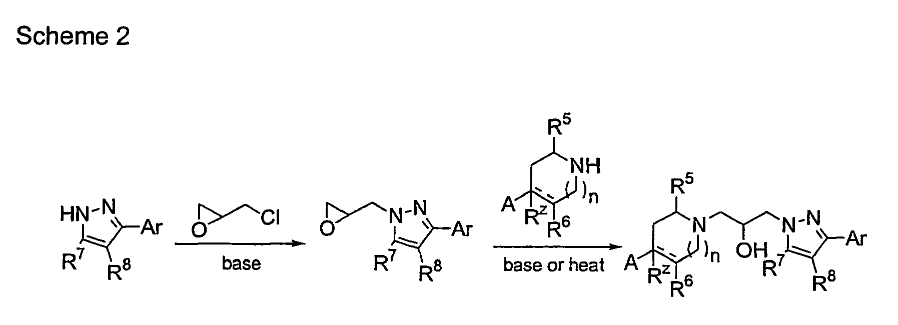

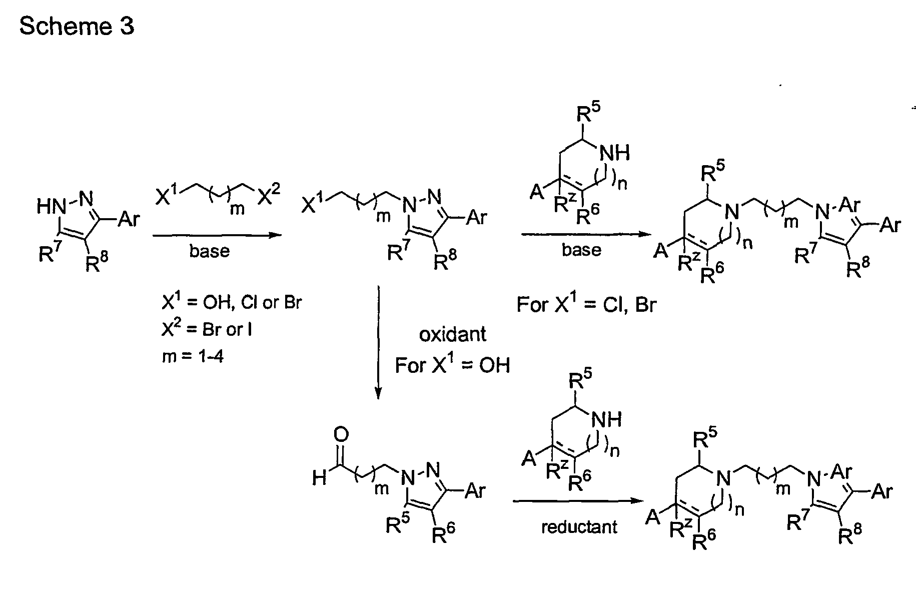

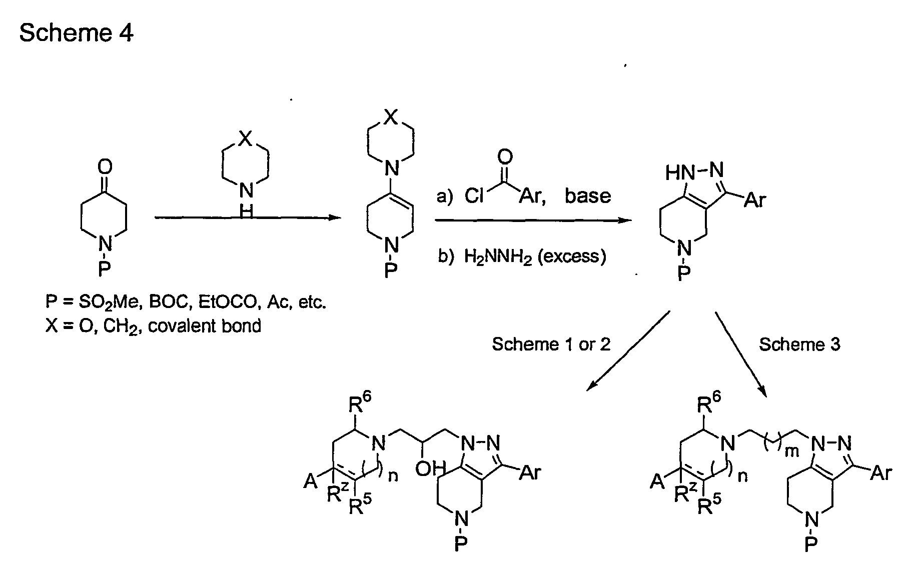

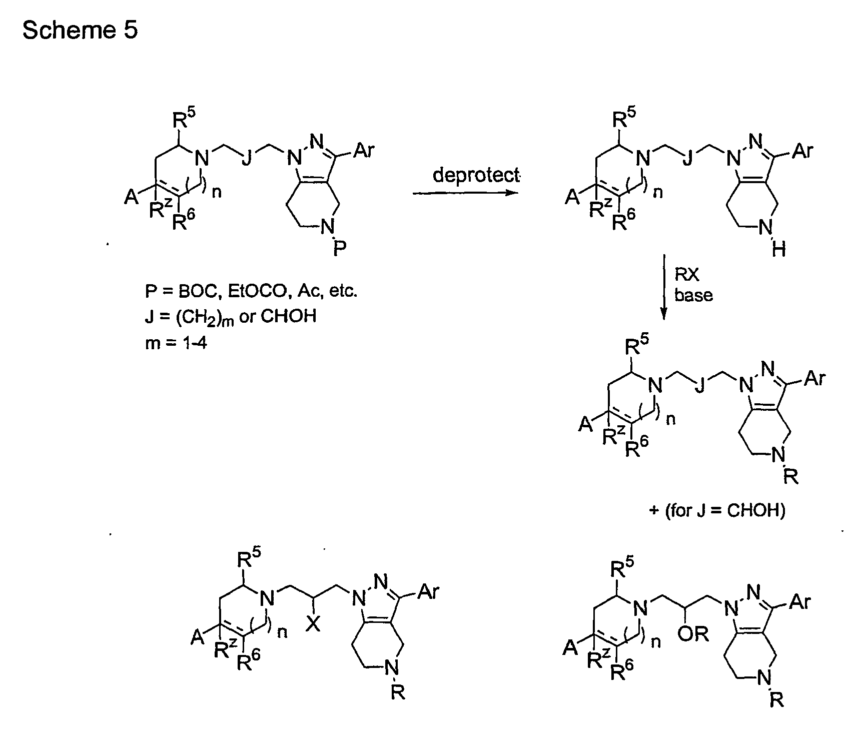

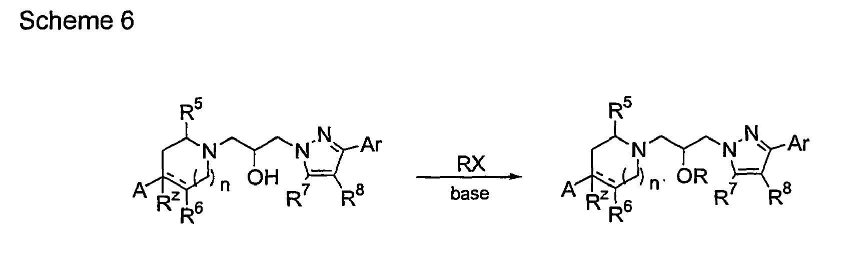

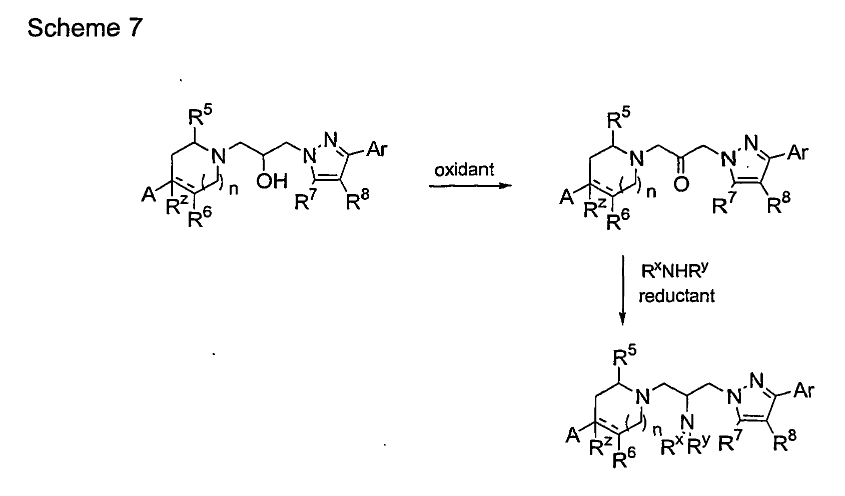

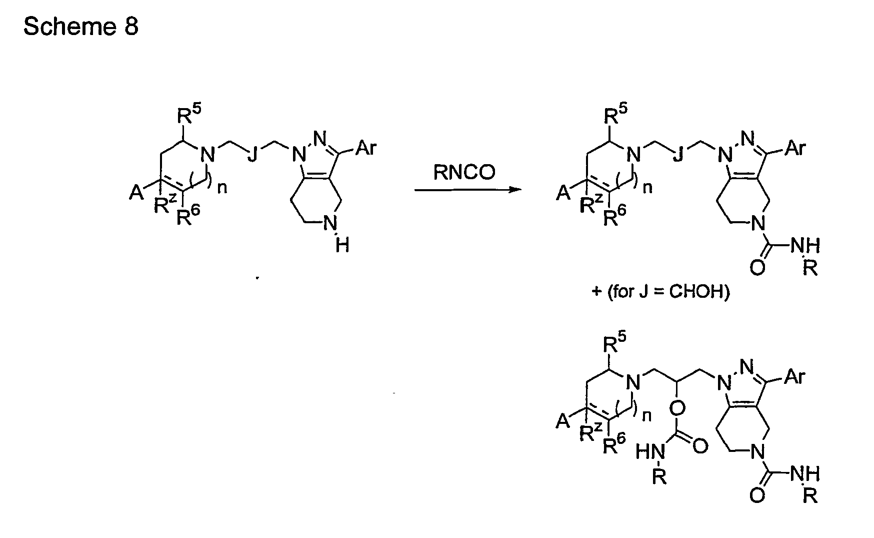

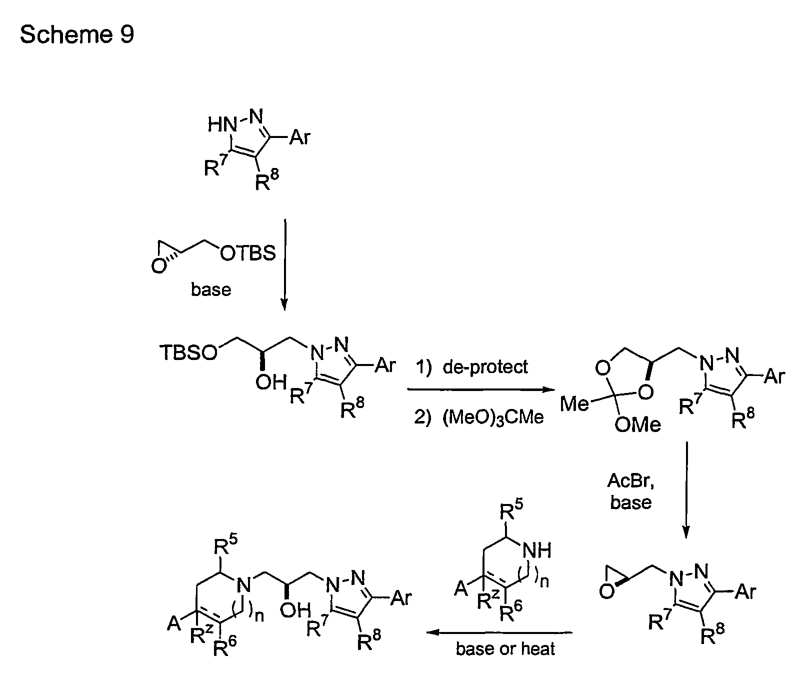

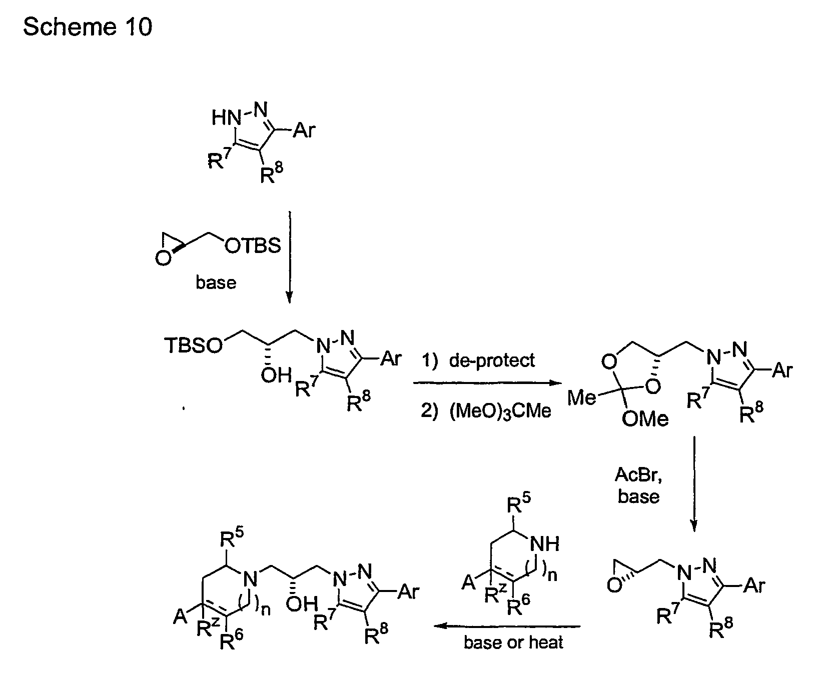

### D. Formulation and Administration

The present compounds inhibit the proteolytic activity of human cathepsin S and therefore are useful as a medicine especially in the manufacture of a pharmaceutical composition for treating patients suffering from allergic disorders or conditions which are modulated or regulated by the inhibition of cathepsin S activity.

The invention features the use of a compound in the manufacture of a pharmaceutical composition for treating a subject with an allergic condition mediated by cathepsin S, said compound being of formula I as shown in claim 1. The invention also provides the use of a compound in the manufacture of a pharmaceutical composition for inhibiting cathepsin S activity in a subject, wherein the compound is of formula I as shown in claim 1.

In view of their inhibitory effect on the proteolytic activity of human cathepsin S the compounds of the present invention may be formulated into various pharmaceutical forms for administration purposes. To prepare these pharmaceutical compositions, an effective amount of a particular compound, in base or acid addition salt form, as the active ingredient is intimately mixed with a pharmaceutically acceptable carrier.

A carrier may take a wide variety of forms depending on the form of preparation desired for administration. These pharmaceutical compositions are desirably in unitary dosage form suitable, preferably, for oral administration or parenteral injection. For example, in preparing the compositions in oral dosage form, any of the usual pharmaceutical media may be employed. These include water, glycols, oils, alcohols and the like in the case of oral liquid preparations such as suspensions, syrups, elixirs and solutions; or solid carriers such as starches, sugars, kaolin, lubricants, binders, disintegrating agents and the like in the case of powders, pills, capsules and tablets. In view of their ease in administration, tablets and capsules represent the most advantageous oral dosage unit form, in which case solid pharmaceutical carriers are generally employed. For parenteral compositions, the carrier will usually comprise sterile water, at least in large part, though other ingredients, for example, to aid solubility, may be included. Injectable solutions, for example, may be prepared in which the carrier comprises saline solution, glucose solution or a mixture of saline and glucose solution. Injectable suspensions may also be prepared in which case appropriate liquid carriers, suspending agents and the like may be employed. In the compositions suitable for percutaneous administration, the carrier optionally comprises a penetration enhancing agent and/or a suitable wetting agent, optionally combined with suitable additives of any nature in minor proportions, which additives do not cause a significant deleterious effect to the skin. Such additives may facilitate the administration to the skin and/or may be helpful for preparing the desired compositions. These compositions may be administered in various ways, e.g., as a transdermal patch, as a spot-on, as an ointment. Acid addition salts of the compounds of formula I, due to their increased water solubility over the corresponding base form, are more suitable in the preparation of aqueous compositions.

It is especially advantageous to formulate the aforementioned pharmaceutical compositions in dosage unit form for ease of administration and uniformity of dosage. Dosage unit form as used in the specification herein refers to physically discrete units suitable as unitary dosages, each unit containing a predetermined quantity of active ingredient calculated to produce the desired therapeutic effect in association with the required pharmaceutical carrier. Examples of such dosage unit forms are tablets (including scored or coated tablets), capsules, pills, powder packets, wafers, injectable solutions or suspensions, teaspoonfuls, tablespoonfuls and the like, and segregated multiples thereof.

Pharmaceutically acceptable acid addition salts include the therapeutically active non-toxic acid addition salt forms which the disclosed compounds are able to form. The latter can conveniently be obtained by treating the base form with an appropriate acid. Appropriate acids comprise, for example, inorganic acids such as hydrohalic acids, e.g. hydrochloric or hydrobromic acid; sulfuric; nitric; phosphoric and the like acids; or organic acids such as, for example, acetic, propanoic, hydroxyacetic, lactic, pyruvic, oxalic, malonic, succinic, maleic, fumaric, malic, tartaric, citric, methanesulfonic, ethanesulfonic, benzenesulfonic, *p-*toluenesulfonic, cyclamic, salicylic, *p-*aminosalicylic, palmoic and the like acids. The term addition salt also comprises the solvates which the disclosed componds, as well as the salts thereof, are able to form. Such solvates are for example hydrates, alcoholates and the like. Conversely the salt form can be converted by treatment with alkali into the free base form.

Stereoisomeric form defines all the possible isomeric forms which the compounds of formula (I) may possess. Unless otherwise mentioned or indicated, the chemical designation of compounds denotes the mixture of all possible stereochemically isomeric forms, said mixtures containing all diastereomers and enantiomers of the basic molecular structure. More in particular, stereogenic centers may have the (R)- or (S)-configuration; substituents on bivalent cyclic saturated radicals may have either the cis- or trans-configuration. The invention encompasses stereochemically isomeric forms including diastereoisomers, as well as mixtures thereof in any proportion of the disclosed compounds. The disclosed compounds may also exist in their tautomeric forms. Such forms although not explicitly indicated in the above and following formulae are intended to be included within the scope of the present invention.

Those of skill in the treatment of allergic disorders or conditions mediated by the cathepsin S enzyme could easily determine the effective daily amount from the test results presented hereinafter and other information. In general it is contemplated that a therapeutically effective dose would be from 0.001 mg/kg to 5 mg/kg body weight, more preferably from 0.01 mg/kg to 0.5 mg/kg body weight. It may be appropriate to administer the therapeutically effective dose as two, three, four or more sub-doses at appropriate intervals throughout the day. Said sub-doses may be formulated as unit dosage forms, for example, containing 0.05 mg to 250 mg, and in particular 0.5 to 50 mg of active ingredient per unit dosage form. Examples include 2 mg, 4 mg, 7 mg, 10 mg, 15 mg, 25 mg, and 35 mg dosage forms. Compounds of the invention may also be prepared in time-release or subcutaneous or transdermal patch formulations. Disclosed compound may also be formulated as a spray or other topical or inhalable formulations.

The exact dosage and frequency of administration depends on the particular compound of formula (I) used, the particular condition being treated, the severity of the condition being treated, the age, weight and general physical condition of the particular patient as well as other medication the patient may be taking, as is well known to those skilled in the art. Furthermore, it is evident that said effective daily amount may be lowered or increased depending on the response of the treated patient and/or depending on the evaluation of the physician prescribing the compounds of the instant invention. The effective daily amount ranges mentioned herein are therefore only guidelines.

The next section includes detailed information relating to the preparation, characterization, and use of the disclosed compounds.

### E. Examples

### EXAMPLE 1

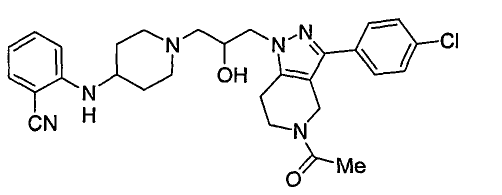

### 2-(1-{3-[5-Acetyl-3-(4-chloro-phenyl)-4,5,6,7-tetrahydro-pyrazolo[4,3-c]pyridin-1-yl]-2-hydroxy-propyl}-piperidin-4-ylamino)-benzonitrile.

### A.1-[3-(4-Chloro-phenyl)-1,4,6,7-tetrahydro-pyrazolo[4,3-c] pyridin-5-yl]-ethanone.

To a stirred solution of 50 g (0.35 mol) of *N-*acetyl-4-piperidone and 31 g (0.35 mol) of morpholine in benzene (350 mL) was added a catalytic amount (- 0.25 g) of *p-*toluenesulfonic acid. The mixture was heated to reflux for 10 h with a Dean-Stark trap. The solvent was removed under reduced pressure to give a brown oil. The crude product was diluted with CH₂Cl₂ (175 mL) and 50.0 mL (0.35 mol) of Et₃N was added. The mixture was cooled to 0°C and a solution of 45.0 mL (0.35 mol) of 4-chlorobenzoyl chloride in CH₂Cl₂ (50 mL) was added slowly by dropping funnel over 1 h. The mixture was allowed to warm to room temperature and stirred overnight. The reaction was then diluted with 1 *N* HCl (150 mL) and stirred vigorously for 3 h. The aqueous layer was extracted with CH₂Cl₂ (3 x 250 mL) and the combined extracts were dried over Na₂SO₄ and the solvent was removed under reduced pressure. The crude oil was diluted with EtOH (350 mL) and cooled to 0°C. To this stirred solution was slowly added 33.0 mL (1.06 mol) of hydrazine and the mixture was allowed to warm to room temperature and stir overnight during which time a white precipitate formed. The volume of the reaction was reduced to ∼150 mL and EtOAc (750 mL) was added to the mixture. The suspension was stirred vigorously for 2 h and was filtered then washed with EtOAc (2 x 200 mL) and dried under vacuum to afford 41.4 g (42% over 3 steps) of a pale yellow solid. TLC (silica, 5% MeOH/CH₂Cl₂): R*_{f}* = 0.3. MS (electrospray): m/z calculated for C₁₄H₁₄ClN₃O [M+H]⁺ 276.08, observed 276.0. ¹H NMR (400 MHz, CDCl₃, a mixture of amide rotamers): 7.65 (d, *J* = 8.4 Hz, 2H), 7.64 (d, *J* = 9.3 Hz, 2H), 7.58 (d, *J* = 10.5 Hz, 2H), 7.55 (d, *J* = 8.5 Hz, 2H), 4.94 (s, 2H), 4.78 (s, 2H), 4.08 (t, *J* = 5.9 Hz, 2H), 3.90 (t, *J* = 5.8 Hz, 2H), 3.02 (t, *J* = 5.8 Hz, 2H), 2.96 (t, *J* = 5.9 Hz, 2H), 2.36 (s, 3H), 2.31 (s, 3H).

### B.1-[3-(4-Chloro-phenyl)-1-oxiranylmethyl-1,4,6,7-tetrahydro-pyrazolo[4,3-c]pyridin-5-yl]-ethanone.

To a stirred solution of 1.00 g (3.63 mmol) of 1-[3-(4-chloro-phenyl)-1,4,6,7-tetrahydro-pyrazolo[4,3-c]pyridin-5-yl]-ethanone and 2.85 mL (36.3 mmol) of epichlorohydrin was added 1.30 g (3.99 mmol) of solid Cs₂CO₃. The reaction was stirred for 48 h and the solvent was removed under reduced pressure. The residue was then diluted with H₂O (50 mL) and EtOAc (50 mL). The layers were separated, and the organic layer was washed with H₂O (25 mL) and brine (25 mL), dried over Na₂SO₄ and the solvent was removed under reduced pressure. Purification by flash chromatography (silica, 0-15% acetone/CH₂Cl₂) afforded 0.72 g (60%) of a white solid. TLC (silica, 5% MeOH/CH₂Cl₂): R*_{f}* = 0.5. MS (electrospray): *m*/*z* calculated for C₁₇H₁₈ClN₃O₂ [M+H]⁺, 332.11, observed 332.0. ¹H NMR (400 MHz, CDCl₃, a mixture of amide rotamers): 7.60 (d, *J* = 8.6 Hz, 2H), 7.54 (d, *J* = 8.4 Hz, 2H), 7.40 (d, *J* = 8.6 Hz, 2H), 7.36 (d, *J* = 8.4 Hz, 2H), 4.80 and 4.73 (A and B of AB quartet, *J*_{ab} = 15.8 Hz, 2H), 4.60 (s, 2H), 4.47 (dd, *J* = 15.3, 2.5 Hz, 1H), 4.42 (dd, *J* = 15.0, 2.7 Hz, 1H), 4.11 (dd, *J* = 5.3, 2.5 Hz, 1H), 4.08 (dd, *J* = 5.1, 3.3 Hz, 1 H), 3.99-3.85 (m, 2H), 3.73 (dt, *J* = 5.9, 1.8 Hz, 2H), 3.37 (m, 2H), 2.87-2.80 (m, 3H), 2.80-2.69 (m, 3H), 2.53 (dd, *J* = 4.7, 2.5 Hz, 1H), 2.48 (dd, *J* = 4.6, 2.6, 1H), 2.19 (s, 3H), 2.15 (s, 3H).

### C. 1-{3-(4-Chloro-phenyl)-1-[3-(1,4-dioxa-8-aza-spiro[4.5]dec-8-yl)-2-hydroxypropyl]-1,4,6,7-tetrahydro-pyrazolo[4,3-c]pyridin-5-yl}-ethanone.

To a stirred solution of 3.20 g (9.64 mmol) of 1-[3-(4-chloro-phenyl)-1-oxiranylmethyl-1,4,6,7-tetrahydro-pyrazolo[4,3-c]pyridin-5-yl]-ethanone and 2.07 g (14.5 mmol) of 1,4-dioxa-8-azaspiro[4.5]decane in CH₂Cl₂ (65 mL) was added 1.79 g (2.89 mmol) of Yb(OTf)₃•H₂O. The reaction was stirred overnight and was then directly purified by flash chromatography (silica, 0-5% MeOH/CH₂Cl₂) to afford 3.70 g (81%) of the title compound. TLC (silica, 5% MeOH/CH₂Cl₂): R*_{f}* = 0.35. MS (electrospray), *m*/*z* calculated for C₂₄H₃₁ClN₄O₄ [M⁺+H], 475.20, observed 475.1.

### D. 1-{3-[5-Acetyl-3-(4-chloro-phenyl)-4,5,6,7-tetrahydro-pyrazolo[4,3-c]pyridin-1-yl]-2-hydroxy-propyl}-piperidin-4-one.

A suspension of 0.50 g (0.96 mmol) of 1-{3-(4-chloro-phenyl)-1-[3-(1,4-dioxa-8-aza-spiro[4.5]dec-8-yl)-2-hydroxy-propyl]-1,4,6,7-tetrahydro-pyrazolo[4,3-c]pyridin-5-yl}-ethanone in 1 *N* HCl (2.0 mL) was heated to 65°C for 48 h in a sealed vessel. The reaction was allowed to cool to room temperature and was diluted with CHCl₃ (20 mL) and saturated NaHCO₃ (20 mL). The aqueous phase was extracted with CHCl₃ (2 x 10 mL) and the combined organic extracts were dried over Na₂SO₄ and the solvent was removed under reduced pressure. The crude material was then diluted with Ac₂O (3.0 mL) and was stirred for 48 h. The solvent was removed under reduced pressure and the crude material was pumped down overnight. The resulting solid was dissolved in MeOH (5.0 mL) and a catalytic amount (0.05 g) of K₂CO₃ was added to the mixture and stirring was continued overnight. The reaction was then diluted with H₂O (20 mL) and CH₂Cl₂ (20 mL) and the layers were separated. The aqueous phase was extracted with CH₂Cl₂ (2 x 10 mL) and the combined organic extracts were dried over Na₂SO₄ and the solvent was removed under reduced pressure. Purification by flash chromatography (silica, 0-10% MeOH/CH₂Cl₂) afforded 0.29 g (65% over 3 steps) of a white solid. TLC (silica, 5% MeOH/CH₂Cl₂): R_{f} = 0.35. MS (electrospray); *m*/*z* calculated for C₂₂H₂₇ClN₄,O₃, [M+H]⁺, 431.18, observed 431.1. ¹H NMR (400 MHz, CDCl₃, a mixture of amide rotamers): 7.59 (d, *J* = 8.3 Hz, 1H), 7.53 (d, *J* = 8.6 Hz, 1 H), 7.41 (d, *J* = 8.5 Hz, 1H), 7.37 (d, *J* = 8.5 Hz, 1H), 4.85 and 4.73 (A and B of AB quartet, *J*_{ab} = 15.8 Hz, 1H), 4.62 (s, 1H), 4.26-4.12 (m, 2H), 4.09-3.68 (m, 4H), 3.49 (s, 1.5H), 3.28 (s, 1.5H).

### E. 2-(1-{3-[5-Acetyl-3-(4-chloro-phenyl)-4,5,6,7-tetrahydro-pyrazolo[4,3-c]pyridin-1-yl]-2-hydroxy-propyl}-piperidin-4-ylamino)-benzonitrile.

To a stirred solution of 50.0 mg (116.0 µmol) of 5-1-{3-[5-acetyl-3-(4-chlorophenyl)-4,5,6,7-tetrahydro-pyrazolo[4,3-c]pyridin-1-yl]-2-hydroxy-propyl}-piperidin-4-one and 9.6 mg (82.5 µmol) of 2-aminobenzonitrile in AcOH (0.5 mL) was added 130.0 mg (917.0 µmol) Na₂SO₄ and the reaction was allowed to stir for 1 h. To this mixture was added 58.0 mg (275.0 µmol) NaBH(OAc)₃ and the reaction was stirred for 48 h. The mixture was diluted with CH₂Cl₂ (20 mL) and saturated NaHCO₃ (20 mL). The aqueous phase was extracted with CH₂Cl₂ (2 x 10 mL) and the combined organic extracts were dried over Na₂SO₄ and the solvent was removed under reduced pressure. Purification by flash chromatography (silica, 0-5% MeOH/CH₂Cl₂) afforded 9.0 mg (20%) of a white solid. TLC (silica, 5% MeOH/CH₂Cl₂): R*_{f}* = 0.2. MS (electrospray): *m*/*z* calculated for C₂₉H₃₃ClN₆O₂, [M+H]⁺, 533.24, observed 533.3. ¹H NMR (400 MHz, CDCl₃, a mixture of amide rotamers): 7.58 (d, *J* = 8.6 Hz, 1H), 7.52 (d, *J* = 8.4 Hz, 1 H), 7.43-7.34 (m, 4H), 6.69 (dt, *J* = 7.6, 4.0 Hz, 1H), 6.64 (d, *J* = 8.6 Hz, 1H), 4.83 and 4.73 (A and B of AB quartet, *J*_{ab} = 15.7 Hz, 1H), 4.61 (s, 1H), 4.44 (d, *J* = 7.3 Hz, 1H), 4.33-4.14 (m, 2H), 4.11-3.84 (m, 2H), 3.83-3.67 (m, 1H), 3.55-3.43 (m, 1H), 3.17-2.94 (m, 1H), 2.93-2.75 (m, 2H), 2.74-2.54 (m, 2H), 2.21 (s, 1.5H), 2.16 (s, 1.5H), 2.23-1.53 (m, 9H).

### EXAMPLE 2

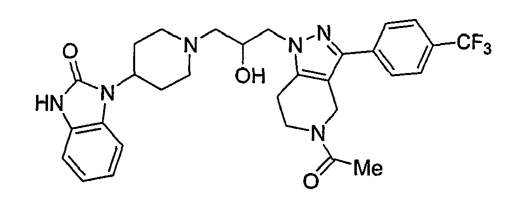

### 1-(1-{3-[5-Acetyl-3-(4-trifluoromethyl-phenyl)-4,5,6,7-tetrahydro-pyrazolo[4,3-c]pyridin-1-yl]-2-hydroxy-propyl}-piperidin-4-yl)-1,3-dihydro-benzoimidazol-2-one.

### A. 1-[3-(4-Trifluoromethyl-phenyl)-1,4,6,7-tetrahydro-pyrazolo[4,3-c]pyridin-5-yl]-ethanone.

A solution of *N*-acetyl-4-piperidone (2.82 g, 20 mmol), morpholine (1.93 mL, 22 mmol) and *p-*toluenesulfonic acid (5 mg) in benzene (8.5 mL) was refluxed for 8 h in a Dean-Stark apparatus. The solvent was removed and the residue dissolved in CH₂Cl₂ (20 mL). Triethylamine (3.1 mL) was added and *p-*trifluoromethylbenzoyl chloride (3.27 mL, 22 mmol) in CH₂Cl₂ (4 mL) was added dropwise into the solution at 0 °C. The reaction mixture was stirred at 25 °C for 24 h and diluted with aqueous HCl (5%, 25 mL). After stirring for another 30 min, the organic layer was separated, washed with H₂O (20 mL), dried (Na₂SO₄), and concentrated. The residue was dissolved in EtOH (95%, 18 mL) and treated at 0 °C with hydrazine (2.9 mL, 60 mmol). The mixture was stirred at 25°C for 3 h and H₂O (4 mL) was added. Most of the volatiles were removed and the residue extracted with CH₂Cl₂ (50 mL). The organic layer was separated, washed with H₂O (20 mL), dried over Na₂SO₄, and concentrated. Column chromatography (silica, 5% MeOH/CH₂Cl₂) provided 5.1 g (83%) of a white powder. TLC (silica, 10% MeOH/CH₂Cl₂): R*_{f}* = 0.30. MS (electrospray): *m*/*z* 332.0 ([M+Na]⁺, C₁₅H₁₄F₃N₃O requires 309.1). ¹H NMR (CDCl₃, 400 MHz, a mixture of two rotamers): 7.73-7.67 (m, 4H), 4.85 (s, 1.2H), 4.68 (s, 0.8H), 3.96 (t, *J* = 4.5 Hz, 0.8H), 3.78 (t, *J* = 4.5 Hz, 1.2H), 2.89 (t, *J* = 4.5 Hz, 1.2H), 2.83 (t, *J* = 4.5 Hz, 0.8H), 2.23 (s, 1.8H), 2.18 (s, 1.2H).

### B.1-[1-Oxiranylmethyl-3-(4-trifluoromethyl-phenyl)-1,4,6,7-tetrahydropyrazolo[4,3-c]pyridin-5-yl]-ethanone.

A solution of 1-[3-(4-trifluoromethyl-phenyl)-1,4,6,7-tetrahydro-pyrazolo[4,3-c]pyridin-5-yl]-ethanone (2.4 g, 7.77 mmol) in DMF (15 mL) was treated with cesium carbonate (5.05 g, 15.5 mmol) and epichlorohydrin (6.1 mL, 77.7 mmol) at 25 °C and stirred for 24 h before it was diluted with EtOAc (100 mL) and H₂O (50 mL). The organic layer was separated, washed with H₂O (2 x 50 mL), brine (50 mL), dried over Na₂SO₄, and concentrated. Column chromatography (silica, 10% acetone/CH₂Cl₂) provided 2.30 g (81%) of a white powder. TLC (silica, 10% MeOH/CH₂Cl₂): R*_{f}* = 0.35. MS (electrospray): *m*/*z* 388.0 ([M+Na]⁺, C₁₈H₁₈F₃N₃O₂ requires 365.1). ¹H NMR (CDCl₃, 400 MHz, a mixture of two rotamers): 7.77 and 7.63 (AB pattern, *J*_{ab} = 8.2 Hz, 2H), 7.71 and 7.67 (AB pattern, *J*_{ab} = 8.4 Hz, 2H), 4.82 and 4.76 (AB pattern, *J*_{ab} = 15.5 Hz, 1.2H), 4.58 (s, 0.8H), 4.45-4.35 (m, 1H), 4.08-4.02 (m, 1H), 3.92-3.80 (m, 1H), 3.70-3.63 (m, 1H), 3.30 (m, 1H), 2.80-2.67 (m, 3H), 2.48-2.42 (m, 1H), 2.13 (s, 1.3H), 2.08 (s, 1.7H).

### C. 1-(1-{3-[5-Acetyl-3-(4-trifluoromethyl-phenyl)-4,5,6,7-tetrahydropyrazolo[4,3-c]pyridin-1-yl]-2-hydroxy-propyl}-piperidin-4-yl)-1,3-dihydro-benzoimidazol-2-one.

A solution of 1-[1-oxiranylmethyl-3-(4-trifluoromethyl-phenyl)-1,4,6,7-tetrahydropyrazolo[4,3-c]pyridin-5-yl]-ethanone (1.17 g, 3.2 mmol) in DMF (10 mL) was treated with ytterbium(III) triflate (0.4 g, 0.64 mmol) and 4-(2-keto-1-benzimidazolinyl)piperidine (1.04 g, 4.8 mmol) at 25°C and stirred for 48 h before it was diluted with CH₂Cl₂ (100 mL) and H₂O (50 mL). The organic layer was separated, washed with H₂O (2 x 50 mL), dried over Na₂SO₄, and concentrated. Flash column chromatography (silica, 5% MeOH/CH₂Cl₂) afforded 1.71 g (92%) of a white powder. TLC (silica, 10% MeOH/CH₂Cl₂): R*_{f}* = 0.25. MS (electrospray): *m*/*z* 583.5 ([M+H]⁺, C₃₀H₃₃F₃N₆O₃ requires 582.3). ¹H NMR (CDCl₃, 400 MHz, a mixture of two rotamers): 9.30 (br s, 0.5H), 9.25 (br s, 0.5H), 7.82 and 7.68 (AB pattern, *J*_{ab} = 8.2 Hz, 2H), 7.76 and 7.72 (AB pattern, *J*_{ab} = 8.4 Hz, 2H), 7.25-7.05 (m, 4H), 4.92 and 4.80 (AB pattern, *J*_{ab} = 15.6 Hz, 1.1H), 4.70 (s, 0.9H), 4.40-3.70 (m, 7H), 3.20-2.82 (m, 4H), 2.60-2.45 (m, 4H), 2.35-2.25 (m, 1H), 2.25 (s, 1.5H), 2.20 (s, 1.5H), 1.90-1.87 (m, 2H).

### EXAMPLE 3

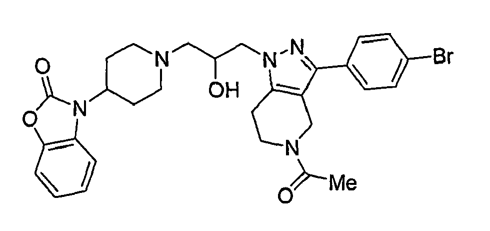

### 3-(1-{3-[5-Acetyl-3-(4-bromo-phenyl)-4,5,6,7-tetrahydro-pyrazolo[4,3-c]pyridin-1-yl]-2-hydroxy-propyl}-piperidin-4-yl)-3H-benzooxazol-2-one.

### A. 1-[3-(4-Bromo-phenyl)-1,4,6,7-tetrahydro-pyrazolo[4,3-c]pyridin-5-yl]-ethanone.

A flask equipped with a Dean-Stark trap, was charged with *N-*acetyl-4-piperidone (100.1g, 709 mmol), piperidine (68 mL, 779 mmol), *p*TsOH (3.7 g) and benzene (500 mL). The mixture was heated to 125 °C. After 17 h the mixture was allowed to cool and divided into two portions. A solution of *p-*bromobenzoyl chloride (70.0 g, 319 mmol) in CH₂Cl₂ (400 mL) was added dropwise to a 0 °C solution of the enamine (ca. 355 mmol) in CH₂Cl₂ (320 mL) over 15 h. The mixture was then allowed to warm to 23 °C and stirred for an additional 5 h. The solution was treated with 1 *N* HCl (500 mL) and stirred vigorously for 1.5 h. The layers were separated and the aqueous layer was extracted with CH₂Cl₂ (2 x 300 mL). The combined extracts were washed with sat. aqueous NaHCO₃ (300 mL), H₂O (300 mL), brine (300 mL), dried over Na₂SO₄ and concentrated. The residue was dissolved in MeOH (300 mL) and treated with NH₂NH₂ (50.0 mL, 1.59 mol). The mixture was stirred for 17 h before the precipitate formed was collected by filtration and air dried to give 52 g (50%) of the title compound which was suitable for use without further purification. TLC (silica, 5% MeOH/CH₂Cl₂): R*_{f}* = 0.3. MS (electrospray): *m*/*z* calculated for C₁₄H₁₅⁷⁹BrN₃O [M+H]⁺, 320.04, found 320. ¹H NMR (CD₃OD/CDCl₃, 400 MHz, a mixture of amide rotamers): 7.53 and 7.35 (A and B of AA'BB', *J* = 8.5 Hz, 2H), 7.51 and 7.39 (A and B of AA'BB', *J* = 8.6 Hz, 2H), 4.72 (s, 2H), 4.58 (s, 2H), 3.85 (t, *J* = 5.9 Hz, 2H), 3.71 (t, *J* = 5.8 Hz, 2H), 2.81, (t, *J* = 5.8 Hz, 2H), 2.74, (t, *J* = 5.8 Hz, 2H), 2.16 (s, 3H), 2.11 (s, 3H).

### B.1-[3-(4-Bromo-phenyl)-1-oxiranylmethyl-1,4,6,7-tetrahydro-pyrazolo[4,3-c]pyridin-5-yl]-ethanone.

Cs₂CO₃ (11.58 g, 35.5 mmol) was added to a solution of 1-[3-(4-bromo-phenyl)-1,4,6,7-tetrahydro-pyrazolo[4,3-c]pyridin-5-yl]-ethanone (7.59 g, 23.7 mmol) and epichlorohydrin (20 mL, 234 mmol) in DMF (100 mL). The mixture was stirred for 18 h then diluted with EtOAc (800 mL) and washed with saturated aqueous NaHCO₃ (2 x 100 mL), H₂O (2 x 100 mL), and brine (100 mL). The NaHCO₃ layer was extracted with EtOAc (2 x 150 mL). The combined washes were extracted with EtOAc (2 x 100 mL). The combined extracts were dried over Na₂SO₄ and concentrated. Column chromatography (silica, 10-20% acetone/CH₂Cl₂) afforded 4.98 g (56%) of the title compound. HPLC , *t*_{R} = 4.90 min. (Reverse phase conditions: HP 1100 LCMS, Phenomenex luna 2.1 x 150 mm column, 60% MeOH/ H₂O (0.5% AcOH) to 90%MeOH/ H₂O (0.5% AcOH), held at initial conditions for 2 min then ramped to final conditions over 5 min.) MS (electrospray): *m*/*z* calculated for C₁₇H₁₉⁷⁹BrN₃O₂, [M+H]⁺, 376.07, found 376.0. ¹H NMR (CDCl₃, 400 MHz, a mixture of amide rotamers): 7.47 (d with fine splittings, *J* = 8.5, Hz, 2H), 7.44 (m, 4H), 7.38 (d with fine splittings, *J* = 8.5, Hz, 2H), 4.71 and 4.64 (A and B of AB quartet, *J*_{ab} = 15.7 Hz, 2H), 4.51 (s, 2H), 4.39 (dd, *J* = 15.1, 2.5 Hz, 1H), 4.34 (dd, *J* = 15.0, 2.9 Hz, 1H), 4.02 (dd, *J* = 5.2, 3.9 Hz, 1H), 3.98 (dd, *J* = 5.3, 3.7 Hz, 1H), 3.83 (m, 2H), 3.64 (m, 2H), 3.25 (br m, 2H), 2.80-2.60 (m, 6H), 2.46 (dd, *J* = 4.6, 2.6 Hz, 1H), 2.38 (dd, *J* = 4.6, 2.6 Hz, 1H), 2.10 (s, 3H), 2.06 (s, 3H).

### C. 4-(2-Oxo-benzooxazol-3-yl)-piperidine-1-carboxylic acid tert-butyl ester.

To a stirred solution of 1.00 g (5.01 mmol) of *tert*-butyl 4-oxo-1-piperidinecarboxylate and 0.55 g (5.01 mmol) of 2-aminophenol in CH₂Cl₂ (15 mL) under nitrogen at rt was added 1.62 g (7.52 mmol) of NaBH(OAc)₃ in one portion and the mixture was stirred for 14 h. The mixture was diluted with CH₂Cl₂ (50 mL) and saturated NaHCO₃ (75 mL) and the layers were separated. The aqueous layer was extracted with CH₂Cl₂ (2 x 25 mL) and the combined organic layers were washed with brine, dried over Na₂SO₄, and the solvent was removed under reduced pressure. The crude solid was diluted with CH₂Cl₂ (15 mL) and 0.89 g (5.51 mmol) of carbonyldiimidazole was added in one portion and mixture was stirred for 16 h. The mixture was diluted with CH₂Cl₂ (50 mL) and 1 *N* HCl (50 mL) and the layers were separated. The aqueous layer was extracted with CH₂Cl₂ (2 x 25 mL) and the combined organic layers were washed with brine, dried over Na₂SO₄, and the solvent was removed under reduced pressure. Flash chromatography (silica, 0-5% acetone/CH₂Cl₂) afforded 1.59 g (99%) of a white solid. TLC (silica, 5% acetone/CH₂Cl₂): R*_{f}* = 0.6. MS (electrospray): *m*/*z* calculated for C₁₇H₂₂N₂O₄, [M+Na]⁺, 341.1, observed 341.1.

### D. 3-Piperidin-4-yl-3H-benzooxazol-2-one.

To a stirred solution of 1.00 g (2.87 mmol) of 4-(2-oxo-benzooxazol-3-yl)-piperidine-1-carboxylic acid tert-butyl ester in CH₂Cl₂ (6.0 mL) was added TFA (6.0 mL) and the mixture was stirred for 12 h. The solvents were removed under reduced pressure and the crude solid was diluted in MeOH (10 mL) and saturated NaHCO₃ (15 mL) was added to the mixture and stirring was continued for 10 min. The solution was diluted with CH₂Cl₂ (30 mL) and the layers were separated. The aqueous phase was extracted with CH₂Cl₂ (2 x 20 mL) and the organic layers were combined, dried over Na₂SO₄ and the solvent was removed under reduced pressure to afford 1.02 g (88%) of a pale yellow solid. TLC (silica, 10% MeOH/CH₂Cl₂): R*_{f}* = 0.1. MS (electrospray): *m*/*z* calculated for C₁₂H₁₄N₂O₂, [M+H]⁺, 219.11, observed 219.1.

### E. 3-(1-{3-[5-Acetyl-3-(4-bromo-phenyl)-4,5,6,7-tetrahydro-pyrazolo[4,3-c]pyridin-1-yl]-2-hydroxy-propyl}-piperidin-4-yl)-3H-benzooxazol-2-one.

To a stirred mixture of 0.025 g (0.066 mmol) of 3-piperidin-4-yl-3H-benzooxazol-2-one and 0.015 g (0.066 mmol) of 1-[3-(4-bromo-phenyl)-1-oxiranylmethyl-1,4,6,7-tetrahydro-pyrazolo[4,3-c]pyridin-5-yl]-ethanone in EtOH (0.5 mL) was added 0.01 mL (0.066 mmol) of Et₃N. The mixture was heated to 80 °C in a sealed vessel for 16 h. The reaction was cooled and the solvent was removed under reduced pressure. Flash chromatography (silica, 0-5% MeOH/CH₂Cl₂) afforded 0.030 g (79%) of a white foam. TLC (silica, 5% MeOH/CH₂Cl₂): R*_{f}* = 0.4. MS (electrospray): *m*/*z* calculated for C₂₉H₃₂BrN₅O₄, [M+H]⁺, 594.16, observed 594.2. ¹H NMR (400 MHz, CDCl₃, a mixture of amide rotamers): 7.60-7.43 (m, 4H), 7.23-7.06 (m, 4H), 4.83 and 4.73 (A and B of AB quartet, *J*_{ab} = 15.4 Hz, 1H), 4.61 (s, 1H), 4.38-3.66 (m, 7H), 3.37-3.02 (m, 2H), 2.99-2.28 (m, 6H), 2.21 (s, 1.5H), 2.16 (s, 1.5H), 1.99-1.83 (m, 3H).

### EXAMPLE 4

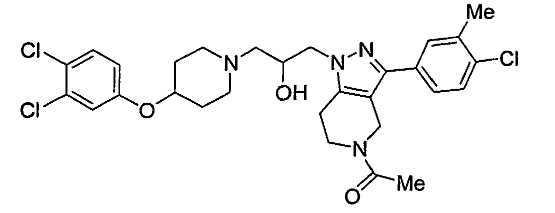

### 1-(3-(4-Chloro-3-methyl-phenyl)-1-{3-[4-(3,4-dichloro-phenoxy)-piperidin-1-yl]-propyl}-1,4,6,7-tetrahydro-pyrazolo[4,3-c]pyridin-5-yl)-ethanone.

### A. 1-[3-(4-Chloro-3-methyl-phenyl)-1,4,6,7-tetrahydro-pyrazolo[4.3-c]pyridin-5-yl]-ethanone.

To a stirred solution of 1-acetyl-4-piperidone (3 g, 0.021 mol) and morpholine (1.86 g, 0.21 mol) in benzene (21 mL) was added a catalytic amount (~0.015 g) of *p*-toluenesulfonic acid. The mixture was heated to reflux for 10 h under a Dean-Stark trap. The solvent was removed under reduced pressure to give a brown oil. The crude product was diluted with CH₂Cl₂ (10.5 mL), and Et₃N (3.0 mL, 0.021 mol) was added. The mixture was cooled to 0 °C, and a solution of 3-methyl-4-chlorobenzoyl chloride (2.7 mL, 0.021 mol) in CH₂Cl₂ (3.0 mL) was added slowly by dropping funnel over 1 h. The mixture was allowed to warm to room temperature and stir overnight. The reaction mixture was then diluted with 1 *N* HCl (9.0 mL) and stirred vigorously for 3 h. The aqueous layer was extracted with CH₂Cl₂ (3 x 15 mL). The combined extracts were dried over Na₂SO₄, and the solvent was removed under reduced pressure. The crude oil was diluted with EtOH (21 mL) and cooled to 0 °C. To this stirred solution was slowly added hydrazine (2.0 mL, 0.064 mol), and the mixture was allowed to warm to room temperature and stir overnight, during which time a white precipitate formed. The volume of the reaction mixture was reduced to ~9 mL, and EtOAc (45 mL) was added. The suspension was stirred vigorously for 2 h and was filtered then washed with EtOAc (2 x 12 mL) and dried under vacuum to afford 4.93 g (81% over 3 steps) of a pale yellow solid. TLC (silica, 10% acetone/CH₂Cl₂): R*_{f}* = 0.2. MS (electrospray): exact mass calculated for C₁₅H₁₆ClN₃O, 289.10; *m*/*z* found, 290.1 [M⁺+H].

### B. 1-[3-(4-Chloro-3-methyl-phenyl)-1-oxiranylmethyl-1,4,6,7-tetrahydropyrazolo[4,3-c]pyridin-5-yl]-ethanone.

Cs₂CO₃ (11 g, 33.8 mmol) was added to a solution of 1-[3-(4-chloro-3-methylphenyl)-1,4,6,7-tetrahydro-pyrazolo[4,3-c]pyridin-5-yl]-ethanone (4.9 g, 16.9 mmol) in DMF (49 mL), which was then stirred for 15 min. Epichlorohydrin (13.2 mL, 169 mmol) was added, and the mixture was stirred under N₂ at room temperature for 16 h. EtOAc (250 mL) was added to the reaction mixture, which was then stirred for 5 min. The resulting solution was washed with water (2 x 50 mL) and brine (1 x 50 mL). The organic extracts were dried over Na₂SO₄ and concentrated. The residue was purified by column chromatography (silica, 10-20% acetone/CH₂Cl₂) to obtain 3.8 g (65%) of a white solid. TLC (silica, 10% acetone/CH₂Cl₂): R*_{f}* = 0.3. MS (electrospray): exact mass calculated for C₁₈H₂₀ClN₃O₂, 345.12; *m*/*z* found, 346.1 [M⁺+H], 368.0 [M⁺+Na],

### C. 4-(3,4-Dichlorophenoxy)-piperidinium trifluoroacetate.

A suspension of 0.69 g (20.0 mmol) of triphenylphosphine (polymer supported, 3 mmol P/g) in CH₂Cl₂ (4.0 mL) was stirred for 15 min to swell the resin. To this suspension was added 0.20 g (1.00 mmol) of 1-*tert*-butoxycarbonyl-4-piperidinol, 0.16 g (1.00 mmol) of 3,4-dichlorophenol; and 0.35 g (1.50 mmol) of di-*tert*-butyl azodicarboxylate. The reaction was stirred for 4 h and was filtered and the resin was washed with 5% MeOH/CH₂Cl₂ (2 x 20 mL) and Et₂O (20 mL). The organic layers were combined and the solvent was removed. The crude oil was diluted with CH₂Cl₂ (2.0 mL) and TFA (2.0 mL) and the mixture was stirred overnight. The solvent was removed under reduced pressure to afford the crude TFA salt which was used without further purification. TLC (silica, 10% MeOH/CH₂Cl₂): R*_{f}* = 0.1. MS (electrospray): *m*/*z* calculated for C₁₁H₁₃Cl₂NO, [M+H]⁺, 246.04, observed 246.1.

### D. 1-(3-(4-Chloro-3-methyl-phenyl)-1-{3-[4-(3,4-dichloro-phenoxy)-piperidin-1-yl]-propyl}-1,4,6,7-tetrahydro-pyrazolo[4,3-c]pyridin-5-yl)-ethanone.

To a stirred solution of 25.0 mg (0.066 mmol) of 1-[3-(4-chloro-3-methylphenyl)-1-oxiranylmethyl-1,4,6,7-tetrahydro-pyrazolo[4,3-c]pyridin-5-yl]-ethanone and 25.0 mg (0.10 mmol) of 4-(3,4-dichlorophenoxy)-piperidinium trifluoroacetate in EtOH (0.5 mL) was added 0.019 mL (0.014 mmol) of Et₃N. The mixture was heated to 80 °C in a sealed vessel for 16 h. The reaction was cooled and the solvent was removed under reduced pressure. Flash chromatography (0-5% MeOH/CH₂Cl₂) afforded 28 mg (74%) of a pale yellow foam. TLC (silica, 5% MeOH/CH₂Cl₂): R*_{f}* = 0.5. MS (electrospray): *mlz* calculated for C₂₉H₃₃Cl₃N₄O₃, [M+H]⁺, 591.16, observed 591.2. ¹H NMR (400 MHz, CDCl₃, a mixture of amide rotamers): 7.51 (d, *J* = 6.9 Hz, 1H), 7.41-7.29 (m, 3H), 6.99 (d, *J* = 2.9 Hz, 1H), 6.74 (dd, *J* = 9.0, 3.1 Hz, 1H), 4.82 and 4.73 (A and B of AB quartet, *J*_{ab} = 15.7 Hz, 1H), 4.60 (s, 1H), 4.46-3.93 (m, 4H), 3.92-3.83 (m, 1H), 3.82-3.68 (m, 1H), 3.08-2.51 (m, 6H), 2.43 (s, 1.5H), 2.41 (s, 1.5H), 2.21 (s, 1.5H), 2.15 (s, 1.5H) 2.00-1.83 (m, 3H), 1.75-1.39 (m, 4H).

### EXAMPLE 5

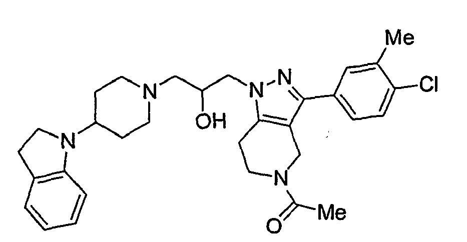

### 1-(3-(4-Chloro-3-methyl-phenyl)-1-{3-[4-(2,3-dihydro-indol-1-yl)-piperidin-1-yl]-2-hydroxy-propyl}-1,4,6,7-tetrahydro-pyrazolo[4,3-c]pyridin-5-yl)ethanone.

### A. 1-Piperidin-4-yl-2,3-dihydro-1H-indole.

Indoline (11.0 g, 92 mmol) and N-BOC-4-piperidone (18.4 g, 92 mmol) were set stirring in 300 mL of CH₂Cl₂ under an atmosphere of nitrogen at rt. Acetic acid (5.5 mL, 96 mmol) was then added. After 1.5 h sodium triacetoxyborohydride (27.4 g, 129 mmol) was added and the mixture was left stirring for 4 days. The mixture was quenched by the slow addition of saturated NaHCO₃. The organics were separated, dried (MgSO₄) and the solvent was evaporated under reduced pressure to give 28 g (100%) of a clear dark green liquid. The crude material was brought up in 1:1 TFA/CH₂Cl₂ (100 mL) and stirred at room temperature. After 45 min the solvent was evaporated under reduced pressure, the oil brought up in EtOAc, and cooled on ice to form a beige precipitate. The solid was filtered, washed with Et₂O and air dried to give 22.5 g (57%) of a white solid as a TFA salt. MS (electrospray): exact mass calculated for C₁₃H₁₈N₂, 202.15; *m*/*z* found, 203.2. ¹H NMR (400MHz, DMSO-*d*₆): 8.74 (br s, 1H), 8.46 (br s, 1H), 7.07 (m, 2H), 6.63 (m, 2H), 3.81 (br s, 1H), 3.46 (m, 2H), 3.37 (m, 2H), 3.12 (m, 2H), 2.95 (m, 2H), 1.86 (m, 4H).

### B. 1-(3-(4-Chloro-3-methyl-phenyl)-1-{3-[4-(2,3-dihydro-indol-1-yl)-piperidin-1-yl]-2-hydroxy-propyl}-1,4,6,7-tetrahydro-pyrazolo[4,3-c]pyridin-5-yl)-ethanone.

1-Piperidin-4-yl-2,3-dihydro-1H-indole (TFA salt) (506 mg, 1.18 mmol) and 1-[3-(4-chloro-3-methyl-phenyl)-1-oxiranylmethyl-1,4,6,7-tetrahydro-pyrazolo[4,3-c]pyridin-5-yl]-ethanone (261 mg, 0.75 mmol) were set stirring in 20 mL of EtOH and heated to 80 °C. After 20 h the mixture was cooled, evaporated, brought up in EtOAc and washed with saturated NaHCO₃. The organics were dried (MgSO₄) and evaporated to give a clear golden oil. Flash chromatography (silica, 100% acetone) gave 260 mg (63%) of a white solid. TLC (silica, 100% acetone): R*_{f}* = 0.10. MS (electrospray): exact mass calculated for C₃₁H₂₈CIN₅O₂, 547.27; *m*/*z* found, 548.3. ¹H NMR 400MHz, CDCl₃): 7.64 (m, 1H), 7.43 (m, 2H), 7.16 (m, 2H), 6.72 (s, 1H), 6.50 (m, 1H), 4.88 (m, 1H), 4.73 (s, 1H), 4.28 (m, 2H), 4.13 (m, 2H), 3.92 (m, 2H), 3.47 (m, 3H), 30.9 (m, 6H), 2.55 (m, 6H), 2.27 (m, 3H), 1.84 (m, 4H).

### EXAMPLE 6

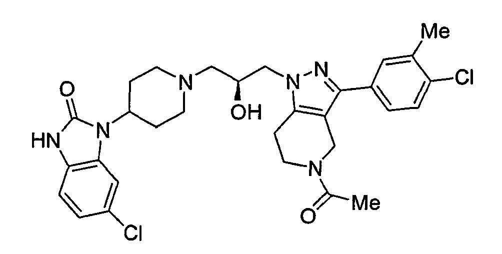

### (S)-1-(1-{3-[5-Acetyl-3-(4-chloro-3-methyl-phenyl)-4,5,6,7-tetrahydropyrazolo[4,3-c]pyridin-1-yl]-2-hydroxy-propyl}-piperidin-4-yl)-6-chloro-1,3-dihydro-benzoimidazol-2-one.

### A. (R)-1-[3-(4-Chloro-3-methyl-phenyl)-1-(2,3-dihydroxy-propyl)-1,4,6,7-tetrahydro-pyrazolo[4,3-c]pyridin-5-yl]-ethanone.

A solution of KHMDS (0.5 M, 8.4 mL, 4.1 mmol) was added to a solution of 1-[3-(4-chloro-3-methyl-phenyl)-1,4,6,7-tetrahydro-pyrazolo[4,3-c]pyridin-5-yl]-ethanone (1.01 g, 3.49 mmol) in DMF (8.5 mL). The mixture was stirred for 1 h then (2*R*)-1-*tert*-butyldimethylsilylglycidol (1.97 g, 10.5 mmol) was added. The mixture was stirred for 17 h then partitioned between EtOAc (500 mL) and saturated aqueous NaHCO₃ (100 mL). The EtOAc layer was washed with H₂O (3 x 100 mL), and brine (100 mL). The combined washes were extracted with EtOAc (2 x 100 mL). The combined extracts were dried over Na₂SO₄ and concentrated. The residue was dissolved in MeOH (50 mL) and treated with CSA (171 mg). The resulting mixture was stirred for 24 h then concentrated to near dryness. The residue was diluted with EtOAc (300 mL), washed with NaHCO₃ (100 mL), dried over Na₂SO₄ and concentrated. Flash chromatography (silica, 5-10% MeOH/CH₂Cl₂) provided 652 mg (50%) of the non-racemic diol. TLC (silica, 10% MeOH/CH₂Cl₂): R*_{f}* = 0.2. MS (electrospray): *m*/*z* calculated for C₁₈H₂₃³⁵ClN₃O₃ ([M+H]⁺, 364.14, found 364.1.

### B. (R)-1-[3-(4-Chloro-3-methyl-phenyl)-1-oxiranylmethyl-1,4,6,7-tetrahydropyrazolo[4,3-c]pyridin-5-yl]-ethanone.

(*R*)-1-[3-(4-Chloro-3-methyl-phenyl)-1-(2,3-dihydroxy-propyl)-1,4,6,7-tetrahydro-pyrazolo[4,3-c]pyridin-5-yl]-ethanone (452 mg, 1.24 mmol) and pyridinium *p*-toluenesulfonate (85 mg) were combined in MeC(OMe)₃ (50 mL) and briefly sonicated. The mixture was stirred for 17 h, concentrated, and the residue dissolved in CH₂Cl₂ (8 mL). The solution was cooled to 0 °C and treated with AcBr (0.15 mL, 2.0 mmol). After 5 h the mixture was partitioned between EtOAc (300 mL) and saturated aqueous NaHCO₃ (75 mL). The EtOAc layer was washed with H₂O (75 mL) and brine (75 mL), dried over Na₂SO₄ and concentrated. The residue was combined with K₂CO₃ (243 mg, 1.84 mmol) in MeOH (50 mL) and stirred for 3 h then worked up as described above. Purification by column chromatography (silica, 10-40% acetone/CH₂Cl₂) gave 159 mg (37%) of the title compound. Chiral HPLC (Daicel OD, 0.5% Et₂NH/MeOH) analysis indicated >95% optical purity. HPLC (reverse phase conditions), *t*_{R} = 4.97 min. MS (electrospray): exact mass calculated for C₁₈H₂₀CIN₃O₂ [M⁺+Na], 368.11; *m*/*z* found 368.05. ¹H NMR (CDCl₃, 400 MHz, a mixture of amide rotamers): 7.54 (br d, *J* = 6.3 Hz, 2H), 7.41-7.35 (m, 3H), 7.29 (dd, *J* = 8.2, 1.9 Hz, 1H), 4.81 and 4.74 (A and B of AB quartet, *J*_{ab} = 15.7 Hz, 2H), 4.60 (s, 2H), 4.48 (dd, *J* = 15.2, 2.4 Hz, 1H), 4.42 (dd, *J* = 15.4, 2.8 Hz, 1H), 4.13 (t, *J* = 4.7 Hz, 1H), 4.09 (dd, *J* = 4.6 Hz, 1H), 3.93 (m, 2H), 3.74 (t, *J* = 5.8 Hz, 1H), 3.73 (t, *J* = 5.8 Hz, 1H), 3.34 (m, 2H), 2.85-2.75 (m, 6H), 2.53 (dd, *J* = 4.6, 2.5 Hz, 1H), 2.48 (dd, *J* = 4.6, 2.6 Hz, 1H), 2.43 (s, 3H), 2.41 (s, 3H), 2.20 (s, 3H), 2.15 (s, 3H).

### C. 4-(5-Chloro-2-nitro-phenylamino)-piperidine-1-carboxylic acid ethyl ester.

To a solution of 2.03 g (11.6 mmol) of 4-chloro-2-fluoro-nitrobenzene in DMF (12.0 mL) at rt was added 2.00 g (11.6 mmol) of ethyl 4-amino-1-piperidinecarboxylate. A yellow precipitate formed within 30 min and the reaction was further diluted with DMF (12.0 mL) and CH₂Cl₂ (5.0 mL) and was shaken at 300 RPM overnight. The solvent was removed under reduced pressure and the resulting solid was dried under vacuum. The crude product was purified by flash chromatography (silica, 0-5% MeOH/CH₂Cl₂) to afford 2.83 g (81%) of the title compound. TLC (silica, 5% MeOH/CH₂Cl₂): R*_{f}* = 0.4. MS (electrospray): *m*/*z* calculated for C₁₄H₁₈ClN₃O₄ [M⁺+Na] 350.09, observed 350.0. ¹H NMR (400 MHz, CDCl₃): 8.13 (apparent d, *J* = 9.1 Hz, 2H), 6.84 (d, *J* = 2.0 Hz, 1H), 6.62 (dd, *J* = 9.4, 2.3 Hz, 1H), 4.15 (q, *J* = 14.9, 7.3 Hz, 2H), 4.08 (br d, *J* = 12.4 Hz, 2H), 3.70-3.58 (m, 1 H), 3.17-3.05 (m, 2H), 2.07 (br dd, *J* = 13.1, 3.1 Hz, 2H), 1.63-1.50 (m, 2H), 1.28 (t, *J* = 7.0 Hz, 3H).

### D. 4-(6-Chloro-2-oxo-2,3-dihydro-benzoimidazol-1-yl)-piperidine-1-carboxylic acid ethyl ester.

To a stirred solution of 0.50 g (1.52 mmol) of 4-(5-chloro-2-nitro-phenylamino)-piperidine-1-carboxylic acid ethyl ester in EtOH (15.0 mL) was added concentrated HCl (3.0 mL) followed by 0.99 g (15.2 mmol) of zinc powder. After 1h, additional concentrated HCl (1.5 mL) followed by 0.99 g (15.2 mmol) of zinc powder was added and the reaction was stirred for 1.5 h. The mixture was filtered through a pad of celite and was washed with 5% MeOH/CH₂Cl_{2.} The mixture was diluted with saturated NaHCO₃ and a precipitate formed. The layers were separated and the aqueous phase was extracted (3 x 5% MeOH/CH₂Cl₂). The combined organic layers were dried over Na₂SO₄ and the solvent was removed under reduced pressure to afford a brown oil. The crude oil was diluted with CH₂Cl₂ (15.0 mL) and 0.64 mL (4.56 mmol) of Et₃N was added followed by 0.45 g (1.52 mmol) of triphosgene. The reaction was allowed to stir overnight and was then diluted with 1 N NaOH (20 mL) and stirred for an additional 1h. The layers were separated and the aqueous phase was extracted with CH₂Cl₂ (3×20 mL). The combined organic extracts were dried over Na₂SO₄ and the solvent was removed under reduced pressure. Purification by flash chromatography (silica, 0-5% MeOH/CH₂Cl₂) afforded 0.33 g (67% over 2 steps) of the title compound. TLC (silica, 5% MeOH/CH₂Cl₂): R*_{f}* = 0.5. MS (electrospray): *m*/*z* calculated for C₁₅H₁₈ClN₃O₃ [M⁺+Na] 346.10, observed 346.0. ¹H NMR (400 MHz, CDCl₃): 9.41 (s, 1 H), 7.11 (d, *J* = 2.0 Hz, 1H), 7.04 (d, *J* = 1.8 Hz, 1H), 7.02 (s, 1 H), 4.48-4.33 (m, 3H), 4.20 (q, *J* = 7.1 Hz, 2H), 2.92 (t, *J* = 12.5 Hz, 2H), 2.30 (dq, *J* = 12.9,4.6 Hz, 2H), 2.10 (d, *J* = 12.6 Hz, 2H), 1.31 (t, *J* = 7.1 Hz, 3H).

### E. 6-Chloro-1-piperidin-4-yl-1,3-dihydro-benzoimidazol-2-one.

A suspension of 0.20 g (0.62 mmol) of 4-(6-chloro-2-oxo-2,3-dihydro-benzoimidazol-1-yl)-piperidine-1-carboxylic acid ethyl ester in 10% NaOH (0.62 mL) was heated to 105 °C for 6 h and then cooled. The solution was adjusted to pH 1 (conc. HCl) and then back to pH 10 (NaOH). Then, the mixture was diluted with 5% MeOH/CH₂Cl₂ (-30 mL) until both layers were clear. The layers were separated and the aqueous phase was extracted with 5% MeOH/CH₂Cl₂ (2 × 30 mL). The combined organic layers were dried over Na₂SO₄ and the solvent was removed under reduced pressure to afford 0.12 g (76%) of a light brown solid. TLC (silica, 10% MeOH/CH₂Cl₂): R*_{f}*= 0.1. MS (electrospray): *m*/*z* calculated for C₁₂H₁₄ClN₃O [M⁺+H] 252.08, observed 252.1. ¹H NMR (400 MHz, CDCl₃): 7.27 (d, *J* = 1.6 Hz, 2H), 7.02 (d, *J* = 1.6 Hz, 1H), 7.01 (s, 1H), 4.38 (m, 1H), 3.30 (br d, *J* = 11.9 Hz, 2H), 2.82 (dt, *J* = 12.3, 2.0 Hz, 2H), 2.35 (dq, *J* = 12.3, 3.5 Hz, 2H), 1.85 (br dd, *J* = 12.1, 2.1 Hz, 2H).

### F. (S)-1-(1-{3-[5-Acetyl-3-(4-chloro-3-methyl-phenyl)-4,5,6,7-tetrahydropyrazolo[4,3-c]pyridin-1-yl]-2-hydroxy-propyl}piperidin-4-yl)-6-chloro-1,3-dihydro-benzoimidazol-2-one.

(*R*)-1-[3-(4-Chloro-3-methyl-phenyl)-1-oxiranylmethyl-1,4,6,7-tetrahydropyrazolo[4,3-c]pyridin-5-yl]-ethanone (31 mg, 0.10 mmol) and 6-chloro-1-piperidin-4-yl-1,3-dihydro-benzoimidazol-2-one (36 mg, 0.17 mmol) were combined in EtOH (0.3 mL) and heated to 70°C. After 18 h the mixture was allowed to cool, diluted with CH₂Cl₂ and purified by preparative TLC (silica, 8% MeOH/CH₂Cl₂) to give 7 mg (12%) of the title compound. HPLC (reverse phase conditions), *t*_{R} = 3.49 min. MS (electrospray): *m*/*z* calculated for C₃₀H₃₅³⁵Cl₂N₆O₃ [M⁺+H] 597.22, found 597.20. ¹H NMR (CDCl₃, 400 MHz, a mixture of amide rotamers): 9.16 (br d, *J* = 10.1 Hz, 1H),7.55(br m, 1H), 7.40-7.28 (m, 2H), 7.18 (br s, 1H), 7.03 and 6.98 (A and B of ABX (with fine splittings), *J*_{ab} = 8.4 Hz, 2H), 4.85 and 4.74 (A and B of ABX (with fine splittings), *J*_{ab} = 15.7 Hz, 1H), 4.62 (s, 1 H), 4.29-4.18 (m, 4H), 4.09-4.00 (m, 2H), 3.91-3.71 (m, 2H), 3.16-2.78 (m, 4H), 2.55-2.50 (m, 4H), 2.43 (s, 1.5H), 2.41 (s, 1.5H), 2.23 (m, 1H), 2.21 (s, 1.5H), 2.16 (s, 1.5H), 1.84 (br s, 2H).

### EXAMPLE 7

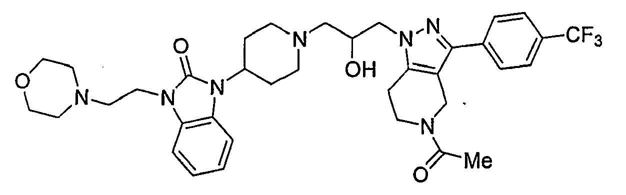

### 1-(1-{3-[5-Acetyl-3-(4-trifluoromethyl-phenyl)-4,5,6,7-tetrahydro-pyrazolo[4,3-c]pyridin-1-yl]-2-hydroxy-propyl}-piperidin-4-yl)-3-(2-morpholin-4-yl-ethyl)-1,3-dihydro-benzoimidazol-2-one.

A solution of 1-(1-{3-[5-acetyl-3-(4-trifluoromethyl-phenyl)-4,5,6,7-tetrahydropyrazolo[4,3-c]pyridin-1-yl]-2-hydroxy-propyl}-piperidin-4-yl)-1,3-dihydro-benzoimidazol-2-one (130 mg, 0.22 mmol) in DMF (1 mL) was treated with cesium carbonate (146 mg, 0.45 mmol) and 4-(2-chloroethyl)morpholine hydrochloride (329 mg, 2.2 mmol) at 25 °C and stirred for 24 h before it was diluted with EtOAc (10 mL) and H₂O (5 mL). The organic layer was separated, washed with H₂O (2 x 5 mL), dried over Na₂SO₄, and concentrated. Column chromatography (silica, 5% MeOH/CH₂Cl₂) afforded 124 mg (81%) of a white powder. TLC (10% MeOH/CH₂Cl₂): R*_{f}* = 0.31. MS (electrospray): *m*/*z* 696.3 ([M+H]⁺, C₃₆H₄₄F₃N₇O₄ requires 695.3). ¹H NMR (CDCl₃, 400 MHz, a mixture of two rotamers): 7.82 and 7.65 (AB pattern, *J*_{ab} = 8.2 Hz, 2H), 7.74 and 7.68 (AB pattern, *J*_{ab} = 8.4 Hz, 2H), 7.23-7.05 (m, 4H), 4.92 and 4.80 (AB pattern. *J*_{ab} = 15.6 Hz, 1.2H), 4.69 (s, 0.8H), 4.38-4.00 (m, 5H), 4.02 (t, *J* = 7.0 Hz, 2H), 3.92-3.70 (m, 2H), 3.70 (t, *J* = 4.5 Hz, 4H), 3.15-2.80 (m, 4H), 2.70 (t, *J* = 7.1 Hz, 2H), 2.60-2.20 (m, 9H), 2.24 (s, 1.6H), 2.18 (s, 1.4H), 1.85-1.75 (m, 2H).

### EXAMPLE 8

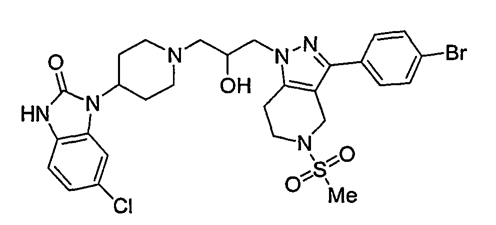

### 1-(1-{3-[3-(4-Bromo-phenyl)-5-methanesulfonyl-4,5,6,7-tetrahydro-pyrazolo[4,3-c]pyridin-1-yl]-2-hydroxy-propyl}-piperidin-4-yl)-6-chloro-1,3-dihydro-benzoimidazol-2-one.

### A. 3-(4-Bromo-phenyl)-1,4,6,7-tetrahydro-pyrazolo[4,3-c]pyridine-5-carboxylic acid tert-butyl ester.

To a stirred solution of 500.0 g (2.51 mol) of 1-*tert-*butoxycarbonyl-4-piperidone and 87.1 g (2.76 mol) of morpholine in benzene (1.25 L) was added a catalytic amount (- 0.25 g) of *p*-TsOH. The mixture was heated to reflux for 36 h with a Dean-Stark trap. The solvent was removed under reduced pressure to give a brown oil, which solidified on standing. The crude product was divided and 335.0 g (1.25 mol) of the enamine was diluted with CH₂Cl₂ (1.25 L) and 175.0 mL (1.25 mol) of Et₃N was added. The mixture was cooled to 0 °C and a solution of 275.0 g (1.25 mol) of 4-bromobenzoyl chloride in CH₂Cl₂ (150 mL) was added slowly by dropping funnel over 1 h. The mixture was allowed to warm to rt and stir overnight. The reaction was then diluted with 1 *N* HCl (450 mL) and stirred vigorously for 3 h. The aqueous layer was extracted with CH₂Cl₂ (3 x 500 mL) and the combined extracts were dried over Na₂SO₄ and the solvent was removed under reduced pressure. The crude oil was diluted with EtOH (850 mL) and cooled to 0 °C. To this stirred solution was slowly added 120.0 g (3.75 mol) of hydrazine and the mixture was allowed to warm to rt and stir overnight during which time a white precipitate formed. The volume of the reaction was reduced to ~350 mL and EtOAc (1.50 L) was added to the mixture. The suspension was stirred vigorously for 2 h and was filtered then washed with EtOAc (2 x 500 mL) and dried under vacuum to afford 309.0 g (62% over 3 steps) of a white solid. TLC (silica, 5% MeOH/CH₂Cl₂): R*_{f}* = 0.3. MS (electrospray): *m*/*z* calculated for C₁₇H₂₀BrN₃O₂ [M⁺+H] 378.07, observed 378.0. ¹H NMR (400 MHz, CDCl₃): 7.65-7.26 (m, 4H), 4.64 (br s, 2H), 3.84-3.68 (br m, 2H), 2.87-2.74 (br m, 2H), 1.48 (br s, 9H).

### B. 3-(4-Bromophenyl)-4,5,6,7-tetrahydro-1H-pyrazolo[4,3-c]pyridinium trifluoroacetate.

To a stirred solution of 10.0 g (26.4 mmol) of the 3-(4-bromo-phenyl)-1,4,6,7-tetrahydro-pyrazolo[4,3-c]pyridine-5-carboxylic acid tert-butyl ester in CH₂Cl₂ (26.0 mL) was added 26.0 mL of TFA. The resulting mixture was allowed to stir overnight. The solvent was removed under reduced pressure and the solid was dried in vacuo. The dried solid was suspended in Et₂O and stirred vigorously for 2 h and then filtered and dried in vacuo to give 10.1 g of a white solid, which was used without further purification. TLC (silica, 10% MeOH/CH₂Cl₂): R*_{f}* = 0.05. MS (electrospray): *m*/*z* calculated for C₁₂H₁₂BrN₃ [M⁺+H] 278.02, observed 278.0.

### C. 3-(4-Bromo-phenyl)-5-methanesulfonyl-4,5,6,7-tetrahydro-1H-pyrazolo[4,3-c]pyridine.

To a stirred solution of 3.11 g (11.1 mmol) of 3-(4-bromophenyl)-4,5,6,7-tetrahydro-1 H-pyrazolo[4,3-*c*]pyridinium trifluoroacetate and 4.71 mL (33.5 mmol) of Et₃N in DMF (55 mL) was slowly added 1.21 mL (15.6 mmol) of methanesulfonyl chloride. After 2.5 h, the solvent was removed under reduced pressure and the residue was diluted with CH₂Cl₂ (100 mL) and saturated NaHCO₃ (100 mL). The layers were separated and the aqueous phase was extracted with CH₂Cl₂ (2 x 30 mL). The combined organic layers were dried over Na₂SO₄ and the solvent was removed under reduced pressure. Purification by column chromatography (silica, 0-5% MeOH/CH₂Cl₂) afforded 2.01 g (50%) of the title compound. TLC (silica, 5% MeOH/CH₂Cl₂): R*_{f}* = 0.3. MS (electrospray): *m*/*z* calculated for C₁₃H₁₄BrN₃O₂S [M⁺+H] 356.00, observed 356.0.

### D. 3-(4-Bromo-phenyl)-5-methanesulfonyl-1-oxiranylmethyl-4,5,6,7-tetrahydro-1H-pyrazolo[4,3-c]pyridine.

To a stirred solution of 2.50 g (7.00 mmol) of 3-(4-bromo-phenyl-5-methanesulfonyl-4,5,6,7-tetrahydro-1 H-pyrazolo[4,3-c]pyridine and 5.52 mL (70.0 mmol) of epichlorohydrin was added 2.50 g (7.72 mmol) of solid Cs₂CO₃. The reaction was allowed to stir for 48 h and the solvent was removed under reduced pressure. The residue was then diluted with H₂O (150 mL) and EtOAc (150 mL). The layers were separated, and the organic layer was washed with H₂O (50 mL) and brine (50 mL), dried over Na₂SO₄ and the solvent was removed under reduce pressure. Purification by flash chromatography (silica, 0-20% acetone/CH₂Cl₂) afforded 1.52 g (53%) of a white solid. TLC (silica, 5% MeOH/CH₂Cl₂): R*_{f}* = 0.5. MS (electrospray): *m*/*z* calculated for C₁₆H₁₈BrN₃O₃S [M⁺+H] 412.03, observed 412.0. ¹H NMR (400 MHz, CDCl₃): 7.54 and 7.47 (A and B of AA'BB', *J* = 8.6 Hz, 4H), 4.56-4.45 (m, 3H), 4.10 (dd, *J* = 15.1, 5.4 Hz, 1H), 3.73-3.58 (m, 2H), 3.38-3.32 (m, 1H), 2.96-2.87 (m, 2H), 2.86 (s, 3H), 2.83 (dd, *J* = 4.4, 4.2 Hz, 1H), 2.48 (dd, *J* = 4.6, 2.6 Hz, 1H).

### E. 1-(1-{3-[3-(4-Bromo-phenyl)-5-methanesulfonyl-4,5,6,7-tetrahydropyrazolo[4,3-c]pyridin-1-yl]-2-hydroxy-propyl}-piperidin-4-yl)-6-chloro-1,3-dihydro-benzoimidazol-2-one.

A stirred solution of 25.0 mg (0.061 mmol) of 3-(4-bromo-phenyl)-5-methanesulfonyl-1-oxiranylmethyl-4,5,6,7-tetrahydro-1 H-pyrazolo[4,3-c]pyridine and 19.0 mg (0.073 mmol) of 6-chloro-1-piperidin-4-yl-1,3-dihydro-benzoimidazol-2-one in EtOH (0.5 mL) was heated to 80°C in a sealed vessel for 16 h. The reaction was cooled and the solvent was removed under reduced pressure. The crude product was purified by column chromatography (silica, 0-5% MeOH/CH₂Cl₂) to afford 0.025 g (63%) of the title compound. TLC (silica, 5% MeOH/CH₂Cl₂): R*_{f}* = 0.4. MS (electrospray): *m*/*z* calculated for C₂₈H₃₂BrClN₆O₄S [M⁺+H] 663.11, observed 663.0. ¹H NMR (400 MHz, CDCl₃): 10.2 (s, 1H), 7.52 and 7.46 (A and B of AA'BB', *J* = 8.6 Hz, 4H), 7.15 (br d, *J* = 1.5 Hz, 1H), 7.04-6.95 (m, 2H), 4.52 and 4.49 (A and B of AB quartet, *J*_{ab} = 14.5 Hz, 2H), 4.33-4.14 (m, 3H), 4.07-3.97 (m, 1H), 3.74-3.58 (m, 2H), 3.17-2.89 (m, 4H), 2.86 (s, 3H), 2.57-2.30 (m, 5H), 2.20 (t, *J* = 11.1 Hz, 1H), 1.87-1.73 (m, 2H).

### EXAMPLE 9

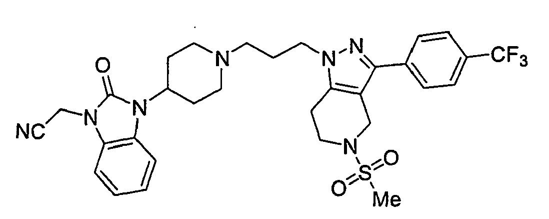

### [3-(1-{3-[5-Methanesulfonyl-3-(4-trifluoromethyl-phenyl)-4,5,6,7-tetrahydropyrazolo[4,3-c]pyridin-1-yl]-propyl}-piperidin-4-yl)-2-oxo-2,3-dihydro-benzoimidazol-1-yl]-acetonitrile.

### A. 3-(4-Trifluoromethyl-phenyl)-1,4,6,7-tetrahydro-pyrazolo[4,3-c]pyridine-5-carboxylic acid tert-butyl ester.

To a stirred solution of 500 g (2.51 mol) of 1-*tert*-butoxycarbonyl-4-piperidone and 87.1 g (2.76 mol) of morpholine in benzene (1.25 L) was added a catalytic amount (~ 0.25 g) of *p*-TsOH. The mixture was heated to reflux for 36 h with a Dean-Stark trap. One half of the solvent was removed under reduced pressure and the resulting solution was cooled and filtered. The filtrate was then concentrated to yield 630 g (94%) of an orange red oil. The eneamine was divided and 320 g (1.19 mol) was diluted with CH₂Cl₂ (1.0 L) and 165.0 mL (1.19 mol) of Et₃N was added. The mixture was cooled to 0 °C and a solution of 225 g (1.08 mol) of 4-trifluoromethylbenzoyl chloride in CH₂Cl₂ (0.5 L) was added slowly by dropping funnel over 1h. The mixture was allowed to warm to rt and stir overnight. The reaction was then diluted with 1*N* HCl (450 mL) and stirred vigorously for 3h. The aqueous layer was extracted with CH₂Cl₂ (3 x 500 mL) and the combined extracts were dried over Na₂SO₄ and the solvent was removed under reduced pressure. The crude oil was diluted with EtOH (1 L) and cooled to 0 °C. To this stirred solution was slowly added 115 g (3.57 mol) of hydrazine and the mixture was allowed to warm to rt and stir overnight during which time a white precipitate formed. The volume of the reaction was reduced to ~500 mL and cooled. The precipitate was collected to afford 285 g (72% from eneamine) of a white solid. ¹H NMR (400 MHz, CDCl₃): 7.63-7.55 (m, 4H), 4.58 (br s, 2H), 3.69-3.62 (br m, 2H), 2.74-2.68 (br m, 2H), 1.47 (s, 9H).

### B. 1-(2-Methoxycarbonyl-ethyl)-3-(4-trifluoromethyl-phenyl)-1,4,6,7-tetrahydropyrazolo[4,3-c]pyridine-5-carboxylic acid tert-butyl ester.

3-(4-Trifluoromethyl-phenyl)-1,4,6,7-tetrahydro-pyrazolo[4,3-c]pyridine-5-carboxylic acid tert-butyl ester (1.85g, 5.04 mmol) and methyl acrylate (0.50 mL, 5.6 mmol) were combined in toluene (30 mL) and heated to 75 °C. The resulting mixture was treated with t-BuONa (100 mg), and heating continued for 48 h. The mixture was allowed to cool and partitioned between EtOAc (300 mL) and NaHCO₃ (75 mL). The aqueous layer was extracted with EtOAc (3 x 75 mL). The combined extracts were dried over Na₂SO₄ and concentrated. Column chromatography (silica, 30-60% EtOAc/hexanes) afforded 343 mg (15%) of the title compound. TLC (silica, 50% EtOAc/hexanes): R_{f} = 0.4. MS (electrospray): *m*/*z* calculated for C₂₂H₂₇F₃N₃O₄ [M⁺+H] 454.20, found 454.1. ¹H NMR (CDCl₃, 400 MHz): 7.75 (br d, *J* = 8.1 Hz, 2H), 7.64 (br s, 2H), 4.63 (br s, 2H), 4.30 (t, *J* = 6.6 Hz, 2H), 3.75 (br s, 2H), 3.68 (s, 3H), 2.98 (t, *J* = 6.6 Hz, 2H), 2.79 (br t, *J* = 5.6 Hz, 2H), 1.48 (s, 9H).

### C. 1-(3-Hydroxy-propyl)-3-(4-trifluoromethyl-phenyl)-1,4,6,7-tetrahydropyrazolo[4,3-c]pyridine-5-carboxylic acid tert-butyl ester.

A solution of LiBH₄ (26 mg, 1.2 mmol) in THF (0.5 mL) was added to a 0 °C solution of 1-(2-methoxycarbonyl-ethyl)-3-(4-trifluoromethyl-phenyl)-1,4,6,7-tetrahydro-pyrazolo[4,3-c]pyridine-5-carboxylic acid tert-butyl ester (317 mg, 0.70 mmol) in THF (4.0 mL). The mixture was stirred for 5 min then additional LiBH₄ (15 mg) was added and stirring continued for 17 h. The mixture was partitioned between EtOAc (80 mL) and saturated aqueous NaHCO₃ (20 mL). The aqueous layer was extracted with EtOAc (2 x 20 mL). The combined extracts were dried over Na₂SO₄ and concentrated. Column chromatography (silica, 0-8% MeOH/CH₂Cl₂) afforded 268 mg (95%) of the title compound. HPLC (reverse phase conditions), *t*_{R} = 6.82 min. MS (electrospray): *m*/*z* calculated for C₂₁H₂₆F₃N₃O₃ [M⁺+Na] 448.18, found 448.10. ¹H NMR (CDCl₃, 400 MHz): 7.73 (br d, *J* = 8.2 Hz, 2H), 7.65 (br s, 2H), 4.64 (br s, 2H), 4.21 (t, *J* = 6.4 Hz, 2H), 3.76 (br s, 2H), 3.66 (t, *J* = 5.7 Hz, 2H), 2.73 (br t, *J* = 5.4 Hz, 2H), 2.04 (q, *J* = 6.1, 2H), 1.48 (s, 9H).

### D. 4-(2-Oxo-2,3-dihydro-benzoimidazol-1-yl)-piperidine-1-carboxylic acid tert-butyl ester.

1-Piperidin-4-yl-1,3-dihydro-benzoimidazol-2-one (7.24 g, 3.4.1 mmol) and di-tert-butyl dicarbonate (9.12 g, 41.0 mmol) were combined in DMF (80 mL) and the mixture heated to 40°C under N₂ for 17h. The mixture was allowed to cool, diluted with EtOAc (800 mL) and washed with saturated aq. NaHCO₃ (150 mL), H₂O (3 x 150 mL) and brine (150 mL). The combined aqueous washes were extracted with EtOAc (2 x 150 mL). The combined extracts were dried over Na₂SO₄ and concentrated to afford 12.4 g of the title compound. TLC (silica, 50% EtOAc/hexanes): R*_{f}* = 0.3. MS (electrospray): *m*/*z* calculated for C₁₇H₂₃N₃O₃ [M⁺+Na] 340.16, found 340.1. ¹H NMR (CDCl₃, 400 MHz): 10.59 (s, 1 H), 7.15-7.11 (m, 2H), 7.08-7.02 (m, 2H), 4.49 (tt, *J* = 8.4, 4.0 Hz, 1H), 4.32 (br s, 2H), 2.89 (br t, *J* = 11.6, 2H), 2.34 (dq, *J* = 12.6, 4.4 Hz, 2H), 1.83 (br d, *J* = 10.5 Hz, 2H) 1.36 (s, 9H).

### E. (2-Oxo-3-piperidin-4-yl-2,3-dihydro-benzoimidazol-1-yl)-acetonitrile.

A solution of 4-(2-oxo-2,3-dihydro-benzoimidazol-1-yl)-piperidine-1-carboxylic acid tert-butyl ester (2.91 g, 9.16 mmol) in THF (10 mL) was added dropwise to a solution of KHMDS (2.19 g, 11.0 mmol) in THF (20 mL). The mixture was stirred for 10 min then bromoacetonitrile (3.2 mL, 46 mmol) was added. The resulting mixture was stirred for 4 h then partitioned between EtOAc (750 mL) and saturated aqueous NaHCO₃ (200 mL). The EtOAc layer was washed with H₂O (3 x 200 mL) and brine (200 mL). The combined washes were extracted with EtOAc (2 x 150 mL). The combined extracts were dried over Na₂SO₄ and concentrated. Column chromatography (silica, 20-60% EtOAc/hexanes) afforded 2.20 g (67%) of 4-(3-cyanomethyl-2-oxo-2,3-dihydro-benzoimidazol-1-yl)-piperidine-1-carboxylic acid tert-butyl ester. The purified material was dissolved in CH₂Cl₂ (40 mL) and diluted with TFA (25 mL). The resulting mixture was stirred for 1h then diluted with CH₂Cl₂ (250 mL) and washed with 1*N* NaOH (100 mL). The aqueous layer was extracted with CH₂Cl₂ (2 x 100 mL). The combined extracts were dried over Na₂SO₄ and concentrated to 1.59 g (95%) of the title compound which was suitable for further use without purification. TLC (silica, 5% MeOH/CH₂Cl₂): R_{f} = 0.1. MS (electrospray): *m*/*z* calculated for C₁₄H₁₇N₄O [M⁺+H] 257.14, found 257.1. ¹H NMR (CDCl₃, 400 Hz): 733-7.29 (m, 1H), 7.17-7.02 (m, 3H), 4.75 (s, 2H), 4.41 (tt, *J* = 12.2, 4.4 Hz, 1 H), 3.28 (br d, *J* = 9.8 Hz, 2H), 3.11 (br s, 1H), 2.80 (t, *J* = 10.0 Hz, 2H), 2.37 (dq, *J* = 12.5, 4.2 Hz, 2H), 1.83 (br d, *J* = 11.8 Hz, 2H).

### F. [3-(1-{3-[5-Methanesulfonyl-3-(4-trifluoromethyl-phenyl)-4,5,6,7-tetrahydropyrazolo[4,3-c]pyridin-1-yl]-propyl}-piperidin-4-yl)-2-oxo-2,3-dihydro-benzoimidazol-1-yl]-acetonitrile.

1-(3-Hydroxy-propyl)-3-(4-trifluoromethyl-phenyl)-1,4,6,7-tetrahydropyrazolo[4,3-c]pyridine-5-cartioxylic acid tert-butyl ester (268 mg, 0.63 mmol) was dissolved in CH₂Cl₂ (10 mL) and TFA (10 mL). The mixture was stirred for 1 h then concentrated to dryness. The residue was dissolved in CH₂Cl₂ (4.0 mL), cooled to 0 °C and treated with *i*-Pr₂NEt (0.36 mL, 2.1 mmol), followed by methanesulfonyl chloride (0.16 mL, 2.1 mmol). The reaction mixture was stirred for 4 h, then diluted with EtOAc (200 mL) and washed with saturated aqueous NaHCO₃ (2 x 25 mL). The washes were extracted with EtOAc (2 x 25 mL). The combined extracts were dried over Na₂SO₄ and concentrated. A portion of the crude mesylate (197 mg, ca. 0.41 mmol) was combined with (2-oxo-3-piperidin-4-yl-2,3-dihydro-benzoimidazol-1-yl)-acetonitrile (321 mg, 1.25 mmol) in CH₂Cl₂ (2.0 mL) and DMF (0.5 mL). The resulting mixture was treated with *i*-Pr₂NEt (0.22 mL, 1.3 mmol) and stirred for 60 h. The reaction mixture was partitioned between EtOAc (150 mL) and saturated aqueous NaHCO₃ (75 mL). The EtOAc layer was washed with H₂O (2 x 75 mL), and brine (75 mL). The combined washes were extracted with EtOAc (3 x 50 mL). The combined extracts were dried over Na₂SO₄ and concentrated. Purification of the residue by preparative TLC (silica, 1% MeOH/CH₂Cl₂ then 25% acetone/CH₂Cl₂) gave 37 mg (14%) of the title compound. HPLC (reverse phase conditions), *t*_{R} = 2.94 min. MS (electrospray): *m*/*z* calculated for C₃₁H₃₅F₃N₇O₃S [M⁺+H] 642.25, found 642.25. ¹H NMR (CDCl₃, 400 MHz): 7.73 and 7.76 (A and B of AA'BB' *J*_{ab} = 8.2 Hz, 4H), 7.26-7.05 (m, 4H), 4.81 (s, 2H), 4.56 (s, 2H), 4.26 (m, 1H), 4.15 (t, *J* = 6.8 Hz, 2H), 3.70 (t, *J* = 5.8 Hz, 2H), 3.03 (br d, *J* = 11.1 Hz, 2H), 2.95 (t, *J* = 5.7 Hz, 2H), 2.91 (s, 3H), 2.43 (m, 4H), 2.12 (m, 4H), 1.82 (br d, *J* = 9.9 Hz, 2H).

### EXAMPLE 10

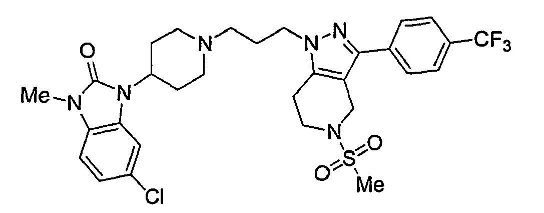

### 5-Chloro-3-(1-{3-,[5-methanesulfonyl-3-(4-trifluoromethyl-phenyl)-4,5,6,7-tetrahydro-pyrazolo[4,3-c]pyridin-1-yl]-propyl}-piperidin-4-yl)-1-methyl-1,3-dihydro-benzoimidazol-2-one.

### A. 1-Methanesulfonyl-piperidin-4-one.

Potassium carbonate (324 g, 2340 mmol) was added to a solution of 4-piperidone monohydrate hydrochloride (90 g, 586 mmol) in chloroform (300 mL) and water (300 mL). The slurry was cooled to 0 °C and treated with methylsulfonyl chloride (136 mL, 1760 mmol) by dropwise addition over a 1 h period. The reaction mixture was allowed to shake for 72 h and was partitioned between CH₂Cl₂ (500 mL) and saturated aqueous NaHCO₃ (500 mL). The aqueous layer was extracted with CH₂Cl₂ (3 X 200 mL). The organic layer was washed with 1% KHSO₄ (250 mL), dried (Na₂SO₄), and concentrated to afford 90.5 g (87%) of a white solid. HPLC (reverse phase conditions), *t*_{R} = 2.19 min. MS (electrospray): exact mass calculated for C₆H₁₁NO₃S, 177.1; *m*/*z* found, 178.1 [M+H]⁺. ¹H NMR (400 MHz, CDCl₃): 3.60 (t, *J* = 6.5 Hz, 4H), 2.89 (s, 3H), 2.59 (t, *J* = 6.3 Hz, 4H).

### B. 5-Methanesulfonyl-3-(4-trifluoromethyl-phenyl)-4,5,6,7-tetrahydro-1H-pyrazolo[4.3-c]pyridine.

*p*-Toluenesulfonic acid (1.34 g, 7.0 mmol) and morpholine (25.83 mL, 296 mmol) were added to a solution of 1-methanesulfonyl-piperidin-4-one (50.0 g. 282 mmol) in benzene (282 mL). The reaction mixture was heated in a flask equipped with a condenser and a Dean-Stark trap at reflux for 15 h. The reaction mixture was cooled and concentrated in vacuo to give the enamine which was used without further purification. The enamine was dissolved in CH₂Cl₂ (200 mL) and cooled to 0°C. To this was added triethylamine (47.2 mL, 339 mmol) followed by dropwise addition of 4-trifluoromethylbenzoyl chloride (42.3 mL, 285 mmol) dissolved in CH₂Cl₂ (82 mL). The reaction mixture was allowed to warm to room temperature and stirred for 20 h. The reaction mixture was washed with 1 *N* HCl (250 mL) and the CH₂Cl₂ layer was separated, dried (Na₂SO₄), and concentrated. The resulting oil was taken up in ethanol (300 mL) and treated with hydrazine (44.3 mL, 1.41 mol) at 0 °C. The reaction mixture was allowed to warm to room temperature and stirred for 24 h. The mixture was concentrated and the resulting solid was filtered with ethanol wash and dried in vacuo to afford 70 g (72%) of 5-methanesulfonyl-3-(4-trifluoromethyl-phenyl)-4,5,6,7-tetrahydro-1H-pyrazolo[4,3-c]pyridine as a white solid. HPLC (reverse phase conditions), *t*_{R} = 6.33 min. MS (electrospray): exact mass calculated for C₁₄H₁₄F₃N₃O₂S, 345.0; *m*/*z* found, 346.0 [M+H]⁺. ¹H NMR (400 MHz, CDCl₃): 7.72 (s, 4H), 4.58 (s, 2H), 3.69 (t, *J* = 5.7 Hz, 2H), 2.99 (t, *J* = 5.7 Hz, 2H), 2.92 (s, 3H).

### C. 3-[5-Methanesulfonyl-3-(4-trifluoromethyl-phenyl)-4,5,6,7-tetrahydropyrazolo[4,3-c]pyridin-1-yl]-propan-1-ol.

Cs₂CO₃ (33.74 g, 103.5 mmol) was added to a solution of 5-methanesulfonyl-3-(4-trifluoromethyl-phenyl)-4,5,6,7-tetrahydro-1 H-pyrazolo[4,3-c]pyridine (29.8 g, 86.3 mmol) in anhydrous DMF (70 mL) and stirred for 25 min. 3-Bromo-1-propanol (8.6 mL, 13.2 g, 94.9 mmol) was added and stirred under N₂ at room temperature for 18 h. Water (500 mL) was added to the reaction and stirred for 5 min. The precipitated material was filtered out and washed with water (4 X 100 mL) and dried in a Freeze Drying System. The crude material (31.0 g) was taken up in anhydrous DMF (65 mL) and Cs₂CO₃ (33.74 g, 103.5 mmol) was added, and stirred for 10 min. 3-Bromo-1-propanol (8.6 mL, 13.2 g, 94.9 mmol) and MeOH (6.0 mL, 4.75 g, 148 mmol) were added and stirring continued under N₂ at rt for 15 h. Water (500 mL) was added to the reaction and stirred for 10 min. The precipitated material was filtered and washed with water (3 X 100 mL). The filter cake was dissolved in CH₂Cl₂ (200 mL) and washed with brine (50 mL), dried (Na₂SO₄), and concentrated. The solid was triturated with Et₂O (200 mL), filtered, then washed with Et₂O, and dried to furnish 16.0 g of the desired compound. The mother liquor was chromatographed (silica, 0-10% acetone/EtOAc) to obtain an additional 3.0 g of the title compound. The combined yield was 54.6%. MS (electrospray): exact mass calculated for C₁₇H₂₀F₃N₃O₃S, 403.12; *m*/*z* found, 404.0 [M+H]⁺, 426.0 [M+Na]⁺. ¹H NMR (400 MHz, CDCl₃): 7.71 (d, *J* = 8.2 Hz, 2H), 7.66 (d, *J* = 8.5 Hz, 2H), 4.55 (s, 2H), 4.23 (t, *J* = 6.5 Hz, 2H), 3.70-3.63 (m, 4H), 2.90 (s, 3H), 2.90 (t, *J* = 5.1 Hz, 2H), 2.62 (t, *J* = 5.9 Hz, 1H), 2.06 (q, *J* = 6.1 Hz, 2H).

### D. 5-Chloro-1-methyl-3-piperidin-4-yl-1,3-dihydro-benzoimidazol-2-one.

To a stirred suspension of 0.97 g (2.99 mmol) of 4-(6-chloro-2-oxo-2,3-dihydro-benzoimidazol-1-yl)-piperidine-1-carboxylic acid ethyl ester in THF (30 mL) was added 0.5 M KHMDS in toluene. This mixture was stirred for 1 h and 0.25 mL (3.89 mmol) of Mel was added in one portion. After 1.5 h the reaction was diluted with 1*N* HCl (75 mL) and EtOAc (75 mL). The layers were separated, and the organic layer was washed with H₂O (50 mL) and brine (50 mL), dried over Na₂SO₄ and the solvent was removed under reduced pressure.
Purification by flash chromatography (silica, 0-5% MeOH/CH₂Cl₂) afforded 0.92 g (91%) of a white solid. A suspension of 0.92 g (2.72 mmol) of the ethyl carbamate in 1:1 EtOH (7.0 mL) and 10% NaOH (7.0 mL) was heated to 110 °C for 36 h and then cooled. The mixture was diluted with EtOAc (30 mL) and H₂O. The layers were separated and the aqueous phase was extracted with EtOAc (2 x 30 mL). The combined organic layers were dried over Na₂SO₄ and the solvent was removed under reduced pressure to afford 0.56 g (78%) of a pale yellow solid. TLC (silica, 10% MeOH/CH₂Cl₂): R*_{f}* = 0.1. MS (electrospray): *m*/*z* calculated for C₁₃H₁₆ClN₃O [M⁺+H] 266.10, observed 266.0. ¹H NMR (400 MHz, CDCl₃): 7.28 (d, *J* = 1.8 Hz, 2H), 7.05 (dd, *J* = 8.3, 2.0 Hz, 1 H), 6.87 (d, *J* = 8.3 Hz, 1 H), 4.41 (tt, *J* = 12.5, 4.3 Hz, 1 H), 3.39 (s, 3H), 3.30 (br d, *J* = 11.9 Hz, 2H), 2.82 (dt, *J* = 12.4, 2.0 Hz, 2H), 2.30 (dq, *J* = 12.3, 4.3 Hz, 2H), 1.81 (br dd, *J* = 12.1, 2.3 Hz, 2H).

### E. 5-Chloro-3-(1-{3-[5-methanesulfonyl-3-(4-trifluoromethyl-phenyl)-4,5,6,7-tetrahydro-pyrazolo[4,3-c]pyridin-1-yl]-propyl}-piperidin-4-yl)-1-methyl-1,3-dihydro-benzoimidazol-2-one.

To a stirred solution of 0.33 mL (3.72 mmol) of oxalyl chloride in CH₂Cl₂ (10 mL) under N₂ at -78 °C was added 0.36 mL (4.96 mmol) of DMSO and the reaction was stirred for 15 min. To this solution was added a solution of 1.0 g (2.48 mmol) of 3-[5-methanesulfonyl-3-(4-trifluoromethyl-phenyl)-4,5,6,7-tetrahydro-pyrazolo[4,3-c]pyridin-1-yl]-propan-1-ol in 10 mL over 10 min and stirring was continued for 25 min. To this solution was added 1.40 mL (9.92 mmol) of Et₃N and the reaction was stirred for 10 min at -78 °C and was then allowed to warm to rt and stir for 1 h. The mixture was diluted with EtOAc (75 mL) and saturated NaHCO₃ (75 mL) and the layers were separated. The combined organic layers were dried over Na₂SO₄ and the solvent was removed under reduced pressure. The resulting solid was dried in vacuo and was suspended in Et₂O (20 mL) and stirred vigorously for 1 h. The solid was filtered and washed with Et₂O (2 x 10 mL) to afford the crude aldehyde which was carried on without further purification. The crude aldehyde, 5-chloro-1-methyl-3-piperidin-4-yl-1,3-dihydro-benzoimidazol-2-one (0.60 g, 2.3 mmol), and 0.20 mL (3.72 mmol) of AcOH was dissolved in CH₂Cl₂ (15 mL) followed by 0.69 g (3.25 mmol) of NaBH(OAc)₃, and the reaction was allowed to stir overnight. The mixture was diluted with CH₂Cl₂ (75 mL) and saturated NaHCO₃ (75 mL) and the layers were separated. The combined organic layers were dried over Na₂SO₄ and the solvent was removed under reduced pressure.
Purification by flash chromatography (silica, 0-4% MeOH/CH₂Cl₂) afforded 1.28 g (79% over 2 steps) of a white solid. TLC (silica, 5% MeOH/CH₂Cl₂): R*_{f}* = 0.5. MS (electrospray): *m*/*z* calculated for C₃₀H₃₄ClF₃N₆O₃S [M⁺+H] 651.21, observed 651.2. ¹H NMR (400 MHz, CDCl₃): 7.71 and 7.63 (A and B of AA'BB", *J_{AB}* = 8.17 Hz, 4H), 7.16 (d, *J* = 1.8 Hz, 1H). 7.04 (d, *J* = 8.3,1.8 Hz, 1 H), 6.86 (d, *J* = 8.3 Hz, 1 H), 4.55 (s, 2H), 4.23 (tt, *J* = 12.4, 4.3 Hz, 1 H), 4.13 (t, *J* = 6.7 Hz, 2H), 3.69 (t, *J* = 5.7 Hz, 2H), 3.36 (s, 3H), 3.0 (d, *J* = 11.6 Hz, 2H), 2.95 (t, *J* = 5.7 Hz, 2H), 2.90 (s, 3H), 2.45-2.32 (m, 4H), 2.16-2.04 (m, 4H), 1.76 (dd, *J* = 11.9, 2.0 Hz, 2H). ¹³C NMR (100 MHz, CDCl₃): 171.0, 153.7, 144.7, 137.1, 136.8, 129.3, 129.0, 128.7, 126.4, 125.5 (q, *J* = 3.8 Hz), 122.7, 120.7, 109.8, 109.2, 108.0, 60.3, 54.7, 53.0, 51.3, 46.8, 43.1, 42.4, 36.8, 29.0, 27.2, 27.0, 22.3, 21.0, 14.1.

### EXAMPLE 11

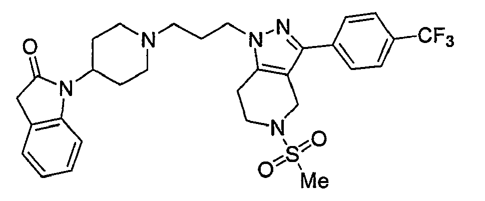

### 1-(1-{3-[5-Methanesulfonyl-3-(4-trifluoromethyl-phenyl)-4,5,6,7-tetrahydropyrazolo[4,3-c]pyridin-1-yl]-propyl}-piperidin-4-yl)-1,3-dihydro-indol-2-one.

### A. Methyl 2-nitrophenylacetate.

2-Nitrophenylacetic acid (60 g, 0.3 mol) was set stirring in 250 mL of methanol. Sulfuric acid (0.5 mL) was added and the mixture heated to reflux. After 20 h the mixture was cooled and evaporated under reduced pressure to give a clear yellow oil. The oil was brought up in EtOAc and washed with saturated NaHCO₃. The organics were dried (MgSO₄) and evaporated to give 63 g (98%) of the ester as a clear orange liquid. TLC (silica, 25% EtOAc/hexanes): R*_{f}* = 0.36. ¹H NMR (400MHz, CDCl₃): 8.24 (m, 1H), 7.16 (m, 1H), 7.60 (m, 1H). 7.47 (m, 1H), 4.15 (s, 2H), 3.83 (s, 3H).

### B. Methyl 2-aminophenylacetate.

Methyl 2-nitrophenylacetate (10.1 g, 51.2 mmol) in 125 mL of methanol containing 221 mg of 10% Pd/C was placed on the Parr hydrogenator at 40 psi. After 5 h the mixture was filtered through celite and evaporated under reduced pressure to give a clear red oil. The solvent was evaporated under reduced pressure to give 8.5 g (100%) of methyl 2-aminophenylacetate as a clear red oil. TLC (silica, EtOAc/hexanes): R*_{f}* = 0.24. ¹H NMR (400MHz, CDCl₃): 7.21 (m, 2H), 6.86 (m, 2H), 3.81 (s, 3H), 3.70 (s, 2H).

### C. 4-(2-Oxo-2,3-dihydro-indol-1-yl)-piperidine-1-carboxylic acid tert-butyl ester.

Methyl 2-aminophenylacetate (3.0 g, 18.2 mmol) and 1-*tert*-butoxycarbonyl-4-piperidone (4.5 g, 22.6 mmol) were set stirring in 50 mL of CH₂Cl₂ under an atmosphere of nitrogen. Sodium triacetoxyborohydride (5.4 g, 25.5 mmol) was added followed by 1 mL of acetic acid. After 20 h at rt the mixture was quenched by the slow addition of saturated NaHCO₃. After stirring for 30 min, the organics were separated, dried (MgSO₄), and evaporated to afford 7.5 g of a purple oil. Purification by column chromatography (silica, 10-50% EtOAc/hexanes) gave 3.9 g (62%) of the title compound. TLC (silica, 25% EtOAc/hexanes): R*_{f}* = 0.15. ¹H NMR (400MHz, CDCl₃): 7.25 (m, 2H), 7.01 (m, 2H), 4.40 (m, 1 H), 3.53 (s, 2H), 2.83 (m, 2H), 2.32 (m, 2H), 1.70 (m, 2H), 1.51 (s, 9H).

### D. 1-Piperidin-4-yl-1,3-dihydro-indol-2-one.

4-(2-Oxo-2,3-dihydro-indol-1-yl)-piperidine-1-carboxylic acid tert-butyl ester (3.9 g, 12.3 mmol) was set stirring in 30 mL of 1:1 TFA/CH₂Cl₂. After 45 min the solvent was evaporated under reduced pressure to give a clear purple oil. The oil was brought up in diethyl ether and cooled on ice to give a precipitate. The solid was filtered, washed with ether and air dried to give 4.0 g (100%) of the title compound as a TFA salt. ¹H NMR (400MHz, DMSO-*d₆*): 8.6 (br s, 1H), 7.27 (m, 3H), 7.03 (m, 1H), 4.45 (m, 1H), 3.56 (s, 2H), 3.42 (m, 2H), 3.09 (m, 2H), 2.53 (m, 2H), 1.78 (m, 2H).

### E. 1-(1-{3-[5-Methanesulfonyl-3-(4-trifluoromethyl-phenyl)-4,5,6,7-tetrahydropyrazolo[4,3-c]pyridin-1-yl]-propyl}-piperidin-4-yl)-1,3-dihydro-indol-2-one.

3-[5-Methanesulfonyl-3-(4-trifluoromethyl-phenyl)-4,5,6,7-tetrahydropyrazolo[4,3-c]pyridin-1-yl]-propan-1-ol (304 mg, 0.754 mmol) was set stirring in 5 mL of CH₂Cl₂ at rt under nitrogen. Dess-Martin reagent (394 mg, 0.929 mmol) was added in one portion and the reaction mixture was left stirring. After 1.5 h the mixture was added to a stirring solution of thiosulphate (10 equiv) in 20 mL of water and 5 mL of saturated NaHCO₃. After 2 h the organic layer was separated, dried (MgSO₄) and evaporated to give the aldehyde as a light yellow solid. The above aldehyde (303 mg, 0.754 mmol) and 1-piperidin-4-yl-1,3-dihydro-indol-2-one were set stirring in 15 mL of CH₂Cl₂ containing Et₃N (0.15 mL, 1.1 mmol). A solution of Na(AcO)₃BH in 5 mL of CH₂Cl₂ was added dropwise via pipette over 10 min and the mixture was left to stir overnight. The mixture was quenched with saturated NaHCO₃ and the organic layer separated. The organics were dried (MgSO₄) and evaporated to a clear purple oil. Purification with column chromatography (silica, 50-100% acetone/CH₂Cl₂) gave 240 mg (53%) of a light pink solid. TLC (silica, 50% acetone/CH₂Cl₂): R*_{f}* = 0.17. ¹H NMR (400MHz, DMSO-*d*₆): 7.82 (m, 4H), 7.24 (m, 2H), 7.11 (m, 1H), 6.98 (m, 1H), 4.49 (s, 2H), 4.12 (m, 3H), 3.54 (s, 2H), 3.32 (s, 4H), 2.95 (m, 7H), 2.32 (m, 4H), 1.99 (m, 4H), 1.55 (m, 2H).

### EXAMPLE 12

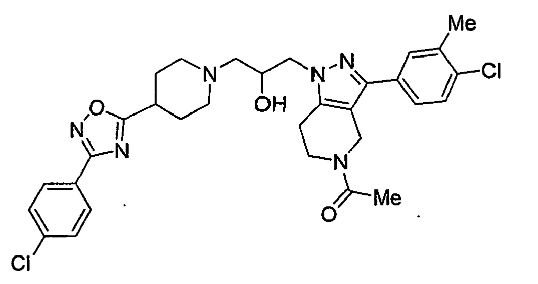

### 1-[3-(4-Chloro-3-methyl-phenyl)-1-(3-{4-[3-(4-chloro-phenyl)-[1,2,4]oxadiazol-5-yl]-piperidin-1-yl}-2-hydroxy-propyl)-1,4,6,7-tetrahydro-pyrazolo[4,3-c]pyridin-5-yl]-ethanone.

1-[3-(4-Chloro-3-methyl-phenyl)-1-oxiranylmethyl-1,4,6,7-tetrahydropyrazolo[4,3-c]pyridin-5-yl]-ethanone (0.069 g. 0.20 mmol) was dissolved in CH₂Cl₂ (1 mL), and 4-[3-(4-chlorophenyl)-1,2,4-oxadiazol-5-yl]piperidine (0.105 g, 0.4 mmol) was added, followed by Yb(OTf)₃ (0.031 g, 0.22 mmol). The mixture was stirred at room temperature for 16 h. Preparative TLC (silica, 7.5% MeOH/CH₂Cl₂) afforded 84 mg (69%) of the title compound. MS (electrospray): exact mass calculated for C₃₁H₃₄Cl₂N₆O₃, 608.21; *m*/*z* found, 609.2 [M⁺+H]. ¹H NMR (400 MHz, CDCl₃, 1:1 mixture of rotamers): 8.00 (d, *J* = 8.4 Hz, 2H), 7.56-7.53 (m, 1H), 7.48-7.42 (d, *J* = 8.6 Hz, 2H), 7.41-7.30 (m, 2H), 4.84 and 4.73 (A and B of AB quartet *J* = 15.6 Hz, 1 H), 4.62 (br s, 1H), 4.25-4.13 (m, 2H), 4.10-3.98 (m, 2H), 3.90-3.70 (m, 2H), 3.04-2.71 (m, 5H), 2.51-2.40 (m, 6H), 2.30-2.15 (m, 6H), 2.10-1.90 (m, 2H).

### EXAMPLE 13

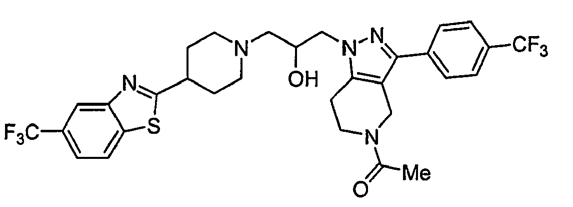

### 1-[1-{2-Hydroxy-3-[4-(5-trifluoromethyl-benzothiazol-2-yl)-piperidin-1-yl]-propyl}-3-(4-trifluoromethyl-phenyl)-1,4,6,7-tetrahydro-pyrazolo[4,3-c]pyridin-5-yl]-ethanone.

### A. 2-Piperidin-4-yl-5-trifluoromethyl-benzothiazole.

To a stirred solution of 5 g (29.2 mmol) of 1-acetyl-piperidine-4-carboylic acid in toluene (100 mL) and a catalytic amount of DMF (1 mL) was added dropwise 2.4 mL of oxalyl chloride (33.3 mmol). The reaction mixture was allowed to stir at room temperature overnight. A 20 mL aliquot (6 mmol) of the acid chloride solution was then placed in a separate flask and treated with a solution of 1.4 g (6.10 mmol) of 2-amino-4-(trifluoromethyl)thiophene hydrochloride in triethyl amine (4 mL). The reaction was then heated to 80°C for 30 min and then partitioned between ethyl acetate (50 mL) and water (20 mL) and separated. The aqueous layer was further extracted with EtOAc (2 x 30 mL). The combined organic layers were then washed with water (25 mL), brine, dried over Na₂SO₄, and the solvent was removed under reduced pressure. This was then heated to 60°C in a 1 *N* HCl/MeOH solution overnight with stirring. The reaction mixture was cooled and concentrated to dryness. The solid was then taken back up in 35 mL MeOH and stirred over sodium bicarbonate (1 g) for 1 h then filtered and stripped to give 1.05 g (60%) of the desired product which was used without further purification. MS (electrospray): exact mass calculated for C₁₃H₁₃F₃N₂S, 286.08; *m*/*z* found, 287.1 [M+H]⁺. ¹H NMR (CDCl₃, 400 MHz): 8.25 (s, 1H), 7.98 (d, *J* = 8.41 Hz, 1H), 7.65 (d, *J* = 8.41 Hz, 1H), 3.38 (tt, *J* = 11.35, 4.11 Hz, 1H), 3.28 (ddd, *J* = 13.69, 11.74, 2.74 Hz, 1H), 3.16 (ddd, *J* = 13.89, 11.15, 2.74 Hz, 1H), 2.85 (m, 1H), 2.25 (br m, 2H), 1.97 (br m, 2H).

### B. 1-[1-{2-Hydroxy-3-[4-(5-trifluoromethyl-benzothiazol-2-yl)-piperidin-1-yl]-propyl}-3-(4-trifluoromethyl-phenyl)-1,4,6,7-tetrahydro-pyrazolo[4,3-c]pyridin-5-yl]-ethanone.

A solution of 63 mg (0.22 mmol) 2-piperidin-4-yl-5-trifluoromethyl-benzothiazole was dissolved in 4 mL EtOH and treated with 40 mg (0.11 mmol) of 1-[1-oxiranylmethyl-3-(4-trifluoromethyl-phenyl)-1,4,6,7-tetrahydro-pyrazolo[4,3-c]pyridin-5-yl]-ethanone. The solution was heated to 60 °C overnight. The solvent was then removed by rotary evaporation and the crude product was purified by column chromatography (silica, 0-10% MeOH/EtOAc) to afford 57 mg (80%) of a white solid. MS (electrospray): exact mass calculated for C₃₁H₃₁F₆N₅O₂S, 651.21; *m*/*z* found, 652.2 [M+H]⁺. ¹H NMR (CDCl₃, 400 MHz, a mixture of amide rotamers): 8.24 (s, 1H), 7.97 (d, *J* = 8.41 Hz, 1H), 7.78 (d, *J* = 8.41 Hz, 1H), 7.70 (m, 2H), 7.65 (d, *J* = 8.41 Hz, 1H), 7.60 (dd, *J* = 8.41, 1.37 Hz, 1H), 4.88 and 4.76 (A and B of AB quartet, *J* = 15.85 Hz, 1H), 4.66 (br s, 1H), 4.25-4.15 (m, 2H), 4.08-3.99 (m, 1.5H), 3.91-3.83 (m, 0.5H), 3.82-3.68 (m, 1H), 3.16 (tt, *J* = 11.35, 3.52 Hz, 1H), 3.12-3.06 (m, 1H), 3.02-2.97 (m, 1H), 2.9-2.87 (m, 1.4H), 2.87-2.75 (m, 0.6H), 2.55-2.43 (m, 3H), 2.27-2.17 (m, 3H), 2.21 (s, 1.5H), 2.17 (s, 1.5H), 2.04-1.87 (m, 2H).

### EXAMPLE 14

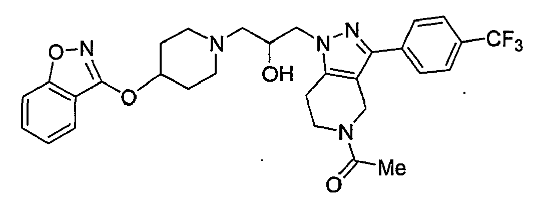

### 1-[1-{3-[4-(Benzo[d]isoxazol-3-yloxy)-piperidin-1-yl]-2-hydroxy-propyl}-3-(4-trifluoromethyl-phenyl)-1,4,6,7-tetrahydro-pyrazolo[4,3-c]pyridin-5-yl]-ethanone.

### A. 4-(Benzo[d]isoxazol-3-yloxy)-piperidine-1-carboxylic acid tert-butyl ester.

To a stirred solution of 263 mg of *t*-butyl-4-hydroxy-1-piperidinecarboxylate (1.3 mmol) in 5 mL of dry DMF was added 52 mg of 60% NaH in mineral oil (1.3 mmol). After stirring at room temperature for 10 min, 100 mg (0.65 mmol) of 3-chloro-1,2-benzisoxazole in DMF (1 mL) was added. The mixture was stirred at 40°C overnight and then partitioned between EtOAc (50 mL) and water (20 mL) and separated. The aqueous layer was further extracted with EtOAc (2 x 30 mL). The combined organic layers were then washed with water (25 mL), brine, dried over Na₂SO₄, and the solvent was removed under reduced pressure to give crude product. Purification by chromatography (silica, gradient elution of 40% hexanes/CH₂Cl₂ to 100%CH₂Cl₂) gave 176 mg (85%) product as a light yellow solid. MS (electrospray): exact mass calculated for C₁₇H₂₂N₂O₄, 318.16; *m*/*z* found, 341.1 [M+Na]⁺. ¹H NMR (CDCl₃, 400 MHz): 7.64 (dt, *J* = 8.02, 1.17 Hz, 1H), 7.53 (ddd, *J* = 8.41, 7.04, 1.17 Hz, 1H), 7.43 (dt, *J* = 8.41, 0.78 Hz, 1H), 7.27 (ddd, *J* = 8.02, 7.04, 0.78 Hz, 1H), 5.07 (m, 1H), 3.87-3.77 (br m, 2H), 3.30 (m, 2H), 2.17-2.10 (br m, 2H), 1.93-1.84 (br m, 2H), 1.48 (s, 9H).

B. 1-[1-{3-[4-(Benzo[d]isoxazol-3-yloxy)-piperidin-1-yl]-2-hydroxy-propyl}-3-(4-trifluoromethyl-phenyl)-1,4,6,7-tetrahydro-pyrazolo[4,3-c]pyridin-5-yl]-ethanone. A solution of 176 mg (0.55 mmol) of 4-(benzo[d]isoxazol-3-yloxy)-piperidine-1-carboxylic acid tert-butyl ester in CH₂Cl₂(2 mL) was treated with trifluoroacetic acid (0.5 mL) at room temperature overnight. The solvent was then removed and the crude product dissolved in methanol and stirred over 100 mg of sodium bicarbonate for 1 h, the solid was then filtered off and the filtrate concentrated. The crude piperidine was then dissolved in 4 mL EtOH and treated with 202 mg (0.55 mmol) of 1-[1-oxiranylmethyl-3-(4-trifluoromethyl-phenyl)-1,4,6,7-tetrahydro-pyrazolo[4,3-c]pyridin-5-yl]-ethanone The solution was heated to 60 °C overnight. The solvent was then removed by rotary evaporation and the crude product was purified by column chromatography (silica, 0-10% MeOH/EtOAc) to afford 220 mg (68%) of a white solid. MS (electrospray): exact mass calculated for C₃₀H₃₂F₃N₅O₄, 583.24; *m*/*z* found, 584.2 [M+H]⁺. ¹H NMR (CDCl₃, 400 MHz, a mixture of amide rotamers): 7.77 (d, *J* = 8.22 Hz, 1H), 7.69 (m, 2H), 7.66-7.61 (m, 2H), 7.54-7.49 (m, 1H), 7.41 (d, *J* = 8.41 Hz, 1H), 7.28-7.23 (m, 1H), 4.93 (br m, 1H), 4.88 and 4.75 (A and B of AB quartet, *J* = 15.65 Hz, 1H), 4.65 (br s, 1H), 4.24-4.18 (m, 0.75H), 4.18-4.09 (m, 1.25H), 4.07-3.98 (m, 1.5H), 3.91-3.79 (m, 0.5H), 3.79-3.67 (m, 1H), 3.02-2.85 (m, 2.4H), 2.85-2.70 (m, 1.6H), 2.61-2.52 (m, 1H), 2.51-2.40 (m, 2H), 2.39-2.30 (m, 1H), 2.24-2.12 (br m, 2H), 2.20 (s, 1.5H), 2.16 (s, 1.5H), 2.02-1.86 (m, 2H).

### EXAMPLE 15

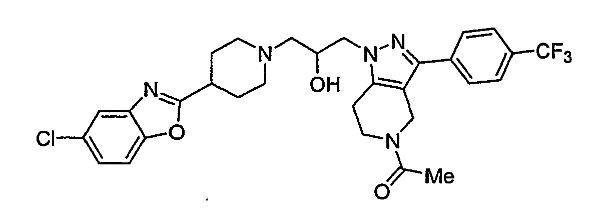

### 1-[1-{3-[4-(5-Chloro-benzooxazol-2-yl)-piperidin-1-yl]-2-hydroxy-propyl}-3-(4-trifluoromethyl-phenyl)-1,4,6,7-tetrahydro-pyrazolo[4,3-c]pyridin-5-yl]-ethanone.

### A. 5-Chloro-2-piperidin-4-yl-benzooxazole.

A flask was charged with 1.35 mL (10 mmol) of methyl isonipicotate, 1.43 g (10 mmol) of 2-amino-4-chlorophenol, and 5 g of polyphosphoric acid. The flask was then heated to 180 °C for 5 h. The reaction mixture was then poured into water while still warm and treated with 50% KOH solution until pH 12. This was then extracted with CH₂Cl₂ (3 x 50 mL), then washed with water (25 mL), brine, dried over Na₂SO₄, and the solvent was removed under reduced pressure to give 1.53 g (57%) of crude product which was used without further purification. MS (electrospray): exact mass calculated for C₁₂H₁₃ClN₂O, 236.07; *m*/*z* found, 237.1 [M+H]⁺.

### B. 1-[1-{3-[4-(5-Chloro-benzooxazol-2-yl)-piperidin-1-yl]-2-hydroxy-propyl}-3-(4-trifluoromethyl-phenyl)-1,4,6,7-tetrahydro-pyrazolo[4,3-c]pyridin-5-yl]-ethanone.

A solution of 130 mg (0.55 mmol) of 5-chloro-2-piperidin-4-yl-benzooxazole was dissolved in 4 mL EtOH and treated with 100 mg (0.27 mmol) of 1-[1-oxiranylmethyl-3-(4-trifluoromethyl-phenyl)-1,4,6,7-tetrahydro-pyrazolo[4,3-c]pyridin-5-yl]-ethanone. The solution was heated to 60 °C overnight. The solvent was then removed by rotary evaporation and the crude product was purified by column chromatography (silica, 0-10% MeOH/EtOAc) to afford 156 mg (95%) of a white solid. MS (electrospray): exact mass calculated for C₃₀H₃₁ClF₃N₅O₃, 601.21; *m*/*z* found, 602.2 [M+H]⁺. ¹H NMR (CDCl₃, 400 MHz, a mixture of amide rotamers):7.76 (d, *J* = 8.41 Hz, 1H), 7.71 and 7.67 (A and B of AB quartet, *J* = 8.41 Hz, 2H), 7.65-7.61 (m, 2H), 7.38 (d, *J* = 8.61 Hz, 1H), 7.26 (dd, *J* = 8.61, 1.96, 1H), 4.86 and 4.74 (A and B of AB quartet, *J* = 15.65 Hz, 1H), 4.64 (br s, 1H), 4.24-4.10 (m, 2.3H), 4.07-3.97 (m, 1.7H), 3.89-3.67 (m, 2H), 3.06-3.00 (m, 1H), 3.00-2.90 (m, 2H), 2.90-2.74 (m, 2H), 2.51-2.38 (m, 3H), 2.25-2.10 (m, 2.3H), 2.20 (s, 1.5H), 2.15 (s, 1.5H), 2.06-1.83 (m, 2.7H).

### EXAMPLE 16

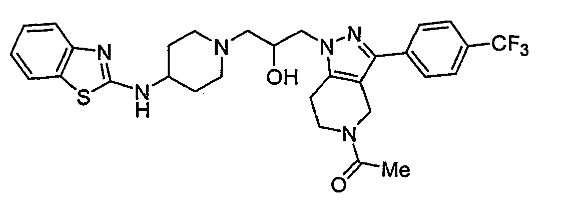

### 1-[1-{3-[4-(Benzothiazol-2-ylamino)-piperidin-1-yl]-2-hydroxy-propyl}-3-(4-trifluoromethyl-phenyl}-1,4,6,7-tetrahydro-pyrazolo[4,3-c]pyridin-5-yl]-ethanone.

### A. 4-(Benzothiazol-2-ylamino}-piperidine-1-carboxylic acid tert-butyl ester.

To a stirred solution of 300 mg (1.77 mmol) of 2-chlorobenzothiazole in dry DMF (3.5 mL) was added 2.9 g of cesium carbonate (8.8 mmol) and 535 mg of *tert-*butyl-4-hydroxy-1-piperidinecarboxylate (2.66 mmol). The mixture was stirred at room temperature for 4 h before it was partitioned between EtOAc (70 mL) and water (30 mL) and separated. The aqueous layer was further extracted with EtOAc (2 x 50 mL). The combined organic layers were washed with water (25 mL), brine, dried over Na₂SO₄, and the solvent was removed under reduced pressure. Purification by flash chromatography (silica, 0-15% EtOAc/hexanes) afforded 220 mg (37%) of the desired product as a white solid. MS (electrospray): exact mass calculated for C₁₇H₂₃N₃O₂S, 333.15; *m*/*z* found, 334.2 [M+H]⁺. ¹H NMR (CDCl₃, 400 MHz): 7.65 (t, *J* = 7.63 2H), 7.36 (ddd, *J* = 8.41, 7.43, 1.37 Hz, 1H), 7.22 (dt, *J* = 7.63, 1.17 Hz, 1H), 5,36 (m, 1H), 3.79-3.70 (br m, 2H), 3.36 (m, 2H), 2.12-2.04 (br m, 2H), 1.92-1.82 (br m, 2H), 1.48 (s, 9H).

B. 1-[1-{3-[4-(Benzothiazol-2-ylamino)-piperidin-1-yl]-2-hydroxy-propyl}-3-(4-trifluoromethyl-phenyl)-1,4,6,7-tetrahydro-pyrazolo[4,3-c]pyridin-5-yl]-ethanone. A solution of 220 mg (0.66 mmol) of 4-(benzothiazol-2-ylamino)-piperidine-1-carboxylic acid tert-butyl ester in dichloromethane (2 mL) was treated with trifluoroacetic acid (0.5 mL) at room temperature overnight. The solvent was then removed and the crude product dissolved in MeOH and stirred over 100 mg of sodium bicarbonate for 1 h. The solid was filtered off and the filtrate concentrated. The crude piperidine was then dissolved in 4 mL EtOH and treated with 220 mg (0.60 mmol) of 1-[1-oxiranylmethyl-3-(4-trifluoromethylphenyl)-1,4,6,7-tetrahydro-pyrazolo[4,3-c]pyridin-5-yl]-ethanone. The solution was heated to 60 °C overnight. The solvent was then removed by rotary evaporation and the crude product was purified by column chromatography (silica, 0-10% MeOH/EtOAc) to afford 240 mg (66%) of a white solid. MS (electrospray): exact mass calculated for C₃₀H₃₃F₃N₆O₂S: 598.23; *m*/*z* found, 599.3 [M+H]⁺. ¹H NMR (CDCl₃, 400 MHz, a mixture of amide rotamers): 7.78 (d, *J* = 8.22 Hz, 1H), 7.72 and 7.68 (A and B of AB quartet, *J* = 8.41 Hz, 2H), 7.64 (d, *J* = 8.22 Hz, 1H), 7.56 (bd, *J* = 8.02 Hz, 1H), 7.51 (bd, *J* = 8.02 Hz, 1H), 7.29 (bd, *J* = 7.63 Hz, 1H), 7.08 (bt, *J* = 7.63 Hz, 1H), 5.29 (br s, 1H), 4.88 and 4.75 (A and B of AB quartet, *J* = 15.65 Hz, 1H), 4.65 (br s, 1 H), 4.23-4.16 (m, 1H), 4.16-4.08 (m, 1H), 4.06-3.98 (m, 2H), 3.92-3.65 (m, 3H), 3.03-2.70 (m, 4H), 2.52-2.41 (m, 3H), 2.26-2.18 (m, 1H), 2.21 (s, 1.5H), 2.16 (s, 1.5H), 2.16-2.08 (m, 2H), 1.66-1.44 (m, 2H).

### EXAMPLE 17

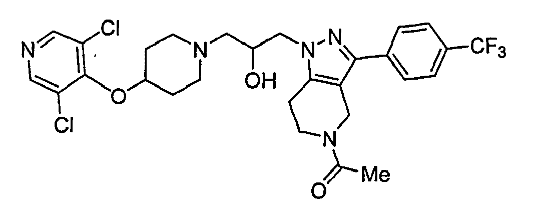

### 1-[1-{3-[4-(3,5-Dichloro-pyridin-4-yloxy)-piperidin-1-yl]-2-hydroxy-propyl}-3-(4-trifluoromethyl-phenyl)-1,4,6,7-tetrahydro-pyrazolo[4,3-c]pyridin-5-yl]-ethanone.

A. 4-(3,5-Dichloro-pyridin-4-yloxy)-piperidine-1-carboxylic acid tert-butyl ester. To a stirred solution of 828 mg (4.12 mmol) of *tert*-butyl-4-hydroxy-1-piperidinecarboxylate in 10 mL of dry DMF was added 165 mg of 60% NaH in mineral oil (4.12 mmol). After stirring at room temperature for 10 min, 500 mg (2.74 mmol) of 3,4,5-trichloropyridine was added. The mixture was stirred at 80 °C overnight and then partitioned between EtOAc (50 mL) and water (20 mL) and separated. The aqueous layer was further extracted with EtOAc (2 x 30 mL). The combined organic layers were washed with water (25 mL), brine, dried over Na₂SO₄, and the solvent was removed under reduced pressure. Column chromatography (silica, 60-100% CH₂Cl₂/hexanes) gave 265 mg (28%) of desired product. MS (electrospray): exact mass calculated for C₁₅H₂₀Cl₂N₂O₃, 346.09; *m*/*z* found, 369.1 [M+Na]⁺. ¹H NMR (CDCl₃, 400 MHz): 8.45 (s, 2H), 4.66 (m, 1H), 3.90-3.80 (br m, 2H), 3.26 (m, 2H), 1.96-1.83 (br m, 4H),1.47 (s, 9H).

### B. 1-[1-{3-[4-(3,5-Dichloro-pyridin-4-yloxy)-piperidin-1-yl]-2-hydroxy-propyl}-3-(4-trifluoromethyl-phenyl)-1,4,6,7-tetrahydro-pyrazolo[4,3-c]pyridin-5-yl]-ethanone.

A solution of 103 mg (0.30 mmol) of 4-(3,5-dichloro-pyridin-4-yloxy)-piperidine-1-carboxylic acid tert-butyl ester in CH₂Cl₂ (2 mL) was treated with trifluoroacetic acid (0.5 mL) at room temperature overnight. The solvent was then removed and the crude product dissolved in MeOH and stirred over 100 mg of sodium bicarbonate for 1 h. The solid was filtered off and the filtrate concentrated. The crude piperidine was then dissolved in 4 mL EtOH and treated with 100 mg (0.27 mmol) of 1-[1-oxiranylmethyl-3-(4-trifluoromethylphenyl)-1,4,6,7-tetrahydro-pyrazolo[4,3-c]pyridin-5-yl]-ethanone. The solution was heated to 60°C overnight. The solvent was then removed by rotary evaporation and the crude product was purified by column chromatography (silica, 0-10% MeOH/EtOAc) to afford 90 mg (54%) of a white solid. MS (electrospray): exact mass calculated for C₂₈H₃₀Cl₂F₃N₅O₃, 611.17; *m*/*z* found, 612.2 [M+H]⁺. ¹H NMR (CDCl₃, 400 MHz, a mixture of amide rotamers): 8.44 (s, 2H), 7.77 (d, *J* = 8.41 Hz, 1H), 7.72 and 7.68 (A and B of AB quartet, *J* = 8.41 Hz, 2H), 7.65 (d, *J* = 8.41 Hz, 1H), 4.88 and 4.76 (A and B of AB quartet, *J* = 15.65 Hz, 1 H), 4.66 (br s, 1H), 4.55 (br s, 1 H), 4.26-4.08 (m, 2H), 4.08-3.98 (m, 2H), 3.91-3.69 (m, 2H), 3.03-2.92 (m, 1.6H), 2.91-2.85 (m, 0.8H), 2.85-2.75 (m, 1.6H), 2.52-2.40 (m, 3H), 2.35-2.24 (br m, 1H), 2.22 (s, 1.5H), 2.17 (s, 1.5H), 2.03-1.90 (m, 4H).

### EXAMPLE 18

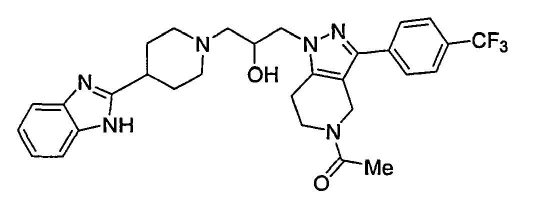

### 1-[1-{3-[4-(1 H-Benzoimidazol-2-yl)-piperidin-1-yl]-2-hydroxy-propyl}-3-(4-trifluoromethyl-phenyl)-1,4,6,7-tetrahydro-pyrazolo[4,3-c]pyridin-5-yl]-ethanone.

### A. 2-Piperidin-4-yl-1H-benzoimidazole.

A flask was charged with 1.35 mL (10 mmol) of methyl isonipicotate, 1.0 g (10 mmol) of 1,2-phenylenediamine and 5 g of polyphosphoric acid. The flask was then heated to 180 °C for 5 h. The reaction mixture was then poured into water while still warm and treated with 50% KOH solution until pH 12. This was then extracted with CH₂Cl₂ (3 x 50 mL), washed with water (25 mL), brine, dried over Na₂SO₄, and the solvent was removed under reduced pressure to give 530 mg (27%) of crude product which was used without further purification. MS (electrospray): exact mass calculated for C₁₂H₁₅N₃, 201.13; *m*/*z* found, 202.1 [M+H]⁺. ¹H NMR (400 MHz, DMSO-*d*₆):12.1 (br s, 1H), 7.49 (br m, 1H), 7.38 (br m, 1H), 7.09 (br m, 2H), 3.00 (dt, *J* = 12.13, 3.33 Hz, 2H), 2.88 (tt, *J* = 11.54, 3.74 Hz, 1H), 2.57 (dt, *J* = 12.13, 2.35 Hz, 2H), 1.90 (m, 2H), 1.66 (m, 2H).

### B. 1-[1-{3-[4-(1 H-Benzoimidazol-2-yl)-piperidin-1-yl]-2-hydroxy-propyl}-3-(4-trifluoromethyl-phenyl)-1,4,6,7-tetrahydro-pyrazolo[4,3-c]pyridin-5-yl]-ethanone.

A solution of 83 mg (0.41 mmol) of 2-piperidin-4-yl-1H-benzoimidazole was dissolved in 4 mL EtOH and treated with 100 mg (0.27 mmol) of 1-[1-oxiranylmethyl-3-(4-trifluoromethyl-phenyl)-1,4,6,7-tetrahydro-pyrazolo[4,3-c]pyridin-5-yl]-ethanone. The solution was heated to 60 °C overnight. The solvent was then removed by rotary evaporation and the crude product was purified by column chromatography (silica, 0-10% MeOH/EtOAc) to afford 55 mg (36%) of a white solid. MS (electrospray): exact mass calculated for C₃₀H₃₃F₃N₆O₂, 566.26; *m*/*z* found, 567.3 [M+H]⁺. ¹H NMR (CDCl₃, 400 MHz, a mixture of amide rotamers):10.66 (br s, 0.5H), 10.57 (br s, 0.5H), 7.73 (bd, *J* = 8.41 Hz, 1H), 7.72-7.63 (m, 3H), 7.60 (bd, *J* = 8.41 Hz, 1H), 7.39-7.32 (m, 1H), 7.23-7.13 (m, 2H), 7.02 (br s, 1), 4.86 and 4.75 (A and B of AB quartet, *J* = 15.85 Hz, 1.25H), 4.64 (br s, 1H), 4.21-4.06 (m, 2H), 4.06-3.81 (m, 2H), 3.80-3.63 (m, 1H), 3.80-3.69 (m, 1H), 3.00-2.68 (m, 5H), 2.44-2.36 (m, 2H), 2.39-2.23 (m, 2H), 2.19 (s, 1.6H), 2.15 (s, 1.4H), 2.13-2.00 (m, 4H), 2.00-1.80 (m, 2H).

### EXAMPLE 19

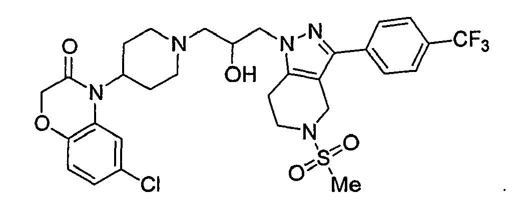

### 6-Chloro-4-(1-{2-hydroxy-3-[5-methanesulfonyl-3-(4-trifluoromethyl-phenyl)-4,5,6,7-tetrahydro-pyrazolo[4,3-c]pyridin-1-yl]-propyl}-piperidin-4-yl)-4H-benzo[1,4]oxazin-3-one.

### A. 5-Methanesulfonyl-1-oxiranylmethyl-3-(4-trifluoromethyl-phenyl)-4,5,6,7-tetrahydro-1H-pyrazolo[4,3-c]pyridine.

5-Methanesulfonyl-3-(4-trifluoromethyl-phenyl)-4,5,6,7-tetrahydro-1H-pyrazolo[4,3-c]pyridine (10.0 g, 29.0 mmol) and epichlorohydrin (24 mL, 307 mmol) were set stirring in DMF (150 mL) containing Cs₂CO₃ (10.4 g, 31.9 mmol). After stirring at room temperature for 4 days the mixture was evaporated, brought up in EtOAc and washed with waster. The organics were dried (MgSO₄) and evaporated to give a light yellow solid. Column chromatography (silica, 5% acetone/CH₂Cl₂) gave 4.1 g (35%) of a white solid. TLC (silica, 5% acetone/CH₂Cl₂): R*_{f}* = 0.28. MS (electrospray): exact mass calculated for C₁₇H₁₈F₃N₃O₃S, 401.10; *m*/*z* found, 402.1 [M+H]⁺. ¹H NMR (400 MHz, CDCl₃); 7.84 (d, *J* = 8.3 Hz, 2H), 7.79 (d, *J* = 8.3 Hz, 2H), 4.70-4.62 (m, 3H), 4.25 (d, *J* = 5.4 Hz, 1H), 3.90-3.70 (m, 2H), 3.47 (m, 1H), 3.10-2.9 (m, 6H), 2.65-2.60 (m, 1H).

### B. 4-(5-Chloro-2-hydroxy-phenylamino)-piperidine-1-carboxylic acid tert-butyl ester.

2-Amino-4-chloro-phenol (30.0 g, 209 mmol) and 4-oxo-piperidine-1-carboxylic acid tert-butyl ester (46.0 g, 231 mmol) were set stirring in dichloromethane (600 mL). Sodium triacetoxyborohydride (58.0 g, 274 mmol) was added in portions over 10 min. Acetic acid (12 mL, 210 mmol) was then added and the mixture left to stir for 18 h. Saturated NaHCO₃ was added and the organics seperated. The organics were dried (MgSO₄) and evaporated to give 56 g (82%) of a light beige solid. TLC (silica, 50% EtOAc/hexanes): R*_{f}* = 0.66. MS (electrospray): exact mass calculated for C₁₆H₂₃ClN₂O₃, 326.14; *m*/*z* found, 349.1 [M+Na]⁺ . ¹H NMR (400 MHz, DMSO-*d*₆): 6.70 (d, *J* = 8.3 Hz, 1H), 6.63 (s, 1H), 6.47 (d, *J* = 8.2 Hz, 1H), 3.97 (d, *J* = 12.2 Hz, 2H), 3.55-3.50 (m, 1H), 2.93 (br s, 2H), 1.93 (d, *J* = 11.1 Hz, 2H), 1.48 (s, 9H), 1.35 (d, *J* = 11.2 Hz, 2H).

### C. 4-[(5-Chloro-2-hydroxy-phenyl)-ethoxycarbonylmethyl-amino]-piperidine-1-carboxylic acid tert-butyl ester.

4-(5-Chloro-2-hydroxy-phenylamino)-piperidine-1-carboxylic acid tert-butyl ester (15.6 g, 47.7 mmol) was set stirring in THF (200 mL) and cooled to 5 °C. Sodium hydride (1.37 g, 54.2 mmol) was added in portions over 10 min and the mixture left to stir for 1 h. Ethyl bromoacetate (5.8 mL, 52.3 mmol) was added and the ice bath removed. After stirring for 20 h the mixture was evaporated, brought up in EtOAc and washed with water. The organics were dried (MgSO₄) and evaporated to give 22.5 g of a deep red oil. The oil was purified (silica, 5% acetone/CH₂Cl₂) to give 12.9 g (65%) of a clear orange liquid. TLC (silica, 5% acetone/CH₂Cl₂): R*_{f}* = 0.43. MS (electrospray): exact mass calculated for C₂₀H₂₉ClN₂O₅, 412.18; *m*/*z* found, 413.2 [M+H]⁺. ¹H NMR (400 MHz, CDCl₃): 6.75-6.62 (m, 3H), 4.71 (s, 1H), 4.37 (q, *J* = 7.2 Hz, 2H), 4.14 (br s, 2H), 3.55-3.50 (m, 1H), 3.08 (br t, 2H), 2.14 (m, 2H), 1.65-1.45 (m, 12H), 1.41 (t, *J* = 7.2 Hz, 3H).

### D. 4-(6-Chloro-3-oxo-2,3-dihydro-benzo[1,4]oxazin-4-yl)-piperidine-1-carboxylic acid tert-butyl ester.

4-[(5-Chloro-2-hydroxy-phenyl)-ethoxycarbonylmethyl-amino]-piperidine-1-carboxylic acid tert-butyl ester (12.9 g, 31.2 mmol) was set stirring in MeOH (100 mL). A solution of NaOH (2.5 g, 62.5 mmol) in water (100 mL) was added and the mixture stirred at room temperature for 3 h. The mixture was acidified to pH 2 and MeOH evaporated. The aqueous layer was extracted twice with EtOAc. The organics were combined, dried (MgSO₄) and evaporated to give 11 g of a clear orange oil. The oil was set stirring in CH₂Cl₂ (150 mL) and EDC (8.2 g, 42.8 mmol) was added. After 1 h the organics were washed with 1 *N* HCl (100 mL), water (100 mL) and dried (MgSO₄). The solvent was evaporated to give 7.2 g (63%) of a clear orange solid. TLC (silica, 5% acetone/CH₂Cl₂): R*_{f}* = 0.53. MS (electrospray): exact mass calculated for C₁₈H₂₃ClN₂O₄, 366.13; *m*/*z* found, 389.1 [M+Na]⁺. ¹H NMR (400 MHz, CDCl₃): 7.19 (s, 1H), 7.11-7.00 (m, 2H), 4.60 (s, 2H), 4.50-4.30 (m, 3H), 3.00-2.80 (m, 2H), 2.70-2.60 (m, 2H), 1.86 (d, *J* = 11.4 Hz, 2H), 1.60 (s, 9H).

### E. 6-Chloro-4-piperidin-4-yl-4H-benzo[1,4]oxazin-3-one.

4-(6-Chloro-3-oxo-2,3-dihydro-benzo[1,4]oxazin-4-yl)-piperidine-1-carboxylic acid tert-butyl ester (7.2 g, 19.6 mmol) was set stirring and a 1:1 TFA/CH₂Cl₂ solvent mixture was added. After 1 h the mixture was evaporated under reduced pressure and the resulting red oil brought up in Et₂O. A solid formed and was filtered and air dried to give 7.2 g (96%) of a light beige solid. MS (electrospray): exact mass calculated for C₁₃H₁₅ClN₂O₂, 266.08; *m*/*z* found, 267.1 [M+H]⁺. ¹H NMR (400 MHz, CD₃OD): 7.52 (s, 1H), 7.20-7.00 (m, 2H), 4.60 (s, 2H), 4.50-4.40 (m, 1H), 3.65-3.55 (m, 2H), 3.28 (t, *J* = 13.1 Hz, 2H), 3.10-3.00 (m, 2H), 2.15 (d, *J* = 13.9 Hz, 2H).

### F. 6-Chloro-4-(1-{2-hydroxy-3-[5-methanesulfonyl-3-(4-trifluoromethyl-phenyl)-4,5,6,7-tetrahydro-pyrazolo[4.3-c]pyridin-1-yl]-propyl}-piperidin-4-yl)-4H-benzo[1,4]oxazin-3-one.

6-Chloro-4-piperidin-4-yl-4H-benzo[1,4]oxazin-3-one (252 mg, 0.66 mmol) and 5-methanesulfonyl-1-oxiranylmethyl-3-(4-trifluoromethyl-phenyl)-4,5,6,7-tetrahydro-1H-pyrazolo[4,3-c]pyridine (209 mg, 0.52 mmol) were set stirring in EtOH (10 mL) containing Et₃N (115 µL, 0.83 mmol) at 70°C. After 2 days the mixture was cooled, evaporated, brought up in EtOAc and washed with saturated NaHCO₃. The organics were dried (MgSO₄) and evaporated to give a clear golden oil. The oil was purified (silica, 50% acetone/CH₂Cl₂) to give 191 mg (55%) of a white solid. TLC (silica, 50% acetone/CH₂Cl₂): R*_{f}* = 0.38. MS (electrospray): exact mass calculated for C₃₀H₃₃ClF₃N₅O₅S, 667.18; m/z found, 668.2 [M+H]⁺. ¹H NMR (400 MHz, CDCl₃): 7.83 (d, J = 8.3 Hz, 2H), 7.77 (d, J = 8.3 Hz, 2H), 7.21 (s, 1H), 7.10-7.00 (m, 2H), 4.68 (d, J = 5.1 Hz, 2H), 4.58 (s, 2H), 4.40-4.10 (m, 4H), 3.90-3.70 (s, 2H), 3.30-3.0 (m, 4H), 3.00 (s, 3H), 2.90-2.70 (m, 2H), 2.65-2.50 (m, 3H), 2.35-2.20 (m, 2H), 1.88 (d, J = 11.3 Hz, 2H).

### EXAMPLE 20

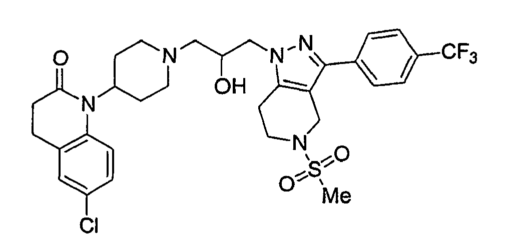

### 6-Chloro-1-(1-{2-hydroxy-3-[5-methanesulfonyl-3-(4-trifluoromethyl-phenyl)-4,5,6,7-tetrahydro-pyrazolo[4,3-c]pyridin-1-yl]-propyl}-piperidin-4-yl)-3,4-dihydro-1H-quinolin-2-one.

### A. 3-(2-Amino-5-chloro-phenyl)-acrylic acid ethyl ester.

2-Amino-5-chlorobenzaldehyde (7.58 g, 48.7 mmol) and 36 g (103 mmol) of (carbethoxymethylene)triphenylphosphorane were added in benzene (300 mL) and heated to reflux for 20 h. The reaction mixture was cooled and concentrated to give an orange oil. The oil was brought up in Et₂O and precipitated appeared. This was filtered and washed with Et₂O. The organics were evaporated to give a clear orange oil. The oil was purified by column chromatography (silica, 10-40% EtOAc/hexanes) to obtain 10.4 g (95%) of a yellow solid. MS (electrospray): exact mass calculated for C₁₁H₁₂ClNO₂, 225.06; m/z found, 226.1 [M⁺+H]. ¹H NMR (400 MHz, CDCl₃): 7.69 (d, J = 15.85 Hz, 1H), 7.30 (d, J = 2.54 Hz, 1H), 7.07 (dd, J = 6.26 Hz, 2.35 Hz, 1H), 6.60 (d, J = 8.61 Hz, 1H), 6.30 (d, J = 15.85 Hz, 1H), 4.22 (dd, J = 7.24 Hz, 7.24 Hz, 2H), 3.98 (br s, 2H), 1.30 (t, J = 7.04 Hz, 3H).

### B. 4-[4-Chloro-2-(2-ethoxycarbonyl-vinyl)-phenylamino]-piperidine-1-carboxylic acid tert-butyl ester.

3-(2-Amino-5-chloro-phenyl)-acrylic acid ethyl ester (10.4 g, 46 mmol) and 4-oxo-piperidine-1-carboxylic acid tert-butyl ester (13.8 g, 69 mmol) were set stirring in CH₂Cl₂ (230 mL). Sodium triacetoxyborohydride (14.6 g, 69 mmol) was added in portions over 10 min. Acetic acid (1.3 mL, 25 mmol) was then added and the mixture left to stir. After 18 h saturated NaHCO₃ was added and the organics separated. The organics were dried over Na₂SO₄ and concentrated. The residue was purified by column chromatography (silica, 20-50% EtOAc/hexanes) to obtain 12.4 g (66%) of a light beige solid. TLC (silica, 25% EtOAc/hexanes): R*_{f}* = 0.5. MS (electrospray): exact mass calculated for C₂₁H₂₉ClN₂O₄, 408.18; *m*/*z* found, 409.1 [M⁺+H]. ¹H NMR (400 MHz, CDCl₃): 7.64 (d, *J* = 15.65 Hz, 1H), 7.29 (d, *J* = 2.35 Hz, 1H), 7.14 (dd, *J* = 6.26 Hz, 2.54 Hz, 1H), 6.59 (d, *J* = 9.00 Hz, 1H), 6.28 (d, *J* = 15.65 Hz, 1H), 4.23 (dd, *J* = 7.04 Hz, 7.04 Hz, 2H), 4.11-3.98 (m, 2H), 3.81 (br s, 1H), 3.46-3.36 (m, 1H), 2.89 (t, *J* = 11.74 Hz, 2H), 2.04-1.95 (m, 2H), 1.44 (s, 9H), 1.42-1.33 (m, 2H), 1.30 (t, *J* = 7.24 Hz, 3H).

### C. 4-[4-Chloro-2-(2-ethoxycarbonyl-ethyl)-phenylamino]-piperidine-1-carboxylic acid tert-butyl ester.

4-[4-Chloro-2-(2-ethoxycarbonyl-vinyl)-phenylamino]-piperidine-1-carboxylic acid tert-butyl ester (12.4 g, 30.4 mmol) in EtOAc (150 mL) containing PtO₂ (1 g) was placed on a Parr hydrogenator at 60 psi H₂. After 18 h the mixture was filtered through celite and evaporated to give a clear brown liquid. The liquid was purified by column chromatography (silica, 20-50% EtOAc/hexanes) to obtain 5.7 g (46%) of the title compound. TLC (silica, 25% EtOAc/hexanes): R*_{f}* = 0.5. MS (electrospray): exact mass calculated for C₂₁H₃₁ClN₂O₄, 410.2; *m*/*z* found, 411.2 [M⁺+H]. ¹H NMR (400 MHz, CDCl₃): 7.05 (dd, *J* = 6.06 Hz, 2.54 Hz, 1H), 6.99 (d, *J* = 2.35 Hz, 1H), 6.55 (d, *J* = 8.61 Hz, 1H), 4.13 (dd, *J* = 7.04 Hz, 3.13 Hz, 2H), 4.11-3.98 (m, 2H), 3.81 (br s, 1H), 3.72 (t, *J* = 6.26 Hz, 2H), 3.46-3.36 (m, 1H), 2.75 (t, *J* = 7.43 Hz, 2H), 2.60 (t, *J* = 7.04, 2H), 2.04-1.95 (m, 2H), 1.46 (s, 9H), 1.42-1.33 (m, 2H), 1.26 (t, *J* = 7.24 Hz, 3H).

### D. 4-[2-(2-Carboxy-ethyl)-4-chloro-phenylamino]-piperidine-1-carboxylic acid tert-butyl ester.

4-[4-Chloro-2-(2-ethoxycarbonyl-ethyl)-phenylamino]-piperidine-1-carboxylic acid tert-butyl ester (5.7 g, 13.9 mmol) was set stirring in MeOH (40 mL). A solution of NaOH (1.4 g, 34.7 mmol) in water (10 mL) was added and the mixture stirred at room temperature. After 3 h the mixture was acidified to pH 7 and MeOH was evaporated. The aqueous layer was extracted with CH₂Cl₂ (3 x 100 mL). The organics were combined, dried over Na₂SO₄ and concentrated to afford 3.9 g (73%) of the desired product. TLC (silica, 50% EtOAc/hexanes): R*_{f}* = 0.4. MS (electrospray): exact mass calculated for C₁₉H₂₇ClN₂O₄, 382.17; *m*/*z* found, 381.1 [M⁻-H].

### E. 4-(6-Chloro-2-oxo-3,4-dihydro-2H-quinolin-1-yl-piperidine-1-carboxylic acid tert-butyl ester.

4-[2-(2-Carboxy-ethyl)-4-chloro-phenylamino]-piperidine-1-carboxylic acid tert-butyl ester (3.9 g, 10.1 mmol) and EDC (2.9 g, 15.3 mmol) were set stirring in CH₂Cl₂ (50 mL) for 2 h. The reaction mixture was dissolved in CH₂Cl₂ (150 mL), washed with water (2 x 50 mL) and brine (1 x 50 mL). The organic layer was dried over Na₂SO₄ and concentrated. The residue was purified by column chromatography (silica, 30-50% EtOAc/hexanes) to obtain 1.9 g (52%) of the desired product. TLC (silica, 50% EtOAc/hexanes): R*_{f}* = 0.67. MS (electrospray): exact mass calculated for C₁₉H₂₅ClN₂O₃, 364.16; *m*/*z* found, 365.1 [M⁺+H]. ¹H NMR (400 MHz, CDCl₃): 7.14-7.08 (m, 2H), 6.98 (d, *J* = 8.61 Hz, 1H), 4.33-3.98 (m, 3H), 2.75 (t, *J* = 7.83 Hz, 4H), 2.55-2.36 (m, 4H), 1.70-1.65 (m, 2H), 1.44 (s, 9H).

### F. 6-Chloro-1-piperidin-4-yl-3,4-dihydro-1H-quinolin-2-one.

4-(6-Chloro-2-oxo-3,4-dihydro-2H-quinolin-1-yl)-piperidine-1-carboxylic acid tert-butyl ester (1.2 g, 3.28 mmol) was set stirring in 1:1 TFA/CH₂Cl₂. After 45 min the mixture was evaporated and the golden oil brought up in Et₂O. A solid formed and was filtered, washed with Et₂O and air dried to give 1.3 g (93%) of a white solid. MS (electrospray): exact mass calculated for C₁₃H₁₇ClN₂O, 264.10; *m*/*z* found, 265.1 [M⁺+H].

### G. 6-Chloro-1-(1-{2-hydroxy-3-[5-methanesulfonyl-3-(4-trifluoromethyl-phenyl)-4,5,6,7-tetrahydro-pyrazolo[4,3-c]pyridin-1-yl]-propyl}piperidin-4-yl)-3,4-dihydro-1H-quinolin-2-one.

6-Chloro-1-piperidin-4-yl-3,4-dihydro-1H-quinolin-2-one (270 mg, 0.62 mmol) and 5-methanesuffonyl-1-oxiranylmethyl-3-(4-trifluoromethyl-phenyl)-4,5,6,7-tetrahydro-1H-pyrazolo[4,3-c]pyridine (165 mg, 0.41 mmol) were set stirring in EtOH (10 mL) containing Et₃N (97 µL, 0.70 mmol) at 80°C. After 16 h the mixture was cooled, evaporated, brought up in dichloromethane and washed with water. The organics were dried over Na₂SO₄ and concentrated. The residue was purified by column chromatography (silica, 5-10% MeOH/CH₂Cl₂) to obtain 205 mg (75%) of a white solid. TLC (silica, 10% MeOH/CH₂Cl₂): R*_{f}* = 0.75. MS (electrospray): exact mass calculated for C₃₁H₃₅ClF₃N₅O₄S, 665.21; *m*/*z* found, 666.2 [M⁺+H]. ¹H NMR (CDCl₃, 400 MHz): 7.69 (d, *J* = 8.41 Hz, 2H), 7.62 (d, *J* = 8.41 Hz, 2H), 7.16-7.08 (m, 2H), 7.00 (d, *J* = 9.00 Hz, 1H), 4.52 (dd, *J* = 14.28 Hz, 5.48 Hz, 2H), 4.18 (dd, *J* = 10.56 Hz, 3.13 Hz, 1H), 4.14-4.04 (m, 2H), 4.03-3.96 (m, 1H), 3.72-3.56 (m, 2H), 3.10-2.96 (m, 2H), 2.95-2.86 (m, 2H), 2.85 (s, 3H), 2.75 (t, *J* = 6.26 Hz, 2H), 2.69-2.54 (m, 2H), 2.53-2.47 (m, 2H), 2.44-2.31 (m, 3H), 2.15-2.05 (m, 1H), 1.71-1.62 (m, 2H).

### EXAMPLE 21

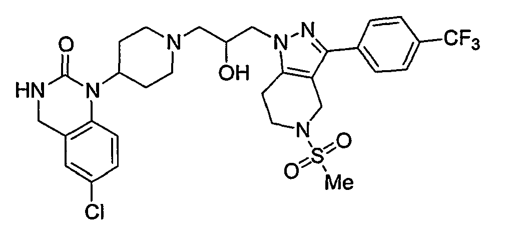

### 6-Chloro-1-(1-{2-hydroxy-3-[5-methanesulfonyl-3-(4-trifluoromethyl-phenyl)-4,5,6,7-tetrahydro-pyrazolo[4,3-c]pyridin-1-y1]-propyl}-piperidin-4-yl)-3,4-dihydro-1H-quinazolin-2-one.

### A. Spiro[piperidine-4,2'(1'H)-6'-chloro-3',4'-dihydro-4'-oxo-quinazoline]-1-carboxylic acid tert-butyl ester.

To a stirred solution of 2-amino-5-chlorobenzamide (5.67 g, 33.2 mmol) and 4-oxo-piperidine-1-carboxylic acid tert-butyl ester (6.62 g, 33.2 mmol) in benzene (70 mL) was added a catalytic amount (-0.3 g) of *p*-toluenesulfonic acid. The mixture was heated to reflux for 20 h under a Dean-Stark trap. The resulting suspension was concentrated. Saturated NaHCO₃ (68 mL) was added. The mixture was extracted with EtOAc and the precipitated crystals in the aqueous layer was collected by filtration. The solid was washed with water and dried to afford 11.22 g (96%) of the desired product. MS (electrospray): exact mass calculated for C₁₇H₂₂ClN₃O₃, 351.13; *m*/*z* found, 352.1 [M⁺+H]. ¹H NMR (CD₃OD, 400 MHz): 7.50 (d, *J* = 2.54 Hz, 1H), 7.13 (dd, *J* = 6.06 Hz, 2.54 Hz, 1H), 6.65 (d, *J* = 8.61 Hz, 1H), 3.56-3.47 (m, 2H), 3.36-3.25 (m, 2H), 1.79-1.66 (m, 4H), 1.32 (s, 9H).

### B. 4-(2-Aminomethyl-4-chloro-phenylamino)-piperidine-1-carboxylic acid tert-butyl ester.

Spiro[piperidine-4,2'(1'H)-6'-chloro-3',4'-dihydro-4'-oxo-quinazoline]-1-carboxylic acid tert-butyl ester (1 g, 2.8 mmol) and borane-tetrahydrofurane complex (1.0 M, 9.9 mL, 9.9 mmol) were added in THF (10 mL) and heated to reflux for 6 h. The reaction mixture was cooled and poured into ice water. The resulting suspension was extracted with CH₂Cl₂ (2 x 100 mL). The organics were dried and concentrated. The residue was purified by column chromatography (silica, 5-10% MeOH/CH₂Cl₂) to obtain 795 mg (79%) of the product. MS (electrospray): exact mass calculated for C₁₇H₂₆ClN₃O₂, 339.17; *m*/*z* found, 362.1 [M⁺+Na]. ¹H NMR (CDCl₃, 400 MHz): 7.07 (dd, *J* = 6.06 Hz, 2.54 Hz, 1H), 6.97 (d, *J* = 2.54 Hz, 1H), 6.54 (d, *J* = 8.61 Hz, 1H), 3.94-3.70 (m, 4H), 3.48-3.38 (m, 1H), 3.05 (t, *J* = 11.15 Hz, 2H), 2.68-2.55 (m, 1H), 2.02-1.90 (m, 4H), 1.46 (s, 9H).

### C. 4-(6-Chloro-2-oxo-3,4-dihydro-2H-quinazolin-1-yl)-piperidine-1-carboxylic acid tert-butyl ester.

1,1'-Carbonyldiimidazole (0.51 g, 3.15 mmol) was added to a solution of 4-(2-aminomethyl-4-chloro-phenylamino)-piperidine-1-carboxylic acid tert-butyl ester (0.79 g, 2.25 mmol) in CH₃CN (10 mL) over 3 h with stirring at 50°C. The reaction mixture was then cooled to room temperature and stirred for additional 2 h. The reaction mixture was dissolved in CH₂Cl₂ (100 mL), washed with water (2 x 10 mL), brine (1 x 10 mL). The organic layer was dried over Na₂SO₄ and concentrated. The residue was purified by column chromatography (silica, 30-50% EtOAc/hexanes) to obtain 0.46 g (63%) of the desired product. TLC (silica, 50% EtOAc/hexanes): R*_{f}* = 0.5. MS (electrospray): exact mass calculated for C₁₈H₂₄ClN₃O₃, 365.15; *m*/*z* found, 388.1 [M⁺+Na]. ¹H NMR (CDCl₃, 400 MHz): 7.18 (dd, *J* = 6.26 Hz, 2.54 Hz, 1H), 7.05 (d, *J* = 2.15 Hz, 1H), 6.94 (d, *J* = 9.00 Hz, 1H), 6.29 (s, 1H), 4.32-4.18 (m, 4H), 4.13-4.02 (m, 1H), 2.88-2.71 (m, 2H), 2.64-2.50 (m, 2H), 1.82-1.73 (m, 2H), 1.49 (s, 9H).

### D. 6-Chloro-1-piperidin-4-yl-3,4-dihydro-1H-quinazolin-2-one.

4-(6-Chloro-2-oxo-3,4-dihydro-2H-quinazolin-1-yl)-piperidine-1-carboxylic acid tert-butyl ester (0.52 g, 1.42 mmol) was set stirring in 1:1 TFA/CH₂Cl₂. After 45 min the mixture was evaporated and the golden oil brought up in Et₂O. A solid formed and was filtered, washed with Et₂O and air dried to give 0.52 g (97%) of an off-white solid. MS (electrospray): exact mass calculated for C₁₃H₁₆ClN₃O, 265.10; *m*/*z* found, 266.1 [M⁺+H].

### E. 6-Chloro-1-(1-{2-hydroxy-3-[5-methanesulfonyl-3-(4-trifluoromethyl-phenyl)-4,5,6,7-tetrahydro-pyrazolo[4,3-c]pyridin-1-yl]-propyl}-piperidin-4-yl)-3,4-dihydro-1H-quinazolin-2-one.

6-Chloro-1-piperidin-4-yl-3,4-dihydro-1H-quinazolin-2-one (183 mg, 0.42 mmol) and 5-methanesulfonyl-1-oxiranylmethyl-3-(4-trifluoromethyl-phenyl)-4,5,6,7-tetrahydro-1H-pyrazolo[4,3-c]pyridine (112 mg, 0.28 mmol) were set stirring in EtOH (10 mL) containing Et₃N (66 µL, 0.47 mmol) at 80°C. After 16 h the mixture was cooled, evaporated, brought up in CH₂Cl₂ and washed with water. The organics were dried over Na₂SO₄ and concentrated. The residue was purified by column chromatography (silica, 5-10% MeOH/CH₂Cl₂) to obtain 141 mg (76%) of a white solid. TLC (silica, 10% MeOH/CH₂Cl₂): R*_{f}* = 0.6. MS (electrospray): exact mass calculated for C₃₀H₃₄ClF₃N₆O₄S, 666.20; m/z found, 667.2 [M⁺+H]. ¹H NMR (CDCl₃, 400 MHz): 7.70 (d, *J* = 7.83 Hz, 2H), 7.63 (d, *J* = 8.02 Hz, 2H), 7.12 (dd, *J* = 6.65 Hz, 2.35 Hz, 1H), 7.01 (br s, 1H), 6.92 (d, *J* = 9.00 HZ, 1H), 5.44 (br s, 1H), 4.54 (dd, *J* = 14.67 Hz, 6.46 Hz, 2H), 4.23-4.08 (m, 4H), 4.05-3.97 (m, 1H), 3.92-3.80 (m, 1H), 3.74-3.57 (m, 2H), 3.14-2.99 (m 2H), 2.97-2.87 (m, 2H), 2.86 (s, 3H), 2.78-2.57 (m, 2H), 2.48-2.32 (m, 3H), 2.10 (t, *J* = 11.50 Hz 1H), 1.80-1.70 (m, 2H).

### EXAMPLE 22

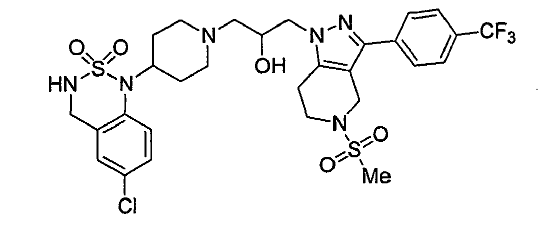

### 1-[4-(6-Chloro-2,2-dioxo-3,4-dihydro-2H-2λ⁶-benzo[1,2,6]thiadiazin-1-yl)-piperidin-1-yl]-3-[5-methanesulfonyl-3-(4-trifluoromethyl-phenyl)-4,5,6,7-tetrahydro-pyrazolo[4,3-c]pyridin-1-yl]-propan-2-ol.

### A. 4-(6-Chloro-2,2-dioxo-3,4-dihydro-2H-2λ⁶-benzo[1,2,6]thiadiazin-1-yl)-piperidine-1-carboxylic acid tert-butyl ester.

A solution of 4-(2-aminomethyl-4-chloro-phenylamino)-piperidine-1-carboxylic acid tert-butyl ester (678 mg, 2 mmol) and sulfamide (596 mg, 6.2 mmol) in pyridine (12 mL) was heated to reflux for 6 h. The reaction mixture was then cooled to room temperature and poured into ice water (50 mL). The solution was extracted with CH₂Cl₂ (4 x 100 mL). The organic extracts was dried over Na₂SO₄ and concentrated. The residue was purified by column chromatography (silica, 30-50% EtOAc/hexanes) to obtain 767 mg (96%) of the desired product. TLC (silica, 50% EtOAc/hexanes): R*_{f}* = 0.75. MS (electrospray): exact mass calculated for C₁₇H₂₄ClN₃O₄S, 401.12; *m*/*z* found, 400.1 [M⁻-H]. ¹H NMR (CDCl₃, 400 MHz): 7.13 (dd, *J* = 6.46 Hz, 2.15 Hz, 1H), 7.00 (d, *J* = 1.96 Hz, 1H), 6.92 (d, *J* = 8.60 Hz, 1H), 5.54 (br s, 1H), 4.35 (s, 2H), 4.11-3.81 (m, 3H), 2.62 (br s, 2H), 1.90-1.66 (m, 4H), 1.34 (s, 9H).

### B. 6-Chloro-1-piperidin-4-yl-3,4-dihydro-1H-benzo[1,2,6]thiadiazine 2,2-dioxide.

4-(6-Chloro-2,2-dioxo-3,4-dihydro-2H-2l6-benzo[1,2,6]thiadiazin-1-yl)-piperidine-1-carboxylic acid tert-butyl ester (767 mg, 1.91 mmol) was set stirring in 1:1 TFA/CH₂Cl₂. After 45 min the mixture was evaporated and the golden oil brought up in Et₂O. A solid formed and was filtered, washed with Et₂O and air dried to give 730 mg (91%) of an off-white solid. MS (electrospray): exact mass calculated for C₁₂H₁₆ClN₃O₂S, 301.07; *m*/*z* found, 302.0 [M⁺+H].

### C. 1-[4-(6-Chloro-2,2-dioxo-3,4-dihydro-2H-2λ⁶-benzo[1,2,6]thiadiazin-1-yl)-piperidin-1-yl]-3-[5-methanesulfonyl-3-(4-trifluoromethyl-phenyl)-4,5,6,7-tetrahydro-pyrazolo[4,3-c]pyridin-1-yl]-propan-2-ol.

6-Chloro-1-piperidin-4-yl-3,4-dihydro-1H-benzo[1,2,6]thiadiazine 2,2-dioxide (440 mg, 1.03 mmol) and 5-methanesulfonyl-1-oxiranylmethyl-3-(4-trifluoromethyl-phenyl)-4,5,6,7-tetrahydro-1H-pyrazolo[4,3-c]pyridine (415 mg, 1.03 mmol) were set stirring in EtOH (20 mL) containing Et₃N (215 µL, 1.54 mmol) at 80 °C. After 16 h the mixture was cooled, evaporated, brought up in CH₂Cl₂ and washed with water. The organics were dried over Na₂SO₄ and concentrated. The residue was purified by column chromatography (silica, 0-5% MeOH/CH₂Cl₂) to obtain 229 mg (32%) of a white solid. TLC (silica, 5% MeOH/CH₂Cl₂): R*_{f}* = 0.8. MS (electrospray): exact mass calculated for C₂₉H₃₄ClF₃N₆O₅S₂, 702.17; m/z found, 703.2 [M⁺+H]. ¹H NMR (CDCl₃, 400 MHz, a mixture of two rotamers): 7.66 (d, *J* = 8.61 Hz, 2H), 7.60 (d, *J* = 8.61 Hz, 2H), 7.16 (dd, *J* = 6.85 Hz, 1.96 Hz, 1H), 6.98 (s, 1H), 6.95 (d, *J* = 9.00 HZ, 1H), 4.47 (s, 2H), 4.33 (s, 2H), 4.16-3.99 (m, 2H), 3.98-3.90 (m, 1H), 3.89-3.78 (m, 1H), 3.62-3.52 (m, 2H), 3.05-2.95 (m, 1H), 2.93-2.84 (m, 2H), 2.82 (s, 3H), 2.81-2.76 (m, 1H), 2.33 (d, *J* = 6.46 Hz, 2H), 2.25 (t, *J* = 11.24 Hz, 1H), 2.09-1.90 (m, 3H), 1.90-1.78 (m, 2H).

### EXAMPLE 23

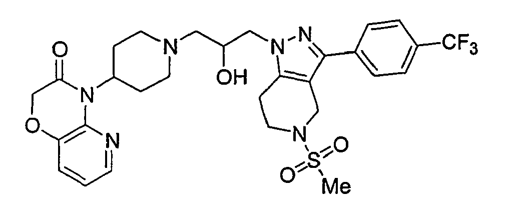

### 4-(-{2-Hydroxy-3-[5-methanesulfonyl-3-(4-trifluoromethyl-phenyl)-4,5,6,7-tetrahydro-pyrazolo[4,3-c]pyridin-1-yl]-propyl}-piperidin-4-yl)-4H-pyrido[3,2-b][1,4]oxazin-3-one.

### A. 4-(3-Hydroxy-pyridin-2-ylamino)-piperidine-1-carboxylic acid tert-butyl ester.

To a stirring solution of 4.7 g (0.042 mol) of 2-amino-3-hydroxypyridine and 12.75 g (0.064 mol) of 4-oxo-piperidine-1-carboxylic acid tert-butyl ester in CH₂Cl₂/AcOH (150 mL/60 mL) was added 10 g (0.070 mol) of Na₂SO₄. After 3.5 h, 9.9 g (0.047) of sodium triacetoxyborohydride was added in three portions, and the mixture was stirred at room temperature for 15 h. The reaction was then quenched with NaHCO₃ (150 mL), extracted with CH₂Cl₂ (500 mL), washed with NaHCO₃(2 x 100 mL), and the combined aqueous layers were extracted with EtOAc (150 mL). The combined organic layers were dried over Na₂SO₄, concentrated, and purified using flash chromatography (silica, 3-10% MeOH/CH₂Cl₂) to afford 5.9 g (48%) of a beige powder. MS (electrospray): exact mass calculated for C₁₅H₂₃N₃O₃, 293.17; *m*/*z* found, 294.2 [M+H]⁺. ¹H NMR (400 MHz, CDCl₃): 7.52 (dd, *J* = 5.3 Hz, 1.3 Hz, 1H), 6.79 (dd, *J* = 7.6 Hz, 1.3 Hz, 1H), 6.40 (dd, *J* = 7.6 Hz, 5.3 Hz, 1H), 4.06-3.94 (m, 3H), 3.02-2.86 (m, 2H), 2.72 (br s, 1H), 2.06-1.97 (m, 2H), 1.42 (s, 9H), 1.46-1.28 (m, 2H).

### B. 4-(3-Ethoxycarbonylmethoxy-pyridin-2-ylamino)-piperidine-1-carboxylic acid tert-butyl ester.

A stirring solution of 1.4 g (0.0048 mol) of 4-(3-hydroxy-pyridin-2-ylamino)-piperidine-1-carboxylic acid tert-butyl ester was dissolved in THF (24 mL) was cooled to 0 °C, and 0.13 g (0.0052 mol) of NaH was added. After 30 min, 0.8 g (0.0052 mol) of ethyl bromoacetate was added, and reaction was allowed to warm to room temperature and stirred overnight. Saturated NaHCO₃ (20 mL) was added and the reaction mixture was partitioned between EtOAc (200 mL) and saturated NaHCO₃ (75 mL). The organic layer was washed with water (50 mL) and NaCl (50 mL), dried over Na₂SO₄, and concentrated to afford 0.9 g (49%) of a white powder. MS (electrospray): exact mass calculated for C₁₉H₂₉N₃O₅, 379.21; *m*/*z* found, 380.2 [M+H]⁺. ¹H NMR (400 MHz, CDCl₃): 7.74 (d, *J* = 5.3 Hz, 1H), 6.76 (d, *J* = 7.8 Hz, 1H), 6.46 (dd, *J* = 7.8 Hz, 5.3 Hz, 1H), 5.05 (d, *J* = 7.33 Hz, 1H), 4.59 (s, 2H), 4.26 (q, *J* = 7.3Hz, 2H), 4.18-3.92 (m, 3H), 2.97 (t, *J* = 11.6 Hz, 2H), 2.06 (d, *J* = 12.1 Hz, 2H), 1.46 (s, 9H), 1.46-1.34 (m, 2H) 1.29 (t, *J* = 7.3 Hz, 3H).

### C. 4-3-Oxo-2,3-dihydro-pyrido[3,2-b][1.4]oxazin-4-yl)-piperidine-1-carboxylic acid tert-butyl ester.

To a stirring solution of 0.9 g (0.0023 mol) of 4-(3-ethoxycarbonylmethoxy-pyridin-2-ylamino)-piperidine-1-carboxylic acid tert-butyl ester in H₂O/MeOH (1 mL/11 mL) was added 0.05 g (0.0023 mol) of LiOH. After 6 h, the solvent was removed under reduced pressure. The residue was dissolved in DMF (12 mL) and to the stirring solution was added 1.82 g (0.0048 mol) of HATU. After 3 h, the reaction was partitioned between EtOAc (250 mL) and saturated NaHCO₃ (100 mL), and washed with water (3 x 100 mL). The combined aqueous layers were extracted with EtOAc (100 mL). The combined organic layers were washed with brine (100 mL), dried over Na₂SO₄, and concentrated to afford 0.37 g (46%) of a white solid. MS (electrospray): exact mass calculated for C₁₇H₂₃N₃O₄, 333.17; *m*/*z* found, 356.1 [M+Na]⁺. ¹H NMR (400 MHz, CDCl₃): 7.97 (dd, *J* = 4.8 Hz, 1.5 Hz, 1H), 7.25 (dd, *J* = 8.1 Hz, 1.5 Hz, 1H), 6.96 (dd, *J* = 8.1 Hz, 4.8 Hz, 1H), 5.03 (tt, *J* = 11.9 Hz, 4.0 Hz, 1H), 4.55 (s, 2H), 4.13 (d, *J* = 10.9 Hz, 2H), 2.82-2.69 (m, 2H), 2.68 (qd, *J* = 12.4 Hz, 4.0 Hz, 2H), 1.65 (d, *J* = 12.1 Hz, 2H), 1.46 (s, 9H).

### D. 4-Piperidin-4-yl-4H-pyrido[3,2-b][1,4]oxazin-3-one.

To a stirring solution of 0.37 g (0.0011 mol) of 4-(3-oxo-2,3-dihydro-pyrido[3,2-b][1,4]oxazin-4-yl)-piperidine-1-carboxylic acid tert-butyl ester in CH₂Cl₂ (2.5 mL) was added 2.5 mL of TFA. After 2.5 h, the solvent was removed. The residue was partitioned between EtOAc (200 mL) and 1 *N* NaOH (150 mL). The aqueous layer was extracted with EtOAc (3 x 100 mL) and the combined organic layers were dried over Na₂SO₄, and concentrated to afford 0.24 g (94%) of a white/pink solid. ¹H NMR (400 MHz, CDCl₃): 7.87 (dd, *J* = 4.8 Hz, 1.5 Hz, 1H), 7.25 (dd, *J* = 7.8 Hz, 1.8 Hz, 1H), 6.84 (dd, *J* = 7.8 Hz, 4.8 Hz, 1H), 4.98-4.83 (m, 1H), 4.45 (s, 2H), 3.90 (s, 1H), 3.06 (d, *J* = 8.3 Hz, 2H), 2.65-2.53 (m, 4H), 1.65-1.53 (m, 2H).

### E. 4-(1-{2-Hydroxy-3-[5-methanesulfonyl-3-(4-trifluoromethyl-phenyl)-4,5,6,7-tetrahydro-pyrazolo[4,3-c]pyridin-1-yl]-propyl}-piperidin-4-yl)-4H-pyrido[3,2-b][1,4]oxazin-3-one.

To a stirring solution of 0.24 g (0.001 mol) of 4-piperidin-4-yl-4H-pyrido[3,2-b][1,4]oxazin-3-one in EtOH/Dichloroethane (2 mL/ 2 mL) was added 0.27 g ( 0.0007 mol) of 5-methanesulfonyl-1-oxiranylmethyl-3-(4-trifluoro-methylphenyl)-4,5,6,7-tetrahydro-1H-pyrazolo[4,3-c]pyridine. The reaction mixture was heated to 80°C and stirred for 16 h. The solvent was then removed under reduced pressure, and the crude product was purified using flash chromatography (30% acetone/CH₂Cl₂), affording 0.42 g (96%) of a white solid. MS (electrospray): exact mass calculated for C₂₉H₃₃F₃N₆O₅S, 634.22; *m*/*z* found, 635.3 [M+H]⁺. ¹H-NMR (400 MHz, CDCl₃): 8.00 (dd, *J* = 4.8 Hz, 1.5 Hz, 1 H), 7.71 and 7.67 (A and B of AA'BB' quartet, *J_{ab}* = 8.4 Hz, 4H), 7.22 (dd, *J* = 7.9 Hz, 1.5 Hz, 1H), 6.94 (dd, *J* = 7.9 Hz, 4.8 Hz, 1H), 4.94 (tt, *J* = 12.1 Hz, 4.0 Hz, 1H) 4.57 and 4.55 (A and B of AB quartet, *J_{ab}* = 14.5 Hz, 2H), 4.57 (s, 2H), 4.25-4.02 (m, 3H), 3.78-3.61 (m, 2H), 3.16-2.90 (m, 4H), 2.90 (s, 3H), 2.89-2.76 (m, 1H), 2.56-2.43 (m, 3H) 2.23 (t, *J* = 11.2 Hz, 1H), 1.67 (d, *J* = 11.3 Hz, 2H).

### EXAMPLE 24

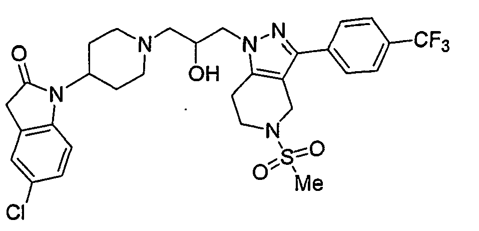

### 5-Chloro-1-(1-{2-hydroxy-3-[5-methanesulfonyl-3-(4-trifluoromethyl-phenyl)-4,5,6,7-tetrahydro-pyrazolo[4,3-c]pyridin-1-yl]-propyl}-piperidin-4-yl)-1,3-dihydro-indol-2-one.

### A. 2-(5-Chloro-2-nitro-phenyl)-malonic acid diethyl ester.

Sodium hydride (2.94 g, 123 mmol) was set stirring in DMSO (100 mL) and heated to 100 °C. Diethyl malonate (17.5 mL, 115 mmol) in DMSO (30 mL) was added and after 10 min a clear red solution was obtained. 2,4-Dichloronitrobenzene in DMSO (50 mL) was added. After 1.5 h the mixture was cooled and added to water (1000 mL). The product was extracted with ether. The organics were dried (MgSO₄) and evaporated to a clear yellow oil (10 g, 59%). TLC (silica, 20% EtOAc/hexanes): R*_{f}* = 0.36. MS (electrospray): exact mass calculated for C₁₃H₁₄ClNO₆, 315.05; *m*/*z* found, 338.0 [M+Na]⁺. ¹H NMR (400 MHz, CDCl₃): 8.05 (d, *J* = 8.7 Hz, 1H), 7.55-7.40 (m, 2H), 5.30 (s, 1H), 4.30 (q, *J* = 7.1 Hz, 4H), 1.31 (t, *J* = 7.1 Hz, 6H).

### B. (5-Chloro-2-nitro-phenyl)-acetic acid ethyl ester.

2-(5-Chloro-2-nitro-phenyl)-malonic acid diethyl ester (10.3 g, 32.6 mmol) in DMSO (200 mL) containing LiCl (2.9 g, 68.4 mmol) and water (0.6 mL, 33.3 mmol) was set stirring and heated to 100 °C. After 5 h the mixture was cooled to room temperature and added to water (750 mL). The product was extracted with two portions of EtOAc. The organics were combined, washed with water, dried (MgSO₄) and evaporated to give 5.9 g (75%) of a clear yellow oil. TLC (silica, 25% EtOAc/hexanes): R*_{f}* = 0.50. ¹H NMR (400 MHz, CDCl₃): 8.21 (d, *J* = 8.8 Hz, 1H), 7.56 (dd, *J* = 8.8, 2.3 Hz, 2H), 7.47 (d, *J* = 2.3 Hz, 1H), 4.30 (q, *J* = 7.2 Hz, 2H), 4.12 (s, 2H), 1.38 (t, *J* = 7.1 Hz, 3H).

### C. (2-Amino-5-chloro-phenyl)-acetic acid ethyl ester.

(5-Chloro-2-nitro-phenyl)-acetic acid ethyl ester (5.9 g, 24.2 mmol) in benzene (125 mL) containing PtO₂ (500 mg) was placed on a Parr hydrogenator at 40 psi H₂. After 18 h the mixture was filtered through celite and evaporated to give a clear brown liquid. The liquid was purified (silica, 25% EtOAc/hexanes) to give 3.3 g (64%) of a clear golden liquid. TLC (silica, 25% EtOAc/hexanes): R*_{f}* = 0.30. MS (electrospray): exact mass calculated for C₁₀H₁₂ClNO₂, 213.06; *m*/*z* found, 214.1 [M+H]⁺. ¹H NMR (400 MHz, CDCl₃): 7.20-7.10 (m, 2H), 6.78 (d, *J* = 8.3 Hz, 1H), 4.26 (q, *J* = 7.2, 2H), 1.18 (t, *J* = 7.1 Hz, 3H).

### D. 4-(5-Chloro-2-oxo-2,3-dihydro-indol-1-yl)-piperidine-1-carboxylic acid tert-butyl ester.

(2-Amino-5-chloro-phenyl)-acetic acid ethyl ester (3.3 g, 15.4 mmol), 4-oxo-piperidine-1-carboxylic acid tert-butyl ester (4.6 g, 23 mmol) were set stirring in CH₂Cl₂ (50 mL) and sodium triacetoxyborohydride (4.9 g, 23.1 mmol) was added followed by acetic acid (3 mL). After 5 days saturated NaHCO₃ was added and the organics separated. The organics were dried (MgSO₄) and evaporated to give 7.5 g of a clear golden oil. The oil was purified (silica, 50% EtOAc/hexanes) to give 3.4 g (63%) of a white solid. TLC (silica, 25% EtOAc/hexanes): R*_{f}* = 0.18. MS (electrospray): exact mass calculated for C₁₈H₂₃ClN₂O₃, 350.14; *m*/*z* found, 373.1 [M+Na]⁺. ¹H NMR (400 MHz, CDCl₃): 7.40-7.30 (m, 2H), 7.00 (d, *J* = 8.4 Hz, 1H), 4.55-4.45 (m, 1H), 4.40 (m, 2H), 3.63 (s, 2H), 2.94 (m, 2H), 2.45-2.30 (m, 2H), 1.82 (m, 2H), 1.62 (s, 9H).

### E. 5-Chloro-1-piperidin-4-yl-1,3-dihydro-indol-2-one.

4-(5-Chloro-2-oxo-2,3-dihydro-indol-1-yl)-piperidine-1-carboxylic acid tert-butyl ester (3.4 g, 9.7 mmol) was set stirring in 1:1 TFA/CH₂Cl₂. After 45 min the mixture was evaporated and the golden oil brought up in Et₂O. A solid formed and was filtered, washed with Et₂O and air dried to give 3.4 g (97%) of a white solid. MS (electrospray): exact mass calculated for C₁₃H₁₅ClN₂O, 250.09; *m*/*z* found, 251.1 [M+H]⁺. ¹H NMR (400 MHz, DMSO-*d*₆); 7.45 (s, 2H), 7.31 (d, *J* = 8.1 Hz, 1H), 4.55-4.45 (m, 1H), 3.68 (s, 2H), 3.50 (d, *J* = 12.3, 2H), 3.14 (m, 2H), 2.70-2.55 (m, 2H), 1.87 (d, *J* = 13.1 Hz, 2H).

### F. 5-Chloro-1-(1-{2-hydroxy-3-[5-methanesulfonyl-3-(4-trifluoromethyl-phenyl)-4,5,6,7-tetrahydro-pyrazolo[4,3-c]pyridin-1-yl]-propyl}-piperidin-4-yl)-1,3-dihydro-indol-2-one.

5-Chloro-1-piperidin-4-yl-1,3-dihydro-indol-2-one (256 mg, 0.70 mmol) and 5-methanesulfonyl-1 -oxiranylmethyl-3-(4-trifluoromethyl-phenyl)-4,5,6,7-tetrahydro-1H-pyrazolo[4,3-c]pyridine (255 mg, 0.64 mmol) were set stirring in EtOH (15 mL) containing Et₃N (107 L, 0.77 mmol) at 80°C. After 20 h the mixture was cooled, evaporated, brought up in CH₂Cl₂ and washed with water. The organics were dried (MgSO₄) and evaporated to give a clear golden oil. The oil was purified (silica, 50% acetone/CH₂Cl₂) to give 225 mg (54%) of a white solid. TLC (silica, 50% acetone/CH₂Cl₂): R*_{f}* = 0.32. MS (electrospray): exact mass calculated for C₃₀H₃₃ClF₃N₅O₄S, 651.19; *m*/*z* found, 652.2 [M+H]⁺. ¹H NMR (400 MHz, CDCl₃): 7.82 (d, *J* = 8.1 Hz, 2H), 7.76 (d, *J* = 8.1 Hz, 2H), 7.40-7.25 (m, 2H), 7.04 (d, *J* = 8.1 Hz, 2H), 4.66 (d, *J* = 4.0 Hz, 2H), 4.40-4.10 (m, 4H), 4.05-3.70 (m, 3H), 3.59 (s, 2H), 3.30-3.0 (m, 4H), 2.99 (s, 3H), 2.70-2.40 (m, 5H), 2.28 (m, 2H).

### EXAMPLE 25

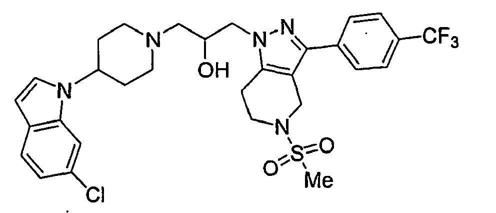

### 1-[4-(6-Chloro-indol-1-yl)-piperidin-1-yl]-3-[5-methanesulfonyl-3-(4-trifluoromethyl-phenyl)-4,5,6,7-tetrahydro-pyrazolo[4,3-c]pyridin-1-yl]-propan-2-ol.

### A. 5-Chloro-2-(2,2-dimethoxy-ethyl)-phenylamine.

To a stirred solution of 10.3 g (60 mmol) of 4-chloro-2-nitrotoluene in dry DMF (120 mL) was added 16.45 g of N,N-dimethylformamide dimethylacetal (138 mmol). The mixture was heated to 140 °C for 18 h after which the solvent was removed under reduced pressure and the residue diluted with 150 mL of MeOH and 15.2 mL of chlorotrimethylsilane (120 mmol). The reaction mixture was then heated to 60 °C overnight. Methanol was then removed under reduced pressure and the residue was taken up in EtOH and transferred to a Parr bottle. 100 mg of 10% Platinum on carbon was added and the reaction mixture was put under 2 atmospheres of hydrogen on a Parr shaker for 8 h. When the reaction was completed the catalyst was removed by filtration and the filtrate was concentrated under reduced pressure. The crude aniline was used without further purification. TLC (silica, 35% EtOAc/hexanes): R*_{f}* = 0.4. MS (electrospray): exact mass calculated for C₁₀H₁₄ClNO₂, 215.07; *m*/*z* found, 216.1 [M+H]⁺.

### B. 4-[5-Chloro-2-(2,2-dimethoxy-ethyl)-phenylamino]-piperidine-1-carboxylic acid tert-butyl ester.

To a stirred solution of 2 g of 5-chloro-2-(2,2-dimethoxy-ethyl)-phenylamine, (9.27 mmol) in 50 mL of acetic acid was added 3.7 g of 4-oxo-piperidine-1-carboxylic acid tert-butyl ester (18.5 mmol). The reaction mixture was allowed to stir for 1 h at room temperature before the portion wise addition of 5.9 g of sodium triacetoxyborohydride (27.9 mmol). The reaction mixture was allowed to stir an additional 5 h before removing the solvent under reduced pressure. The crude product was partitioned between CH₂Cl₂ (250 mL) and water. The aqueous layer was further extracted with CH₂Cl₂ (2 x 75 mL). The combined organic layers were then washed with 1 N NaOH (2 x 50 mL), brine, dried over Na₂SO₄, and concentrated. Purification by chromatography (silica, 10-25% EtOAc/hexanes) afforded 1.5 g (71%) of desired product. TLC (silica, 35% EtOAc/hexanes): R*_{f}* = 0.49. MS (electrospray): exact mass calculated for C₂₀H₃₁ClN₂O₄, 398.20; *m*/*z* found, 399.2 [M+H]⁺. ¹H NMR (CDCl₃, 400 MHz): 6.94 (d, *J* = 7.83 Hz, 1H), 6.61 (dd, *J* = 7.83, 2.02 Hz, 1H), 6.57 (d, *J* = 2.02 Hz, 1 H), 4.87 (br s, 1H), 4.40 (t, *J* = 5.31 Hz, 1H), 3.97 (br m, 2H), 3.36 (s, 6H), 3.02 (m, 2H), 2.78 (d, *J* = 5.05 Hz, 2H), 2.00 (m, 2H), 1.47 (s, 9H), 1.37 (m, 2H).

### C. 6-Chloro-1-piperidin-4-yl-1H-indole.

To a stirred solution of 1.03 g (2.59 mmol) of 4-[5-chloro-2-(2,2-dimethoxyethyl)-phenylamino]-piperidine-1-carboxylic acid tert-butyl ester in 15 mL toluene was added 1.0 g (5.2 mmol) of *p*-toluenesulfonic acid. The reaction mixture was heated to 60°C for 20 min, allowed to cool to room temperature and quenched with 100 mL of *sat.* aqueous NaHCO₃ then extracted with EtOAc (3 x 75 mL). The combined organic layers were washed with brine, dried over Na₂SO₄, and concentrated to afford 590 mg (98%) of the desired product as a pink oil. MS (electrospray): exact mass calculated for C₁₃H₁₅ClN₂, 234.09; m/z found, 235.1 [M+H]⁺. ¹H NMR (CDCl₃, 400 MHz, a mixture of amide rotamers): 7.52 (d, *J* = 8.34 Hz, 1H), 7.38 (br s, 1H), 7.21 (d, *J* = 3.28 Hz, 1H), 7.06 (dd, *J* = 8.34, 1.77 Hz, 1H), 6.49 (d, *J* = 3.28 Hz, 1H), 4.24 (m, 1H), 3.30 (m, 2H), 2.85 (dt, *J* = 12.38, 2.53 Hz, 2H), 2.08 (m, 2H), 1.94 (m, 2H).

### D. 1-[4-(6-Chloro-indol-1-yl)-piperidin-1-yl]-3-[5-methanesulfonyl-3-(4-trifluoromethyl-phenyl)-4,5,6,7-tetrahydro-pyrazolo[4,3-c]pyridin-1-yl]-propan-2-ol.

To a stirred solution of 86 mg (0.21 mmol) of 5-methanesulfonyl-1-oxiranylmethyl-3-(4-trifluoromethyl-phenyl)-4,5,6,7-tetrahydro-1H-pyrazolo[4,3 -c]pyridine in 4 mL of EtOH was added 50 mg (0.39 mmol) of 6-chloro-1-piperidin-4-yl-1H-indole. The solution was heated to 60°C overnight. The solvent was then removed by rotary evaporation and the crude product was purified by column chromatography (silica, gradient elution from 0-5% 2 *N* NH₃/MeOH in CH₂Cl₂) to afford 64 mg (48%) of a white solid. MS (electrospray), exact mass calculated for C₃₀H₃₃ClF₃N₅O₃S: 635.19; *m*/*z* found, 636.2 [M+H]⁺. HPLC (reverse phase conditions 10-90%), t*_{R}* = 4.88 min. ¹H NMR (CDCl₃, 400 MHz): 7.72 and 7.67 (A and B of AB quartet, *J* = 8.80 Hz, 4H), 7.52 (d, *J* = 8.41, 1H), 7.34 (s, 1H), 7.18 (d, *J* = 3.33 Hz, 1H), 7.07 (dd, *J* = 8.41, 1.76 Hz, 1H), 6.50 (d, *J* = 3.33 Hz, 1H), 4.59 and 4.54 (A and B of AB quartet, *J* = 14.48 Hz, 2H), 4.24 (dd, *J* = 13.69, 2.39 Hz, 1H), 4.21-4.14 (m, 2H), 4.05 (dd, *J* = 13.69, 6.46 Hz, 1H), 3.69 (m, 2H), 3.15 (br d, *J* = 11.54 Hz, 1H), 3.11-2.91 (m, 3H), 2.60-2.48 (m, 3H), 2.28 (dt, *J* = 11.74, 2.15 Hz, 1H), 2.13-1.93 (m, 4H).

### EXAMPLE 26

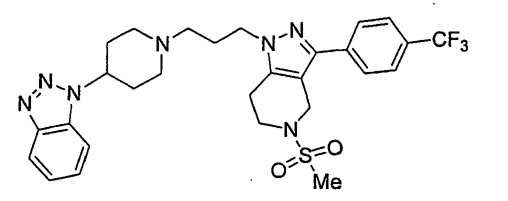

### 1-(1-{3-[5-Methanesulfonyl-3-(4-trifluoromethyl-phenyl)-4,5,6,7-tetrahydropyrazolo[4,3-c]pyridin-1-yl]-propyl}-piperidin-4-yl)-1H-benzotriazole.

To a stirred solution of 3-[5-methanesulfonyl-3-(4-trifluoromethyl-phenyl)-4,5,6,7-tetrahydro-pyrazolo[4,3-c]pyridin-1-yl]-propionaldehyde (0.084 g, 0.21 mmol) in CH₂Cl₂ (0.5 mL) were added 1-piperidin-4-yl-1H-benzotriazole hydrochloride (Maybridge Chemicals, 0.050 g, 0.21 mmol), Et₃N (0.1 mL) and glacial AcOH (12 L, 0.21 mmol) in that order and stirred for 20 min. NaBH(OAc)₃ (0.058 g, 0.27 mmol) was added and stirred under nitrogen overnight. Saturated NaHCO₃ (1 mL) was added and stirred for 30 min. The layers were separated and the aqueous layer was extracted with CH₂Cl₂ (3 mL). The combined organic extracts were washed with brine (3 mL), dried over Na₂SO₄, and removed under reduced pressure. MPLC of the crude afforded the desired compound as a white solid (0.098 g, 80 %). TLC (silica, 12% MeOH/CH₂Cl₂): R*_{f}* = 0.44. MS (electrospray): exact mass calculated for C₂₈H₃₂F₃N₇O₂S, 587.23; *m*/*z* found 588.2 [M+H]⁺. ¹H NMR (400 MHz, CDCl₃): 8.00 (d, *J* = 8.4 Hz, 1H), 7.66 (d, *J* = 8.2 Hz, 2H), 7.59 (d, *J* =8.2 Hz, 2H), 7.50 (d, *J* = 8.4 Hz, 1H), 7.41 (dt, *J* = 0.9, 7.6 Hz, 1H), 7.30 (dt, *J* = 0.9, 7.6 Hz, 1H), 4.59 (br t, *J* = 11.2 Hz, 1H), 4.50 (s, 2H), 4.10 (t, *J* = 6.7 Hz, 2H), 3.63 (t, *J* = 5.8 Hz, 2H), 3.00 (br d, *J* = 12.0 Hz, 2H), 2.89 (t, *J* = 5.8 Hz, 2H), 2.86 (s, 3H), 2.38-2.27 (m, 4H), 2.17-1.99 (m, 6H).

### EXAMPLE 27

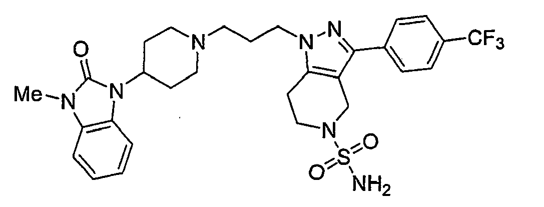

### 1-{3-[4-(3-Methyl-2-oxo-2,3-dihydro-benzoimidazol-1-yl)-piperidin-1-yl]-propyl}-3-(4-trifluoromethyl-phenyl)-1,4,6,7-tetrahydro-pyrazolo[4,3-c]pyridine-5-sulfonic acid amide.

### A. 1-(3-Oxo-propyl)-3-(4-trifluoromethyl-phenyl)-1,4,6,7-tetrahydropyrazolo[4,3-c]pyridine-5-carboxylic acid tert-butyl ester.

Dess-Martin periodinane (1.43 g, 3.36 mmol) was added portion wise to a stirred solution of 1-(3-hydroxy-propyl)-3-(4-trifluoromethyl-phenyl)-1,4,6,7-tetrahydropyrazolo[4,3-c]pyridine-5-carboxylic acid tert-butyl ester (1.30 g, 3.05 mmol) in CH₂Cl₂ (15 mL) at 0 °C under N₂. Then the reaction was stirred at 0 °C for 15 min and allowed to warm to room temperature. After stirring at room temperature for 1.5 h the reaction was diluted with Et₂O (50 mL) and saturated NaHCO₃ (15 mL) was added slowly (caution! gas evolution). Then Na₂S₂O₃.5H₂O (5.31 g, 21.4 mmol) was added and stirred for 30 min. The layers were separated and the aqueous layer was extracted with Et₂O (2 x 30 mL). The combined extracts were washed with brine, dried (Na₂SO₄) and concentrated. MPLC (1-10% MeOH/CH₂Cl₂) afforded the aldehyde in 79% yield (1.02 g). TLC (silica, 10% MeOH/CH₂Cl₂): R*_{f}* = 0.67. MS (electrospray) calculated for C₂₁H₂₄F₃N₃O₃, 424.2 ([M+H]⁺), *m*/*z* found, 424.2. ¹H NMR (400 MHz, CDCl₃): 9.82 (s, 1H), 7.65 (br d, *J* = 8.0 Hz, 2H), 7.54 (br s, 2H), 4.53 (s, 2H), 4.21 (t, *J* = 6.2 Hz, 2H), 3.68 (br s, 2H), 3.04 (t, *J* = 6.2 Hz, 2H), 2.70 (t, *J* = 5.6 Hz, 2H), 1.39 (s, 9H).

### B. 1-{3-[4-(3-Methyl-2-oxo-2,3-dihydro-benzoimidazol-1-yl)-piperidin-1-yl]-propyl}-3-(4-trifluoromethyl-phenyl)-1,4,6,7-tetrahydro-pyrazolo[4,3-c]pyridine-5-carboxylic acid tert-butyl ester.

To a stirred solution of 1-(3-oxo-propyl)-3-(4-trifluoromethyl-phenyl)-1,4,6,7-tetrahydro-pyrazolo[4,3-c]pyridine-5-carboxylic acid tert-butyl ester (0.99 g, 23.6 mmol) in CH₂Cl₂ (20 mL) were added 1-methyl-3-piperidin-4-yl-1,3-dihydro-benzoimidazol-2-one (0.60 g, 25.9 mmol) and glacial AcOH (0.13 mL, 23.6 mmol) in that order and stirred for 20 min. NaBH(OAc)₃ (0.65 g, 30.6 mmol) was added and stirred under nitrogen for 2 h. Saturated NaHCO₃ (20 mL) was added and stirred for 30 min, and the layers were separated. The organic extract was washed with brine, dried over Na₂SO₄, and concentrated under reduced pressure. MPLC of the crude afforded the desired compound as a white solid (1.27 g, 85%). TLC (silica, 7% MeOH/CH₂Cl₂): R*_{f}* = 0.35. MS (electrospray): exact mass calculated for C₃₄H₄₁F₃N₆O₃, 638.32; *m*/*z* found, 639.3 [M+H]⁺, 661.2 [M+Na]⁺. ¹H NMR (400 MHz, CDCl₃): 7.81 (br d, *J* = 8.0 Hz, 2H), 7.68 (br s, 2H), 7.25 (dd, *J* = 1.6, 7.5 Hz, 1H), 7.15-7.07 (m, 2H), 7.02(dd, *J* = 1.6, 7.9 Hz, 1H), 4.70 (br s, 2H), 4.38 (tt, *J* = 4.2, 12.4 Hz, 1H), 4.18 (t, *J* = 6.8 Hz, 2H), 3.82 (s, 2H), 3.45 (s, 3H), 3.07 (d, *J* = 11.6 Hz, 2H), 2.84 (t, *J* = 5.5 Hz, 2H), 2.53-2.42 (m, 2H), 2.44 (t, *J* = 6.7 Hz, 2H), 2.21-2.03 (m, 4H), 1.84 (d, *J* = 12.0 Hz, 2H), 1.52 (s, 9H).

### C. 1-Methyl-3-(1-{3-[3-(4-trifluoromethyl-phenyl)-4,5,6,7-tetrahydropyrazolo[4,3-c]pyridin-1-yl]-propyl}-piperidin-4-yl)-1,3-dihydro-benzoimidazol-2-one.

1-{3-[4-(3-Methyl-2-oxo-2,3-dihydro-benzoimidazol-1-yl)-piperidin-1-yl]-propyl}-3-(4-trifluoromethyl-phenyl)-1,4,6,7-tetrahydro-pyrazolo[4,3-c]pyridine-5-carboxylic acid tert-butyl ester (1.19 g, 1.86 mmol) was dissolved in trifluoroacetic acid (5 mL) and CH₂Cl₂ (5 mL) and allowed to stir at room temperature for 2 h. The reaction mixture was concentrated, diluted with CH₂Cl₂, and washed with saturated NaHCO₃. The organic layer was dried over Na₂SO₄ and concentrated to afford 1-methyl-3-(1-{3-[3-(4-trifluoromethylphenyl)-4,5,6,7-tetrahydro-pyrazolo[4,3-c]pyridin-1-yl]-propyl}-piperidin-4-yl)-1,3-dihydro-benzoimidazol-2-one (0.955 g, 96%) as a white foam. TLC (silica, 10% MeOH/CH₂Cl₂): R*_{f}* = 0.19. MS (electrospray) calculated for C₂₉H₃₃F₃N₆O, 539.3 ([M+H]⁺), *m*/*z* found, 539.3.

### D. 1-{3-[4-(3-Methyl-2-oxo-2,3-dihydro-benzoimidazol-1-yl)-piperidin-1-yl]-propyl}-3-(4-trifluoromethyl-phenyl)-1,4,6,7-tetrahydro-pyrazolo[4,3-c]pyridine-5-sulfonic acid (N-t-butoxy carbonyl)amide.

To a solution of chlorosulfonyl isocyanate (0.018 mL, 0.209 mmol) in CH₂Cl₂ (0.150 mL) was added 2-methyl-2-propanol (0.020 mL, 0.209 mmol) and the solution was stirred at room temperature for 15 min. This solution was then added dropwise to a solution of 1-methyl-3-(1-{3-[3-(4-trifluoromethyl-phenyl)-4,5,6,7-tetrahydro-pyrazolo[4,3-c]pyridin-1-yl]-propyl}-piperidin-4-yl)-1,3-dihydro-benzoimidazol-2-one (75 mg, 0.139 mmol) and triethylamine (0.039 mL, 0.279 mmol) in CH₂Cl₂ (0.4 mL). An additional 0.15 mL of CH₂Cl₂ was used to transfer all of the material to the reaction mixture. The reaction mixture was allowed to stir overnight. Column chromatography (silica, 2-10% MeOH/CH₂Cl₂) gave 93 mg (93%) of the title compound. TLC (silica, 5% MeOH/CH₂Cl₂): R*_{f}* = 0.24. MS (electrospray): calculated for C₃₄H₄₂F₃N₇O₅S, 718.3 ([M+H]⁺); *m*/*z* found, 718.3.

### E. 1-{3-[4-(3-Methyl-2-oxo-2,3-dihydro-benzoimidazol-1-yl)-piperidin-1-yl]-propyl}-3-(4-trifluoromethyl-phenyl)-1,4,6,7-tetrahydro-pyrazolo[4,3-c]pyridine-5-sulfonic acid amide.

1-{3-[4-(3-Methyl-2-oxo-2,3-dihydro-benzoimidazol-1-yl)-piperidin-1-yl]-propyl}-3-(4-trifluoromethyl-phenyl)-1,4,6,7-tetrahydro-pyrazolo[4,3-c]pyridine-5-sulfonic acid (*N-t-*butoxy carbonyl)amide (75 mg, 0.105 mmol) was dissolved in trifluoroacetic acid (0.75 mL) and CH₂Cl₂ (0.75 mL). The reaction mixture was allowed to stir for 2 h, concentrated, diluted with CH₂Cl₂ (25 mL) and washed with saturated NaHCO₃. The organic layer was dried over Na₂SO₄, concentrated, and purified by silica gel chromatography (5-10% MeOH/CH₂Cl₂) to afford 1-{3-[4-(3-methyl-2-oxo-2,3-dihydro-benzoimidazol-1-yl)-piperidin-1-yl]-propyl}-3-(4-trifluoromethyl-phenyl)-1,4,6,7-tetrahydro-pyrazolo[4,3-c]pyridine-5-sulfonic acid amide (15 mg, 23%). MS (electrospray) calculated for C₂₉H₃₄F₃N₇O₃S, 618.2 ([M+H]⁺), *m*/*z* found, 618.2. ¹H NMR (400 MHz, CDCl₃): 7.72 (d, *J* = 8.2 Hz, 2H), 7.63 (d, *J* = 8.2 Hz, 2H), 7.22 (br s, 1H), 7.04-7.11 (m, 2H), 6.95-7.00 (m, 1H), 5.02 (br s, 1H), 4.53 (s, 1H), 4.08-4.36 (m, 3H), 3.68 (br t, *J* = 5.9 Hz, 2H), 3.38 (s, 3H), 2.95-3.01 (m, 2H), 2.41-2.70 (m, 4H), 2.11-2.34 (m, 4H), 1.52-1.94 (m, 6H).

### EXAMPLE 28

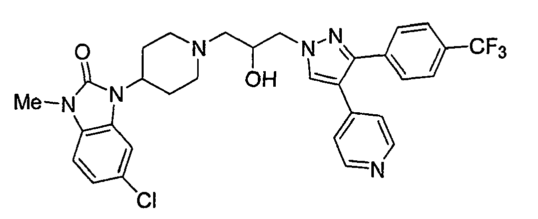

### 5-Chloro-3-(1-{2-hydroxy-3-[4-pyridin-4-yl-3-(4-trifluoromethyl-phenyl)-pyrazol-1-yl]-propyl}-piperidin-4-yl)-1-methyl-1,3-dihydro-benzoimidazol-2-one.

### A. 4-[1-Oxiranylmethyl-3-(4-trifluoromethyl-phenyl)-1H-pyrazol-4-yl]-pyridine.

To a solution of 4-[3-(4-trifluoromethyl-phenyl-1H-pyrazol-4-yl]-pyridine (0.5 g, 1.73 mmol) and epichlorohydrin (1.35 mL, 17.3 mmol) in DMF (2 mL) was added cesium carbonate (0.676 g, 2.07 mmol). The reaction mixture was allowed to stir for 24 h, diluted with EtOAc and washed successively with saturated NaHCO₃, water, and brine. The organic layer was dried over Na₂SO₄, concentrated and partially purified by running through a plug of silica gel (5% acetone/CH₂Cl₂) to afford 4-[1-oxiranylmethyl-3-(4-trifluoromethylphenyl)-1H-pyrazol-4-yl]-pyridine (0.198 g, 33%) as an unstable oil. TLC (silica, 20% acetone/CH₂Cl₂): *R_{f}* = 0.39. MS (electrospray): exact mass calculated for C₁₈H₁₄F₃N₃O, 346.1 [M+H]⁺, *m*/*z* found, 346.1.

### B. 5-Chloro-3-(1-{2-hydroxy-3-[4-pyridin-4-yl-3-(4-trifluoromethyl-phenyl)-pyrazol-1-yl]-propyl}-piperidin-4-yl)-1-methyl-1,3-dihydro-benzoimidazol-2-one.

To a solution of 4-[1-oxiranylmethyl-3-(4-trifluoromethyl-phenyl)-1 H-pyrazol-4-yl]-pyridine (68 mg, 0.197 mmol) and 5-chloro-1-methyl-3-piperidin-4-yl-1,3-dihydro-benzoimidazol-2-one (0.055 g, 0.207 mmol) in EtOH (1 mL) was added triethylamine (0.027 mL, 0.197 mmol). The reaction mixture was heated at 80 °C overnight, concentrated, and purified by column chromatography (silica, 2-10% MeOH/CH₂Cl₂) to afford the title compound (0.026 g, 22%). MS (electrospray): exact mass calculated for C₃₁H₃₀ClF₃N₆O₂, 611.2 [M+H]⁺, *m*/*z* found, 611.2. ¹H NMR (400 MHz, CDCl₃): 8.59 (br s, 2H), 8.20 (s, 1H), 7.67 (d, *J* = 8.2 Hz, 2H), 7.61 (d, *J* = 5.9 Hz, 2H), 7.55 (d, *J* = 8.2 Hz, 2H), 7.35 (br s, 1H), 7.09 (dd, *J* = 8.2, 1.8 Hz, 1H), 6.89 (d, *J* = 8.2 Hz, 1H), 4.55-4.60 (m, 2H), 4.39 (d, *J* = 14.2, 4.1 Hz, 1H), 4.31 (d, *J* = 14.2, 6.1 Hz, 1H), 3.80-3.90 (m, 2H), 3.37 (s, 3H), 3.18-3.33 (m, 2H), 3.02-3.17 (m, 2H), 2.77-2.95 (m, 2H), 1.99 (t, *J* = 12.4 Hz, 2H).

### EXAMPLE 29

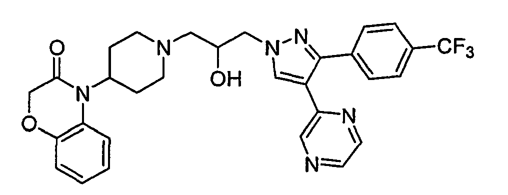

### 4-(1-{2-Hydroxy-3-[4-pyrazin-2-yl-3-(4-trifluoromethyl-phenyl)-pyrazol-1-yl]-propyl}-piperidin-4-yl)-4H-benzo[1,4]oxazin-3-one.

### A. 4-(2-Hydroxy-phenylamino)-piperidine-1-carboxylic acid tert-butyl ester.

2-Aminophenol (15.0 g, 137 mmol) and 4-oxo-piperidine-1-carboxylic acid tert-butyl ester (27.4 g, 138 mmol) were set stirring in CH₂Cl₂ (200 mL) at room temperature. Sodium triacetoxyborohydride (40.8 g, 193 mmol) was added in portions over 10 min followed by acetic acid (7.8 mL, 136 mmol). After 18 h saturated NaHCO₃ was added, the organics separated, dried (MgSO₄) and evaporated to give 36.4 g (91%) of a beige solid. TLC (silica, 50% EtOAc/hexanes): R*_{f}* = 0.56. MS (electrospray): exact mass calculated for C₁₆H₂₄N₂O₃, 292.18; *m*/*z* found, 315.1 [M+Na]⁺. ¹H NMR (400 MHz, CDCl₃): 9.20 (s, 1H), 6.80-6.50 (m, 3H), 6.40 (t, *J* = 6.1 Hz, 1H), 4.30 (d, *J* = 8.7 Hz, 1H), 3.88 (d, *J* = 12.6 Hz, 2H), 3.45-3.35 (m, 1H), 3.00-2.75 (br s, 2H), 1.88 (d, *J* = 10.5 Hz, 2H), 1.40 (s, 9H), 1.30-1.20 (m, 2H).

### B. 4-(2-Ethoxycarbonylmethoxy-phenylamino)piperidine-1-carboxylic acid tert-butyl ester.

A mixture of NaH (1.56 g, 65 mmol) in THF (100 mL) was set stirring and cooled to 5°C. 4-(2-Hydroxy-phenylamino)-piperidine-1-carboxylic acid tert-butyl ester (17.5 g, 60 mmol) in THF (100 mL) was added dropwise over 30 min. After 2 h ethyl bromoacetate (7.3 mL, 66 mmol) was added. After stirring at room temperature for 24 h saturated NH₄Cl (100 mL) was added and the organics evaporated. The aqueous layer was extracted with EtOAc (2 x 150 mL). The organics were combined, dried (MgSO₄) and evaporated to give 24 g of a deep red liquid. The liquid was purified (silica, 5% acetone/CH₂Cl₂) to give 21.4 g (94%) of a clear orange liquid. TLC (silica, 5% acetone/CH₂Cl₂): R*_{f}* = 0.48. MS (electrospray): exact mass calculated for C₂₀H₃₀N₂O₅, 378.22; *m*/*z* found, 379.2 [M+H]⁺. ¹H NMR (400 MHz, DMSO): 7.02 (m, 1H), 6.90-6.70 (m, 3H), 4.74 (s, 2H), 4.37 (q, *J* = 7.1 Hz, 2H), 4.13 (br s, 2H), 3.60-3.50 (m, 1H), 3.08 (m, 2H), 2.16 (m, 2H), 1.60-1.50 (m, 2H), 1.58 (s, 9H), 1.41 (t, *J* = 7.1 Hz, 3H).

### C. 4-(2-Carboxymethoxy-phenylamino)-piperidine-1-carboxylic acid tert-butyl ester.

4-(2-Ethoxycarbonylmethoxy-phenylamino)-piperidine-1-carboxylic acid tert-butyl ester (21.4 g, 56.5 mmol) was set stirring in MeOH (150 mL). A solution of NaOH (4.5 g, 112.5 mmol) in water (150 mL) was added. After 3 h the mixture was acidified to pH 4 with 6 *N* HCl. MeOH was removed under reduced pressure and the aqueous layer extracted with EtOAc (2 x 150 mL). The organics were combined, dried (MgSO₄) and evaporated to give 20 g (100%) of a brown solid. MS (electrospray): exact mass calculated for C₁₈H₂₆N₂O₅, 350.18; *m*/*z* found, 351.2 [M+H]⁺.

### D. 4-(3-Oxo-2,3-dihydro-benzo[1,4]oxazin-4-yl)-piperidine-1-carboxylic acid tert-butyl ester.

4-(2-Carboxymethoxy-phenylamino)-piperidine-1-carboxylic acid tert-butyl ester (22 g, 63 mmol) was set stirring in CH₂Cl₂ (200 mL). EDC (13 g, 68 mmol) was added in one portion. After 30 min 1 *N* HCl was added. The organics were seperated, dried (MgSO₄) and evaporated to give 17 g (81 %) of a clear brown oil. TLC (silica, 5% acetone/CH₂Cl₂): R*_{f}*= 0.45. MS (electrospray): exact mass calculated for C₁₈H₂₄N₂O₄, 332.17; *m*/*z* found, 259.1 [M-BOC+H]⁺. ¹H NMR (400 MHz, CDCl₃): 7.30-7.20 (m, 1H), 7.15-7.10 (m, 3H), 4.61 (s, 2H), 4.60-4.45 (m, 1H), 4.45-4.30 (br s, 2H), 2.88 (t, *J* = 12.5 Hz, 2H), 2.65 (dd, *J* = 12.6, 4.5 Hz, 2H), 1.87 (d, *J* = 12.4 Hz, 2H), 1.60 (s, 9H).

### E. 4-Pperidin-4-yl-4H-benzo[1,4]oxazin-3-one.

4-(3-Oxo-2,3-dihydro-benzo[1,4]oxazin-4-yl)-piperidine-1-carboxylic acid tert-butyl ester (17 g, 51 mmol) and 1:1 TFA/ CH₂Cl₂ (40 mL) were combined and set stirring. After 45 min the mixture was evaporated to give a clear brown oil. The oil was set stirring and Et₂O was added (300 mL). A solid formed and was filtered, washed with Et₂O and air dried to give 16 g (90%) of a light beige solid. MS (electrospray): exact mass calculated for C₁₃H₁₆N₂O₂, 232.12; *m*/*z* found, 233.1 [M+H]⁺. ¹H NMR (400 MHz, CD₃OD): 7.44 (dd, *J* = 6.5, 1.4 Hz, 1H), 7.20-7.7.10 (m, 3H), 4.58 (s, 2H), 4.55-4.45 (m, 1H), 4.65-4.55 (m, 2H), 3.27 (dt, *J* = 13.0, 2.3 Hz, 2H), 3.05 (dd, *J* = 12.3, 4.1 Hz, 2H), 2.15 (d, *J* = 13.8 Hz, 2H).

### F. 2-[1-Oxiranylmethyl-3-(4-trifluoromethyl-phenyl)-1H-pyrazol-4-yl]-pyrazine.

To a solution of 2-[3-(4-trifluoromethyl-phenyl)-1H-pyrazol-4-yl]-pyrazine (200 mg, 0.69 mmol) and epichlorohydrin (0.540 mL, 6.9 mmol) in DMF (2 mL) was added cesium carbonate (450 mg, 1.38 mmol). The reaction mixture was allowed to stir for 24 h, diluted with EtOAc, and washed with saturated NaHCO₃, water, and brine. The organic layer was dried over Na₂SO₄, concentrated and purified by column chromatography (silica, 5% acetone/CH₂Cl₂) to afford 2-[1-oxiranylmethyl-3-(4-trifluoromethyl-phenyl)-1H-pyrazol-4-yl]-pyrazine (141 mg, 59%). TLC (silica, 20% acetone/CH₂Cl₂): *R_{f}* = 0.38. MS (electrospray) *m*/*z* 347.1 (347.1, calculated for C₁₇H₁₃F₃N₄O, M⁺+H). ¹H NMR (400 MHz, CDCl₃): 8.51 (dd, *J* = 2.8, 1.8 Hz, 1H), 8.45 (d, *J* = 1.5 Hz, 1H), 8.38 (d, *J* = 12.8 Hz, 1H), 8.01 (s, 1H), 7.66 (d, *J* = 8.6 Hz, 1H), 7.62 (d, *J* = 8.6 Hz, 1H), 4.57 (dd, *J* = 14.7, 3.1 Hz, 1H), 4.21 (dd, *J* = 14.7, 6.1 Hz, 1H), 3.44 (m, 1H), 2.91 (t, *J* = 4.5 Hz, 1H), 2.62 (dd, *J* = 4.0, 2.5 Hz, 1H).

### G. 4-(1-{2-Hydroxy-3-[4-pyrazin-2-yl-3-(4-trifluoromethyl-phenyl)-pyrazol-1-yl]-propyl}-piperidin-4-yl)-4H-benzo[1,4]oxazin-3-one.

To a solution of 2-[1-oxiranylmethyl-3-(4-trifluoromethyl-phenyl)-1H-pyrazol-4-yl]-pyrazine (76 mg, 0.220 mmol) and 4-piperidin-4-yl-4H-benzo[1,4]oxazin-3-one (61 mg, 0.231 mmol) in EtOH (1.1 mL) was added triethylamine (0.031 mL, 0.220 mmol). The reaction mixture was heated to 80 °C overnight, concentrated, and purified by column chromatography (silica, 5-10% MeOH/CH₂Cl₂) to afford 4-(1-{2-hydroxy-3-[4-pyrazin-2-yl-3-(4-trifluoromethylphenyl)-pyrazol-1-yl]-propyl}-piperidin-4-yl)-4H-benzo[1,4]oxazin-3-one (27 mg, 21 %). TLC (silica, 5% MeOH/CH₂Cl₂): *R_{f}* = 0.09. MS (electrospray): *m*/*z* 579.2 (579.2, calculated for C₃₀H₂₉F₃N₆O₃, M⁺+H). ¹H NMR (400 MHz, CDCl₃): 8.53 (s, 1H), 8.48 (s, 1H), 8.40 (s, 1H), 8.11 (s, 1H), 7.73 (d, *J* = 8.2 Hz, 2H), 7.63 (d, *J* = 8.2 Hz, 2H), 7.16 (d, *J* = 5.4 Hz, 1H), 7.00-7.03 (m, 3H), 4.49 (s, 2H), 4.39 (d, *J* = 10.8 Hz, 1H), 3.13 (d, *J* = 11.9 Hz,1H), 2.96 (d, *J* = 11.9 Hz, 1H), 2.59-2.80 (m, 2H), 2.40-2.55 (m, 3H), 2.17 (t, *J* = 11.9 Hz, 1H),1.77 (d, *J* = 11.9 Hz, 2H).

### EXAMPLE 30

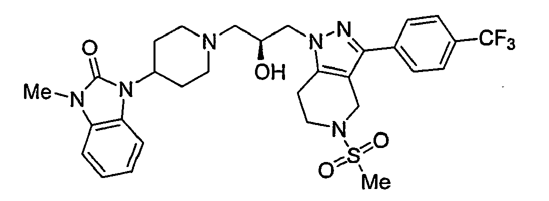

### (S)-1-(1-{2-Hydroxy-3-[5-methanesulfonyl-3-(4-trifluoromethyl-phenyl)-4,5,6,7-tetrahydro-pyrazolo[4,3-c]pyridin-1-yl]-propyl}-piperidin-4-yl)-3-methyl-1,3-dihydro-benzoimidazol-2-one.

### A. 4-(2-Oxo-2,3-dihydro-benzoimidazol-1-yl)-piperidine-1-carboxylic acid tert-butyl ester.

1-Piperidin-4-yl-1,3-dihydro-benzoimidazol-2-one (7.24 g, 34.1 mmol) and di-tert-butyl dicarbonate (9.12 g, 41.0 mmol) were combined in DMF (80 mL) and the mixture heated to 40 °C under N₂ for 17 h. The mixture was allowed to cool, diluted with EtOAc (800 mL) and washed with saturated NaHCO₃ (150 mL), H₂O (3 x 150 mL) and brine (150 mL). The combined aqueous washes were extracted with EtOAc (2 x 150 mL). The combined extracts were dried over Na₂SO₄ and concentrated, to give 4-(2-oxo-2,3-dihydro-benzoimidazol-1-yl)-piperidine-1-carboxylic acid tert-butyl ester (12.36 g, 94%). TLC (silica, 50% EtOAc/hexanes): R*_{f}* = 0.3. MS (electrospray): exact mass calculated for C₁₇H₂₃N₃O₃, 340.16; m/z found, 340.1 [M + Na]⁺. ¹H NMR (CDCl₃, 400 MHz): 10.59 (s, 1H), 7.15-7.11 (m, 2H), 7.08-7.02 (m, 2H), 4.49 (tt, *J* = 8.4, 4.0 Hz, 1H), 4.32 (br s, 2H), 2.89 (br t, *J* = 11.6, 2H), 2.34 (dq, *J* = 12.6, 4.4 Hz, 2H), 1.83 (br d, *J* = 10.5 Hz, 2H) 1.36 (s, 9H).

### B. 1-Methyl-3-piperidin-4-yl-1,3-dihydro-benzoimidazol-2-one.

A solution of KHMDS (5.07 g, 25.4 mmol) in THF (40 mL plus a 10 mL rinse) was added via cannula to a solution of 4-(2-oxo-2,3-dihydro-benzoimidazol-1-yl)-piperidine-1-carboxylic acid tert-butyl ester (6.64 g, 20.2 mmol) in THF (20 mL). The mixture was stirred for 25 min then iodomethane (5.2 mL, 84 mmol) was added. The resulting mixture was stirred for 45 min then diluted with EtOAc (700 mL). The EtOAc was washed with H₂O (3 x 200 mL), saturated NaHCO₃ (150 mL) and brine (150 mL). The combined washes were extracted with EtOAc (2 x 150 mL). The combined extracts were dried over Na₂SO₄ and concentrated. The crude reaction mixture was purified by column chromatography (silica, 15-60% EtOAc/hexanes) to give the methylated adduct (5.21 g, 78%). The purified material was dissolved in a mixture of CH₂Cl₂ (40 mL) and TFA (35 mL). The mixture was stirred for 4 h then concentrated in vacuo. The residue was dissolved in CH₂Cl₂ (300 mL) and washed with saturated NaHCO₃ (100 mL). The aqueous layer was extracted with 5%MeOH/CH₂Cl₂ (4 x 150 mL). The combined extracted were dried over Na₂SO₄ and concentrated to yield the title compound (3.85 g, containing inorganic salts) which was suitable for further use. TLC (silica, 5% MeOH/CH₂Cl₂): R*_{f}* = 0.1. MS (electrospray): exact mass calculated for C₁₃H₁₈N₃O, 232.14; *m*/*z* found 232.1 [M + H]⁺. ¹H NMR (CDCl₃, 400 Hz): 7.27-7.29 (m, 1H), 7.05-7.12 (m, 2H), 6.99 (dd, *J* = 6.1, 2.1 Hz, 1H), 4.45 (tt, *J* = 12.5, 4.2 Hz, 1H), 3.42 (s, 3H), 3.27 (dd, *J* = 10.2, 2.1 Hz, 2H), 2.81 (dt, *J* = 2.4, 12.4 Hz, 2H), 2.35 (dq, *J* = 12.5, 4.2 Hz, 2H), 2.26 (br s, 1H), 1.83 (dd, *J* = 12.1, 2.1 Hz, 2H).

### C. (R)-tert-Butyl-dimethyl-oxiranylmethoxy-silane.

tert-Butyl-chloro-dimethylsilane (12.9 g, 85.5 mmol) followed by Et₃N (19 mL, 136 mmol) was added to a 0 °C solution of (S)-(+)-glycidol (5.0 g, 67 mmol) in CH₂Cl₂ (200 mL). The solution was allowed to warm to 23 °C with stirring over 17 h. The resulting pink solution was diluted with Et₂O (800 mL) and stirred an additional 30 min. The Et₂O layer was washed with saturated aqueous NaHCO₃ (200 mL), H₂O (2 x 100 mL), brine (100 mL), dried over Na₂SO₄ and concentrated. Purification by column chromatography (silica, 5-10% Et₂O/hexanes) gave (*R*)-tert-Butyl-dimethyl-oxiranylmethoxy-silane (10.01 g, 79%). TLC (silica, 10% Et₂O/hexanes): R*_{f}* 0.5. ¹H NMR (CDCl₃, 400 MHz): 3.85 (dd, *J* = 11.9, 3.2 Hz, 1H), 3.66 (dd, *J* = 11.9, 4.8 Hz, 1H), 3.09 (m, 1H), 2.77 (dd, *J* = 5.0, 4.2 Hz, 1H), 2.64 (dd, *J* = 5.2, 2.7 Hz, 1H), 0.90 (s, 9H), 0.08 (s, 3H), 0.07 (s, 3H).

### D. (R)-3-[5-Methanesulfonyl-3-(4-trifluoromethyl-phenyl)-4,5,6,7-tetrahydropyrazolo[4,3-c]pyridin-1-yl]-1-propane-1,2-diol.

CS₂CO₃ (1.88 g, 5.77 mmol) was added to a solution of (*R*)-tert-Butyl-dimethyl-oxiranylmethoxy-silane (2.72 g, 14.4 mmol) and 5-methanesulfonyl-3-(4-trifluoromethyl-phenyl)-4,5,6,7-tetrahydro-1H-pyrazolo[4,3-c]pyridine (1.70g, 4.81 mmol) in DMF (13 mL). The mixture ws stirred at room temperature for 5 days, then partitioned between EtOAc (400 mL) and saturated NaHCO₃ (100 mL). The EtOAc layer was washed with H₂O (3 x 75 mL) and brine (100 mL), dried over Na₂SO₄ and concentrated. The residue was dissolved in MeOH (125 mL) and treated with CSA (800 mg). The mixture was stirred for 20 h then concentrated. The residue was re-dissolved in EtOAc (200 mL), washed with saturated NaHCO₃ (100 mL), dried over Na₂SO₄ and concentrated. Purification by column chromatography (silica, 20-60% acetone/CH₂Cl₂) gave the corresponding diol (0.78 g, 40%). TLC (25% acetone/CH₂Cl₂): R*_{f}* = 0.2. MS (electrospray): exact mass calculated for C₁₇H₂₁F₃N₃O₄S, 420.11; *m*/*z* found, 420.1 [M + H]⁺. ¹H NMR (CD₃OD/CDCl₃, 400 MHz): 7.74 and 7.67 (A and B of AA'BB', *J*_{ab} = 8.3 Hz, 4H), 4.52 (s, 2H), 4.23 (dd, *J* = 13.0, 3.0 Hz, 1H), 4.04-4.11 (m, 2H), 3.64 (t, *J* = 5.9 Hz, 2H), 3.52 and 3.57 (A and B of ABX, *J*_{ab} = 11.4, *J*ₐₓ = 4.8, *J*_{bx} = 4.9 Hz, 2H), 2.98 (m, 2H), 2.91 (s, 3H).

### E. (R)-5-Methanesulfonyl-1-oxiranylmethyl-3-(4-trifluoromethyl-phenyl)-4,5,6,7-tetrahydro-1H-pyrazolo[4,3-c]pyridine.

PpTs (271 mg, 1.1 mmol) and (R)-3-[5-methanesulfonyl-3-(4-trifluoromethylphenyl)-4,5,6,7-tetrahydro-pyrazolo[4,3-c]pyridin-1-yl]-propane-1,2-diol (317 mg, 0.756 mmol) were combined in trimethylorthoacetate (30 mL). The mixture was stirred for 18 h then diluted with EtOAc (125 mL), washed with saturated NaHCO₃ (2 x 50 mL), brine (50 mL), dried over Na₂SO₄ and concentrated. Purification by chromatography (silica, 100%EtOAc) gave the corresponding orthoacetate (313 mg, 0.678 mmol). The purified orthoacetate was dissolved in CH₂Cl₂ (2.25 mL), cooled to 0 °C, and treated with MeOH (25 µL) and AcBr (110 µL, 1.48 mmol). The mixture was allowed to warm over 3 h, then partitioned between EtOAc (50 mL) and saturated NaHCO₃ (20 mL). The EtOAc layer was washed with saturated NaHCO₃ (2 x 20 mL). The combined washes were extracted with EtOAc (3 x 20 mL). The combined extracts were dried over Na₂SO₄ and concentrated. The residue was dissolved in EtOH (40 mL) and treated with KOEt (1.0 mL, 40 wt% solution in EtOH). After 1 h the mixture was concentrated to ca. 20 mL and worked up as above. Purification by column chromatography (silica, 100% EtOAc) gave the epoxide (189 mg, 62%). TLC (100% EtOAc): R*_{f}* = 0.35. MS (electrospray): exact mass calculated for C₁₇H₁₉F₃N₃O₃S, 402.10; *m*/*z* found, 402.1 M + H]⁺. ¹H NMR (CDCl₃, 400 MHz): 7.72 and 7.67 (A and B of AA'BB', *J*_{ab} = 8.3 Hz, 4H), 4.57 and 4.53 (A and B of AB, *J*_{ab} = 12.9 Hz, 2H), 4.52 (dd, *J* = 15.2, 2.7 Hz, 1H), 4.12 (dd, *J* = 15.2, 5.4 Hz, 1H), 3.67 (m, 2H), 3.36 (m, 1H), 2.92 (m, 2H), 2.88 (s, 3H), 2.85 (dd, *J* = 4.4, 4.3 Hz, 1H), 2.49 (dd, *J* = 4.6, 2.6 Hz, 1H).

### F. (S)-1-(1-{2-Hydroxy-3-[5-methanesulfonyl-3-(4-trifluoromethyl-phenyl)-4,5,6,7-tetrahydro-pyrazolo[4,3-c]pyridin-1-yl]-propyl}-piperidin-4-yl)-3-methyl-1,3-dihydro-benzoimidazol-2-one.

A solution of (*R*)-5-methanesulfonyl-1-oxiranylmethyl-3-(4-trifluoromethylphenyl)-4,5,6,7-tetrahydro-1H-pyrazolo[4,3-c]pyridine (134 mg, 0.334 mmol) and 1-methyl-3-piperidin-4-yl-1,3-dihydro-benzoimidazol-2-one (110 mg, 0.476 mmol) in EtOH (0.8 mL) and dichloroethane (0.8 mL) was heated to 80°C for 18 h. The mixture was then concentrated and the residue purified by column chromatography (silica, 0-50% acetone/CH₂Cl₂) to give the title compound (134 mg, 86%). TLC (20% acetone/CH₂Cl₂) R*_{f}* = 0.3. MS (electrospray): calculated for C₃₀H₃₆F₃N₆O₄S, [M + H]⁺ 633.24; *m*/*z* found, 633.3. ¹H NMR (CDCl₃, 400 MHz): 7.72 and 7.66 (A and B of AA'BB', *J*_{ab} = 8.3 Hz, 4H), 7.15 (dd, *J* = 7.0, 1.7 Hz, 1H ), 7.08 (m, 2H), 6.98 (dd, *J* = 6.6, 1.8 Hz, 1H), 4.60 and 4.55(A and B of AB, *J*_{ab} = 14.5 Hz, 2H), 4.34 (m, 1H), 4.23 (dd, *J* = 13.8, 2.8 Hz, 1H), 4.15 (m, 1H), 4.23 (dd, *J* = 13.8, 6.6 Hz, 1H), 3.71 (m, 2H), 3.40 (s, 3H), 3.08 (m, 2H), 2.96 (m, 2H), 2.89 (s, 3H), 2.56-2.36 (m, 4H), 2.23 (d, *J* = 11.6 Hz, 1H), 1.81 (m, 2H).

### EXAMPLE 31

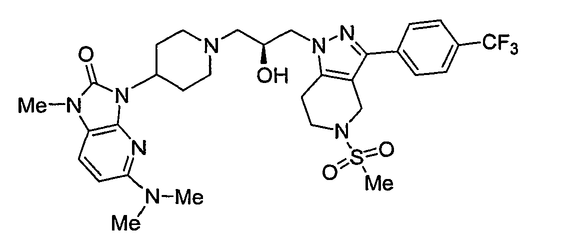

### (S)-5-Dimethylamino-3-(1-{2-hydroxy-3-[5-methanesulfonyl-3-(4-trifluoromethylphenyl)-4,5,6,7-tetrahydro-pyrazolo[4,3-c]pyridin-1-yl]-propyl}-piperidin-4-yl)-1-methyl-1,3-dihydro-imidazo[4,5-b]pyridin-2-one.

### A. 4-(6-Chloro-3-nitro-pyridin-2-ylamino)-piperidine-1-carboxylic acid tert-butyl ester.

A stirring solution of 20 g (0.10 mol) of 2,6-dichloro-3-nitro-pyridine in DMF (245 mL) was cooled to 0 °C. After 5 min, 9.87 g (0.05 mol) of 4-amino-piperidine-1-carboxylic acid tert-butyl ester and 6.8 g (0.05 mol) of K₂CO₃ were added, resulting in a suspension. The mixture was allowed to stir for 5 h at 0 °C. The mixture was then partitioned between water (300 mL) and EtOAc (400 mL). The aqueous layer was then extracted with EtOAc (5 x 400 mL). The organic layer was dried over anhydrous Na₂SO₄, and concentrated to give a brown oil. The product was purified using silica gel chromatography (silica, 100%CH₂Cl₂, then 10% EtOAc/hexanes) to afford 8.99 g (51%) of the desired product as a bright yellow solid. MS (electrospray): exact mass calculated for C₁₅H₂₁ClN₄O₄, 356.13; *m*/*z* found, 379.1 [M+Na]⁺. ¹H NMR (400 MHz, CDCl₃): 8.36 (d, *J* = 8.4 Hz, 1H), 8.27 (d, *J* = 7.3 Hz, 1H), 6.62 (d, *J* = 8.4 Hz, 1H), 4.38-4.26 (m, 1H), 4.14-3.96 (m, 2H), 3.01 (t, *J* = 11.6 Hz, 2H), 2.05 (dd, *J* = 12.4 Hz, 3.03 Hz, 2H), 1.58-1.44 (m, 2H), 1.47 (s, 9H).

### B. 4-(6-Dimethylamino-3-nitro-pyridin-2-ylamino)-piperidine-1-carboxylic acid tert-butyl ester.

To a stirring solution of 6 g (0.016 mol) of 4-(6-chloro-3-nitro-pyridin-2-ylamino)-piperidine-1-carboxylic acid tert-butyl ester in MeOH/CH₂Cl₂(84 mL/15 mL) was added 2.2 g (0.05 mol) of dimethylamine in THF (25 mL). The reaction mixture was stirred at room temperature for 16 h, and was then concentrated. The crude product was then dissolved in CH₂Cl₂ (400 mL) and washed with saturated NaHCO₃ (2 x 200 mL). The washes were combined and extracted with EtOAc (100 mL). The combined organic layers were dried over Na₂SO₄ and concentrated to afford 6.1 g (99%) of the desired product as a bright yellow solid. MS (electrospray): exact mass calculated for C₁₇H₂₇N₅O₄, 365.21; *m*/*z* found, 388.19 [M+Na]⁺. ¹H NMR (400 MHz, CDCl₃): 8.74 (d, *J* = 7.07 Hz, 1H), 8.18 (d, *J* = 9.4 Hz, 1H), 5.97 (d, *J* = 7.3 Hz, 1H), 4.28-4.16 (m, 1H), 4.07-3.93 (m, 2H), 3.17 (s, 6H), 3.01 (t, *J* = 11.9 Hz, 2H), 2.05 (dd, *J* = 12.4 Hz and 3.03 Hz, 2H), 1.60-1.50 (m, 2H), 1.47 (s, 9H).

### C. 4-(5-Dimethylamino-1-methyl-2-oxo-1,2-dihydro-imidazo[4,5-b]pyridin-3-yl)-piperidine-1-carboxylic acid tert-butyl ester.

A stirring solution of 5.3 g (0.014 mol) of 4-(6-dimethylamino-3-nitro-pyridin-2-ylamino)-piperidine-1-carboxylic acid tert-butyl ester in methanol/EtOAc (73 mL/15 mL) was degassed. 10% Pd/C (1.17 g, 0.5 mmol) was added as a suspension in EtOH (5 mL), followed by ammonium formate (4.5 g, 0.073 mol). The mixture was stirred at room temperature for 3 h. The reaction mixture was then filtered through celite and the filtrate was concentrated, giving a purple oil. The residue was then dissolved in THF (73 mL), and 11.7 g (0.073 mol) of CDI was added, and the reaction was heated to 98 °C and stirred for 16 h. The mixture was then cooled and concentrated. The crude product was then partitioned between EtOAc (800 mL) and NaHCO₃ (100 mL), and the organic layer was washed with water (5 x 100 mL) and NaCl (100 mL). The combined aqueous layers were back-extracted with EtOAc (150 mL). The resulting organic layers were combined and dried over Na₂SO₄ and concentrated. The residue (2.4 g) was dissolved in THF (73 mL). To this stirring solution was added KHMDS (3.46 g, 0.017 mol) and iodomethane (10.3 g, 0.072 mol), and the mixture was allowed to stir for 20 min. The solvent was then concentrated, and the crude product was partitioned between EtOAc (600 mL) and NaHCO₃ (200 mL). The organic layer was washed with NaHCO₃ (150 mL), dried over Na₂SO₄, and concentrated. Purification using flash chromatography (silica, 80% EtOAc/hexanes) afforded 2.4 g (67% yield, 3 steps, based upon using 2/3 material at methylation stage) of desired product as a white solid. MS (electrospray): exact mass calculated for C₁₉H₂₉N₅O₃, 375.23; *m*/*z* found, 276.17 [M+H-100]⁺. ¹H NMR: (400MHz, CDCl₃): 7.02 (d, *J* = 8.6 Hz, 1 H), 6.15 (d, *J* = 8.6 Hz, 1 H), 4.46 (tt, *J* = 12.0 Hz and 4.0 Hz, 1 H), 4.38-4.11(m, 2H), 3.33 (s, 3H), 3.01 (s, 6H), 2.95-2.73 (m, 2H), 2.73-2.55 (m, 2H), 1.77-1.61 (m, 2H), 1.47 (s, 9H).

### D. 5-Dimethylamino-1-methyl-3-piperidin-4-yl-1,3-dihydro-imidazo[4,5-b]pyridin-2-one.

To a stirring solution of 1.07 g (0.0028 mol) of 4-(5-dimethylamino-1-methyl-2-oxo-1,2-dihydro-imidazo[4,5-b]pyridin-3-yl)-piperidine-1-carboxylic acid tert-butyl ester in CH₂Cl₂ (7 mL) was added 7 mL of TFA. After 35 min, the solvent was removed. The residue was partitioned between EtOAc (200 mL) and 1 *N* NaOH (150 mL). The aqueous layer was extracted with EtOAc (3 x 100 mL) and the combined organic layers were dried over Na₂SO₄ and concentrated to afford 0.74 g (96%) of 5-dimethylamino-1-methyl-3-piperidin-4-yl-1,3-dihydroimidazo[4,5-b]pyridin-2-one as a white/pink solid. ¹H NMR (400MHz, CDCl₃): 6.95 (d, *J* = 8.3 Hz, 1 H), 6.08 (d, *J* = 8.3 Hz, 1 H), 4.35 (tt, *J* = 12.1 Hz, 4.0 Hz, 1H), 3.25 (s, 3H), 3.14 (d, *J* = 12.4 Hz, 2H) 2.97 (s, 6H), 2.66 (td, *J* = 12.9 Hz, 1.3 Hz, 2H), 2.53 (qd, *J* = 12.4 Hz, 4.0 Hz, 2H), 1.69 (d, *J* = 11.9 Hz, 2H).

### E. (S)-5-Dimethylamino-3-(1-{2-hydroxy-3-[5-methanesulfonyl-3-(4-trifluoromethyl-phenyl)-4,5,6,7-tetrahydro-pyrazolo[4,3-c]pyridin-1-yl]-propyl} piperidin-4-yl)-1-methyl-1,3-dihydro-imidazo[4,5-b]pyridin-2-one.

To a stirring solution of 0.24 g (0.0009 mol) of 5-dimethylamino-1-methyl-3-piperidin-4-yl-1,3-dihydro-imidazo[4,5-b]pyridin-2-one in EtOH/Dichloroethane (1.5 mL/1.5 mL) was added 0.23 g (0.0005 mol) of (*R*)-5-methanesulfonyl-1-oxiranylmethyl-3-(4-trifluoromethyl-phenyl)-4,5,6,7-tetrahydro-1H-pyrazolo[4,3-c]pyridine. The reaction mixture was heated to 80°C and stirred for 16 h and concentrated. The crude product was then dissolved in CH₂Cl₂ (40 mL) and purified using flash chromatography (0-6% MeOH/CH₂Cl₂) affording 0.38 g (97%) of the desired product as a white solid. MS (electrospray): exact mass calculated for C₃₁H₃₉F₃N₈O₄S, 676.28; *m*/*z* found, 677.28 [M+H]⁺. ¹H NMR (400 MHz, CDCl₃):7.71 and 7.67 (A and B of AA'BB' quartet , *J_{ab}* = 8.3 Hz, 4H), 7.03 (d, *J* = 8.6 Hz, 1 H), 6.16 (d, *J* = 8.6 Hz, 1 H), 4.58 and 4.56 (A and B of AB quartet, *J_{ab}* = 14.5 Hz, 2H), 4.36 (tt, *J* = 12.1 Hz, 4.04 Hz, 1 H), 4.25-4.01 (m, 4H), 3.77-3.60 (m, 2H), 3.33 (s, 3H), 3.16-3.04 (m, 2H), 3.03 (s, 6H), 2.99-2.90 (m, 2H), 2.88 (s, 3H), 2.77 (qd, *J* = 12.1 Hz, 3.54 Hz, 2H), 2.56-2.42 (m, 3H), 2.21 (t, *J* =11.6Hz, 1 H), 1.75 (d, *J* = 11.6 Hz, 2H).

### EXAMPLE 32

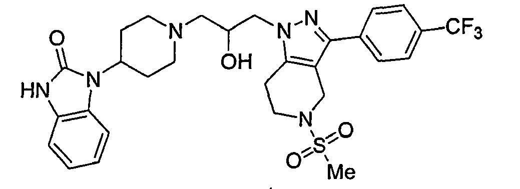

### 1-(1-{2-Hydroxy-3-[5-methanesulfonyl-3-(4-trifluoromethyl-phenyl)-4,5,6,7-tetrahydro-pyrazolo[4,3-c]pyridin-1-yl]-propyl}-piperidin-4-yl)-1,3-dihydro-benzoimidazol-2-one.

### EXAMPLE 33

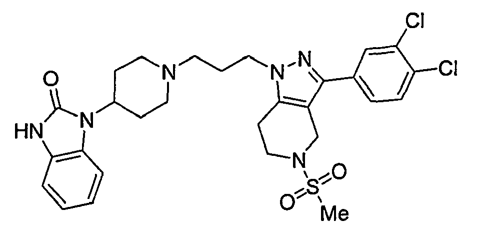

### 1-(1-{3-[3-(3,4-Dichloro-phenyl)-5-methanesulfonyl-4,5,6,7-tetrahydropyrazolo[4,3-c]pyridin-1-yl]-propyl}-piperidin-4-yl)-1,3-dihydro-benzoimidazol-2-one.

### EXAMPLE 34

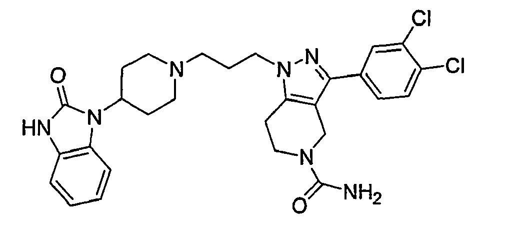

### 3-(3,4-Dichloro-phenyl)-1-{3-[4-(2-oxo-2,3-dihydro-benzoimidazol-1-yl)-piperidin-1-yl]-propyl}-1,4,6,7-tetrahydro-pyrazolo[4,3-c]pyridine-5-carboxylic acid amide.

### EXAMPLE 35

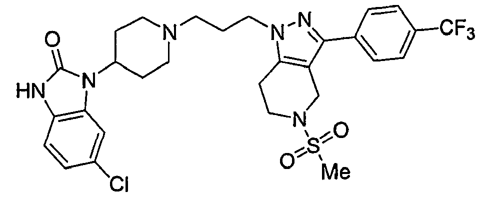

### 6-Chloro-1-(1-{3-[5-methanesulfonyl-3-(4-trifluoromethyl-phenyl)-4,5,6,7-tetrahydro-pyrazolo[4,3-c]pyridin-1-yl]-propyl}-piperidin-4-yl)-1,3-dihydro-benzoimidazol-2-one.

### EXAMPLE 36

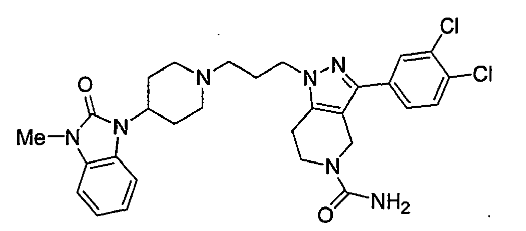

### 3-(3,4-Dichloro-phenyl)-1-{3-[4-(3-methyl-2-oxo-2,3-dihydro-benzoimidazol-1-yl)-piperidin-1-yl]-propyl}-1,4,6,7-tetrahydro-pyrazolo[4,3-c]pyridine-5-carboxylic acid amide.

### EXAMPLE 37

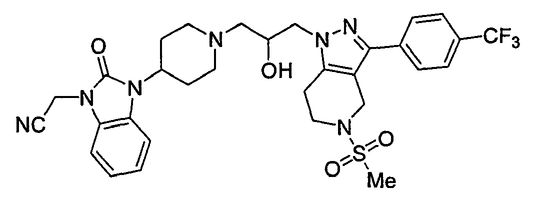

### [3-(1-{2-Hydroxy-3-[5-methanesulfonyl-3-(4-trifluoromethyl-phenyl)-4,5,6,7-tetrahydro-pyrazolo[4,3-c]pyridin-1-yl]-propyl}-piperidin-4-yl)-2-oxo-2,3-dihydro-benzoimidazol-1-yl]-acetonitrile.

### EXAMPLE 38

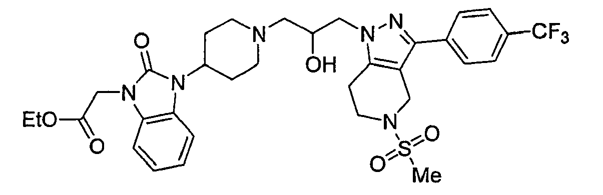

### [3-(1-{2-Hydroxy-3-[5-methanesulfonyl-3-(4-trifluoromethyl-phenyl)-4,5,6,7-tetrahydro-pyrazolo[4,3-c]pyridin-1-yl]-propyl}-piperidin-4-yl)-2-oxo-2,3-dihydro-benzoimidazol-1-yl]-acetic acid ethyl ester.

### EXAMPLE 39

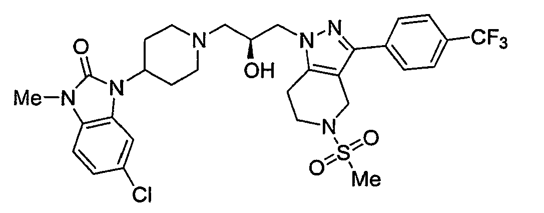

### 5-Chloro-3-(1-{2-hydroxy-3-[5-methanesulfonyl-3-(4-trifluoromethyl-phenyl)-4,5,6,7-tetrahydro-pyrazolo[4,3-c]pyridin-1-yl]-propyl}-piperidin-4-yl)-1-methyl-1,3-dihydro-benzoimidazol-2-one.

### EXAMPLE 40

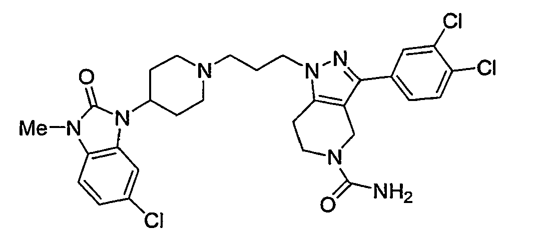

### 1-{3-[4-(6-Chloro-3-methyl-2-oxo-2,3-dihydro-benzoimidazol-1-yl)-piperidin-1-yl]-propyl}-3-(3,4-dichloro-phenyl)-1,4,6,7-tetrahydro-pyrazolo[4,3-c]pyridine-5-carboxylic acid amide.

### EXAMPLE 41

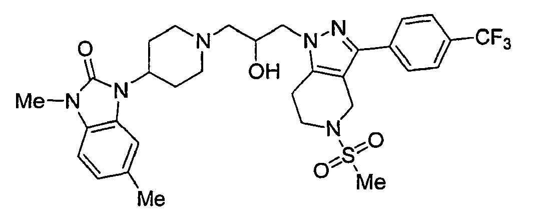

### 3-(1-{2-Hydroxy-3-[5-methanesulfonyl-3-(4-trifluoromethyl-phenyl)-4,5,6,7-tetrahydro-pyrazolo[4,3-c]pyridin-1-yl]-propyl}-piperidin-4-yl)-1,5-dimethyl-1,3-dihydro-benzoimidazol-2-one.

### EXAMPLE 42

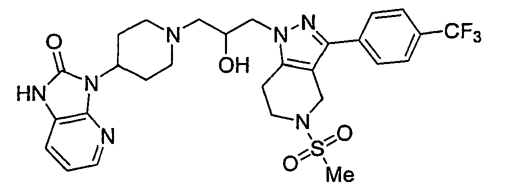

### 3-(1-{2-Hydroxy-3-[5-methanesufonyl-3-(4-trifluoromethyl-phenyl)-4,5,6,7-tetrahydro-pyrazolo[4,3-c]pyridin-1-yl]-propyl}-piperidin-4-yl)-1,3-dihydroimidazo[4,5-b]pyridin-2-one.

### EXAMPLE 43

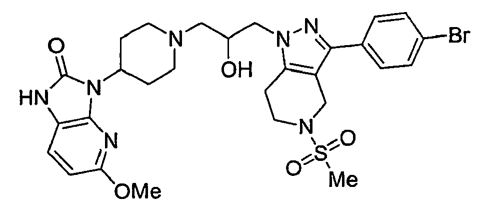

### 3-(1-{3-[3-(4-Bromo-phenyl)-5-methanesulfonyl-4,5,6,7-tetrahydro-pyrazolo[4,3-c]pyridin-1-yl]-2-hydroxy-propyl}-piperidin-4-yl)-5-methoxy-1,3-dihydroimidazo[4,5-b]pyridin-2-one.

### EXAMPLE 44

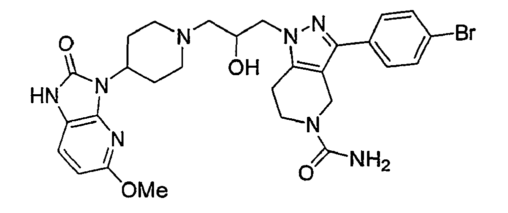

### 3-(4-Bromo-phenyl)-1-{2-hydroxy-3-[4-(5-methoxy-2-oxo-1,2-dihydroimidazo[4,5-b]pyridin-3-yl)-piperidin-1-yl]-propyl}-1,4,6,7-tetrahydropyrazolo[4,3-c]pyridine-5-carboxylic acid amide.

### EXAMPLE 45

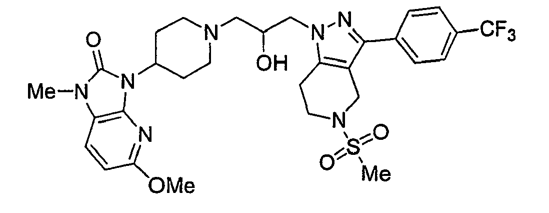

### 3-(1-{2-Hydroxy-3-[5-methanesulfonyl-3-(4-trifluoromethyl-phenyl)-4,5,6,7-tetrahydro-pyrazolo[4,3-c]pyridin-1-yl]-propyl}-piperidin-4-yl)-5-methoxy-1-methyl-1,3-dihydro-imidazo[4,5-b]pyridin-2-one.

### EXAMPLE 46

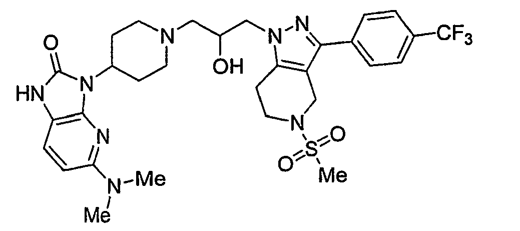

### 5-Dimethylamino-3-(1-{2-hydroxy-3-[5-methanesulfonyl-3-(4-trifluoromethylphenyl)-4,5,6,7-tetrahydro-pyrazolo[4,3-c]pyridin-1-yl]-propyl}-piperidin-4-yl]-1,3-dihydro-imidazo[4,5-b]pyridin-2-one.

### EXAMPLE 47

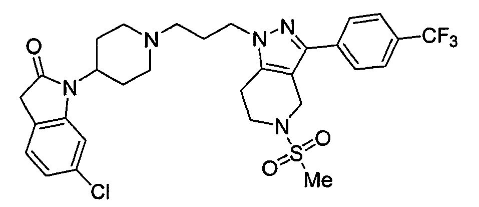

### 6-Chloro-1-(1-{3-[5-methanesulfonyl-3-(4-trifluoromethyl-phenyl)-4,5,6,7-tetrahydro-pyrazolo[4,3-c]pyridin-1-yl]-propyl}-piperidin-4-yl)-1,3-dihydro-indol-2-one.

### EXAMPLE 48

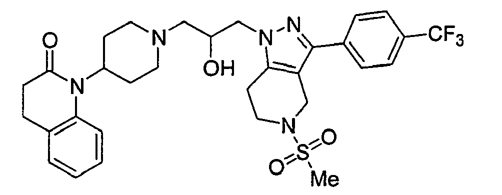

### 1-(1-{2-Hydroxy-3-[5-methanesulfonyl-3-(4-trifluoromethyl-phenyl)-4,5,6,7-tetrahydro-pyrazolo[4,3-c]pyridin-1-yl]-propyl}-piperidin-4-yl)-3,4-dihydro-1H-quinolin-2-one.

### EXAMPLE 49

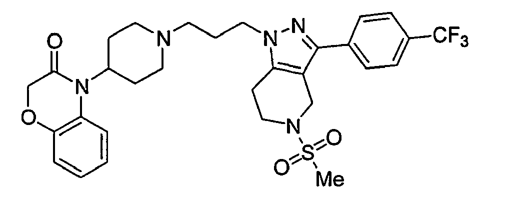

### 4-(1-{3-[5-Methanesulfonyl-3-(4-trifluoromethyl-phenyl)-4,5,6,7-tetrahydro-pyrazolo[4,3-c]pyridin-1-yl]-propyl}-piperidin-4-yl)-4H-benzo[1,4]oxazin-3-one.

### EXAMPLE 50

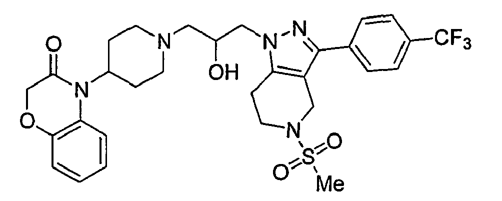

### 4-(1-{2-Hydroxy-3-[5-methanesulfonyl-3-(4-trifluoromethyl-phenyl)-4,5,6,7-tetrahydro-pyrazolo[4,3-c]pyridin-1-yl]-propyl}-piperidin-4-yl)-4H-benzo[1,4]oxazin-3-one.

### EXAMPLE 51

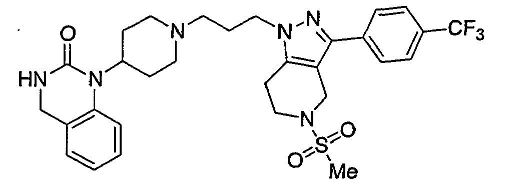

### 1-(1-{3-[5-Methanesulfonyl-3-(4-trifluoromethyl-phenyl)-4,5,6,7-tetrahydropyrazolo[4,3-c]pyridin-1-yl]-propyl}-piperidin-4-yl)-3,4-dihydro-1H-quinazolin-2-one.

### EXAMPLE 52

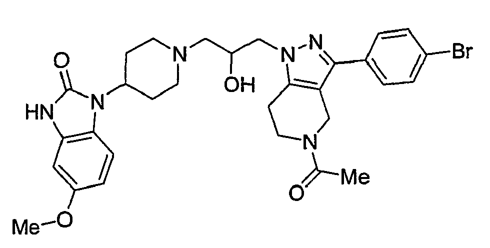

### 1-(1-{3-[5-Acetyl-3-(4-bromo-phenyl)-4,5,6,7-tetrahydro-pyrazolo[4,3-c]pyridin-1-yl]-2-hydroxy-propyl}-piperidin-4-yl)-5-methoxy-1,3-dihydro-benzoimidazol-2-one

### EXAMPLE 53

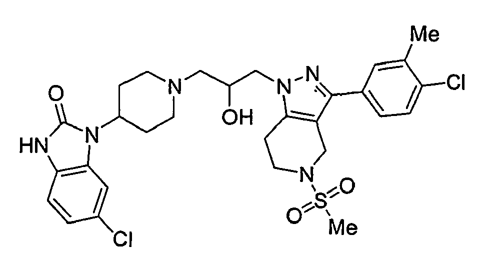

### 6-Chloro-1-(1-{3-[3-(4-chloro-3-methyl-phenyl)-5-methanesulfonyl-4,5,6,7-tetrahydro-pyrazolo[4,3-c]pyridin-1-yl]-2-hydroxy-propyl}-piperidin-4-yl)-1,3-dihydro-benzoimidazol-2-one

### EXAMPLE 54

### Cathepsin S Inhibition Assay.

Recombinant human cathepsin S (CatS) was expressed in the baculovirus system and purified in one step with a thiopropyl-sepharose column. 10-L yielded ~700 mg of CatS and N-terminal sequencing confirmed identity. The assay is run in 100 mM sodium acetate pH 5.0 containing 1 mM DTT and 100 mM NaCl. The substrate for the assay is
(Aedens)EKARVLAEAA(Dabcyl)K-amide

The Kₘ for the substrate is around 5 µM but the presence of substrate inhibition makes kinetic analysis difficult. With 20 µM substrate the assay rate is linear over the range of 1-8 ng CatS in 100 µl reaction. Using 2 ng/well of CatS, the production of product is linear and yields -7-fold signal after 20 min with only 20% loss of substrate. Primary assays are run by quenching the reaction after 20 min with 0.1 % SDS and then measuring the fluorescence. For other assays, measurements are taken every min for 20 min. The rate is calculated from the slope of the increase and the percent inhibition is calculated from this (See Tables 1 and 2 below).

**Table 1**

| EXAMPLE | lC₅₀ (µM) |
|---|---|
| 1 | 0.73 |
| 2 | 0.07 |
| 3 | 0.28 |
| 4 | 0.19 |
| 5 | 1.16 |
| 6 | 0.19 |
| 7 | 0.26 |
| 8 | 0.04 |
| 9 | 0.10 |
| 10 | 0.09 |
| 11 | 0.03 |
| 12 | 0.62 |
| 13 | 0.37 |
| 14 | 0.29 |
| 15 | 0.23 |
| 16 | 0.30 |
| 17 | 1.30 |
| 18 | 0.25 |
| 19 | 0.02 |
| 20 | 0.01 |
| 21 | 0.02 |
| 22 | 0.03 |
| 23 | 0.08 |
| 24 | 0.03 |
| 25 | 0.23 |
| 26 | 0.18 |
| 27 | 0.09 |
| 28 | 0.89 |
| 29 | 0.78 |
| 30 | 0.04 |
| 31 | 0.07 |

**Table 2**

| | |
|---|---|
| EXAMPLE | IC₅₀ (µM) |
| 32 | 0.06 |
| 33 | 0.01 |
| 34 | 0.02 |
| 35 | 0.03 |
| 36 | 0.04 |
| 37 | 0.05 |
| 38 | 0.02 |
| 39 | 0.04 |
| 40 | 0.04 |
| 41 | 0.03 |
| 42 | 0.08 |
| 43 | 0.02 |
| 44 | 0.03 |
| 45 | 0.02 |
| 46 | 0.03 |
| 47 | 0.04 |
| 48 | 0.02 |
| 49 | 0.02 |
| 50 | 0.02 |
| 51 | 0.02 |
| 52 | 0.13 |
| 53 | 0.05 |

### Example 55

### Ex vivo inhibition by cathepsin S inhibitors of the allergenic response

The following assay demonstrates that cathepsin S inhibitors block the response of human T cells to crude allergen extracts.

### Materials and Methods.

Reagents. Glycerinated crude allergen extracts of house dust mites (*Dermataphagoides pteronyssinus, Dermataphagoides farinae*) and ragweed [*Ambrosia trifida* (giant), *Ambrosia artemisiifolia* (short)] were purchased from Hollister-Stier Laboratories (Minneapolis, MN). Concanavalin A (ConA) was purchased from Calbiochem (La Jolla, CA).

Donors. All allergic donors were prescreened for their specific allergies using RAST tests. The HLA class II haplotypes of these donors were determined using PCR.

Cell culture. Human peripheral blood mononuclear cells (PBMC) were purified from blood of allergic donors using Ficoll-Hypaque gradient followed by washes with phosphate buffered saline (PBS). PBMC were cultured in triplicate or duplicate at 0.5-1.0 x 10⁶ cells/well with titrated doses of allergen extracts, in the presence or absence of a known cathepsin S inhibitor, LHVS (morpholinurea-leucine-homo-phenylalanine-vinylsuffonephenyl) (Palmer et al. (1995), J. Med. Chem. 38:3193 and Riese et al. (1996), Immunity 4:357). Serial diluted stock solutions of LHVS were first made in 100% DMSO and then diluted 1:15 in 40% Hydroxypropynyl cyclodextrin (HPCD). Three microliters of LHVS in HPCD was added into PBMC cultures (200 µL/well). After 6 days of culture, 1 µCi/well of ³H-thymidine (TdR) was added. Eighteen hours later, cells were harvested using a Filtermate Harvester (Packard) and counted for ³H-TdR incorporation on Topcount (Packard).

### Inhibition of T cell proliferative responses to house dust mites.

About 10% of most populations are allergic to house dust mites (HDM) of the genus *Dermatophagoides* with *Dermatophagoides pteronyssinus* (Der p) and *D. farinae* (Der f) being the two major species present in varying proportions in most countries. The major clinical manifestations are asthma and perennial rhinitis.

Effect of cathepsin S inhibition on activation of HDM allergen-specific CD4 T cells was tested in an *ex vivo* human T cell-proliferation assay. Culturing PBMC with crude extracts from either *Der p* or *Der f,* resulted in strong proliferation (Figure 1A). This proliferation consisted primarily of allergen-specific CD4 T cells. When cathepsin S activity was blocked by a specific cathepsin S inhibitor, LHVS (cf. Riese et al. (1996) Immunity 4:357) the proliferation was strongly inhibited (Figure 1 B). Inhibition by LHVS was specific for responses induced by HDM extracts since T cell proliferative responses induced by ConA, a pan-T cell mitogen, were not affected. Furthermore, this inhibition was observed for all four HDM-allergic donors tested regardless of the different HLA class II haplotypes (DR4; DR7, 15; DR11, 15; and DR4, 11).

This system is very similar to an *in vivo* situation. The allergic subject would be exposed to a crude mixture of allergens that would lead to the proliferation of T cells and an allergic response. The observation of inhibition of CD4 T cell activation by a cathepsin S inhibitor shows that such inhibitors can be effective in treating a generalized population of patients allergic to house dust mites.

### Inhibition of T cell proliferative responses to ragweed

About 10% of population in US are allergic to ragweed pollen, making it one of the most important allergens in terms of clinical diseases. Allergens from pollens are a common precipitant of rhinitis and asthma in this population.

The effect of cathepsin S inhibition on activation of ragweed allergen-specific CD4 T cells was tested in an *ex vivo* human T cell-proliferation assay. Culturing PBMC with crude extracts from both short and giant ragweed resulted in strong proliferation (Figure 2A). This proliferation consisted mainly of allergen-specific CD4 T cells. When cathepsin S activity was blocked by a specific cathepsin S inhibitor, LHVS (cf. Riese et al. (1996) Immunity 4:357) the proliferation was strongly inhibited (Figure 2B). Inhibition by LHVS was specific for responses induced by ragweed since T cell proliferative responses induced by ConA, a pan-T cell mitogen, were not affected. Furthermore, this inhibition was observed for the two ragweed-allergic donors tested regardless of the different HLA class II haplotypes (DR7, 15 and DR4, 11).

Similar experiments were run using three additional CatS inhibitors, compounds from Examples 8, 52, and 53 above, with the results shown in FIGS. 3A , 3B, 4A, and 4B.

This system is very similar to an *in vivo* situation. The allergic subject would be exposed to a crude mixture of allergens that would lead to the proliferation of T cells and an allergic response. The observation of inhibition of CD4 T cell activation by a cathepsin S inhibitor shows that such inhibitors can be effective in treating a generalized population of patients allergic to ragweed.

### Example 56

### Monitoring cathepsin S inhibition in human blood.

The effect of *in vivo* administration of cathepsin S inhibitors, in a clinical trial setting, can be monitored by measuring accumulation of an intermediate degradation product of invariant chain (li), i.e. the p10li fragment, in blood of dosed subjects. After administration of a cathepsin inhibitor for a certain period of time, for example, between 0.01 and 50 mg/kg/day, to result in a blood concentration of between 1 nM-10 µM, for 16-30 h, blood is drawn and white blood cells are purified, e.g. either by lysis of red blood cells or by a Ficoll-Hypaque gradient centrifugation. Whole cell lysates of WBC are then made and analyzed by either a Western blot assay or an ELISA assay. For the Western blot assay, cell lysates are first resolved on SDS-PAGE gels. After - transferring to nitrocellulose membranes, li and its intermediate degradation products, including the p10li, can be detected using a mouse mAb against li, e.g. Pin 1.1, or rabbit polyclonal antibodies specific for the C-terminus of the p10li fragment or against the entire p10li fragment. For ELISA assay, a pair of antibodies against li, including Pin1.1, and a rabbit polyclonal antibody or a mouse monoclonal antibody specific for p10li, can be used. The same assay can also be applied to monitor the effect of cathepsin S inhibitors *in vivo* in animal studies, for example in monkeys, dogs, pigs, rabbits, guinea pigs, and rodents.

In the present example PBMC from human blood were incubated with the cathepsin S inhibitor, LHVS (morpholinurea-leucine-homo-phenylalanine-vinylsulfonephenyl, also referred to as 4-morpholinecarboxamide, N-[(1S)-3-methyl-1-[[[(1S,2E)-1-(2-phenylethyl)-3-(phenylsulfonyl)-2-propenyl]amino]carbonyl]butyl]-. This compound has been described in US Patent No. 5,976,858 and in Palmer et al. (1995) J. Med. Chem. 38:3193 and Riese et al. (1996) Immunity 4:357. After incubation for 24 h the samples were run using standard SDS-PAGE protocols, transferred to nitrocellulose membranes and probed with an antibody that recognizes the invariant chain including the p10li fragment. In the presence of LHVS the p10li fragment was seen, representing a block in the degradation of li due to inhibition of cathepsin S.

### Example 57

### Monitoring in vivo inhibition of allergenic response by cathepsin S inhibitors.

To demonstrate the efficacy of cathepsin S inhibitors for suppressing allergic responses *in vivo,* allergic volunteers are dosed with cathepsin S inhibitors to levels where invariant chain degradation is inhibited. Allergens are deposited subcutaneously, and the size of the cutaneous reactions are determined at 15 min, 6 h and 24 h. Skin biopsies are performed at 24 h. The immediate weal and flare response is not mediated by a T cell response and is not expected to be influenced by cathepsin S inhibitors, while the late phase induration (noticeable at 6 hours, more pronounced at 24 hours) is characterized by activation and infiltration of CD4 T cells (as well as of eosinophils) and should be inhibited by administration of inhibitors of cathepsin S. The skin biopsies are used to determine the cellular composition in the induration, and cathepsin S treated subjects are expected to have fewer activated CD4 T cells present than placebo-treated subjects.

References for these procedures are provided in Eberlein-Konig et al. (1999) Clin. Exp. Allergy 29:1641-1647 and in Gaga et al. (1991) J. Immunol. 147:816-822.

As controls for the experiment, prednisone and cyclosporine A will be used. Prednisone will inhibit both the immediate and the late phase responses, while cyclosporin A will inhibit only the late phase response.

### F. Other Embodiments

The features and advantages of the invention are apparent to one of ordinary skill in the art. Based on this disclosure, including the summary, detailed description, background, examples, and claims, one of ordinary skill in the art will be able to make modifications and adaptations to various conditions and usages. These other embodiments are also within the scope of the invention.

## Claims

1. The use of a compound in the manufacture of a pharmaceutical composition for treating a subject with an allergic condition, said compound being of formula (I): 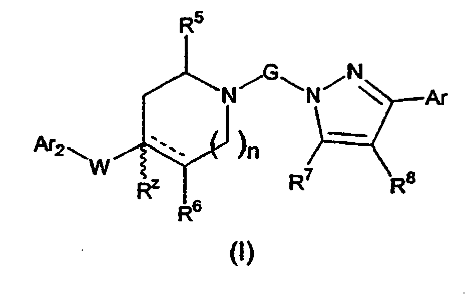 wherein:
Ar₂ is a monocyclic or bicyclic ring system, unsaturated, saturated or aromatic, optionally fused, optionally including between 1 and 5 heteroatom ring moieties independently selected from O, S, N, SO₂, and C=O; said Ar₂ ring system being optionally substituted with between 1 and 4 substituents;
R⁵ and R⁶ are independently selected from hydrogen and C₁₋₆ alkyl;
R⁷ and R⁸ are independently hydrogen, C₁₋₅ alkyl, C₂₋₆ alkenyl, C₁₋₅ alkoxy, C₁₋₅ alkylthio, halogen, or a 4-7 membered carbocyclyl or heterocyclyl; alternatively, R⁷ and R⁸ can be taken together to form an optionally substituted 5- to 7- membered carbocyclic or heterocyclic ring, which ring may be unsaturated or aromatic, and may be optionally substituted with between one and three substituents independently selected from halo, cyano, amino, hydroxy, nitro, R⁴, R⁴O-, R⁴S-, R⁴O(C₁₋₅ alkylene)-, R⁴O(C=O)-, R⁴(C=O)-, R⁴(C=S)-, R⁴(C=O)O-, R⁴O(C=O)(C=O)-, R⁴SO₂, NHR⁴⁴(C=NH)-, NHR⁴⁴SO₂-, and NHR⁴⁴(C=O)-;
R⁴ is H, C₁₋₆alkyl, C₂₋₅ alkenyl, C₁₋₆ heterocyclyl, (C₁₋₅ heterocyclyl)C₁₋₆ alkylene, phenyl benzyl, phenethyl, NH₂, mono- or di(C₁₋₆alkyl)N-, (C₁₋₆ alkoxy)carbonyl- or R⁴²OR⁴³-, wherein R⁴² is H, C₁₋₅ alkyl, C₂₋₅ alkenyl, phenyl, benzyl, phenethyl, C₁₋₅ heterocyclyl, or (C₁₋₆ heterocyclyl)C₁₋₆ alkylene and R⁴³ is C₁₋₅ alkylene, phenylene, or divalent C₁₋₅ heterocyclyl;
R⁴⁴ can be any of the values for R⁴;
n is 0, 1, or 2;
G is C₃₋₆ alkenediyl or C₃₋₆ alkanediyl, optionally substituted with hydroxy, halogen, C₁₋₅alkoxy, C₁₋₅alkyl, oxo, hydroximino, CO₂R^{k}, R^{k}R^{l}N, R^{k}R^{l}NCO₂, (L)-C₁₋₄ alkylene-, (L)-C₁₋₅ alkoxy, N₃, or [(L)-C₁₋₅ alkylene]amino;
each of R^{k} and R^{l} is independently hydrogen, C₁₋₆alkyl, C₃₋₅ alkenyl, phenyl, benzyl, phenethyl, or C₁₋₅ heterocyclyl; alternatively R^{k} and R^{l}, can be taken together to form an optionally substituted 4- to 7- membered heterocyclic ring, which ring may be saturated, unsaturated or aromatic;
L is amino, mono- or di-C₁₋₅ alkylamino, pyrrolidinyl, morpholinyl, piperidinyl homopiperidinyl, or piperazinyl, wherein available ring nitrogens may be optionally substituted with C₁₋₅ alkyl, benzyl, C₂₋₆ acyl, C₁₋₆alkylsulfonyl, or C₁₋₆ alkoxycarbonyl;
Ar represents a monocyclic or bicyclic aryl or heteroaryl ring, optionally substituted with between 1 and 3 substituents independently selected from halogen, C₁₋₅ alkoxy, C₁₋₅ alkyl, C₂₋₅ alkenyl, cyano, azido, nitro, R²²R²³N, R²⁴SO₂, R²⁴S, R²⁴SO, R²⁴OC=O, R²²R²³NC=O, C₁₋₅ haloalkyl, C₁₋₅haloalkoxy, C₁₋₅haloalkylthio, and C₁₋₅ alkylthio;
R²² is hydrogen, C₁₋₆ alkyl, C₃₋₅ alkenyl, phenyl, phenethyl, benzyl, or C₁₋₅ heterocyclyl, C₂₋₈ acyl, aroyl, R³⁸OC=O, R²⁵R²⁶NC=O, R³⁸SO, R³⁸SO₂, R³⁸S or R²⁵R²⁶NSO₂;
R³⁸ is H, C₁₋₅ alkyl, C₃₋₅ alkenyl, phenyl, benzyl, phenethyl C₁₋₅ heterocyclyl;
R²³ is hydrogen, C₁₋₅ alkyl, C₃₋₅ alkenyl, phenyl, benzyl or C₁₋₅ heterocyclyl;
alternatively, R²² and R²³ can be taken together to form an optionally substituted 4- to 7- membered heterocyclic ring, which ring may be saturated, unsaturated or aromatic;
R²⁴ is C₁₋₅ alkyl, C₃₋₅ alkenyl, phenyl, benzyl, or C₁₋₅ heterocyclyl;
R²⁵ and R²⁶ independently are hydrogen, C₁₋₅ alkyl, C₃₋₅ alkenyl, phenyl, benzyl, or C₁₋₅ heterocyclyl;
or, alternatively, R²⁵ and R²⁶ can be taken together to form an optionally substituted 4- to 7-membered heterocyclic ring, which ring may be saturated, unsaturated or aromatic;
W represents O, S, NR²⁷, C=O, (C=O)NH, NH(C=O), CHR²⁸, or a covalent bond;
R^{z} is H or OH and the dashed line is absent; or R^{z} is absent where the dashed line is an sp² bond;
R²⁷ is hydrogen, C₁₋₅alkyl, C₃₋₅ alkenyl, phenyl, naphthyl, benzyl, phenethyl,
C₁₋₅ heterocyclyl, C₂₋₈ acyl, aroyl, R²⁹OC=O, R³⁰R³¹NC=O, R²⁹SO, R²⁹S, R²⁹SO₂, or R³⁰R³¹NSO₂ ;
or, alternatively, R²⁷ and part of Ar₂ can be taken together to form an optionally substituted 5- or 6-membered heterocyclic ring with optionally 1 to 3 additional heteroatom moieties in the ring selected from O, NR⁹, NR¹⁰, N, SO₂, C=O, and S; which ring may be saturated, unsaturated or aromatic; R⁹ and R¹⁰ are independently selected from H, C₁₋₃ alkyl, and -CH₂CO₂(C₁₋₄ alkyl);
R²⁸ is hydrogen, C₁₋₅ alkyl, C₃₋₅ alkenyl, hydroxy, phenyl, benzyl, C₁₋₅ heterocyclyl, R²⁹O, -R³⁰R³¹NC=O, R²⁹S, R²⁹SO, R²⁹SO₂, or R³⁰R³¹NSO₂;
R²⁹ is C₁₋₅, alkyl, C₃₋₅ alkenyl, phenyl, benzyl, or C₁₋₅ heterocyclyl;
R³⁰ and R³¹ are independently selected from hydrogen, C₁₋₅ alkyl, C₃₋₅ alkenyl, phenyl, benzyl, phenethyl, naphthyl, and C₁₋₅ heteroaryl; alternatively, R³⁰ and R³¹ can be taken together to form an optionally substituted 4- to 7-membered ring carbocyclic or heterocyclic ring, which ring may be saturated, unsaturated or aromatic;
wherein each of the above hydrocarbyl or heterocarbyl groups, unless otherwise indicated, and in addition to any specified substituents, is optionally and independently substituted with between 1 and 3 substituents selected from methyl, halomethyl, hydroxymethyl, halo, hydroxy, amino, nitro, cyano, C₁₋₅ alkyl, C₁₋₅ alkoxy, -COOH, C₂₋₆ acyl, [di(C₁₋₄ alkyl)amino]C₂₋₅ alkylene, [di(C₁₋₄ alkyl)amino] C₂₋₅ alkyl-NH-CO-, and C₁₋₅ haloalkoxy;
or a pharmaceutically acceptable salt, amide, or ester thereof; or a stereoisomeric form thereof.

2. A use of claim 1, wherein Ar₂ is selected from 2,5-di(C₁₋₆ alkyl)aminopyrrolyl and the following 6 formulae: 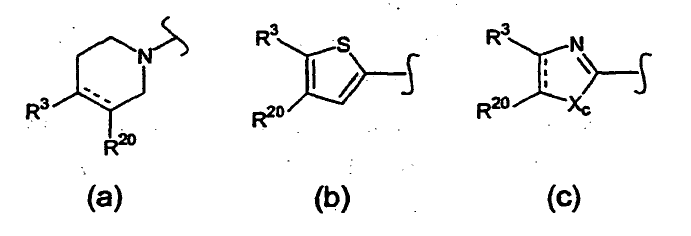 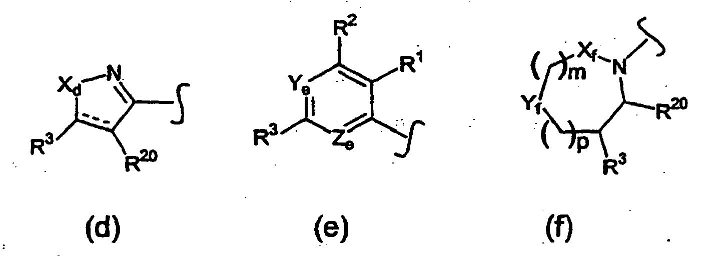 wherein
each dashed line may be an sp² bond or absent;
X_{c} is O, S, or N; and X_{d} is O or S;
R¹ is hydrogen, halogen, C₁₋₅ alkoxy, hydroxy, C₁₋₆alkyl, C₂₋₅ alkenyl, cyano, nitro, R^{a}R^{b}N, C₂₋₈ acyl, C₁₋₅ heterocyclyl, (C₁₋₅ heterocyclyl)C₁₋₅ alkylene, R¹¹S, R¹¹SO, R¹¹SO₂, R^{c}OC=O, R^{c}R^{d}NC=O, or R^{c}R^{d}NSO₂; or R¹ can be taken together with R²⁷ as provided below,
R² is hydrogen, halogen, C₁₋₅ alkoxy, hydroxy, C₁₋₅ alkyl, C₂₋₅ alkenyl, cyano, nitro, R^{a}R^{f}N, C₁₋₅ heterocyclyl, or C₂₋₆ acyl;
R³ is hydrogen, halogen, C₁₋₅ alkoxy, hydroxy, C₁₋₅ alkyl, C₂₋₅ alkenyl, cyano, nitro, R^{g}R^{h}N, C₂₋₈ acyl, C₁₋₅ heterocyclyl, R^{h}OC=O, R^{g}R^{h}NC=O, or R^{g}R^{h}NSO₂;
R^{a} is selected from hydrogen, C₁₋₅alkyl, C₃₋₅ alkenyl, phenyl, benzyl, phenethyl, C₁₋₅ heterocyclyl, C₂₋₈ acyl, aroyl, RⁱOC=O, RⁱR^{j}NC=O, R¹²SO, R¹²SO₂, R¹²S, and RⁱR^{j}NSO₂;
R^{e} is selected from hydrogen, C₁₋₅alkyl, C₃₋₅ alkenyl, phenyl; benzyl, phenethyl, C₁₋₅ heterocyclyl, C₂₋₈ acyl, aroyl, R³²OC=O, R³²R³³NC=O, R¹³SO, R¹³SO₂, R¹³S, and R³²R³³NSO₂;
R^{m} is selected from hydrogen, C₁₋₅ alkyl, C₃₋₅ alkenyl, phenyl, benzyl, phenethyl, C₁₋₅ heterocyclyl, C₂₋₈ acyl, aroyl, R³⁴OC=O, R³⁴R³⁵NC=O, R¹⁵SO, R¹⁵SO₂, R¹⁵S, and R³⁴R³⁵NSO₂
R^{o} is selected from hydrogen, C₁₋₅ alkyl, C₃₋₅ alkenyl, phenyl, benzyl, phenethyl, C₁₋₅ heterocyclyl, C₂₋₈ acyl, aroyl, R³⁶OC=O, R³⁸R³⁷NC=O, R¹⁹SO, R¹⁹SO₂, R¹⁹S, and R³⁸R³⁷NSO₂;
each of R^{b}, R^{f}, Rⁿ, R^{p}, R³², R³³, R³⁴, R³⁵, R³⁶ , R³⁷, R³⁹, and R⁴⁰ is independently selected from hydrogen, C₁₋₅ alkyl, C₃₋₅ alkenyl, phenyl, benzyl, phenethyl, and C₁₋₅ heteroaryl;
alternatively, R^{a} and R^{b}, R^{e} and R^{f}, R^{m} and Rⁿ, and R^{o} and R^{p}, independently, can be taken together to form an optionally substituted 4-to 7- membered heterocyclic ring, which ring may be saturated, unsaturated or aromatic;
each of R¹¹, R¹², R¹³, R¹⁴ , R¹⁵, R¹⁶, R¹⁹ , R³⁸, and R⁴¹ is independently C₁₋₅ alkyl, C₃₋₅ alkenyl, phenyl, benzyl, phenethyl, or C₁₋₅ heterocyclyl;
each of R^{c} and R^{d}, and Rⁱ and R^{j} are independently, are hydrogen, C₁₋₅ alkyl, C₃₋₅ alkenyl, phenyl, benzyl, phenethyl, or C₁₋₅ heteroaryl;
alternatively, R^{c} and R^{d}, and Rⁱ and R^{j}, independently, can be taken together to form an optionally substituted 4- to 7- membered heterocyclic ring, which ring may be saturated, unsaturated or aromatic;
R^{g} is hydrogen, C₁₋₅ alkyl, C₃₋₅ alkenyl, phenyl, benzyl, phenethyl, or C₁₋₅ heterocyclyl, C₂₋₈ acyl, aroyl, R¹⁷OC=O, R¹⁷R¹⁸NC=O, R¹⁶S, R¹⁶SO, R¹⁶SO₂, or R¹⁷R¹⁸NSO₂;
R^{h} is hydrogen, C₁₋₅ alkyl, C₃₋₅ alkenyl, phenyl, benzyl, phenethyl or C₁₋₅ heterocyctyl;
alternatively, R^{g} and R^{h} can be taken together to form an optionally substituted 4- to 7- membered heterocyclic ring, which ring may be saturated, unsaturated or aromatic;
R¹⁷ and R¹⁸ independently are hydrogen, C₁₋₅ alkyl, C₃₋₅ alkenyl, phenyl, benzyl, or C₁₋₅ heterocyclyl;
alternatively, R¹⁷ and R¹⁸ can be taken together to form an optionally substituted 4- to 7- membered heterocyclic ring, which ring may be saturated, unsaturated or aromatic;
Yₑ is nitrogen or R²⁰DC;
Zₑ is nitrogen or R²¹C;
R²⁰ is hydrogen, halogen, C₁₋₅ alkoxy, C₁₋₅ alkyl, C₂₋₅ alkenyl, cyano, nitro, R^{m}RⁿN, C₂₋₈ acyl, R^{m}OC=O, R¹⁴S, R¹⁴SO, or R¹⁴SO₂;
R²¹ is hydrogen, halogen, C₁₋₅ alkoxy, C₁₋₅ alkyl, C₂₋₅ alkenyl, cyano, nitro, R^{o}R^{p}N, C₂₋₈ acyl, R¹⁸OC=O, R¹¹S, R¹¹SO, or R¹¹SO₂;
alternatively, R³ and R²⁰ or R³ and R²¹ can be taken together to form an optionally substituted-5- or 6-membered carbocyclic or heterocyclic ring, which ring may be saturated, unsaturated or aromatic; wherein said ring may be optionally substituted with halo, di(C₁₋₅ alkyl)amino, C₂₋₅ acyl, and C alkoxy;
R²⁷ is hydrogen, C₁₋₅ alkyl, C₃₋₅ alkenyl, phenyl, naphthyl, benzyl, phenethyl, C₁₋₅ heterocyclyl, C₂₋₈ acyl, aroyl, R²⁹OC=O, R³⁰R³¹NC=O, R²⁸SO, R²⁹S, R²⁹SO₂, or R³⁰R³¹NSO₂;
or, alternatively, R²⁷and R¹can be taken together to form an optionally substituted 5- or 6-membered heterocyclic ring with optionally 1 to 3 additional heteroatom moieties in the ring selected from O, NR⁹, NR¹⁰, N, SO₂, C=O, and S; which ring may be saturated, unsaturated or aromatic;
R⁹and R¹⁰ are independently selected from H, C₁₋₃ alkyl, and -CH₂CO₂(C₁₋₄ alkyl);
X_{f} is CHR^{1f}, =N-, NH, C=O, SO₂, CHSR^{1f} wherein, in formula (t), R^{1f} is hydrogen, halogen, C₁₋₅ alkoxy, hydroxy, C₁₋₅ alkyl, C₃₋₆ alkenyl, cyano, nitro, R³⁹R⁴⁰N, C₂₋₈ acyl,C₁₋₅ heterocyclyl, (C₁₋₅, heterocyclyl)C₁₋₅ alkylene, R⁴¹S , R⁴¹SO, R⁴¹SO₂, R³⁹OC=O, R³⁹R⁴⁰NC=O, R³⁹R⁴⁰NSO₂, R⁴¹SO₃- or R³⁹(C=O)O-;
Y_{f} is CH₂, CHR^{2f}, =CR^{2f}, O, or NR^{2f}, wherein R^{2f} is H, C₁₋₇ alkyl, C₃₋₅ alkenyl, C₂₋₈ acyl, C₁₋₅ heterocyclyl, (C₁₋₅ heterocyclyl)-C₁₋₅ alkylene, phenyl, (phenyl)-C₁₋₅ alkylene. (C₃-₇ cycloalkyl)-C₁₋₅ alkylene, (H₂NCO)- C₁₋₅ alkylene, C₁₋₅ haloalkyl, C₁₋₅ cyanoalkyl, (C₁₋₅ alkoxycarbonyl)C₁₋₅ alkylene, and (phenylcarbonyl)NH-;
m is 0 or 1;
p is 0 or 1;
wherein each of the above hydrocarbyl or heterocarbyl groups, unless otherwise indicated, and in addition to any specified substituents, is optionally and independently substituted with between 1 and 3 substituents selected from methyl, halomethyl, hydroxymethyl, halo, hydroxy, amino, nitro, cyano, C₁₋₆ alkyl, C₁₋₅ alkoxy, -COOH, C₂₋₆ acyl, [di(C₁₋₄ alkyl)amino]C₂₋₅ alkylene, [di(C₁₋₄alkyl)amino] C₂₋₅ alkyl-NH-CO-, and C₁₋₅ haloalkoxy.

3. A use of claim 2, wherein Ar₂ is selected from formulae (f).

4. A use of claim 2, wherein Ar₂ is formula (e) and R¹ halogen, C₁₋₅ alkoxy, hydroxy, C₁₋₅ alkyl, cyano, nitro, and R^{a}R^{b}N, or R¹ can be taken together with R²⁷ as provided below;
R² is hydrogen, halogen, C₁₋₅ alkoxy, C₁₋₆alkyl, or R^{o}R^{f}N;
R⁸ is hydrogen, halogen, C₁₋₅ alkoxy, hydroxy, C₁₋₅alkyl, cyano, R^{g}R^{h}N;
R⁵ and R⁶ are independently selected from hydrogen and C₁₋₃alkyl;
R⁷ and R⁸ independently are taken together to form an optionally substituted 5-to 7- membered carbocyclic or heterocyclic ring, which ring may be saturated, unsaturated or aromatic;
each of R⁸, R^{e} , R^{m} , and R^{o} is independently selected from hydrogen, C₁₋₅ alkyl, C₂₋₈ acyl, and the respective ROC=O, RRNC=O, RS, RSO, RSO₂, and RRNSO₂ groups;
each of R^{b}, R^{f}, Rⁿ, and R^{p}, is independently selected from hydrogen and C₁₋₅ alkyl;
each of R¹¹, R¹², R¹³, R¹⁴, R¹⁵, R¹⁶, R¹⁹, and R³⁸ is independently C₁₋₅alkyl;
each of R^{c} and R^{d}, Rⁱ and R^{j}, R^{k} and R^{l}, R³²and R³³, R³⁴ and R³⁵, R³⁶ and R³⁷ are independently are hydrogen or C₁₋₅ alkyl, or are taken together to form an optionally substituted 4- to 7- membered heterocyclic ring;
R^{g} is hydrogen, C₁₋₆ alkyl, C₂₋₈ acyl, R¹⁷OC=O, R¹⁷R¹⁸NC=O, R¹⁶S, R¹⁶SO, R¹⁶SO₂, or R¹⁷R¹⁸NSO₂;
R^{h} is hydrogen or C₁₋₅ alkyl;
alternatively, R^{g} and R^{h} can be taken together to form an optionally substituted 4- to 7- membered heterocyclic ring;
R¹⁷ and R¹⁸ independently are hydrogen or C₁₋₆ alkyl;
n is 0 or1;
G is C₃₋₄ alkenediyl or C₃₋₄ alkanediyl, optionally substituted with hydroxy, halogen, C₁₋₅ alkyloxy, (L)-C₁₋₅alkoxy, N₃, or [(L)-C₁₋₆ alkylene]amino;
L is amino, mono- or di-C₁₋₅ alkylamino, pyrrolidinyl, morpholinyl, piperidinyl homopiperidinyl, or piperazinyl, wherein available ring nitrogens may be optionally substituted with C₁₋₅ alkyl, benzyl, C₁₋₅ alkylcarbonyl, or C₁₋₅ alkyloxycarbonyl;
Yₑ is nitrogen or R²⁰C;
Zₑ is nitrogen or R²¹C;
R²⁰ and R²¹ are independently selected from hydrogen, halogen, C₁₋₅ alkoxy, C₁₋₅ alkyl, cyano, nitro, and R^{m}RⁿN or R^{o}R^{p}N, respectively;
alternatively, R³ and R²⁰ or R³ and R²¹ can be taken together to form an optionally substituted 5- or 6-membered carbocyclic or heterocyclic ring;
Ar represents a monocyclic or bicyclic aryl or heteroaryl ring, optionally substituted with between 1 and 3 substituents independently selected from halogen, C₁₋₅alkoxy, C₁₋₅alkyl, cyano, azido, nitro, R²²R²³N, R²⁴SO₂, R²⁴OC=O, R²⁵R²⁶NC=O, CF₃, OCF₃, CF₃S, and C₁₋₅alkylthio;
R²² is hydrogen, C₁₋₅alkyl, phenyl, benzyl, phenethyl, C₁₋₅heterocyclyl, C₂₋₈ acyl, aroyl, R²⁴OC=O, R²⁵R²⁶NC=O, R²⁴SO, R²⁴SO₂, or R²⁵R²⁶NSO₂;
R²³ is hydrogen or C₁₋₅alkyl;
alternatively, R²² and R²³ can be taken together to form an optionally substituted 4- to 7- membered heterocyclic ring;
R²⁴ is hydrogen or C₁₋₅alkyl;
R²⁵ and R²⁶ are independently hydrogen or C₁₋₅alkyl;
or, alternatively, R²⁵ and R²⁶ can be taken together to form an optionally substituted 4- to 7- membered heterocyclic;
W is NR²⁷or CHR²⁸;
R²⁷ is hydrogen, C₁₋₅alkyl, R²⁹OC=O, R³⁰R³¹NC=O, R²⁹SO, R²⁹SO₂, or R³⁰R³¹NSO₂;
or, alternatively, R²⁷ and R¹ can be taken together to form an optionally substituted 5- or 6-membered heterocyclic ring, which ring may be saturated, unsaturated or aromatic;
R²⁸ is hydrogen, hydroxy, C₁₋₅heterocyclyl, phenyl, or C₁₋₅alkyl;
R29 is C₁₋₅alkyl; and
R³⁰ and R³¹ are independently selected from hydrogen, C₁₋₅alkyl; alternatively, R³⁰ and -R³¹ can be taken together to form an optionally substituted 4- to 7-membered heterocyclic.

5. A use of claim 1, wherein
one of R⁵ and R⁶ is H,
R⁷and R⁸ are taken together to form an optionally substituted 6-membered carbocyclic or heterocyclic ring; and
Ar represents a monocyclic ring, optionally substituted with 1 to 2 substituents selected from halogen, C₁₋₆alkyl, cyano, azido, nitro,
R²²R²³N, CF₃ and OCF₃.

6. A use of claim 5, wherein both R⁵ and R⁶ are each H, and Ar is a six membered ring substituted with between 1 and 2 substituents independently selected from halogen, methyl, CF₃, and OCF₃, said substituent or substituents being at the 4-position, or at the 3- and 4-positions.

7. A use of claim 2, wherein R²⁰ and R³ taken together are a six-membered carbocyclic or heterocyclic ring -optionally substituted with between 1 and 3 substituents independently selected from halo, C₁₋₃alkoxy, di(C₁₋₃ alkyl)amino, and C₂₋₅acyl.

8. A use of claim 1, wherein said compound is selected from:
1-(1-{2-Hydroxy-3-[5-methanesulfonyl-3-(4-trifluoromethyl-phenyl)-4,5,6,7-tetrahydro-pyrazolo[4,3-c]pyidin-1-yl-propyl}-piperidin-4-yl)-1,3-dihydro-benzoimidazol-2-one ;
1-(1-{3-[3-(3,4-Dichloro-phenyl)-5-methanesulfonyl-4,5,6,7-tetrahydropyrazolo[4,3-c]pyridin-1-y1]-propyl}-piperidin-4-yl)-1,3-dihydro-benzoimidazol-2-one ;
3-(3,4-Dichloro-phenyl)-1-{3-[4-(2-oxo-2,3-dihydro-benzoimidazol-1-yl)-piperidin-1-yl]-propyl}-1,4,6,7-tetrahydro-pyrazolo[4,3-c]pyridine-5-carboxylic acid amide ;
6-Chloro-1-(1-{3-[5-methanesulfonyl-3-(4-trifluoromethyl-phenyl)-4,5,6,7-tetrahydro-pyrazolo[4,3-c]pyridin-1-yl]-propyl}-piperidin-4-yl)-1,3-dihydro-benzoimidazol-2-one ;
3-(3,4-Dichloro-phenyl)-1-{3-[4-(3-methyl-2-oxo-2,3-dihydro-benzoimidazol-1-yl)-piperidin-1-yl]-propyl}-1,4,6,7-tetrahydro-pyrazolo[4,3-c]pyridine-5-carboxylic acid amide ;
[3-(1-{2-Hydroxy-3-[5-methanesulfonyl-3-(4-trifluoromethyl-phenyl)-4,5,6,7-tetrahydro-pyrazolo[4,3-c]pyridin-1-yl]-propyl)-piperidin-4-yl)-2-oxo-2,3-dihydro-benzoimidazol-1-yl]-acetonitrile ;
[3-(1-{2-Hydroxy-3-[5-methanesulfonyl-3-(4-bifluoromethyl-phenyl)-4,5,6,7-tetrahydro-pyrazolo[4,3-c]pyridin-1-yl]-propyl}-piperidin-4-yl)-2-oxo-2,3-dihydro-benzoimidazol-1-yl]-acetic acid ethyl ester;
5-Chloro-3-(1-{2-hydroxy-3-[5-methanesulfonyl-3-(4-trifluoromethylphenyl)-4,5,6,7-tetrahydro-pyrazolo[4,3-c]pyridin-1-yl]-propyl}-piperidin-4-yl)-1-methyl-1,3-dihydro-benzoimidazol-2-one ;
1-{3-[4-(6-Chloro-3-methyl-2-oxo-2,3-dihydro-benzoimidazol-1-yl)-piperidin-1-yl]-propyl}-3-(3,4-dichloro-phenyl)-1,4,6,7-tetrahydro-pyrazolo[4,3-c]pyridine-5-carboxylic acid amide ;
3-(1-{2-Hydroxy-3-[5-methanesulfonyl-3-(4-trifluoromethyl-phenyl)-4,5,6,7-tetrahydro-pyrazolo[4,3-c]pyridin-1-yl]-propyl}-piperidin-4-yl)-1,5-dimethyl-1,3-dihydro-benzoimidazol-2-one ;
3-(1-{2-Hydroxy-3-[5-methanesulfonyl-3-(4-trifluoromethyl-phenyl)-4,5,6,7-tetrahydro-pyrazolo[4,3-c]pyridin-1-yl]-propyl}-piperidin-4-yl)-1,3-dihydro-imidazo[4,5-b]pyridin-one ;
3-(1-{3-[3-(4-Bromo-phenyl)-5-methanesulfonyl-4,5,6,7-tetrahydropyrazolo[4,3-c]pyridin-1-yl]-2-hydroxy-propyl}-piperidin-4-yl)-5-methoxy-1,3-dihydro-imidazo[4,5-b]pyridin-2-one ;
3-(4-Bromo-phenyl)-1-{2-hydroxy-3-[4-(5-methoxy-2-oxo-1,2-dihydroimidazo[4,5-b]pyridin-3-yl)-piperidin-1-yl]-propyl}-1,4,6,7-tetrahydropyrazolo[4,3-c]pyridine-5-carboxylic acid amide ;
3-(1-{2-Hydroxy-3-[5-methanesulfonyl-3-(4-trifluoromethyl-phenyl)-4,5,6,7-tetrahydro-pyrazolo[4,3-c]pyridin-1-yl]-propyl}-piperidin-4-yl)-5-methoxy-1-methyl-1,3-dihydro-imidazo[4,5-b]pyridin-2-one ;
5-Dimethylamino-3-(1-{2-hydroxy-3-[5-methanesulfonyl-3-(4-trifluoromethyl-phenyl)-4,5,6,7-tetrahydro-pyrazolo[4,3-c]pyridin-1-yl]-propyl}-piperidin-4-yl)-1,3-dihydro-imidazo[4,5-b]pyridin-2-one ;
6-Chloro-1-(1-{3-[5-methanesulfonyl-3-(4-trifluoromethyl-phenyl)-4,5,6,7-tetrahydro-pyrazolo[4,3-c]pyridin-1-yl]-propyl}piperidin-4-yl)-1,3-dihydro-indol-2-one ;
1-(1-{2-Hydroxy-3-[5-methanesulfonyl-3-(4-trifluoromethyl-phenyl)-4,5,6,7-tetrahydro-pyrazolo[4,3-c]pyridin-1-yl]-propyl}-piperidin-4-yl)-3,4-dihydro-1H-quinolin-2-one ;
4-(1-{3-[5-Methanesulfonyl-3-(4-trifluoromethyl-phenyl)-4,5,6,7-tetrahydro-pyrazolo[4,3-c]pyridin-1-yl]-propyl}-piperidin-4-yl)-4H-benzo[1,4]oxazin-3-one ;
4-(1-{2-Hydroxy-3-[5-methanesulfonyl-3-(4-trifluoromethyl-phenyl)-4,5,6,7-tetrahydro-pyrazolo[4,3-c]pyridin-1-yl]-propyl}-piperidin-4-yl)-4H-benzo[1,4]oxazin-3-one ; and
1-(1-{3-[5-Methanesulfonyl-3-(4-trifluoromethyl-phenyl)-4,5,6,7-tetrahydro-pyrazolo[4,3-c]pyridin-1-yl]-propyl}-piperidin-4-yl)-3,4-dihydro-1H-quinazolin-2-one.

9. A use of claim 1, wherein said compound is selected from:
[3-(1-{2-Hydroxy-3-[5-methanesulfonyl-3-(4-trifluoromethyl-phenyl)-4,5,6,7-tetrahydro-pyrazolo[4,3-c]pyridin-1-yl]-propyl}-piperidin-4-yl)-2-oxo-2,3-dihydro-benzoimidazol-1-yl]-acetonitrile; and 4-(1-{2-Hydroxy-3-[5-methanesulfonyl-3-(4-trifluoromethyl-phenyl)-4,5,6,7-tetrahydro-pyrazolo[4,3-c]pyridin-1-yl]-propyl}-piperidin-4-yl)-4H-benzo[1,4]oxazin-3-one.

10. A use of claim 1, wherein said compound is selected from:
2-(1-{3-[5-Acetyl-3-(4-chloro-phenyl)-4,5,6,7-tetrahydro-pyrazolo[4,3-c]pyridin-1-yl]-2-hydroxy-propyl}-piperidin-4-ylamino)-benzonitrile;
1-(1-{3-[5-Acetyl-3-(4-trifluoromethyl-phenyl)-4,5,6,7-tetrahydropyrazolo[4,3-c]pyridin-1-yl]-2-hydroxy-propyl}-piperidin-4-yl)-1,3-dihydro-benzoimidazol-2-one;
3-(1-{3-[5-Acetyl-3-(4-bromo-phenyl)-4,5,6,7-tetrahydro-pyrazolo[4,3-c]pyridin-1-yl]-2-hydroxy-propyl}-piperidin-4-yl)-3H-benzooxazol-2-one;
1-(3-(4-Chloro-3-methyl-phenyl)-1-{3-[4-(3,4-dichloro-phenoxy)-piperidin-1-yl]-propyl}-1,4,6,7-tetrahydro-pyrazolo[4,3-c]pyridin-5-yl)-ethanone;
1-(3-(4-Chloro-3-methyl-phenyl)-1-{3-[4-(2,3-dihydro-indol-1-yl)-piperidin-1-yl]-2-hydroxy-propyl}-1,4,6,7-tetrahydro-pyrazolo[4,3-c]pyridin-5-yl)-ethanone;
(*S*)-1-(1-{3-[5-Acetyl-3-(4-chloro-3-methyl-phenyl)-4,5,6,7-tetrahydropyrazolo[4,3-c]pyridin-1-yl]-2-hydroxy-propyl}-piperidin-4-yl)-6-chloro-1,3-dihydro-benzoimidazol-2-one;
1-(1-{3-[5-Acetyl-3-(4-trifluoromethyl-phenyl)-4,5,6,7-tetrahydropyrazolo[4,3-c]pyridin-1-yl]-2-hydroxy-propyl}-piperidin-4-yl)-3-(2-morpholin-4-yl-ethyl)-1,3-dihydro-benzoimidazol-2-one;
1-(1-{3-[3-(4-Bromo-phenyl)-5-methanesulfonyl-4,5,6,7-tetrahydropyrazolo[4,3-c]pyridin-1-yl]-2-hydroxy-propyl}-piperidin-4-yl)-6-chloro-1,3-dihydro-benzoimidazol-2-one;
[3-(1-{3-[5-Methanesulfonyl-3-(4-trifluoromethyl-phenyl)-4,5,6,7-tetrahydro-pyrazolo[4,3-c]pyridin-1-yl]-propyl}-piperidin-4-yl)-2-oxo-2,3-dihydro-benzoimidazol-1-yl]-acetonitrite;
5-Chloro-3-(1-{3-[5-methanesulfonyl-3-(4-trifluoromethyl-phenyl)-4,5,6,7-tetrahydro-pyrazolo[4,3-c]pyridin-1-yl]-propyl}-piperidin-4-yl)-1-methyl-1,3-dihydro-benzoimidazol-2-one;
1-(1-{3-[5-Methanesulfonyl-3-(4-trifluoromethyl-phenyl)-4,5,6,7-tetrahydro-pyrazolo[4,3-c]pyridin-1-yl]-propyl}-piperidin-4-yl)-1,3-dihydro-indol-2-one;
1-[3-(4-Chloro-3-methyl-phenyl)-1-(3-{4-[3-(4-chloro-phenyl)-[1,2,4]oxadiazol-5-yl]-piperidin-1-yl}-2-hydroxy-propyl)-1,4,6,7-tetrahydropyrazolo[4,3-c]pyridin-5-yl]-ethanone;
1-[1-{2-Hydroxy-3-[4-(5-trifluoromethyl-benzothiazol-2-yl)-piperidin-1-yl]-propyl}-3-(4-trifluoromethyl-phenyl)-1,4,6,7-tetrahydro-pyrazolo[4,3-c]pyridin-5-yl]-ethanone;
1-[1-{3-[4-(Benzo[d]isoxazol-3-yloxy)-piperidin-1-yl]-2-hydroxy-propyl}-3-(4-trifluoromethyl-phenyl)-1,4,6,7-tetrahydro-pyrazolo[4,3-c]pyridin-5-yl]-ethanone;
1-[1-{3-[4-(5-Chloro-benzooxazol-2-yl)-piperidin-1-yl]-2-hydroxy-propyl}-3-(4-trifluoromethyl-phenyl)-1,4,6,7-tetrahydro-pyrazolo[4,3-c]pyridin-5-yl]-ethanone;
1-[1-{3-[4-(Benzothiazol-2-ylamino)-piperidin-1-yl]-2-hydroxy-propyl}-3-(4-trifluoromethyl-phenyl)-1,4,6,7-tetrahydro-pyrazolo[4,3-c]pyridin-5-yl]-ethanone;
1-[1-{3-[4-(3,5-Dichloro-pyridin-4-yloxy)-piperidin-1-yl]-2-hydroxy-propyl}-3-(4-trifluoromethyl-phenyl)-1,4,6,7-tetrahydro-pyrazolo[4,3-c]pyridin-5-yl]-ethanone;
1-[1-{3-[4-(1H-Benzoimidazol-2-yl)-piperidin-1-yl]-2-hydroxy-propyl}-3-(4-trifluommethyl-phenyl)-1,4,6,7-tetrahydro-pyrazolo[4,3-c]pyridin-5-yl]-ethanone;
6-Chloro-4-(1-{2-hydroxy-3-[5-methanesulfonyl-3-(4-trifluoromethylphenyl)-4,5,6,7-tetrahydro-pyrazolo[4,3-c]pyridin-1-yl]-propyl}-piperidin-4-yl)-4H-benzo[1,4]oxazin-3-one;
6-Chloro-1-(1-{2-hydroxy-3-[5-methanesulfonyl-3-(4-trifluoromethylphenyl)-4,5,6,7-tetrahydro-pyrazolo[4,3-c]pyridin-1yl]-propyl}-piperidin-4-yl)-3,4-dihydro-1H-quinolin-2-one;
6-Chloro-1-(1-{2-hydroxy-3-[5-methanesulfonyl-3-(4-trifluoromethylphenyl)-4,5,6,7-tetrahydro-pyrazolo[4,3-c]pyridin-1-yl]-propyl}-piperidin-4-yl)-3,4-dihydro-1H-quinazolin-2-one;
1-[4-(6-Chloro-2,2-dioxo-3,4-dihydro-2H-2λ⁶-benzo[1,2,6]thiadiazin-1-yl)-piperidin-1-yl]-3-[5-methanesulfonyl-3-(4-trifluoromethyl-phenyl)-4,5,6,7-tetrahydro-pyrazolo[4,3-c]pyridin-1-yl]-propan-2-ol;
4-(1-{2-Hydroxy-3-[5-methanesulfonyl-3-(4-trifluoromethyl-phenyl)-4,5,6,7-tetrahydro-pyrazolo[4,3-c]pyridin-1-yl]-propyl}-piperidin-4-yl)-4H-pyrido[3,2-b][1,4]oxazin-3-one;
5-Chloro-1-(1-{2-hydroxy-3-[5-methanesulfonyl-3-(4-trifluoromethylphenyl)-4,5,6,7-tetrahydro-pyrazolo[4,3-c]pyridin-1-yl]-propyl}-piperidin-4-yl)-1,3-dihydro-indol-2-one;
1-[4-(6-Chloro-indol-1-yl)-piperidin-1-yl]-3-[5-methanesulfonyl-3-(4-trifluoromethyl-phenyl)-4,5,6,7-tetrahydro-pyrazolo[4,3-c]pyridin-1-yl]-propan-2-ol;
1-(1-{3-[5-Methanesulfonyl-3-(4-trifluoromethyl-phenyl)-4,5,6,7-tetrahydro-pyrazolo[4,3-c]pyridin-1-yl]-propyl}-piperidin-4-yl)-1H-benzotriazole;
1-{3-[4-(3-Methyl-2-oxo-2,3-dihydro-benzoimidazol-1-yl)-piperidin-1-yl]-propyl}-3-(4-trifluoromethyl-phenyl)-1,4,6,7-tetrahydro-pyrazolo[4,3-c]pyridine-5-sulfonic acid amide;
5-Chloro-3-(1-{2-hydroxy-3-[4-pyridin-4-yl-3-(4-trifluoromethyl-phenyl)-pyrazol-1-yl]-propyl}-piperidin-4-yl)-1-methyl-1,3-dihydro-benzoimidazol-2-one;
4-(1-{2-Hydroxy-3-[4-pyrazin-2-yl-3-(4-trifluoromethyl-phenyl)-pyrazol-1-yl]-propyl}-piperidin-4-yl)-4H-benzo[1,4]oxazin-3-one;
(*S*)-1-(1-{2-Hydroxy-3-[5-methanesulfonyl-3-(4-trifluoromethyl-phenyl)-4,5,6,7-tetrahydro-pyrazolo[4,3-c]pyridin-1-yl]-propyl}-piperidin-4-yl)-3-methyl-1,3-dihydro-benzoimidazol-2-one; and
(*S*)-5-Dimethylamino-3-(1-{2-hydroxy-3-[5-methanesulfonyl-3-(4-trifluoromethyl-phenyl)-4,5,6,7-tetrahydro-pyrazolo[4,3-c]pyridin-1-yl]-propyl}-piperidin-4-yl)-1-methyl-1,3-dihydro-imidazo[4,5-b]pyridin-2-one.

11. A use of claim 1, wherein said compound is selected from:
1-(1-{3-[5-Acetyl-3-(4-bromo-phenyl)-4,5,6,7-tetrahydro-pyrazolo[4,3-c]pyridin-1-yl]-2-hydroxy-propyl}-piperidin-4-yl)-5-methoxy-1,3-dihydro-benzoimidazol-2-one;
6-Chloro-1-(1-{3-[3-(4-chloro-3-methyl-phenyl)-5-methanesulfonyl-4,5,6,7-tetrahydro-pyrazolo[4,3-c]pyridin-1-yl]-2-hydroxy-propyl}-piperidin-4-yl)-1,3-dihydro-benzoimidazol-2-one;

12. A use of claim 1, wherein said pharmaceutical composition is formulated in a dosage amount appropriate for the treatment of an allergic condition.

## Patentansprüche

1. Verwendung einer Verbindung zur Verstellung einer pharmazeutischen Zusammensetzung zur Behandlung eines Patienten mit einem allergischen Zustand,
wobei die Verbindung Formel (I) besitzt: 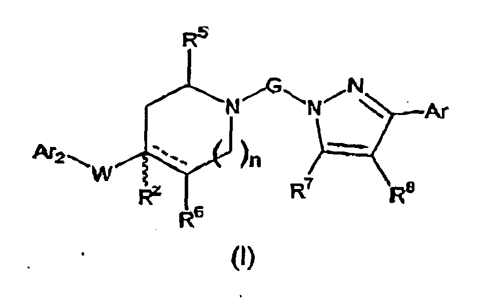 worin:
Ar₂ ein monocyclisches oder bicyclisches Ringsystem ist, ungesättigt, gesättigt oder aromatisch, gegebenenfalls kondensiert, gegebenenfalls einschließlich zwischen 1 und 5 Heteroatomringeinheiten, die unabhängig ausgewählt sind aus O, S, N, SO₂ und C=O; wobei das Ar₂-Ringsystem gegebenenfalls mit zwischen 1 und 4 Substituenten substituiert ist;
R⁵ und R⁶ unabhängig ausgewählt sind aus Wasserstoff und C₁₋₅-Alkyl;
R⁷ und R⁸ unabhängig Wasserstoff, C₁₋₅-Alkyl, C₂₋₅-Alkenyl, C₁₋₅-Alkoxy, C₁₋₅-Alkylthio, Halogen oder ein 4- bis 7-gliedriges Carbocyclyl oder Heterocyclyl sind; alternativ R⁷ und R⁸ zusammengenommen sein können, um einen gegebenenfalls substituierten 5- bis 7-gliedrigen carbocyclischen oder heterocyclischen Ring zu bilden, wobei dieser Ring ungesättigt oder aromatisch sein kann und gegebenenfalls substituiert sein kann mit zwischen einem und drei Substituenten, die unabhängig ausgewählt sind aus Halo, Cyano, Amino, Hydroxy, Nitro, R⁴, R⁴O-, R⁴S-, R⁴O(C₁₋₅-Alkylen)-, R⁴O(C=O)-, R⁴(C=O)-, R⁴(C=S)-, R⁴(C=O)O-, R⁴O(C=O)(C=O)-, R⁴SO₂, NHR⁴⁴(C=NH)-, NHR⁴⁴SO₂- und NHR⁴⁴(C=O)-;
R⁴ H, C₁₋₅-Alkyl, C₂₋₅-Alkenyl, C₁₋₅-Heterocyclyl, (C₁₋₅-Heterocyclyl)-C₁₋₆-alkylen, Phenyl, Benzyl, Phenethyl, NH₂, Mono- oder Di-(C₁₋₆-alkyl)N-, (C₁₋₆-Alkoxy)-carbonyl- oder R⁴²OR⁴³- ist, wobei R⁴² H, C₁₋₅-Alkyl, C₂₋₅-Alkenyl, Phenyl, Benzyl, Phenethyl, C₁₋₅-Heterocyclyl oder (C₁₋₅-Heterocyclyl)-C₁₋₆-alkylen ist und R⁴³ C₁₋₅-Alkylen, Phenylen oder zweiwertiges C₁₋₅-Heterocyclyl ist;
R⁴⁴ irgendeiner der Werte für R⁴ sein kann;
n 0, 1 oder 2 ist;
G C₃₋₆-Alkendiyl oder C₃₋₆-Alkandiyl ist, gegebenenfalls substituiert mit Hydroxy, Halogen, C₁₋₅-Alkoxy, C₁₋₅-Alkyl, Oxo, Hydroximino, CO₂R^{k}, R^{k}R^{l}N, R^{k}R^{l}NCO₂, (L)-C₁₋₄-Alkylen-, (L)-C₁₋₅-Alkoxy, N₃ oder [(L)-C₁₋₅-Alkylen]-amino;
R^{k} und R¹ jeweils unabhängig Wasserstoff, C₁₋₅-Alkyl, C₃₋₅-Alkenyl, Phenyl, Benzyl, Phenethyl oder C₁₋₅-Heterocyclyl ist; alternativ R^{k} und R¹ zusammengenommen sein können, um einen gegebenenfalls substituierten 4- bis 7-gliedrigen heterocyclischen Ring zu bilden, wobei dieser Ring gesättigt, ungesättigt oder aromatisch sein kann;
L Amino, Mono- oder Di-C₁₋₅-alkylamino, Pyrrolidinyl, Morpholinyl, Piperidinyl, Homopiperidinyl oder Piperazinyl ist, wobei verfügbare Ringstickstoffe gegebenenfalls mit C₁₋₅-Alkyl, Benzyl, C₂₋₅-Acyl, C₁₋₅-Alkylsulfonyl oder C₁₋₅-Alkoxycarbonyl substituiert sein können,
Ar für einen monocyclischen oder bicyclischen Aryl- oder Heteroarylring steht, gegebenenfalls substituiert mit zwischen 1 und 3 Substituenten, die unabhängig ausgewählt sind aus Halogen, C₁₋₅-Alkoxy, C₁₋₅-Alkyl, C₂₋₅-Alkenyl, Cyano, Azido, Nitro, R²²R²³N, R²⁴SO₂, R²⁴S, R²⁴SO, R²⁴OC=O, R²²R²³NC=O, C₁₋₅-Haloalkyl, C₁₋₅-Haloalkoxy, C₁₋₅-Haloalkylthio und C₁₋₅-Alkylthio;
R²² Wasserstoff, C₁₋₅-Alkyl, C₃₋₅-Alkenyl, Phenyl, Phenethyl, Benzyl oder C₁₋₅-Heterocyclyl, C₂₋₈-Acyl, Aroyl, R³⁸OC=O, R²⁵R²⁶NC=O, R³⁸SO, R³⁸SO₂, R³⁸S oder R²⁵R²⁶NSO₂ ist;
R³⁸ H, C₁₋₅-Alkyl, C₃₋₅-Alkenyl, Phenyl, Benzyl, Phenethyl oder C₁₋₅-Heterocyclyl ist;
R²³ Wasserstoff, C₁₋₅-Alkyl, C₃₋₅-Alkenyl, Phenyl, Benzyl oder C₁₋₅-Heterocyclyl ist; alternativ R²² und R²³ zusammengenommen sein können, um einen gegebenenfalls substituierten 4- bis 7-gliedrigen heterocyclischen Ring zu bilden, wobei dieser Ring gesättigt, ungesättigt oder aromatisch sein kann;
R²⁴ C₁₋₅-Alkyl, C₃₋₅-Alkenyl, Phenyl, Benzyl oder C₁₋₅-Heterocyclyl ist;
R²⁵ und R²⁶ unabhängig Wasserstoff, C₁₋₅-Alkyl, C₃₋₅-Alkenyl, Phenyl, Benzyl und C₁₋₅-heterocyclyl sind; oder, alternativ, R²⁵ und R²⁶ zusammengenommen sein können, um einen gegebenenfalls substituierten 4- bis 7-gliedrigen heterocyclischen Ring zu bilden, wobei dieser Ring gesättigt, ungesättigt oder aromatisch sein kann;
W für O, S, NR²⁷, C=O, (C=O)NH, NH(C=O), CHR²⁸ oder eine kovalente Bindung steht;
R^{z} H oder OH ist und die gestrichelte Linie nicht vorhanden ist; oder R^{z} vorhanden ist, wobei die gestrichelte Linie eine sp²-Bindung ist;
R²⁷ Wasserstoff, C₁₋₅-Alkyl, C₃₋₅-Alkenyl, Phenyl, Naphthyl, Benzyl, Phenethyl, C₁₋₅-Heterocyclyl, C₂₋₈-Acyl, Aroyl, R²⁹OC=O, R³⁰R³¹NC=O, R²⁹SO, R²⁹S, R²⁹SO₂ oder R³⁰R³¹NSO₂ ist; oder, alternativ, R²⁷ und ein Teil von Ar₂ zusammengenommen sein können, um einen gegebenenfalls substituierten 5- oder 6-gliedrigen heterocyclischen Ring zu bilden, mit gegebenenfalls 1 bis 3 zusätzlichen Heteroatomeinheiten im Ring, ausgewählt aus O, NR⁹, NR¹⁰, N, SO₂, C=O und S; wobei dieser Ring gesättigt, ungesättigt oder aromatisch sein kann; R⁹ und R¹⁰ unabhängig ausgewählt sind aus H, C₁₋₃-Alkyl und -CH₂CO₂(C₁₋₄-Alkyl);
R²⁸ Wasserstoff, C₁₋₅-Alkyl, C₃₋₅-Alkenyl, Hydroxy, Phenyl, Benzyl, C₁₋₅-Heterocyclyl, R²⁹O, R³⁰R³¹NC=O, R²⁹S, R²⁹SO, R²⁹SO₂ oder R³⁰R³¹NSO₂ ist;
R²⁹ C₁₋₅-Alkyl, C₃₋₅-Alkenyl, Phenyl, Benzyl oder C₁₋₅-Heterocyclyl ist;
R³⁰ und R³¹ unabhängig ausgewählt sind aus Wasserstoff, C₁₋₅-Alkyl, C₃₋₅-Alkenyl, Phenyl, Benzyl, Phenethyl, Naphthyl und C₁₋₅-Heteroaryl; alternativ R³⁰ und R³¹ zusammengenommen sein können, um einen gegebenenfalls substituierten 4- bis 7-gliedrigen carbocyclischen oder heterocyclischen Ring zu bilden, wobei dieser Ring gesättigt, ungesättigt oder aromatisch sein kann;
wobei jede der obigen Hydrocarbyl- oder Heterocarbylgruppen, sofern nicht anders angegeben und zusätzlich zu jeglichen spezifizierten Substituenten, gegebenenfalls und unabhängig mit zwischen 1 und 3 Substituenten substituiert ist, die ausgewählt sind aus Methyl, Halomethyl, Hydroxymethyl, Halo, Hydroxy, Amino, Nitro, Cyano, C₁₋₅-Alkyl, C₁₋₅-Alkoxy, -COOH, C₂₋₆-Acyl, [Di-(C₁₋₄-alkyl)-amino]-C₂₋₅-alkylen, [Di-(C₁₋₄-alkyl)-amino]-C₂₋₅-alkyl-NH-CO- und C₁₋₅-Haloalkoxy;
oder ein pharmazeutisch annehmbares Salz, Amid oder Ester davon; oder eine stereoisomere Form davon.

2. Verwendung nach Anspruch 1, wobei Ar₂ ausgewählt ist aus 2,5-Di-(C₁₋₆-alkyl)-aminopyrrolyl und den folgenden 6 Formeln: 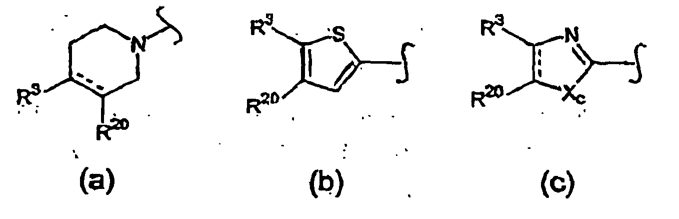 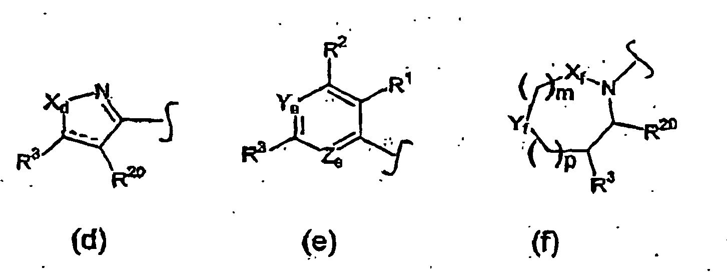 worin
jede gestrichelte Linie eine sp²-Bindung oder nicht vorhanden sein kann;
X_{c} O, S oder N ist; und X_{d} O oder S ist;
R¹ Wasserstoff, Halogen, C₁₋₅-Alkoxy, Hydroxy, C₁₋₅-Alkyl, C₂₋₅-Alkenyl, Cyano, Nitro, R^{a}R^{b}N, C₂₋₈-Acyl, C₁₋₅-Heterocyclyl, (C₁₋₅-Heterocyclyl)-C₁₋₅-alkylen, R¹¹S, R¹¹SO, R¹¹SO₂, R^{c}OC=O, R^{c}R^{d}NC=O oder R^{c}R^{d}NSO₂ ist; oder R¹ mit R²⁷, wie unten angegeben, zusammengenommen sein kann;
R² Wasserstoff, Halogen, C₁₋₅-Alkoxy, Hydroxy, C₁₋₅-Alkyl, C₂₋₅-Alkenyl, Cyano, Nitro, R^{c}R^{f}N, C₁₋₅-Heterocyclyl oder C₂₋₈-Acyl ist;
R³ Wasserstoff, Halogen, C₁₋₅-Alkoxy, Hydroxy, C₁₋₅Alkyl, C₂₋₅-Alkenyl, Cyano, Nitro, R^{g}R^{h}N, C₂₋₈-Acyl, C₁₋₅-Heterocyclyl, R^{h}OC=O, R^{g}R^{h}NC=O oder R^{g}R^{h}NSO₂ ist;
R^{a} ausgewählt ist aus Wasserstoff, C₁₋₅-Alkyl, C₃₋₅-Alkenyl, Phenyl, Benzyl, Phenethyl, C₁₋₅-Heterocyclyl, C₂₋₈-Acyl, Aroyl, R^{j}OC=O, RⁱR^{j}NC=O, R¹²SO, R¹²SO₂, R¹²S und RⁱR^{j}NSO₂;
R^{c} ausgewählt ist aus Wasserstoff, C₁₋₅-Alkyl, C₃₋₅-Alkenyl, Phenyl, Benzyl, Phenethyl, C₁₋₅-Heterocyclyl, C₂₋₈-Acyl, Aroyl, R³²OC=O, R³²R³³NC=O, R¹³SO, R¹³SO₂, R¹³S und R³²R³³NSO₂;
R^{m} ausgewählt ist aus Wasserstoff, C₁₋₅-Alkyl, C₃₋₅-Alkenyl, Phenyl, Benzyl, Phenethyl, C₁₋₅-Heterocycyl, C₂₋₈-Acyl, Aroyl, R³⁴OC=O, R³⁴R³⁵NC=O, R¹⁵SO, R¹⁵SO₂, R¹⁵S und R³⁴R³⁵NSO₂;
R^{o} ausgewählt ist aus Wasserstoff, C₁₋₅-Alkyl, C₃₋₅-Alkenyl, Phenyl, Benzyl, Phenethyl, C₁₋₅-Heterocyclyl, C₂₋₈-Acyl, Aroyl, R³⁶OC=O, R³⁶R³⁷NC=O, R¹⁹SO, R¹⁹SO₂, R¹⁹S und R³⁶R³⁷NSO₂;
R^{b}, R^{f}, Rⁿ, R^{p}, R³², R³³, R³⁴, R³⁵, R³⁶, R³⁷, R³⁹ und R⁴⁰ jeweils unabhängig ausgewählt ist aus Wasserstoff, C₁₋₅-Alkyl, C₃₋₅-Alkenyl, Phenyl, Benzyl, Phenethyl und C₁₋₅-Heteroaryl; alternativ R^{a} und R^{b}, R^{c} und R^{f}, R^{m} und Rⁿ und R^{o} und R^{p} unabhängig zusammengenommen sein können, um einen gegebenenfalls substituierten 4- bis 7-gliedrigen heterocyclischen Ring zu bilden, wobei dieser Ring gesättigt, ungesättigt oder aromatisch sein kann;
R¹¹, R¹², R¹³, R¹⁴, R¹⁵, R¹⁶, R¹⁹, R³⁸ und R⁴¹ jeweils unabhängig C₁₋₅-Alkyl, C₃₋₅-Alkenyl, Phenyl, Benzyl, Phenethyl oder C₁₋₅-Heterocyclyl ist;
R^{c} und R^{d} und Rⁱ und R^{j} jeweils unabhängig Wasserstoff, C₁₋₅-Alkyl, C₃₋₅-Alkenyl, Phenyl, Benzyl, Phenethyl oder C₁₋₅-Heteroaryl sind; alternativ R^{c} und R^{d} und Rⁱ und R^{j} unabhängig zusammengenommen sein können, um einen gegebenenfalls substituierten 4- bis 7-gliedrigen heterocyclischen Ring zu bilden, wobei dieser Ring gesättigt, ungesättigt oder aromatisch sein kann;
R^{g} Wasserstoff, C₁₋₅-Alkyl, C₃₋₅-Alkenyl, Phenyl, Benzyl, Phenethyl oder C₁₋₅-Heterocyclyl, C₂₋₈-Acyl, Aroyl, R¹⁷OC=O, R¹⁷R¹⁸NC=O, R¹⁶S, R¹⁶SO, R¹⁶SO₂ oder R¹⁷R¹⁸NSO₂ ist;
R^{h} Wasserstoff, C₁₋₅-Alkyl, C₃₋₅-Alkenyl, Phenyl, Benzyl, Phenethyl oder C₁₋₅-Heterocyclyl ist; alternativ R^{g} und R^{h} zusammengenommen sein könnten, um einen gegebenenfalls substituierten 4- bis 7-gliedrigen heterocyclischen Ring zu bilden, wobei dieser Ring gesättigt, ungesättigt oder aromatisch sein kann;
R¹⁷ und R¹⁸ unabhängig Wasserstoff, C₁₋₅-Alkyl, C₃₋₅-Alkenyl, Phenyl, Benzyl oder C₁₋₅-Heterocyclyl sind; alternativ R¹⁷ und R¹⁸ zusammengenommen sein können, um einen gegebenenfalls substituierten 4- bis 7-gliedrigen heterocyclischen Ring zu bilden, wobei dieser Ring gesättigt, ungesättigt oder aromatisch sein kann;
Yₑ Stickstoff oder R²⁰C ist;
Zₑ Stickstoff oder R²¹C ist;
R²⁰ Wasserstoff, Halogen, C₁₋₅-Alkoxy, C₁₋₅-Alkyl, C₂₋₅-Alkenyl, Cyano, Nitro, R^{m}RⁿN, C₂₋₈-Acyl, R^{m}OC=O, R¹⁴S, R¹⁴SO oder R¹⁴SO₂ ist;
R²¹ Wasserstoff, Halogen, C₁₋₅-Alkoxy, C₁₋₅-Alkyl, C₂₋₅-Alkenyl, Cyano, Nitro, R^{o}R^{p}N, C₂₋₈-Acyl, R¹⁶OC=O, R¹¹S, R¹¹SO oder R¹¹SO₂ ist; alternativ R³ und R²⁰ oder R³ und R²¹ zusammengenommen sein können, um einen gegebenenfalls substituierten 5- oder 6-gliedrigen carbocyclischen oder heterocyclischen Ring zu bilden, wobei dieser Ring gesättigt, ungesättigt oder aromatisch sein kann; wobei der Ring gegebenenfalls mit Halo, Di-(C₁₋₅-alkyl)-amino, C₂₋₅-Acyl und C₁₋₅-Alkoxy substituiert sein kann;
R²⁷ Wasserstoff, C₁₋₅-Alkyl, C₃₋₅-Alkenyl, Phenyl, Naphthyl, Benzyl, Phenethyl, C₁₋₅-Heterocyclyl, C₂₋₈-Acyl, Aroyl, R²⁹OC=O, R³⁰R³¹NC=O, R²⁹SO, R²⁹S, R²⁹SO₂ oder R³⁰R³¹NSO₂ ist; oder, alternativ, R²⁷ und R¹ zusammengenommen sein können, um einen gegebenenfalls substituierten 5- oder 6-gliedrigen heterocyclischen Ring zu bilden, mit gegebenenfalls 1 bis 3 zusätzlichen Heteroatomeinheiten im Ring, ausgewählt aus O, NR⁹, NR¹⁰, N, SO₂, C=O und S; wobei dieser Ring gesättigt, ungesättigt oder aromatisch sein kann;
R⁹ und R¹⁰ unabhängig ausgewählt sind aus H, C₁₋₃-Alkyl und -CH₂CO₂(C₁₋₄-Alkyl);
X_{f} CHR^{1f}, =N-, NH, C=O, SO₂, CHSR^{1f} ist, wobei, in Formel (f), R^{1f} Wasserstoff, Halogen, C₁₋₅-Alkoxy, Hydroxy, C₁₋₅-Alkyl, C₃₋₅-Alkenyl, Cyano, Nitro, R³⁹R⁴⁰, C₂₋₈-Acyl, C₁₋₅-Heterocyclyl, (C₁₋₅-Heterocyclyl)-C₁₋₅-alkylen, R⁴¹S, R⁴¹SO, R⁴¹SO₂, R³⁹OC=O, R³⁹R⁴⁰NC=O, R³⁹R⁴⁰NSO₂, R⁴¹SO₃- oder R³⁹(C=O)O- ist;
Y_{f} CH₂, CHR^{2f}, =CR^{2f}, O oder NR^{2f} ist, wobei R^{2f} H, C₁₋₇-Alkyl, C₃₋₅-Alkenyl, C₂₋₈-Acyl, C₁₋₅-Heterocyclyl, (C₁₋₅-Heterocyclyl)-C₁₋₅-alkylen, Phenyl, (Phenyl)-C₁₋₅-alkylen, (C₃₋₇-Cycloakyl)-C₁₋₅-alkylen, (H₂NCO)-C₁₋₅-Alkylen, C₁₋₅-Haloalkyl, C₁₋₅-Cyanoalkyl, (C₁₋₅-Alkoxycarbonyl)-C₁₋₅-alkylen und (Phenylcarbonyl)NH- ist;
m 0 oder 1 ist;
p 0 oder 1 ist;
wobei jede der obigen Hydrocarbyl- oder Heterocarbylgruppen, sofern nicht anders angegeben und zusätzlich zu jeglichen spezifizierten Substituenten, gegebenenfalls und unabhängig mit zwischen 1 und 3 Substituenten substituiert ist, die ausgewählt sind aus Methyl, Halomethyl, Hydroxymethyl, Halo, Hydroxy, Amino, Nitro, Cyano, C₁₋₅-Alkyl, C₁₋₅-Alkoxy, -COOH, C₂₋₆-Acyl, [Di-(C₁₋₄-alkyl)-amino]-C₂₋₅-alkylen, [Di-(C₁₋₄-alkyl)-amino]-C₂₋₅-alkyl-NH-CO- und C₁₋₅-Haloalkoxy,

3. Verwendung nach Anspruch 2, wobei Ar₂ ausgewählt ist aus den Formeln (f).

4. Verwendung nach Anspruch 2, wobei Ar₂ Formel (e) ist und R¹ Halogen, C₁₋₅-Alkoxy, Hydroxy, C₁₋₅-Alkyl, Cyano, Nitro und R^{a}R^{b}N ist oder R¹ mit R²⁷, wie unten angegeben, zusammengenommen sein kann;
R² Wasserstoff, Halogen, C₁₋₅-Alkoxy, C₁₋₅-Alkyl oder R^{e}R^{f}N ist;
R³ Wasserstoff, Halogen, C₁₋₅-Alkoxy, Hydroxy, C₁₋₅-Alkyl, Cyano, R^{g}R^{h}N ist;
R⁵ und R⁶ unabhängig ausgewählt sind aus Wasserstoff und C₁₋₃-Alkyl;
R⁷ und R⁸ unabhängig zusammengenommen sein können, um einen gegebenenfalls substituierten 5- bis 7-gliedrigen carbocyclischen oder heterocyclischen Ring zu bilden, wobei dieser Ring gesättigt, ungesättigt oder aromatisch sein kann;
R^{a}, R^{c}, R^{m} und R^{o} jeweils unabhängig ausgewählt sind aus Wasserstoff, C₁₋₅-Alkyl, C₂₋₈-Acyl und den entsprechenden ROC=O-, RRNC=O-, RS-, RSO-, RSO₂- und RRNSO₂-Gruppen;
R^{b}, R^{f}, Rⁿ und R^{p} jeweils unabhängig ausgewählt ist aus Wasserstoff und C₁₋₅-Alkyl;
R¹¹, R¹², R¹³, R¹⁴, R¹⁵, R¹⁶, R¹⁹ und R³⁸ jeweils unabhängig C₁₋₅-Alkyl ist;
R^{c} und R^{d}, Rⁱ und R^{j}, R^{k} und R^{l}, R³² und R³³, R³⁴ und R³⁵, R³⁶ und R³⁷ jeweils unabhängig Wasserstoff oder C₁₋₅-Alkyl sind oder zusammengenommen sind, um einen gegebenenfalls substituierten 4- bis 7-gliedrigen heterocyclischen Ring zu bilden;
R^{g} Wasserstoff, C₁₋₅-Alkyl, C₂₋₈-Acyl, R¹⁷OC=O, R¹⁷R¹⁸NC=O, R¹⁶S, R¹⁶SO, R¹⁶SO₂ oder R¹⁷R¹⁸NSO₂ ist;
R^{h} Wasserstoff oder C₁₋₅-Alkyl ist; alternativ R^{g} und R^{h} zusammengenommen sein können, um einen gegebenenfalls substituierten 4- bis 7-gliedrigen heterocyclischen Ring zu bilden;
R¹⁷ und R¹⁸ unabhängig Wasserstoff oder C₁₋₅-Alkyl sind;
n 0 oder 1 ist;
G C₃₋₄-Alkendiyl oder C₃₋₄-Alkandiyl ist, gegebenenfalls substitutiert mit Hydroxy, Halogen, C₁₋₅-Alkyloxy, (L)-C₁₋₅-Alkoxy, N₃ oder [(L)-C₁₋₅-Alkylen]-amino;
L Amino, Mono- oder Di-C₁₋₅-alkylamino, Pyrrolidinyl, Morpholinyl, Piperidinyl, Homopiperidinyl oder Piperazinyl ist, wobei verfügbare Ringstickstoffe gegebenenfalls substituiert sein können mit C₁₋₅-Alkyl, Benzyl, C₁₋₅-Alkylcarbonyl oder C₁₋₅-Alkyloxycarbonyl;
Yₑ Wasserstoff oder R²⁰C ist;
Z_{c} Stickstoff oder R²¹C ist;
R²⁰ und R²¹ unabhängig ausgewählt sind aus Wasserstoff, Halogen, C₁₋₆-Alkoxy, C₁₋₅-Alkyl, Cyano, Nitro und R^{m}RⁿN bzw. R^{o}R^{p}N; alternativ R³ und R²⁰ oder R³ und R²¹ zusammengenommen sein können, um einen gegebenenfalls substituierten 5- oder 6-gliedrigen carbocyclischen oder heterocyclischen Ring zu bilden;
Ar für einen monocyclischen oder bicyclischen Aryl- oder Heteroaryling steht, gegebenenfalls substituiert mit zwischen 1 und 3 Substituenten, die unabhängig ausgewählt sind aus Halogen, C₁₋₅-Alkoxy, C₁₋₅-Alkyl, Cyano, Azido, Nitro, R²²R²³N, R^{2A}SO₂, R²⁴OC=O, R²⁵R²⁶NC=O, CF₃, OCF₃, CF₃S und C₁₋₅-Alkylthio;
R²² Wasserstoff, C₁₋₅-Alkyl, Phenyl, Benzyl, Phenethyl,C₁₋₅-Heterocyclyl, C₂₋₈-Acyl, Aroyl, R²⁴OC=O, R²⁵R²⁶NC=O, R²⁴SO, R²⁴SO₂ oder R²⁵R2²⁶NSO₂ ist;
R²³ Wasserstoff oder C₁₋₅-Alkyl ist; alternativ R²² und R²³ zusammengenommen sein können, um einen gegebenenfalls substituierten 4- bis 7-gliedrigen heterocyclischen Ring zu bilden;
R²⁴ Wasserstoff oder C₁₋₅-Alkyl ist;
R²⁵ und R²⁶ unabhängig Wasserstoff oder C₁₋₅-Alkyl sind; oder, alternativ, R²⁵ und R²⁶ zusammengenommen sein können, um einen gegebenenfalls substituierten 4- bis 7-gliedrigen heterocyclischen Ring zu bilden;
W NR²⁷ oder CHR²⁸ ist;
R²⁷ Wasserstoff, C₁₋₅-Alkyl, R²OC=O, R³⁰R³¹NC=O, R²⁹SO, R²⁹SO₂ oder R³⁰R³¹NSO₂ ist; oder, alternativ, R²⁷ und R¹ zusammengenommen sein können, um einen gegebenenfalls substituierten 5- oder 6-gliedrigen heterocyclischen Ring zu bilden, wobei dieser Ring gesättigt, ungesättigt oder aromatisch sein kann;
R²⁸ Wasserstoff, Hydroxy, C₁₋₅-Heterocyclyl, Phenyl oder C₁₋₅-Alkyl ist;
R²⁹ C₁₋₅-Alkyl ist; und
R³⁰ und R³¹ unabhängig ausgewählt sind aus Wasserstoff, C₁₋₅-Alkyl; alternativ R³⁰ und R³¹ zusammengenommen sein können, um einen gegebenenfalls substituierten 4-bis 7-gliedrigen heterocyclischen Ring zu bilden.

5. Verwendung nach Anspruch 1, wobei
eines von R⁵ und R⁶ H ist;
R⁷ und R⁸ zusammengenommen sind, um einen gegebenenfalls substituierten 6-gliedrigen carbocyclischen und heterocyclischen Ring zu bilden; und
Ar für einen monocyclischen Ring steht, gegebenenfalls substituiert mit 1 bis 2 Substituenten, ausgewählt aus Halogen, C₁₋₅-Alkyl, Cyano, Azido, Nitro, R²²R²³N, CF₃ und OCF₃.

6. Verwendung nach Anspruch 5, wobei beide R⁵ und R⁶ jeweils H sind und Ar ein sechsgliedriger Ring ist, substituiert mit zwischen 1 und 2 Substituenten, die unabhängig ausgewählt sind aus Halogen, Methyl, CF₃ und OCF₃, wobei der Substituent oder die Substituenten an der 4-Position oder an den 3- und 4-Positionen sind.

7. Verwendung nach Anspruch 2, wobei R²⁰ und R³ zusammengenommen ein sechsgliedriger carbocyclischer oder heterocyclischer Ring sind, gegebenenfalls substituiert mit zwischen 1 und 3 Substituenten, die unabhängig ausgewählt sind aus Halo, C₁₋₃-Alkoxy, Di-(C₁₋₃-alkyl)-amino und C₂₋₅-Acyl.

8. Verwendung nach Anspruch 1, wobei die Verbindung ausgewählt ist aus:
1-(1-{2-Hydroxy-3-[5-methansulfonyl-3-(4-trifluormethylphenyl)-4,5,6,7-tetrahydropyrazolo[4,3-c]pyridin-1-yl]-propyl}-piperidin-4-yl)-1,3-dihydrobenzoimidazol-2-on;
1-(1-{3-[3-(3,4-Dichlorphenyl)-5-methansulfonyl-4,5,6,7-tetrahydropyrazolo[4,3-c]pyridin-1-yl]-propyl}-piperidin-4-yl)-1,3-dihydrobenzoimidazol-2-on;
3-(3,4-bichlorphenyl)-1-{3-[4-(2-oxo-2,3-dihydrobenzoimidazol-1-yl)-piperidin-1-yl]-propyl}-1,4,6,7-tetrahydropyrazolo[4,3-c]pyridin-5-carbonsäureamid;
6-Chlor-1-(1-{3-[5-methansulfonyl-3-(4-trifluormethylphenyl)-4,5,6,7-tetrahydropyrazolo[4,3-c]pyridin-1-yl]-propyl}-piperidin-4-yl)-1,3-dihydrobenzoimidazol-2-on;
3-(3,4-Dichlorphenyl)-1-{3-[4-(3-methyl-2-oxo-2,3-dihydrobenzoimidazol-1-yl)-piperidin-1-yl]-propyl}-1,4,6,7-tetrahydropyrazolo[4,3c]pyridin-5-carbonsäureamid;
[3-(1-{2-Hydroxy-3-[5-methansulfonyl-3-(4-trifluormethylphenyl)-4,5,6,7-tetrahydropyrazolo [4,3-c]pyridin-1-yl]-propyl}-piperidin-4-yl)-2-oxo-2,3-dihydrobenzoimidazol-1-yl]-acetonitril;
[3-(1-{2-Hydroxy-3-[5-methansulfonyl-3-(4-trifluormethylphenyl)-4,5,6,7-tetrahydropyrazolo[4,3-c]pyridin-1-yl]-propyl}-piperidin-4-yl)-2-oxo-2,3-dihydrobenzoimidazol-1-yl]-essigsäureethylester;
5-Chlor-3-(1-{2-hydroxy-3-[5-methansulfonyl-3-(4-trifluormethylphenyl)-4,5,6,7-tetrahydropyrazolo[4,3-c]pyridin-1-yl]-propyl}-piperidin-4-yl)-1-methyl-1,3-dihydrobenzoimidazol-2-on;
1-{3-[4-(6-Chlor-3-methyl-2-oxo-2,3-dihydrobenzoimidazol-1-yl}-piperidin-1-yl]-propyl}-3-(3,4-dichlorphenyl)-1,4,6,7-tetrahydropyrazolo[4,3-c]pyridin-5-carbonsäureamid;
3-(1-{2-Hydroxy-3-[5-methansulfonyl-3-(4-trifluormethylphenyl)-4,5,6,7-tetrahydropyrazolo[4,3-c]pyridin-1-yl]-propyl}-piperidin-4-yl)-1,5-dimethyl-1,3-dihydrobenzoimidazol-2-on;
3-(1-{2-Hydroxy-3-[5-methansulfonyl-3-(4-trifluormethylphenyl)-4,5,6,7-tetrahydropyrazolo[4,3-c]pyridin-1-yl]-propyl}-piperidin-4-yl)-1,3-dihydroimidazo[4,5-b]pyridin-2-on;
3-(1-{3-[3-(4-Bromphenyl)-5-methansulfonyl-4,5,6,7-tetrahydropyrazolo[4,3-c]pyridin-1-yl]-2-hydroxypropyl}-piperidin-4-yl)-5-methoxy-1,3-dihydroimidazo[4,5-b]pyridin-2-on;
3-(4-Bromphenyl)-1-{2-hydroxy-3-[4-(5-methoxy-2-oxo-1,2-dihydroimidazo[4,5-b]pyridin-3-yl)-piperidin-1-yl]-propyl}-1,4,6,7-tetrahydropyrazolo[4,3-c]pyridin-5-carbonsäureamid;
3-(1-{2-Hydroxy-3-[5-methansulfonyl-3-(4-trifluormethylphenyl)-4,5,6,7-tetrahydropyrazolo[4,3-c]pyridin-1-yl]-propyl}-piperidin-4-yl)-5-methoxy-1-methyl-1,3-dihydroimidazo[4,5-b]pyridin-2-on;
5-Dimethylamino-3-(1-{2-hydroxy-3-[5-methansulfonyl-3-(4-trifluormethylphenyl)-4,5,6,7-tetrahydropyrazolo[4,3-c]pyridin-1-yl]-propyl}-piperidin-4-yl)-1,3-dihydroimidazo[4,5-b]pyridin-2-on;
6-Chlor-1-(1-{3-[5-methansulfonyl-3-(4-trifluormethylphenyl)-4,5,6,7-tetrahydropyrazolo[4,3-c]pyridin-1-yl]-propyl}-piperidin-4-yl)-1,3-dihydroindol-2-on;
1-(1-{2-Hydroxy-{3-[5-methansulfonyl-3-(4-trifluormethylphenyl)-4,5,6,7-tetrahydropyrazolo [4,3-c]pyridin-1-yl]-propyl}-piperidin-4-yl)-3,4-dihydro-1H-chinolin-2-on;
4-(1-{3-[5-Methansulfonyl-3-(4-trifluormethylphenyl)-4,5,6,7-tetrahydropyrazolo[4,3-o]pyridin-1-yl]-propyl}-piperidin-4-yl)-4H-benzo[1,4]oxazin-3-on;
4-(1-{2-Hydroxy-3-[5-methansulfonyl-3-(4-trifluormethylphenyl)-4,5,6,7-tetrahydropyrazolo[4,3-c]pyridin-1-yl]-propyl}-piperidin-4-yl)-4H-benzo[1,4]oxazin-3-on; und
1-(1-{3-[5-Methansulfonyl-3-(4-trifluormethylphenyl)-4,5,6,7-tetrahydropyrazolo[4,3-c]pyridin-1-yl]-propyl}-piperidin-4-yl)-3,4-dihydro-1H-chinazolin-2-on.

9. Verwendung nach Anspruch 1, wobei die Verbindung ausgewählt ist aus:
[3-(1-{2-Hydroxy-3-[5-methansulfonyl-3-(4-trifluormethylphenyl)-4,5,6,7-tetrahydropyrazolo[4, 3-c]pyridin-1-yl]-propyl}-piperidin-4-yl)-2-oxo-2,3-dihydrobenzoimidazol-1-yl]-acetonitril; und 4-(1-{2-Hydroxy-3-[5-methansulfonyl-3-(4-trifluormethylphenyl)-4,5,6,7-tetrahyldropyrazolo[4,3-c]pyridin-1-yl]-propyl}-piperidin-4-yl)-4H-benzo[1,4]oxazin-3-on.

10. Verwendung nach Anspruch 1, wobei die Verbindung ausgewählt ist aus:
2-(1-{3-[5-Acetyl-3-(4-chlorphenyl)-4,5,6,7-tetrahydropyrazolo[4,3-c]pyridin-1-yl]-2-hydroxypropyl}-piperidin-4-ylamino)-benzonitril;
1-(1-{3-[5-Acetyl-3-(4-trifluormethylphenyl)-4,5,6,7-tetrahydropyrazolo[4,3-c]pyridin-1-yl]-2-hydroxypropyl}-piperidin-4-yl)-1,3-dihydrobenzoimidazol-2-on;
3-(1-{3-[5-Acetyl-3-(4-bromphenyl)-4,5,6,7-tetrahydropyrazolo[4,3-c]pyridin-1-yl]-2-hydroxypropyl}-piperidin-4-yl)-3H-benzooxazol-2-on;
1-(3-(4-Chlor-3-methylphenyl)-1-{3-[4-(3,4-dichlorphenoxy)-piperidin-1-yl]-propyl}-1,4,6,7-tetrahydropyrazolo[4,3-c]pyridin-5-yl)-ethanon;
1-(3-(4-Chlor-3-methylphenyl)-1-{3-[4-(2,3-dihydroindol-1-yl)-piperidin-1-yl]-2-hydroxypropyl}-1,4,6,7-tetrahydropyrazolo[4,3-c]pyridin-5-yl)-ethanon;
(S)-1-(1-{3-[5-Acetyl-3 -(4-chlor-3-methylphenyl)-4,5,6,7-tetrahydropyrazolo[4,3-c]pyridin-1-yl]-2-hydroxypropyl}-piperidin-4-yl)-6-chlor-1,3-dihydrobenzoimidazol-2-on;
1-(1-{3-[5-Acetyl-3-(4-trifluormethylphenyl)-4,5,6,7-tetrahydropyrazolo[4,3-c]pyridin-1-yl]-2-hydroxypropyl}-piperidin-4-yl)-3-(2-morpholin-4-ylethyl)-1,3-dihydrobenzoimidazol-2-on;
1-(1-{3-[3-(4-Bromphenyl)-5-methansulfonyl-4,5,6,7-tetrahydropyrazolo[4,3-c]pyridin-1-yl]-2-hydroxypropyl}-piperidin-4-yl)-6-chlor-1,3-dihydrobenzoimidazol-2-on;
[3-(1-{3-[5-Methansulfonyl-3-(4-trifluormethylphenyl)-4,5,6,7-tetrahydropyrazolo[4, 3-c]pyridin-1-yl]-propyl}-piperidin-4-yl)-2-oxo-2,3-dihydrobenzoimidazol-1-yl]-acetonitril;
5-chlor-(3-(1-3-[5-methansulfonyl-3-(4-trifluormethylphenyl)-4,5,6,7-tetrahydropyrazolo[4,3-c]pyridin-1-yl]-propyl}-piperidin-4-yl)-1-methyl-1,3-dihydrobenzoimidazol-2-on;
1-(1-{3-[5-Methansulfonyl-3-(4-trifluormethylphenyl)-4,5,6,7-tetrahydropyrazolo[4,3-c]pyridin-1-yl]-propyl}-piperidin-4-yl)-1,3-dihydroindol-2-on;
1-[3-(4-Chlor-3-methylphenyl)-1-(3-{4-[3-(4-chlorphenyl)-[1,2,4]oxadiazol-5-yl]-piperidin-1-yl}-2-hydroxypropyl)-1,4,6,7-tetrahydropyrazolo[4,3-c]pyridin-5-yl]-ethanon;
1-[1-{2-Hydroxy-3-[4-(5-trifluormethylbenzothiazol-2-yl)-piperidin-1-yl]-propyl}-3-(4-trifluormethylphenyl)-1,4,6,7-tetrahydropyrazolo[4,3-c]pyridin-5-yl]-ethanon;
1-[1-{3-[4-(Benzo[d]isoxazol-3-yloxy)-piperidin-1-yl]-2-hydroxypropyl}-3-(4-trifluormethylphenyl)-1,4,6,7-tetrahydropyrazolo[4,3-c]pyridin-5-yl]-ethanon;
1-[1-{3-[4-(5-Chlorbenzooxazol-2-yl)-piperidin-1-yl]-2-hydroxypropyl}-3- (4-trifluormethylphenyl)-1,4,6,7-tetrahydropyrazolo[4,3-c]pyridin-5-yl]-ethanon;
1-[1-{3-[4-(Benzothiazol-2-ylamino)-piperidin-1-yl]-2-hydroxypropyl}-3-(4-trifluormethylphenyl)-1,4,6,7-tetrahydropyrazolo[4,3-c]pyridin-5-yl]-ethanon;
1-[1-{3-[4-(3,5-Dichlorpyridin-4-yloxy)-piperidin-1-yl]-2-hydroxypropyl-3-(4-trifluormethylphenyl)-1,4,6,7-tetrahydropyrazolo[4,3-c]pyridin-5-yl]-ethanon;
1-[1-{3-[4-(1H-Benzoimidazol-2-yl)-piperidin-1-yl]-2-hydroxypropyl}-3-(4-trifluormethylphenyl)-1,4,6,7-tetrahydropyrazolo [4,3-c]pyridin-5-yl]-ethanon;
6-Chlor-4-(1-{2-hydroxy-3-[5-methansulfonyl-3-(4-trifluormethyphenyl)-4,5,6,7-tetrahydropyrazolo[4,3-c]pyridin-1-yl]-propyl}-piperidin-4-yl)-4H-benzo[1,4] oxazin-3-on;
6-Chlor-1-(1-{2-hydroxy-3-[5-methansulfonyl-3-(4-trifluormethylphenyl)-4,5,6,7-tetrahydropyrazolo[4,3-c]pyridin-1-yl]-propyl}-piperidin-4-yl)-3,4-dihydro-1H-chinolin-2-on;
6-Chlor-1-(1-{2-hydroxy-3-[5-methansulfonyl-3-(4-trifluormethylphenyl)-4,5,6,7-tetrahydropyrazolo[4,3-c]pyridin-1-yl]-propyl}-piperidin-4-yl)-3,4-dihydro-1H-chinazolin-2-on;
1-[4-(6-Chlor-2,2-dioxo-3,4-dihydro-2H-2λ⁶-benzo[1,2,6]thiadiazin-1-yl)-piperidin-1-yl]-3-[5-methansulfonyl-3-(4-trifluormethylphenyl)-4,5,6,7-tetrahydropyrazolo[4,3-c]pyridin-1-yl]-propan-2-ol;
4-(1-{2-Hydroxy-3-[5-methansulfonyl-3-(4-trifluormethylphenyl)-4,5,6,7-tetrahydropyrazolo[4,3-c]pyridin-1-yl]-propyl}-piperidin-4-yl)-4H-pyrido[3,2-b][1,4]oxazin-3-on;
5-Chlor-1-(1-{2-hydroxy-3-[5-methansulfonyl-3-(4-trifluormethyphenyl)-4,5,6,7-tetrahydropyrazolo[4,3-c]pyridin-1-yl]-propyl}-piperidin-4-yl)-1,3-dihydroindol-2-on;
1-[4-(6-Chlorindol-1-yl)-piperidin-1-yl]-3-[5-methansulfonyl-3-(4-trifluormethylphenyl)-4,5,6,7-tetrahydropyrazolo[4,3-c]pyridin-1-yl]-propan-2-ol;
1-(1-{3-[5-Methansulfonyl-3-(4-trifluormethylphenyl)-4,5,6,7-tetrahydropyrazolo[4,3-c]pyridin-1-yl]-propyl}-piperidin-4-yl)-1H-benzotriazol;
1-{3-[4-(3-Methyl-2-oxo-2,3-dihydrobenzoimidazol-1-yl)-piperidin-1-yl]-propyl}-3-(4-trifluormethylphenyl)-1,4,6,7-tetrahydropyrazolo[4,3-c]pyridin-5-sulfonsäureamid;
5-Chlor-3-(1-{2-hydroxy-3-[4-pyridin-4-yl-3-(4-trifluormethylphenyl)-pyrazol-1-yl]-propyl}-piperidin-4-yl)-1-methyl-1,3-dihydrobenzoimidazol-2-on;
4-(1-{2-Hydroxy-3-[4-pyrazin-2-yl-3-(4-trifluormethylphenyl)-pyrazol-1-yl]-propyl}-piperidin-4-yl)-4H-benzo[1,4]oxazin-3-on;
(S)-1-(1-{2-Hydroxy-3-[5-methansulfonyl-3-(4-trifluormethylphenyl)-4,5,6,7-tetrahydropyrazolo[4,3-c]pyridin-1-yl]-propyl}-piperidin-4-yl)-3-methyl-1,3-dihydrobenzoimidazol-2-on; und
(S)-5-Dimethylamino-3-(1-{2-hydxoxy-3-[5-methansulfonyl-3-(trifluormethylphenyl)-4,5,6,7-tetrahydropyrazolo[4,3-c]pyridin-1-yl]-propyl}-piperidin-4-yl)-1-methyl-1,3-dihydroimidazo [4,5-b]pyridin-2-on.

11. Verwendung nach Anspruch 1, wobei die Verbindung ausgewählt ist aus:
1-(1-{3-[5-Acetyl-3-(4-bromphenyl)-4,5,6,7-tetrahydropyrazolo[4,3c]pyridin-1-yl]-2-hydroxyprapyl}-piperidin-4-yl)-5-methoxy-1,3-dihydrobenzoimidazol-2-on;
6-Chlor-1-(1-{3-[3-(4-chlor-3-methylphenyl)-5-methansulfonyl-4,5,6,7-tetrahydropyrazolo[4,3-c]pyridin-1-yl]-2-hydroxypropyl}-piperidin-4-yl)-1,3-dihydrobenzoimidazol-2-on.

12. Verwendung nach Anspruch 1, wobei die pharmazeutische Zusammensetzung in einer Dosierungsmenge formuliert ist, die geeignet ist zur Behandlung eines allergischen Zustands.

## Revendications

1. Utilisation d'un composé dans la fabrication d'une composition pharmaceutique pour le traitement d'un sujet avec une condition allergique, ledit composé étant de formule (I): 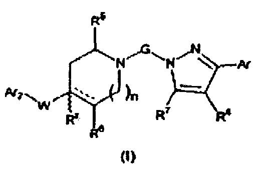 dans laquelle
Ar₂ est un système de cycle monocyclique ou bicyclique, insaturé, saturé ou aromatique, facultativement fusionné, comprenant facultativement entre 1 et 5 fractions de cycle d'hétéroatome indépendamment choisies parmi O, S, N, SO₂ et C=O; ledit système de cycle Ar₂ étant facultativement substitué par entre 1 et 4 substituants;
R⁵ et R⁶ sont indépendamment choisis parmi un atome d'hydrogène et un groupe alkyle en C_{1 à 6};
R⁷ et R⁶ sont indépendamment un atome d'hydrogène, un groupe alkyle en C_{1 à 5}, alcényle en C_{2 à 5}, alcoxy en C₁ à ₅, alkylthio en C_{1 à 5}, un atome d'halogène ou un groupe carbocyclyle ou hétérocyclyle à 4 à 7 chaînons; en variante, R⁷ et R⁸ peuvent être pris ensemble pour former un cycle carbocyclique ou hétérocyclique à 5 à 7 chaînons facultativement substitué, lequel cycle peut être insaturé ou aromatique, et peut être facultativement substitué par entre un et trois substituants indépendamment choisis parmi les groupes halogéno, cyano, amino, hydroxy, nitro, R⁴, R⁴O-, R⁴S-, R⁴O (alkylène en C_{1 à 6}) -, R⁴O (C=O) -, R⁴ (C=O) -, R⁴(C=S)-, R⁴ (C=O) O-, R⁴O(C=0) (C=0) -, R⁴SO₂, NHR⁴⁴ (C=NH) -, NHR⁴⁴SO₂-, et NHR⁴⁴(C=O) -;
R⁴ est H, alkyle en C₁-C₆, alcényle en C_{2 à 5}, hétérocyclyle en C_{1 à 6}, (hétérocyclyle en C_{1 à 5}) alkylène en C_{1 à 6,} phényle, benzyle, phénéthyle, NH₂, mono- ou di(alkyle en C_{1 à 6}) N-, (alcoxy en C_{1 à 6}) carbonyl- ou R⁴²OR⁴³-, où R⁴² est H, un groupe alkyle en C_{1 à 5}, alcényle en C_{2 à 5}, phényle, benzyle, phénéthyle, hétérocyclyle en C_{1 à 5},
ou (hétérocyclyle en C_{1 à 5})alkylène en C_{1 à 6} et R⁴³ est un groupe alkylène en C_{1 à 5}, phénylène, ou hétérocyclyle en C_{1 à 5} divalent;
R⁴⁴ peut être l'une des valeurs pour R⁴;
n vaut 0, 1, ou 2;
G est alcènediyle en C_{3 à 6} ou alcanediyle en C_{3 à 6}, facultativement substitué par un groupe hydroxy, un atome d'halogène, un groupe alcoxy en C_{1 à 5}, alkyle en C_{1 à 5}, oxo, hydroximino, CO₂R^{k}, R^{k}R^{l}N, R^{k}R¹NCO₂, (L)-alkylène en C_{1 à 4}, (L) -alcoxy en C_{1 à 5}, N₃, ou [(L)-alkylène en C_{1 à 5}] amino;
chacun de R^{k} et R^{l} est indépendamment un atome d'hydrogène, un groupe alkyle en C_{1 à 5}, alcényle en C_{3 à 5}, phényle, benzyle, phénéthyle ou hétérocyclyle en C_{1 à 5}; en variante R^{k} et R^{l} peuvent être pris ensemble pour former un cycle hétérocyclique à 4 à 7 chaînons facultativement substitué, lequel cycle peut être saturé, insaturé ou aromatique;
L est un groupe amino, mono ou di-alkylamino en C_{1 à 5}, pyrrolidinyle, morpholinyle, pipéridinyle, homopipéridinyle, ou pipérazinyle, où des atomes d'azote cycliques disponibles peuvent être facultativement substitués par un groupe alkyle en C_{1 à 5}, benzyle, acyle en C_{2 à 5}, alkylsulfonyle en C_{1 à 5}, ou alcoxycarbonyle en C_{1 à 5};
Ar représente un cycle aryle ou hétéroaryle monocyclique ou bicyclique, facultativement substitué par entre 1 et 3 substituants indépendamment choisis parmi un atome d'halogène, un groupe alcoxy en C_{1 à 5}, alkyle en C_{1 à 5}, alcényle en C_{2 à 5}, cyano, azido, nitro, R²²R²³N, R²⁴SO₂, R²⁴ S, R²⁴SO, R²⁴OC=O, R²²R²³NC=O, halogénoalkyle en C_{1 à 5}, halogénoalcoxy en C_{1 à 5}, halogénoalkylthio en C_{1 à 5} et alkylthio en C_{1 à 5};
R²² est un atome d'hydrogène, un groupe alkyle en C_{1 à 5}, alcényle en C_{3 à 5}, phényle, phénéthyle, benzyle, ou hétérocyclyle en C_{1 à 5}, acyle en C_{2 à 8}, aroyle, R³⁸OC=O, R²⁵R²⁶NC=O, R³⁸SO, R³⁸SO₂, R³⁸S ou R²⁵R²⁶NSO₂;
R³⁸ est H, un groupe alkyle en C_{1 à 5}, alcényle en C_{3 à 5}, phényle, benzyle, phénéthyle ou hétérocyclyle en C_{1 à 5};
R²³ est un atome d'hydrogène, un groupe alkyle en C_{1 à 5}, alcényle en C_{3 à 5}, phényle, benzyle ou hétérocyclyle en C_{1 à 5}; en variante, R²² et R²³ peuvent être pris ensemble pour former un cycle hétérocyclique à 4 à 7 chaînons facultativement substitué, lequel cycle peut être saturé, insaturé ou aromatique;
R²⁴ est un groupe alkyle en C_{1 à 5}, alcényle en C_{3 à 5}, phényle, benzyle ou hétérocyclyle en C_{1 à 5};
R²⁵ et R²⁶ sont indépendamment un atome d'hydrogène, un groupe alkyle en C_{1 à 5}, alcényle en C_{3 à 5}, phényle, benzyle ou hétérocyclyle en C_{1 à 5;}
ou, en variante, R²⁶ et R²⁶ peuvent être pris ensemble pour former un cycle hétérocyclique à 4 à 7 chaînons facultativement substitué, lequel cycle peut être saturé, insaturé ou aromatique;
W représente O, S, NR²⁷, C=O, (C=O)NH, NH(C=O) , CHR²⁸, ou une liaison covalente;
R^{z} est H ou OH et la ligne en trait tireté est absente; ou R^{z} est absent où la ligne en trait tireté est une liaison sp²;
R²⁷ est un atome d'hydrogène, un groupe alkyle en C_{1 à 5}, alcényle en C_{3 à 5}, phényle, naphtyle, benzyle, phénéthyle, hétérocyclyle en C_{1 à 5}, acyle en C_{2 à 8}, aroyle, R²⁹ OC=O, R³⁰R³¹NC=O, R²⁹SO, R²⁹S, R²⁹SO₂ ou R³⁰R³¹NSO₂;
ou, en variante, R²⁷ et une partie de Ar₂ peuvent être pris ensemble pour former un cycle hétérocyclique à 5 ou 6 chaînons facultativement substitué avec facultativement 1 à 3 fractions d'hétéroatome supplémentaire dans le cycle choisies parmi O, NR⁹, NR¹⁰, N, SO₂, C=O et S; lequel cycle peut être saturé, insaturé ou aromatique; R⁹ et R¹⁰ sont indépendamment choisis parmi H, alkyle en C_{1 à 3} et -CH₂CO₂ (alkyle en C_{1 à 4});
R²⁸ est un atome d'hydrogène, un groupe alkyle C_{1 à 5}, alcényle en C_{3 à 5}, hydroxy, phényle, benzyle, hétérocyclyle en C_{1 à 5}, R²⁹O, R³⁰R³¹NC=O, R²⁹SO, R²⁹ SO, R²⁹SO₂ ou R³⁰R³¹NSO₂;
R²⁹ est un groupe alkyle en C_{1 à 5}, alcényle en C_{3 à 5}, phényle, benzyle, ou hétérocyclyle en C_{1 à 5};
R³⁰ et R³¹ sont indépendamment choisis parmi un atome d'hydrogène, un groupe alkyle en C_{1 à 5}, alcényle en C_{3 à 5}, phényle, benzyle, phénéthyle, naphtyle et hétéroaryle en C_{1 à 5};
en variante, R³⁰ et R³¹ peuvent être pris ensemble pour former un cycle à 4 à 7 chaînons facultativement substitué carbocyclique ou hétérocyclique lequel cycle peut être saturé, insaturé ou aromatique;
où chacun des groupes hydrocarbyle ou hétérocarbyle ci-dessus, sauf indication contraire, et en plus d'un substituant spécifié quelconque, est facultativement et indépendamment substitué par entre 1 et 3 substituants choisis parmi un groupe méthyle, halogénométhyle, hydroxyméthyle, halogéno, hydroxy, amino, nitro, cyano, alkyle en C_{1 à 5}, alcoxy en C_{1 à 5}, - COOH, acyle en C_{2 à 6}, [di (alkyle en C_{1 à 4}) amino] alkylène en C_{2 à 5}, [di (alkyle en C_{1 à 4}) amino] alkyle en C₂ à ₅-NH-CO- et halogénoalcoxy en C_{1 à 5};
ou un sel, amide ou ester pharmaceutiquement acceptable de celui-ci; ou une forme a stéréoïsomère de celui-ci.

2. Utilisation selon la revendication 1, dans laquelle Ar₂ est choisi parmi le 2,5-di(alkyle en C_{1 à 6}) aminopyrrolyle et les six formules suivantes: 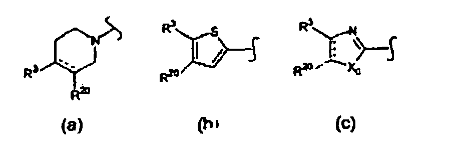 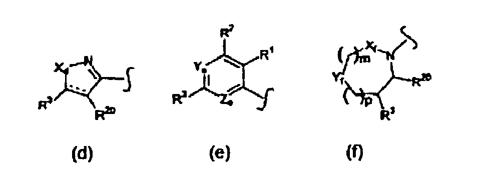 dans lesquelles
chaque ligne en trait tireté peut être une liaison sp² ou absente;
X_{c} est O, S, ou N; et X_{d} est O ou S;
R¹ est un atome d'hydrogène, un atome d'halogène, un groupe alcoxy en C_{1 à 5}, hydroxy, alkyle en C_{1 à 5}, alcényle en C_{2 à 5}, cyano, nitro, R^{a}R^{b}N, acyle en C_{2 à 8}, hétérocyclyle en C_{1 à 5}, (hétérocyclyle en C_{1 à 5}) alkylène en C_{1 à 5}, R¹¹S, R¹¹SO, R¹¹SO₂, R^{c}OC=O, R^{c}R^{d}NC=O ou R^{c}R^{d}NSO₂; ou R¹ peut être pris conjointement avec R²⁷ comme prévu ci-dessous;
R² est un atome d'hydrogène, un atome d'halogène, un groupe alcoxy en C_{1 à 5}, hydroxy, alkyle en C_{1 à 5}, alcényle en C_{2 à 5}, cyano, nitro, R^{e}R^{f}N, hétérocyclyle en C_{1 à 5}, ou acyle en C_{2 à 8};
R³ est un atome d'hydrogène, un atome d'halogène, un groupe alcoxy en C_{1 à 5}, hydroxy, alkyle en C_{1 à 5}, alcényle en C_{2 à 5}, cyano, nitro, R^{g}R^{h}N, acyle en C_{2 à 8}, hétérocyclyle en C_{1 à 5}, R^{h}OC=O, R^{g}R^{h}NC=O, ou R⁹R^{h}NSO₂;
R^{a} est choisi parmi un atome d'hydrogène, un groupe alkyle en C_{1 à 5}, alcényle en C_{3 à 5}, phényle, benzyle, phénéthyle, hétérocyclyle en C_{1 à 5}, acyle en C_{2 à 8}, aroyle, R¹OC=O, RⁱR^{j}NC=O, R¹²SO, R¹²SO₂, R¹²S et RⁱR^{j}NSO₂;
R^{e} est choisi parmi un atome d'hydrogène, un groupe alkyle en C_{1 à 5}, alcényle en C_{3 à 5}, phényle, benzyle, phénéthyle, hétérocyclyle en C_{1 à 5}, acyle en C_{2 à 8}, aroyle, R³²OC=O, R³²R³³NC=O, R¹³SO, R¹³SO₂, R¹³S et R³²R³³NSO₂;
R^{m} est choisi parmi un atome d'hydrogène, un groupe alkyle en C_{1 à 5}, alcényle en C_{3 à 5}, phényle, benzyle, phénéthyle, hétérocyclyle en C_{1 à 5}, acyle en C_{2 à 8}, aroyle, R³⁴OC=O, R³⁴R³⁵NC=O, R¹⁵SO, R¹⁵SO₂, R¹⁵S et R³⁴R³⁵NSO₂;
R^{o} est choisi parmi un atome d'hydrogène, un groupe alkyle en C_{1 à 5}, alcényle en C_{3 à 5}, phényle, benzyle, phénéthyle, hétérocyclyle en C_{1 à 5}, acyle en C_{2 à 8}, aroyle, R³⁸OC=O, R³⁸R³⁷NC=O, R¹⁹SO, R¹⁹SO₂, R¹⁹S et R³⁸R³⁷NSO₂;
chacun de R^{b}, R^{f} , Rⁿ, R^{p}, R³², R³³, R³⁴, R³⁵, R³⁶, R³⁷, R³⁹ et R⁴⁰ est indépendamment choisi parmi un atome d'hydrogène, un groupe alkyle en C_{1 à 5}, alcényle en C_{3 à 5}, phényle, benzyle, phénéthyle et hétéroaryle en C_{1 à 5}; en variante, R^{a} et R^{b}, R^{e} et R^{f}, R^{m} et Rⁿ, et R^{o} et R^{p}, indépendamment, peuvent être pris ensemble pour former un cycle hétérocyclique à 4 à 7 chaînons facultativement substitué, lequel cycle peut être saturé, insaturé ou aromatique;
chacun de R¹¹, R¹², R¹³, R¹⁴, R¹⁵, R¹⁶, R¹⁹, R³⁸ et R⁴¹ est indépendamment un groupe alkyle en C_{1 à 5}, alcényle en C_{3 à 5}, phényle, benzyle, phénéthyle, ou hétérocyclyle en C_{1 à 5};
chacun de R^{c} et R^{d}, et Rⁱ et R^{j} est indépendamment un atome d'hydrogène, un groupe alkyle en C_{1 à 5}, alcényle en C_{3 à 6}, phényle, benzyle, phénéthyle, ou hétéroaryle en C_{1 à 5};
en variante, R^{c} et R^{d}, et Rⁱ et R^{j}, indépendamment, peuvent être pris ensemble pour former un cycle hétérocyclique à 4 à 7 chaînons facultativement substitué, lequel cycle peut être saturé, insaturé ou aromatique;
R^{g} est un atome d'hydrogène, un groupe alkyle en C_{1 à 5}, alcényle en C_{3 à 5}, phényle, benzyle, phénéthyle, or hétérocyclyle en C_{1 à 5}, acyle en C_{2 à 8}, aroyle, R¹⁷OC=O, R¹⁷R¹⁸NC=O, R¹⁶S, R¹⁶SO, R¹⁶SO₂ ou R¹⁷R¹⁸NSO₂;
R^{h} est un atome d'hydrogène, un groupe alkyle en C_{1 à 5}, alcényle en C_{3 à 5}, phényle, benzyle, phénéthyle ou hétérocyclyle en C_{1 à 5};
en variante, R⁹ et R^{h} peuvent être pris ensemble pour former un cycle hétérocyclique à 4 à 7 chaînons facultativement substitué, lequel cycle peut être saturé insaturé ou aromatique;
R¹⁷ et R¹⁸ sont indépendamment un atome d'hydrogène, un groupe alkyle en C_{1 à 5}, alcényle en C_{3 à 5}, phényle, benzyle ou hétérocyclyle en C_{1 à 5};
en variante, R¹⁷ et R¹⁸ peuvent être pris ensemble pour former un cycle hétérocyclique à 4 à 7 chaînons facultativement substitué, lequel cycle peut être saturé, insaturé ou aromatique;
Yₐ est un atome d'azote ou R²⁰C;
Zₑ est un atome d'azote ou R²¹C;
R²⁰ est un atome d'hydrogène, un atome d'halogène, un groupe alcoxy en C_{1 à 5}, alkyle en C_{1 à 5}, alcényle en C_{2 à 5}, cyano, nitro, R^{m}RⁿN, acyle en C_{2 à 8}, R^{m}OC=O, R¹⁴S, R¹⁴SO ou R¹⁴SO₂;
R²¹ est un atome d'hydrogène, un atome d'halogène, un groupe alcoxy en C_{1 à 5}, alkyle en C_{1 à 5}, alcényle en C_{2 à 5}, cyano, nitro, R^{o}R^{p}N, acyle en C_{2 à 8}, R¹⁶OC=O, R¹¹S, R¹¹SO ou R¹¹SO₂;
en variante, R³ et R²⁰ ou R³ et R²¹ peuvent être pris ensemble pour former un cycle carbocyclique ou hétérocyclique à 5 ou 6 chaînons facultativement substitué, lequel cycle peut être saturé, insaturé ou aromatique; où ledit cycle peut être facultativement substitué par un groupe halogéno, di(alkyle en C_{1 à 5}) amino, acyle en C_{2 à 5} et alcoxy en C_{1 à 5};
R²⁷ est un atome d'hydrogène, un groupe alkyle en C_{1 à 5}, alcényle en C_{3 à 5}, phényle, naphtyle, benzyle, phénéthyle, hétérocyclyle en C_{1 à 5}, acyle en C_{2 à 8}, aroyle, R²⁹OC=O, R³⁰R³¹NC=O, R²⁹SO, R²⁹S, R²⁹SO₂ ou R³⁰R³¹NSO₂ ;
ou, en variante, R²⁷ et R¹ peuvent être pris ensemble pour former un cycle hétérocyclique à 5 ou 6 chaînons facultativement substitué par facultativement 1 à 3 fractions d'hétéroatome supplémentaires dans le cycle choisies parmi O, NR⁹, NR¹⁰, N, SO₂, C=O et S; lequel cycle peut être saturé, insaturé ou aromatique;
R⁹ et R¹⁰ sont indépendamment choisis parmi H, un groupe alkyle en C_{1 à 3} et -CH₂CO₂ (alkyle en C_{1 à 4}) ;
X_{f} est CHR^{1f}, =N-, NH, C=O, SO₂, CHSR^{1f} où, dans la formule (f), R^{1f} est un atome d'hydrogène, un atome d'halogène, un groupe alcoxy en C_{1 à 5}, hydroxy, alkyle en C_{1 à 5}, alcényle en C_{3 à 5}, cyano, nitro, R³⁹R⁴⁰N, acyle en C_{2 à 8}, hétérocyclyle en C_{1 à 5}, (hétérocyclyle en C_{1 à 5}) alkylène en C_{1 à 5}, R¹⁴S, R⁴¹SO, R⁴¹SO₂, R³⁹OC=O, R³⁹R⁴⁰NC=O, R³⁹R⁴⁰NSO₂, R⁴¹SO₃- ou R³⁹(C=O)O-;
Y_{f} est CH₂, CHR^{2f}, =CR^{2f}, O ou NR^{2f}, où R^{2f} est H, un groupe alkyle en C_{1 à 7}, alcényle en C_{3 à 5}, acyle en C_{2 à 8}, hétérocyclyle en C_{1 à 5}, (hétérocyclyle en C_{1 à 5})-alkylène en C_{1 à 5}, phényle, (phényl)-alkylène en C_{1 à 5}, (cycloalkyle en C_{3 à 7}) -alkylène en C_{1 à 5}, (H₂NCO)-alkylène en C_{1 à 5}, halogénoalkyle en C_{1 à 5}, cyanoalkyle en C_{1 à 5}, (alcoxycarbonyle en C_{1 à 5}) alkylène en C_{1 à 5} et (phénylcarbonyl)NH-;
m vaut 0 ou 1;
p vaut 0 ou 1;
où chacun des groupes hydrocarbyle ou hétérocarbyle ci-dessus, sauf indication contraire, et en plus d'un substituant spécifié quelconque, est facultativement et indépendamment substitué par entre 1 et 3 substituants choisis parmi les groupes méthyle, halogénométhyle, hydroxyméthyle, halogéno, hydroxy, amino, nitro, cyano, alkyle en C_{1 à 5}, alcoxy en C_{1 à 5}, - COOH, acyle en C_{2 à 6}, [di (alkyle en C_{1 à 4}) amino]alkylène en C_{2 à 5}, [di (alkyle en C_{1 à 4}) amino] alkyle en C_{2 à 5}-NH-CO- et halogénoalcoxy en C_{1 à 5}.

3. Utilisation selon la revendication 2, dans laquelle Ar₂ est choisi parmi les formules (f).

4. Utilisation selon la revendication 2, dans laquelle Ar₂ est la formule (e) et R¹ est un atome d'halogène, un groupe alcoxy en C_{1 à 5}, hydroxy, alkyle en C_{1 à 5}, cyano, nitro et R^{a}R^{b}N, où R¹ peut être pris conjointement avec R²⁷ comme prévu ci-dessous;
R² est un atome d'hydrogène, un atome d'halogène, un groupe alcoxy en C_{1 à 5}, alkyle en C_{1 à 5} ou R^{e}R^{f}N;
R³ est un atome d'hydrogène, un atome d'halogène, un groupe alcoxy en C_{1 à 5}, hydroxy, alkyle en C_{1 à 5}, cyano, R^{g}R^{h}N;
R⁵ et R⁶ sont indépendamment choisis parmi un atome d'hydrogène et un groupe alkyle en C_{1 à 3};
R⁷ et R⁸ sont indépendamment pris ensemble pour former un cycle carbocyclique ou hétérocyclique à 5 à 7 chaînons facultativement substitué, lequel cycle peut être saturé, insaturé ou aromatique;
chacun de R^{a}, R^{e}, R^{m} et R^{o} est indépendamment choisi parmi un atome d'hydrogène, un groupe alkyle en C_{1 à 5}, acyle en C_{2 à 8} et les groupes ROC=O, RRNC=O, RS, RSO, RSO₂ et RRNSO₂ respectifs;
chacun de R^{b}, R^{f}, Rⁿ et R^{p}, est indépendamment choisi parmi un atome d'hydrogène et un groupe alkyle en C_{1 à 5};
chacun de R¹¹, R¹², R¹³, R¹⁴, R¹⁵, R¹⁶, R¹⁹ et R³⁸ est indépendamment un groupe alkyle en C_{1 à 5};
chacun de R^{c} et R^{d}, Rⁱ et R^{j}, R^{k} et R¹, R³² et R³³, R³⁴ et R³⁵, R³⁶ et R³⁷ sont indépendamment un atome d'hydrogène ou un groupe alkyle en C_{1 à 5}, ou sont pris ensemble pour former un cycle hétérocyclique à 4 à 7 chaînons facultativement substitué;
R^{g} est un atome d'hydrogène, un groupe alkyle en C_{1 à 5}, acyle en C_{2 à 8}, R¹⁷OC=O, R¹⁷R¹⁸NC=O, R¹⁶S, R¹⁶SO, R¹⁶SO₂ ou R¹⁷R¹⁸NSO₂;
R^{h} est un atome d'hydrogène ou un groupe alkyle en C_{1 à 5}; en variante, R^{g} et R^{h} peuvent être pris ensemble pour former un cycle hétérocyclique à 4 à 7 chaînons facultativement substitué;
R¹⁷ et R¹⁸ sont indépendamment un atome d'hydrogène ou un groupe alkyle en C_{1 à 5};
n vaut 0 ou 1;
G est un groupe alcènediyle en C_{3 à 4} ou alcanediyle en C_{3 à 4}, facultativement substitué par un groupe hydroxy, un atome d'halogène, un groupe alkyloxy en C_{1 à 5}, (L)-alcoxy en C_{1 à 5}, N₃, ou [(L)-alkylène en C_{1 à 6}] amino;
L est un groupe amino, mono ou di-alkylamino en C_{1 à 5}, pyrrolidinyle, morpholinyle, pipéridinyle, homopipéridinyle ou pipérazinyle, où les atomes d'azote cycliques disponibles peuvent être facultativement substitués par un groupe alkyle en C_{1 à 5}, benzyle, alkylcarbonyle en C_{1 à 5}, ou alkyloxycarbonyle en C_{1 à 5};
Yₑ est un atome d'azote ou R²⁰C;
Zₑ est un atome d'azote ou R²¹C;
R²⁰ et R²¹ sont indépendamment choisis parmi un atome d'hydrogène, un atome d'halogène, un groupe alcoxy en C_{1 à 5}, un groupe alkyle en C_{1 à 5}, cyano, nitro et R^{m}RⁿN ou R^{o}R^{p}N, respectivement;
en variante, R³ et R²⁰ ou R³ et R²¹ peuvent être pris ensemble pour former un cycle carbocyclique ou hétérocyclique à 5 ou 6 chaînons facultativement substitué;
Ar représente un cycle aryle ou hétéroaryle monocyclique ou bicyclique, facultativement substitué par entre 1 et 3 substituants indépendamment choisis parmi un atome d'halogène, un groupe alcoxy en C_{1 à 5}, alkyle en C_{1 à 5}, cyano, azido, nitro, R²²R²³N, R²⁴SO_{2,} R²⁴OC=O, R²⁵R²⁸NC=O, CF₃, OCF₃, CF₃S et alkylthio en C_{1 à 5};
R²² est un atome d'hydrogène, un groupe alkyle en C_{1 à 5}, phényle, benzyle, phénéthyle, hétérocyclyle en C_{1 à 5}, acyle en C_{2 à 5}, aroyle, R²⁴OC=O, R²⁵R²⁶NC=O, R²⁴SO, R²⁴SO₂ ou R²⁵R²⁸NSO₂*;*
R²³ est un atome d'hydrogène ou un groupe alkyle en C_{1 à 5}; en variante, R²² et R²³ peuvent être pris ensemble pour former un cycle hétérocyclique à 4 à 7 chaînons facultativement substitué;
R²⁴ est un atome d'hydrogène ou un groupe alkyle en C_{1 à 5};
R²⁵ et R²⁶ sont indépendamment un atome d'hydrogène ou un groupe alkyle en C_{1 à 5};
ou, en variante, R²⁵ et R²⁶ peuvent être pris ensemble pour former un cycle hétérocyclique à 4 à 7 chaînons facultativement substitué;
W est NR²⁷ ou CHR²⁸;
R²⁷ est un atome d'hydrogène, un groupe alkyle en C_{1 à 5}. R²⁹OC=O, R³⁰R³¹NC=O, R²⁹SO, R²⁸SO₂ ou R³⁰R³¹NSO₂;
ou, en variante, R²⁷ et R¹ peuvent être pris ensemble pour former un cycle hétérocyclique à 5 ou 6 chaînons facultativement substitué, lequel cycle peut être saturé, insaturé ou aromatique;
R²⁸ est un atome d'hydrogène, un groupe hydroxy, hétérocyclyle en C_{1 à 5}, phényle ou alkyle en C_{1 à 5};
R²⁹ est un groupe alkyle en C_{1 à 5}; et
R³⁰ et R³¹ sont indépendamment choisis parmi un atome d'hydrogène, un groupe alkyle en C_{1 à 5}; en variante, R³⁰ et R³¹ peuvent être pris ensemble pour former un cycle hétérocyclique à 4 à 7 chaînons facultativement substitué.

5. Utilisation selon la revendication 1, dans laquelle
l'un de R⁵ et R⁶ est H,
R⁷ et R⁸ sont pris ensemble pour former un cycle carbocyclique ou hétérocyclique à 6 chaînons facultativement substitué; et
Ar représente un cycle monocyclique, facultativement substitué par 1 à 2 substituants choisis parmi un atome d'halogène, un groupe alkyle en C_{1 à 5}, cyano, azido, nitro, R²²R²³N, CF₃ et OCF₃.

6. Utilisation selon la revendication 5, dans laquelle R⁵ et R⁶ sont chacun H, et Ar est un cycle à six chaînons substitué par entre 1 et 2 substituants indépendamment choisis par un atome d'halogène, un groupe méthyle, CF₃ et OCF₃, ledit substituant ou lesdits substituants étant en position 4 ou en position 3 et 4.

7. Utilisation selon la revendication 2, dans laquelle R²⁰ et R³ pris ensemble sont un cycle carbocyclique ou hétérocyclique à six chaînons facultativement substitué par entre 1 et 3 substituants indépendamment choisis parmi un groupe halogéno, alcoxy en C_{1 à 3}, di (alkyle en C_{1 à 3}) amino et acyle en C_{2 à 5}.

8. Utilisation selon la revendication 1, dans laquelle ledit composé est choisi parmi:
La 1-(1-{2-hydroxy-3-[5-méthanesulfonyl-3-(4-trifluorométhyl-phényl)-4,5,6,7-tétrahydro-pyrazolo[4,3-c]pyridin-1-yl]-propyl}-pipéridin-4-yl)-1,3-dihydro-benzoïmidazol-2-one;
la 1-(1-{3-[3-(3,4-dichloro-phényl)-5-méthanesulfonyl-4,5,6,7-tétrahydro-pyrazolo[4,3-c]pyridin-1-yl]-propyl}-pipéridin-4-yl)-1,3-dihydro-benzoïmidazol-2-one;
l'amide d'acide 3-(3,4-dichloro-phényl)-1-{3-[4-(2-oxo-2,3-dihydro-benzoïmidazol-1-yl}-pipéridin-1-yl]-propyl)-1,4,6,7-tétrahydro-pyrazolo[4,3-c]pyridine-5-carboxylique;
la 6-chloro-1-(1-(3-[5-méthanesulfonyl-3-(4-trifluorométhyl-phényl)-4,5,6,7-tétrahydro-pyrazolo[4,3-c]pyridin-1-yl]-propyl}-pipéridin-4-yl}-1,3-dihydrobenzoïmidazol-2-one;
l'amide d'acide 3-(3,4-dichloro-phényl)-1-{3-[4-(3-méthyl-2-oxo-2,3-dihydrobenzoïmidazol-1-yl)-pipéridin-1-yl]-propyl}-1,4,6,7-tétrahydro-pyrazolo[4,3-c]pyridine-5-carboxylique;
le [3-(1-{2-hydroxy-3-[5-méthanesulfonyl-3-(4-trifluorométhyl-phényl)-4,5,6,7-tétrahydro-pyrazolo[4,3-c]pyridin-1-yl]-propyl}-pipéridin-4-yl)-2-oxo-2,3-dihydro-benzoïmidazol-1-yl]-acétonitrile;
l'ester d'éthyle d'acide [3-(1-{2-hydroxy-3-[5-méthanesulfonyl-3-(4-trifluorométhyl-phényl)-4,5,6,7-tétrahydro-pyrazolo[4,3-c]pyridin-1-yl]-propyl}-pipéridin-4-yl)-2-oxo-2,3-dihydro-benzoïmidazol-1-yl]-acétique;
la 5-chloro-3-(1-{2-hydroxy-3-[5-méthanesulfonyl-3-(4-trifluorométhylphényl)-4,5,6,7-tétrahydro-pyrazolo[4,3-c]pyridin-1-yl-propyl]-pipéridin-4-yl)-1-méthyl-1,3-dihydro-benzoïmidazol-2-one;
l'amide d'acide 1-(3-[4-(6-chloro-3-méthyl-2-oxo-2,3-dihydro-benzoïmidazol-1-yl)-pipéridin-1-yl]-propyl)-3-(3,4-dichloro-phényl)-1,4,6,7-tétrahydro-pyrazolo[4,3-c]pyridine-5-carboxylique;
la 3-(1-{2-hydroxy-3-[5-méthanesulfonyl-3-(4-trifluorométhyl-phényl)-4,5,6,7-tétrahydro-pyrazolo[4,3-c]pyridin-1-yl]-propyl}-pipéridin-4-yl)-1,5-diméthyl-1,3-dihydro-benzoïmidazol-2-one;
la 3-(1-{2-hydroxy-3-[5-méthanesulfonyl-3-(4-trifluorométhyl-phényl)-4,5,6,7-tétrahydro-pyrazolo[4,3-c]pyridin-1-yl]-propyl}-pipéridin-4-yl)-1,3-dihydroimidazo[4,5-b]pyridin-2-one;
la 3-(1-(3-[3-(4-bromo-phényl)-5-méthanesulfonyl-4,5,6,7-tétrahydro-pyrazolo[4,3-c]pyridin-1-yl]-2-hydroxypropyl}-pipéridin-4-yl)-5-méthoxy-1,3-dihydro-imidazo[4,5-b]pyridin-2-one;
l'amide d'acide 3-(4-bromo-phényl)-1-{2-hydroxy-3-[4-(5-méthoxy-2-oxo-1,2-dihydroïmidazo[4,5-b]pyridin-3-yl)-pipéridin-1-yl]-propyl}-1,4,6,7-tétrahydro-pyrazolo[4,3-c]pyridine-5-carboxylique;
la 3-(1-{2-hydroxy-3-[5-méthanesulfonyl-3-(4-trifluorométhyl-phényl)-4,5,6,7-tétrahydro-pyrazolo[4,3-c]pyridin-1-yl]-propyl}-pipéridin-4-yl}5-méthoxy-1-méthyl-1,3-dihydro-imidazo[4,5-b]pyridin-2-one;
la 5-diméthylamino-3-(1-{2-hydroxy-3-[5-méthanesulfonyl-3-(4-trifluorométhyl-phényl)-4,5,6,7-tétrahydro-pyrazolo[4,3-c]pyridin-1-yl]-propyl}-pipéridin-4-yl)-1,3-dihydro-imidazo[4,5-b]pyridin-2-one;
la 6-chloro-1-(1-{3-[5-méthanesulfonyl-3-(4-trifluorométhyl-phényl)-4,5,6,7-tétrahydro-pyrazolo[4,3-c]pyridin-1-yl]-propyl}-pipéridin-4-yl)-1,3-dihydro-indol-2-one;
la 1-(1-{2-hydroxy-3-[5-méthanesulfonyl-3-(4-trifluorométhyl-phényl)-4,5,6,7-tétrahydro-pyrazolo[4,3-c]pyridin-1-yl]-propyl}-pipéridin-4-yl)-3,4-dihydro-1H-quinoléin-2-one;
la 4-(1-{3-[5-méthanesulfonyl-3-(4-trifluorométhyl-phényl)-4,5,6,7-tétrahydro-pyrazolo[4,3-c]pyridin-1-yl]-propyl}-pipéridin-4-yl)-4H-benzo[1,4]oxazin-3-one;
la 4-(1-{2-hydroxy-3-[5-méthanesulfonyl-3-(4-trifluorométhyl-phényl)-4,5,6,7-tétrahydro-pyrazolo[4,3-c]pyridin-1-yl]-propyl}-pipéridin-4-yl)-4H-benzo[1,4]oxazin-3-one; et
la 1-(1-{3-[5-méthanesulfonyl-3-(4-trifluorométhyl-phényl)-4,5,6,7-tétrahydro-pyrazolo[4,3-c]pyridin-1-yl]-propyl}-pipéridin-4-yl)-3,4-dihydro-1H-quinazolin-2-one.

9. Utilisation selon la revendication 1, dans laquelle ledit composé est choisi parmi:
le [3-(1-{2-hydroxy-3-[5-méthanesulfonyl-3-(4-trifluorométhyl-phényl)-4,5,6,7-tétrahydro-pyrazolo[4,3-c]pyridin-1-yl]-propyl}-pipéridin-4-yl à 2-oxo-2,3-dihydro-benzoïmidazol-1-yl]-acétonitrile; et la 4-(1-{2-hydroxy-3-[5-méthanesulfonyl-3-(4-trifluorométhyl-phényl)-4,5,6,7-tétrahydro-pyrazolo[4,3-c]pyridin-1-yl]-propyl}-pipéridin-4-yl)-4H-benzo[1,4]oxazin-3-one.

10. Utilisation selon la revendication 1, dans laquelle ledit composé est choisi parmi:
le 2-(1-{3-[5-acétyl-3-(4-chloro-phényl)-4,5,6,7-tétrahydro-pyrazolo[4,3-c]pyridin-1-yl]-2-hydroxy-propyl}-pipéridin-4-ylamino)-benzonitrile;
la 1-(1-(3-[5-acétyl-3-(4-trifluorométhyl-phényl)4,5,6,7-tétrahydro-pyrazolo[4,3-c]pyridin-1-yl]-2-hydroxy-propyl)-pipéridin-4-yl}1,3-dihydrobenzoïmidazol-2-one;
la 3-(1-{3-[5-acétyl-3-(4-bromo-phényl)-4,5,6,7-tétrahydro-pyrazolo[4,3-c]pyridin-1-yl]-2-hydroxy-propyl}-pipéridin-4-yl)-3H-benzooxazol-2-one;
la 1-(3-(4-chloro-3-méthyl-phényl)-1-{3-[4-(3,4-dichloro-phénoxy)-pipéridin-1-yl]-propyl}-1,4,6,7-tétrahydro-pyrazolo[4,3-c]pyridin-5-yl)-éthanone;
la 1-(3-(4-chloro-3-méthyl-phényl)-1-{3-[4-(2,3-dihydro-indol-1-yl)-pipéridin-1-yl]-2-hydroxy-propyl}-1,4,6,7-tétrahydro-pyrazolo[4,3-c]pyridin-5-yl)-éthanone;
la (S)-1-(1-{3-[5-acétyl-3-(4-chloro-3-méthyl-phényl)-4,5,6,7-tétrahydro-pyrazolo[4,3-c]pyridin-1-yl]-2-hydroxypropyl}-pipéridin-4-yl)-6-chloro-1,3-dihydro-benzoïmidazol-2-one;
la 1-(1-{3-[5-acétyl-3-(4-trifluorométhyl-phényl)-4,5,6,7-tétrahydro-pyrazolo[4,3-c]pyridin-1-yl]-2-hydroxypropyl}-pipéridin-4-yl)-3-(2-morpholin-4-yl-éthyl)-1,3-dihydro-benzoïmidazol-2-one;
la 1-(1-{3-[3-(4-bromo-phényl)-5-méthanesulfonyl-4,5,6,7-tétrahydro-pyrazolo[4,3-c]pyridin-1-yl]-2-hydroxypropyl}-pipéridin-4-yl)-6-chloro-1,3-dihydro-benzoïmidazol-2-one;
le [3-(1-{3-[5-méthanesulfonyl-3-(4-trifluorométhyl-phényl)-4,5,6,7-tétrahydro-pyrazolo[4,3-c]pyridin-1-yl]-propyl)-pipéridin-4-yl)-2-oxo-2,3-dihydrobenzoïmidazol-1-yl]-acétonitrile;
la 5-chloro-3-(1-(3-[5-méthanesulfonyl-3-(4-trifluorométhyl-phényl)-4,5,6,7-tétrahydro-pyrazolo[4,3-c]pyridin-1-yl]-propyl}-pipéridin-4-yl}1-méthyl-1,3-dihydro-benzoïmidazol-2-one;
la 1-(1-{3-[5-méthanesulfonyl-3-(4-trifluorométhyl-phényl)-4,5,6,7-tétrahydro-pyrazolo[4,3-c]pyridin-1-yl]-propyl}-pipéridin-4-yl}-1,3-dihydro-indol-2-one;
la 1-[3-(4-chloro-3-méthyl-phényl)-1-(3-[4-[3-(4-chloro-phényl)-[1,2,4]oxadiazol-5-yl]-pipéridin-1-yl}-2-hydroxy-propyl}1,4,6,7-tétrahydro-pyrazolo[4,3-c]pyridin-5-yl]-éthanone;
la 1-[1-{2-hydroxy-3-[4-(5-trifluorométhyl-benzothiazol-2-yl)-pipéridin-1-yl]-propyl)-3-(4-trifluorométhyl-phényl)-1,4,6,7-tétrahydro-pyrazolo[4,3-c]pyridin-5-yl]-éthanone;
la 1-[1-(3-[4-(benzo[d]isoxazol-3-yloxy)-pipéridin-1-yl]-2-hydroxy-propyl}-3-(4-trifluorométhyl-phényl)-1,4,6,7-tétrahydro-pyrazolo[4,3-c]pyridin-5-yl]-éthanone;
la 1-[1-{3-[4-(5-chloro-benzooxazol-2-yl)-pipéridin-1-yl]-2-hydroxy-propyl}-3-(4-trifluorométhyl-phényl)-1,4,6,7-tétrahydro-pyrazolo[4,3-c]pyridin-5-yl]-éthanone;
la 1-[1-{3-[4-(benzothiazol-2-ylamino)-pipéridin-1-yl]-2-hydroxy-propyl)-3-(4-trifluorométhyl-phényl)-1,4,6,7-tétrahydro-pyrazolo[4,3-c]pyridin-5-yl]-éthanone;
la 1-[1-(3-(4-(3,5-dichloro-pyridin-4-yloxy)-pipéridin-1-yl]-2-hydroxy-propyl}-3-(4-trifluorométhyl-phényl)-1,4,6,7-tétrahydro-pyrazolo[4,3-c]pyridin-5-yl]-éthanone;
la 1-[1-{3-[4-(1H-benzoïmidazol-2-yl)-pipéridin-1-yl]-2-hydroxy-propyl}-3-(4-trifluorométhyl-phényl)-1,4,6,7-tétrahydro-pyrazolo[4,3-c]pyridin-5-yl]-éthanone;
la 6-chloro-4-(1-{2-hydroxy-3-[5-méthanesulfonyl-3-(4-trifluorométhylphényl)-4,5,6,7-tétrahydro-pyrazolo[4,3-c]pyridin-1-yl]-propyl}-pipéridin-4-yl)-4H-benzo[1,4]oxazin-3-one;
la 6-chloro-1-(1-{2-hydroxy-3-[5-méthanesulfonyl-3-(4-trifluorométhylphényl)-4,5,6,7-tétrahydro-pyrazolo[4,3-c]pyridin-1-yl]-propyl}-pipéridin-4-yl}-3,4-dihydro-1H-quinoléin-2-one;
la 6-chloro-1-(1-{2-hydroxy-3-[5-méthanesulfonyl-3-(4-trifluorométhylphényl)-4,5,6,7-tétrahydro-pyrazolo[4,3-c]pyridin-1-yl]-propyl}-pipéridin-4-yl)-3,4-dihydro-1H-quinazolin-2-one;
le 1-[4-(6-chloro-2,2-dioxo-3,4-dihydro-2H-2λ⁶-benzo[1,2,6]thiadiazin-1-yl)-pipéridin-1-yl]-3-[5-méthanesulfonyl-3-(4-trifluorométhyl-phényl)-4,5,6,7-tétrahydro-pyrazolo[4,3-c]pyridin-1-yl]-propan-2-ol;
la 4-(1-{2-hydroxy-3-[5-méthanesulfonyl-3-(4-trifluorométhyl-phényl)-4,5,6,7-tétrahydro-pyrazolo[4,3-c]pyridin-1-yl]-propyl}-pipéridin-4-yl)-4H-pyrido[3,2-b][1,4]oxazin-3-one;
la 5-chloro-1-(1-{2-hydroxy-3-[5-méthanesulfonyl-3-(4-trifluorométhylphényl)-4,5,6,7-tétrahydro-pyrazolo[4,3-c]pyridin-1-yl]-propyl}-pipéridin-4-yl)-1,3-dihydro-indol-2-one;
le 1-[4-(6-chloro-indol-1-yl)-pipéridin-1-yl]-3-[5-méthanesulfonyl-3-(4-trifluorométhyl-phényl)-4,5,6,7-tétrahydro-pyrazolo[4,3-c]pyridin-1-yl]-propan-2-ol;
le 1-(1-{3-[5-méthanesulfonyl-3-(4-trifluorométhyl-phényl)-4,5,6,7-tétrahydro-pyrazolo[4,3-c]pyridin-1-yl]-propyl}-pipéridin-4-yl)-1H-benzotriazole;
l'amide d'acide 1-{3-[4-(3-méthyl-2-oxo-2,3-dihydro-benzoïmidazol-1-yl)-pipéridin-1-yl]-propyl)-3-(4-trifluorométhyl-phényl)-1,4,6,7-tétrahydro-pyrazolo[4,3-c]pyridine-5-sulfonique;
la 5-chloro-3-(1-(2-hydroxy-3-[4-pyridin-4-yl à 3-(4-trifluorométhyl-phényl)-pyrazol-1-yl]-propyl)-pipéridin-4-yl)-1-méthyl-1,3-dihydro-benzoïmidazol-2-one;
la 4-(1-(2-hydroxy-3-[4-pyrazin-2-yl-3-(4-trifluorométhyl-phényl)pyrazol-1-yl]-propyl}-pipéridin-4-yl)-4H-benzo[1,4]oxazin-3-one;
la (S)-1-(1-{2-hydroxy-3-[5-méthanesulfonyl-3-(4-trifluorométhyl-phényl)-4,5,6,7-tétrahydro-pyrazolo[4,3-c]pyridin-1-yl]-propyl)-pipéridin-4-y1}3-méthyl-1,3-dihydro-benzoïmidazol-2-one; et
la (S)-5-diméthylamino-3-(1-[2-hydroxy-3-[5-méthanesulfonyl-3-(4-trifluorométhyl-phényl)-4,5,6,7-tétrahydro-pyrazolo[4,3-c]pyridin-1-yl]-propyl}-pipéridin-4-yl)-1-méthyl-1,3-dihydro-imidazo[4,5-b]pyridin-2-one.

11. Utilisation selon la revendication 1, dans laquelle ledit composé est choisi parmi:
La 1-(1-{3-[5-acétyl-3-(4-bromo-phényl)-4,5,6,7-tétrahydro-pyrazolo[4,3-c]pyridin-1-yl]-2-hydroxy-propyl}-pipéridin-4-yl)-5-méthoxy-1,3-dihydrobenzoïmidazol-2-one;
La 6-chloro-1-(1-{3-[3-(4-chloro-3-méthyl-phényl)-5-méthanesulfonyl-4,5,6,7-tétrahydro-pyrazolo[4,3-c]pyridin-1-yl]-2-hydroxy-propyl}-pipéridin-4-yl)-1,3-dihydro-benzoïmidazol-2-one;

12. Utilisation selon la revendication 1, dans laquelle ladite composition pharmaceutique est formulée dans une quantité de dosage appropriée pour le traitement d'une condition allergique.
